# EUROPEAN PATENT SPECIFICATION

(11) **EP 2 830 662 B1**
(45) Date of publication and mention of the grant of the patent: **29.08.2018**
(21) Application number: 13769804.9
(22) Date of filing: 29.03.2013
(51) Int. Cl.: A61K 39/395, A61P 17/14

(54) **METHODS FOR TREATING HAIR LOSS DISORDERS**
VERFAHREN ZUR BEHANDLUNG VON HAARAUSFALL
MÉTHODES DE TRAITEMENT DE TROUBLES SE CARACTÉRISANT PAR LA PERTE DES CHEVEUX

(30) Priority: 29.03.2012 US 201261617225 P; 10.05.2012 US 201261645499 P
(43) Date of publication of application: 04.02.2015
(73) Proprietor: The Trustees of Columbia University in the City of New York, New York, NY 10027 (US)
(72) Inventor: CHRISTIANO, Angela, M., Mahwah, NJ 07430 (US); CLYNES, Raphael, West Nyack, NY 10994 (US)
(74) Representative: Barker Brettell LLP
(86) International application number: PCT/US2013/034688
(87) International publication number: WO 2013/149194

(56) References cited:
- WO-A1-2005/105146
- WO-A2-2009/132202
- US-A- 5 916 792
- US-A1- 2010 221 364
- US-A1- 2011 071 149
- US-A1- 2011 117 159
- US-B1- 7 015 218
- B. Y. CHANG ET AL: "JAK3 Inhibition Significantly Attenuates Psoriasiform Skin Inflammation in CD18 Mutant PL/J Mice", THE JOURNAL OF IMMUNOLOGY, vol. 183, no. 3, 13 July 2009 (2009-07-13) , pages 2183-2192, XP055025639, ISSN: 0022-1767, DOI: 10.4049/jimmunol.0804063
- KARYN SPRINGER ET AL: "Common Hair Loss Disorders", AMERICAN FAMILY PHYSICIAN, vol. 68, no. 1, 1 July 2003 (2003-07-01), pages 93-102, XP055205904,
- ANNE L MOUNSEY ET AL: "Diagnosing and Treating Hair Loss", AMERICAN FAMILY PHYSICIAN, vol. 80, no. 4, 15 August 2009 (2009-08-15), pages 356-362, XP055205907,
- BRITTANY G CRAIGLOW ET AL: "Killing Two Birds with One Stone: Oral Tofacitinib Reverses Alopecia Universalis in a Patient with Plaque Psoriasis", JOURNAL OF INVESTIGATIVE DERMATOLOGY, vol. 134, no. 12, 18 June 2014 (2014-06-18), pages 2988-2990, XP055206017, ISSN: 0022-202X, DOI: 10.1038/jid.2014.260
- JABBARI A ET AL: "Targeting of JAK3 prevents onset of murine alopecia areata", JOURNAL OF INVESTIGATIVE DERMATOLOGY, & 75TH ANNUAL MEETING OF THE SOCIETY-FOR-INVESTIGATIVE-DERMATOLOGY; RALEIGH, NC, USA; MAY 09 -12, 2012, vol. 132, no. Suppl. 1, 1 May 2012 (2012-05-01), page S104, XP002712231,

## Description

### BACKGROUND OF THE INVENTION

Alopecia Areata (AA) is one of the most highly prevalent autoimmune diseases affecting over 5 million individuals in the US, and as many as 140 million worldwide AA leads to hair loss due to the collapse of immune privilege of the hair follicle and subsequent autoimmune destruction. AA is a skin disease which leads to hair loss on the scalp and elsewhere. In some severe cases, it can progress to complete loss of hair on the head or body. Although Alopecia Areata is believed to be caused by autoimmunity, the gene level diagnosis and rationally targeted therapeutics have not been developed. The genetic basis of AA is largely unknown. Its psychological impact on affected patients is devastating, particularly in children.

B.Y. Chang et al., (2009) J. Immunol. 183(3): 2183-92 discloses the treatment of mice having psoriasiform skin disease using R348, a JAK3 inhibitor.

### SUMMARY OF THE INVENTION

An aspect of the invention encompasses a Jak3 inhibitor selected from the group consisting of decernotinib, tofacitinib, JAK3 Inhibitor IV (ZM-39923); NSC114792; PF-956980;. an antisense RNA or antisense DNA that is specific for a nucleic acid encoding a JAK3 polypeptide; and a siRNA that specifically targets the Jak3 gene for use in the treatment of a hair-loss disorder selected from the group consisting of alopecia areata and androgenetic alopecia;. In some embodiments, the siRNA directed to a Jak3 gene is any one of the sequences listed in Table 1. In a further embodiment, the Jak3 inhibitor is intended to be administered subcutaneously, intra-muscularly, intra-peritoneally, intradermally, by intravenous injection; by an infusion; parentally, transdermally, transmucosally, rectally, orally, nasally, or by topical delivery; or a combination thereof. In some embodiments, the Jak3 inhibitor is intended to be administereddaily, weekly, twice weekly, monthly, twice monthly, or yearly. In some embodiments, the Jak3 inhibitor is administered 1 time per week, 2 times per week, 3 times per week, 4 times per week, 5 times per week, 6 times per week, 7 times per week, 8 times per week, 9 times per week, 10 times per week, 11 times per week, 12 times per week, 13 times per week, or 14 times per week. In other embodiments, the subject is administered the Jak3 inhibitor for at least 1 week, at least 2 weeks, at least 3 weeks, at least 4 weeks, at least 5 weeks, at least 6 weeks, at least 8 weeks, at least 12 weeks, or at least 16 weeks. In some embodiments, the Jak3 inhibitor is intended to be administered with a Jak1/2 inhibitor to the subject. In further embodiments, the Jak1/2 inhibitor is intended to be administered simultaneously with the Jak3 inhibitor. Yet in other embodiments, the Jak1/2 inhibitor is intended to be administered in any order with the Jak3 inhibitor. In some embodiments, the Jak1/2 inhibitor is ruxolitinib, figlotinib , AG490, momelotinib, pacritinib, baricitinib, fedratinib, BMS-911543, or lestaurtinib.

The application also discloses a method for inducing hair growth in a subject where the method comprises administering to the subject an effective amount of an inhibitor of a protein tyrosine kinase (PTK) involved in cytokine signaling. The inhibitor may be a Jak3 inhibitor. The inhibitor may be tofacitinib (CP690550). The hair-loss disorder may comprise androgenetic alopecia, telogen effluvium, alopecia areata, tinea capitis, alopecia totalis, hypotrichosis, hereditary hypotrichosis simplex, or alopecia universalis. The method may further comprise determining whether the inhibitor administered induced hair growth in the subject afflicted with a hair loss disorder as compared to the subject's hair growth prior to treatment with the inhibitor. Theinhibitor may be an antibody that specifically binds to a Jak3 protein or a fragment thereof; an antisense RNA or antisense DNA that decreases expression of the gene that encodes the Jak3 protein; an antisense RNA or antisense DNA that decreases expression of the Jak3 protein; a siRNA that specifically targets the Jak3 gene; a small molecule; or a combination thereof. The small molecule may be Janex 1 (WHI-P131), PF-956980, WHI-P154, tofacitinib (CP690550), VX-509, JAK3 Inhibitor IV, NSC114792, or R348. The antibody may specifically bind to a protein comprising SEQ ID NO: 1. The siRNA may be directed to a human nucleic acid sequence comprising SEQ ID NO: 2. The siRNA may be directed to a Jak3 gene is any one of the sequences listed in Table 1. The administering may comprise a subcutaneous, intra-muscular, intra-peritoneal, or intravenous injection; an infusion; oral, nasal, or topical delivery; or a combination thereof The Jak3 inhibitor may be intended to be administered occurs daily, weekly, twice weekly, monthly, twice monthly, or yearly. The Jak3 inhibitor may be intended to be administered 1 time per week, 2 times per week, 3 times per week, 4 times per week, 5 times per week, 6 times per week, 7 times per week, 8 times per week, 9 times per week, 10 times per week, 11 times per week, 12 times per week, 13 times per week, or 14 times per week. The subject is administered the Jak3 inhibitor for at least 1 week, at least 2 weeks, at least 3 weeks, at least 4 weeks, at least 5 weeks, at least 6 weeks, at least 8 weeks, at least 12 weeks, or at least 16 weeks. The method may further comprise administering a Jak1/2 inhibitor to the subject. The Jak1/2 inhibitor may be intended to be administered simultaneously with the administering of the Jak3 inhibitor. Yet in other embodiments, the Jak1/2 inhibitor is intended to be administered sequentially in any order with the Jak3 inhibitor. In some embodiments, the Jak1/2 inhibitor is INCB 018424, GLPG0634, AG490, CYT387, SB1518, LY3009104 (Baricitinib; INCB28050), TG101348, BMS-911543, or CEP-701.

### BRIEF DESCRIPTION OF THE FIGURES

**FIG. 1** shows that treatment of mice with the Jak3 inhibitor (tofacitinib; CP-69055) prevents alopecia areata (AA).

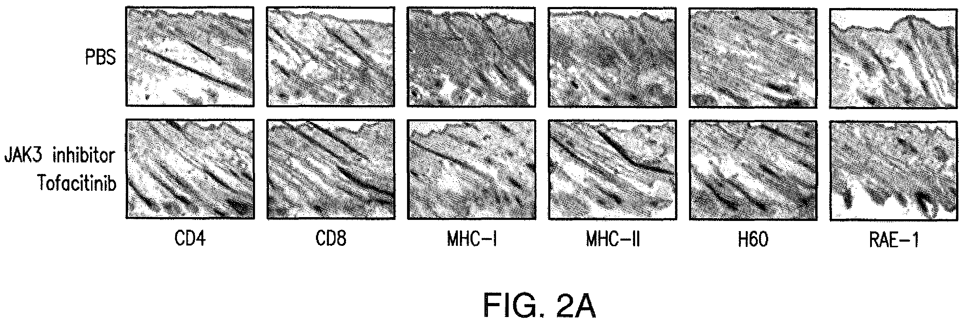

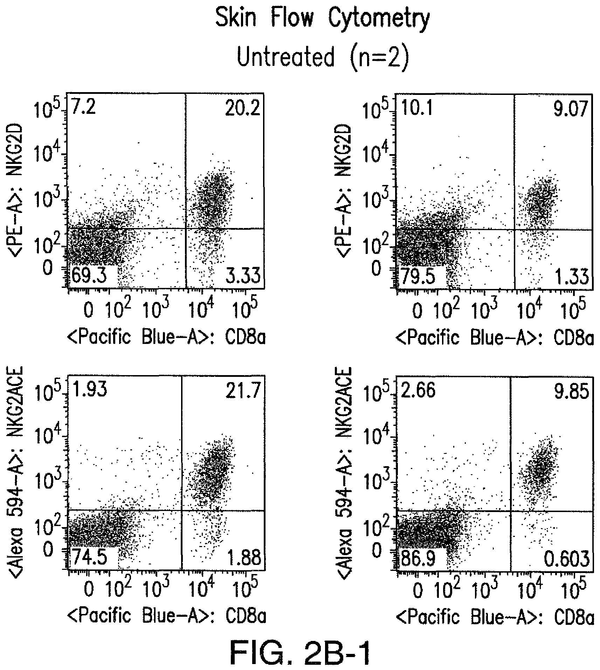

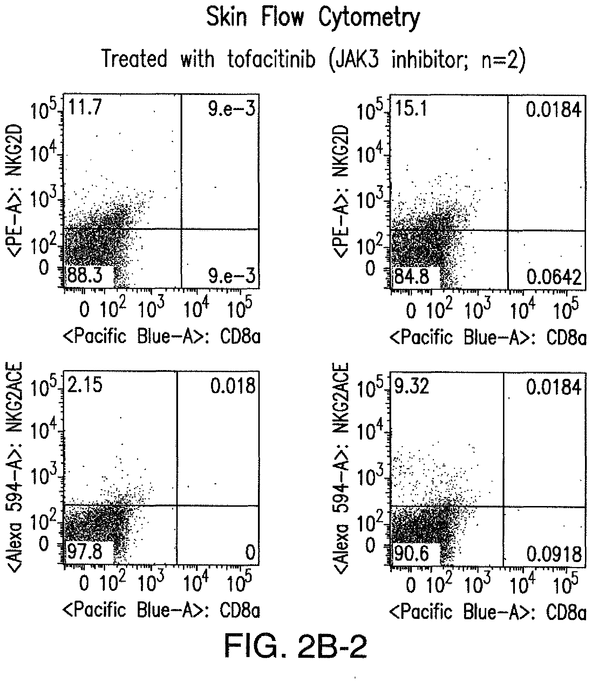

**FIG. 2** shows treatment related elimination of dermal T cell infiltrates and inflammatory biomarkers by immunostaining **(****FIG. 2A****)** and by flow cytometry **(FIG. 2B).** CD8+ NKG2D+ T cells, which represent up to 25% of dermal cells in AA skin, are completely eliminated with treatment. The flow cytometry is of single cell suspensions from the skin. The cells in the right upper quadrant are not observed in normal healthy mice and are CD8+NKG2D+ effector T cells. CD8+NKG2D+ effector T cells are found in alopecic mice after skin grafting. Mice treated with the JAK3 inhibitor do not develop alopecia and their skin is devoid of these cells.
**FIG. 3** are graphs that show treatment elimination of IFN-γ producing "NK-type" CD8 T cells in cutaneous LN, and loss of circulating IFN-inducible chemokines. The bar graphs in the left panel show intracellular flow cytometry of PMA/Iono stimulated LN cells from individually treated and untreated mice stained for CD8.NKG2A/C/D/E.IFNγ. Two mice per group are shown.
**FIG. 4** is a ribbon diagram that shows C3H/HeJ graft recipients were treated with a JAK3 inhibitor (right panel), or PBS. RNA was extracted from skin harvested at weeks 6 (w6) and 13 (w13) following treatment and also from mice that had undergone sham surgery and subsequently interrogated by microarray analysis.

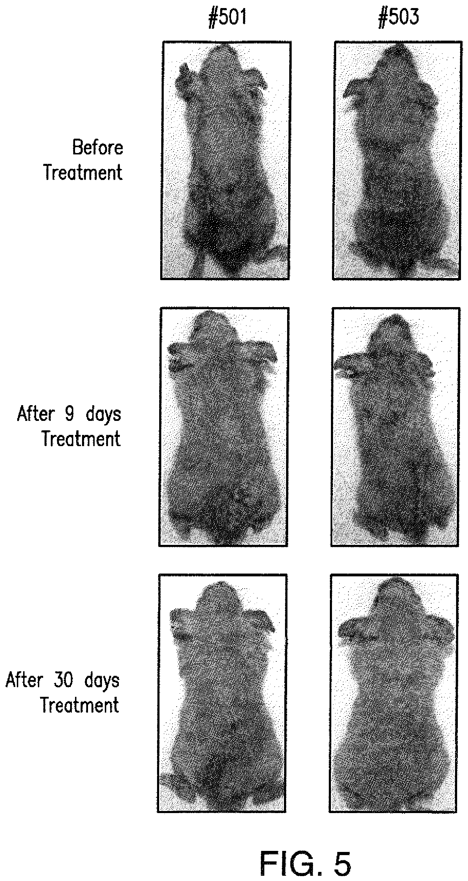

**FIG. 5** shows photographs of mice before (top panel) and after topical treatment (9 days (middle panel) and 30 days (bottom panel)) with cream only.

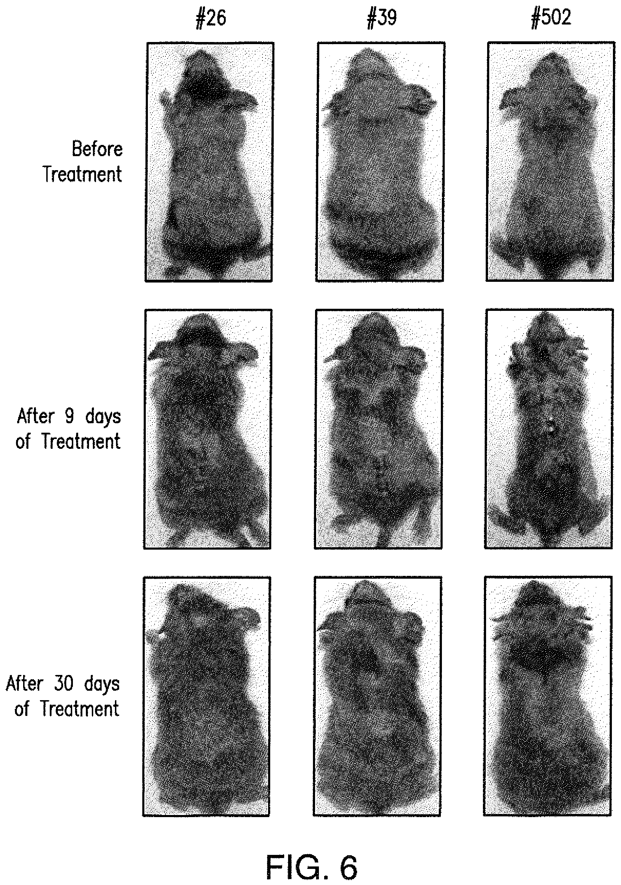

**FIG. 6** shows photographs of mice before (top panel) and after topical treatment (9 days (middle panel) and 30 days (bottom panel)) with the Jak3 inhibitor, tofacitinib.

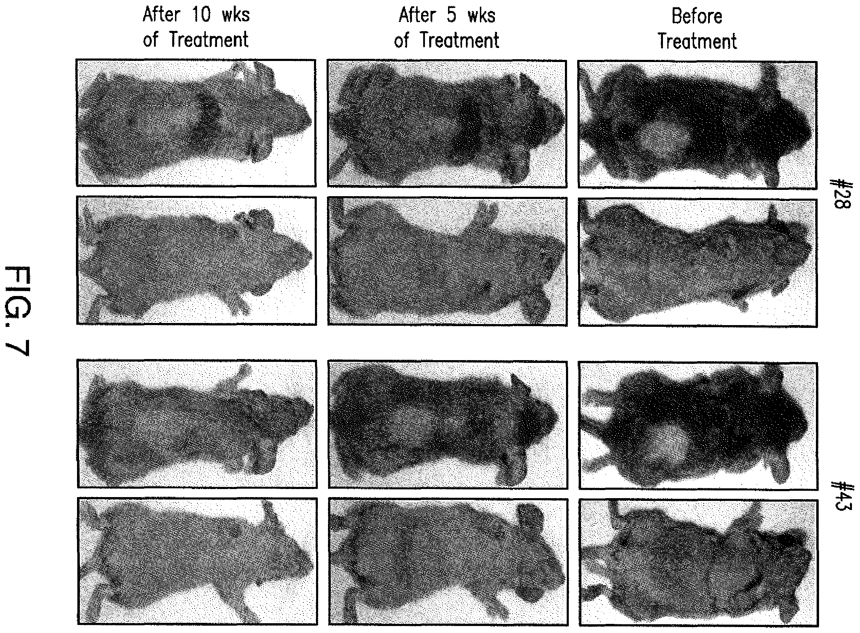

**FIG. 7** shows photographs of mice before (top panel) and after a control infusion (5 weeks (middle panel) and 10 weeks (bottom panel)) without the Jak3 inhibitor, tofacitinib.

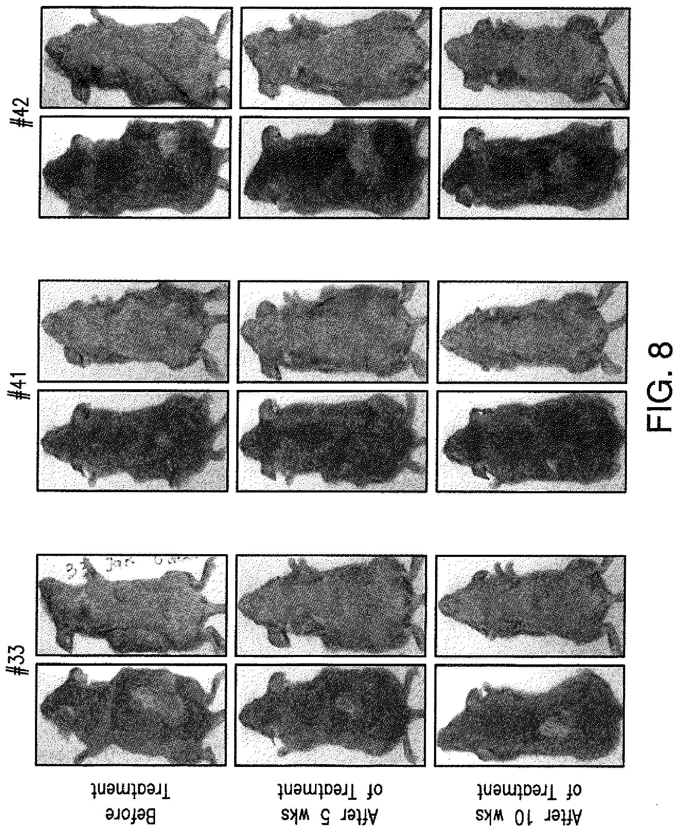

**FIG. 8** shows photographs of mice before (top panel) and after a pump infusion (5 weeks (middle panel) and 10 weeks (bottom panel)) with the Jak3 inhibitor, tofacitinib.

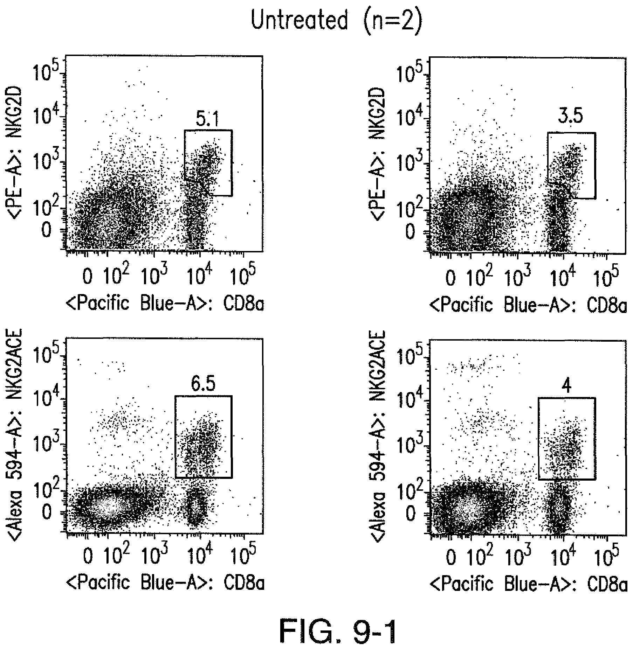

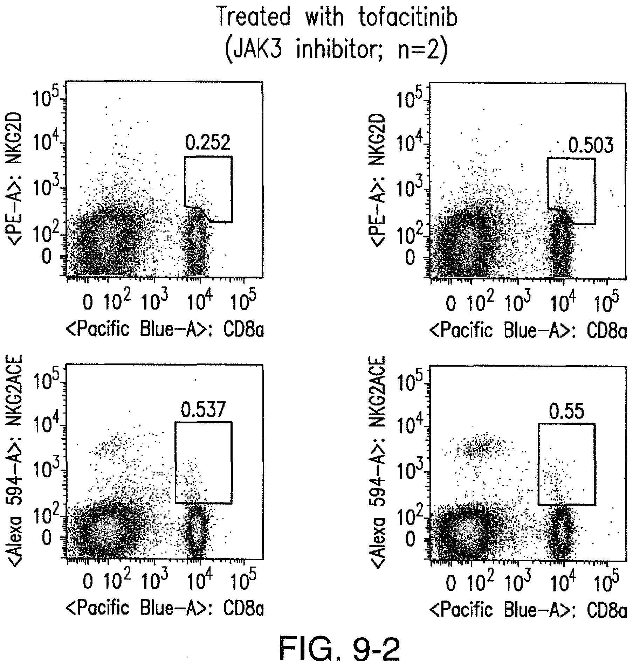

**FIG. 9** shows treatment related elimination of dermal T cell infiltrates and inflammatory biomarkers by flow cytometry. The flow cytometry is of lymph node cells. The cells in the right upper quadrant are not observed in normal healthy mice and represent CD8+NKG2D+ effector T cells. CD8+NKG2D+ effector T cells are found in alopecic mice after skin grafting. Alopecic mice treated with the JAK3 inhibitor exhibit hair regrowth and their lymph nodes are devoid of these cells.

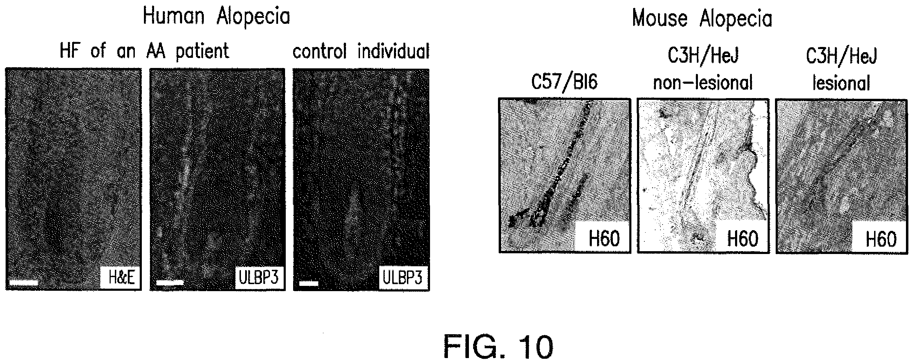

**FIG. 10** shows that NKG2D "stress ligands" are upregulated in the hair follicle, and demonstrates the shared pathogenesis in human and mouse.

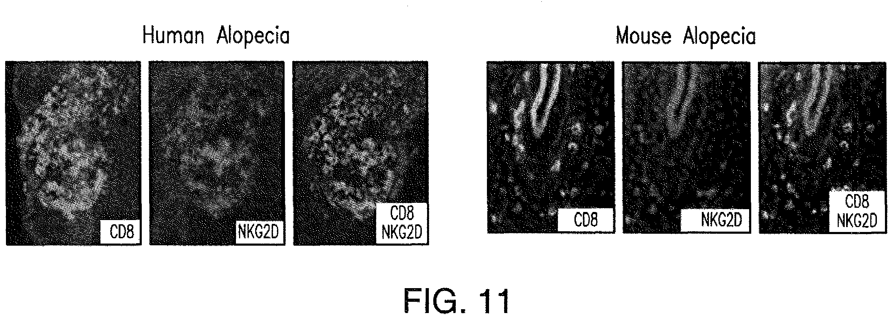

**FIG. 11** is a photomicrograph of an immunostain showing that CD8⁺ NKG2D⁺ T cells infiltrate the hair follicle, and demonstrates the shared pathogenesis in human and mouse.

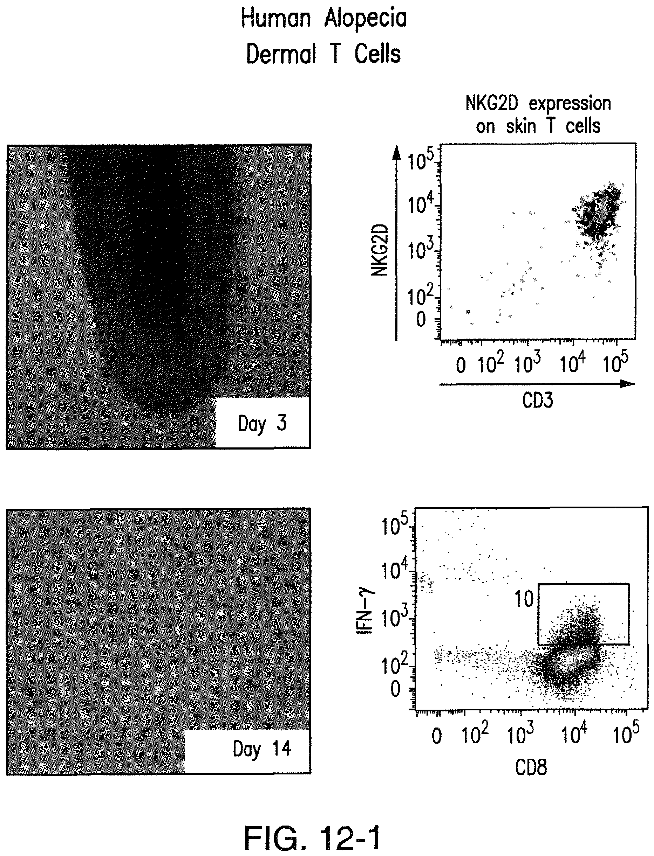

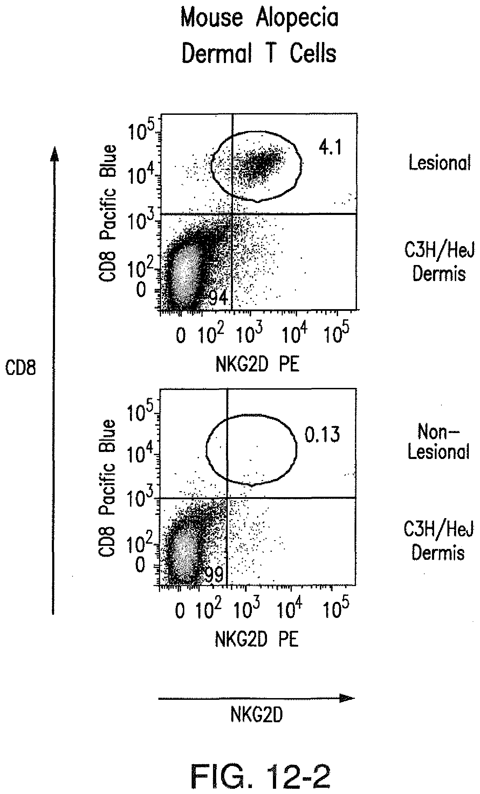

**FIG. 12** shows flow cytometric analysis that shows that NKG2D⁺ CD8⁺ T cells are the dominant infiltrating cells, and demonstrates the shared pathogenesis in human and mouse. (e.g., *see* Clark, et al., (2006) J Invest Dermatol. 126(5):1059-70, reporting a method for isolating T cell crawlouts from the skin).

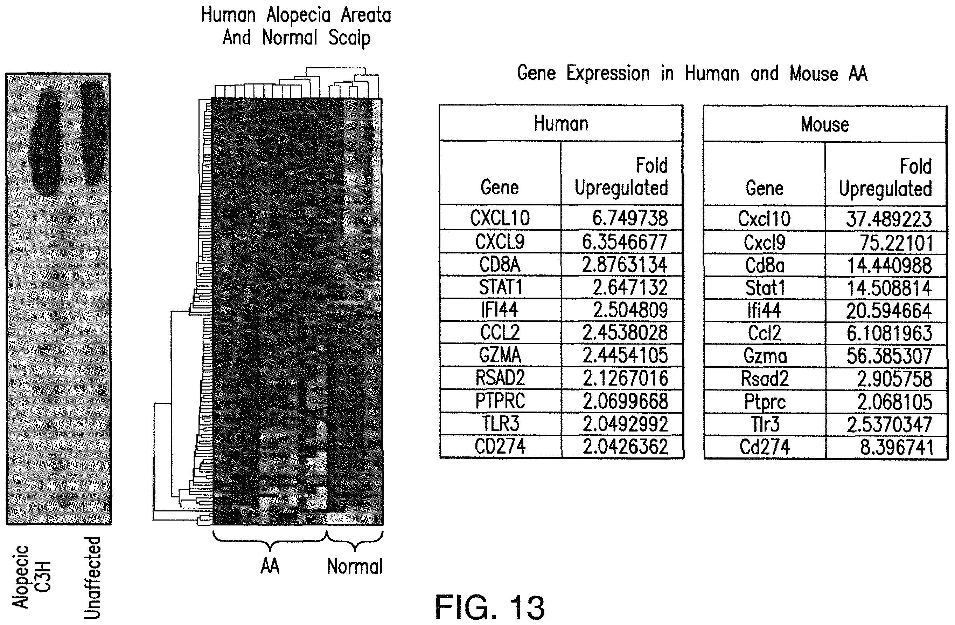

**FIG. 13** shows that comparative transcriptomics reveals a shared IFN response signature, and demonstrates shared pathogenesis in human and mouse.

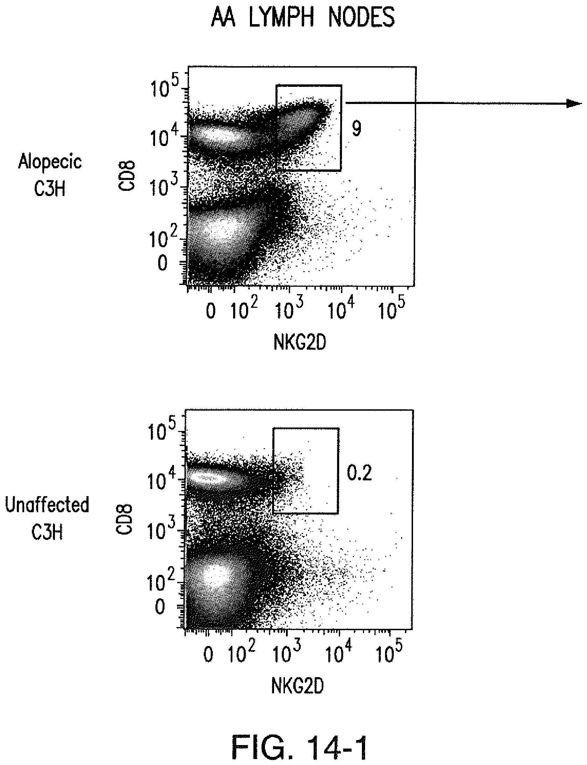

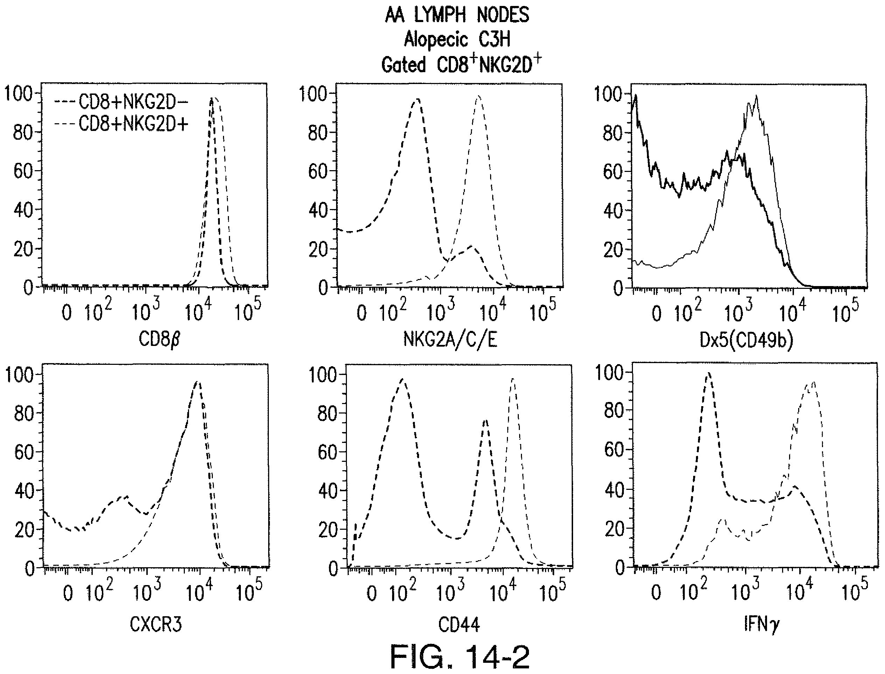

**FIG. 14** shows plots depicting that CD8+NKG2D+ Cytotoxic T Cells are IFN-gamma producing cells bearing NK-type receptors.

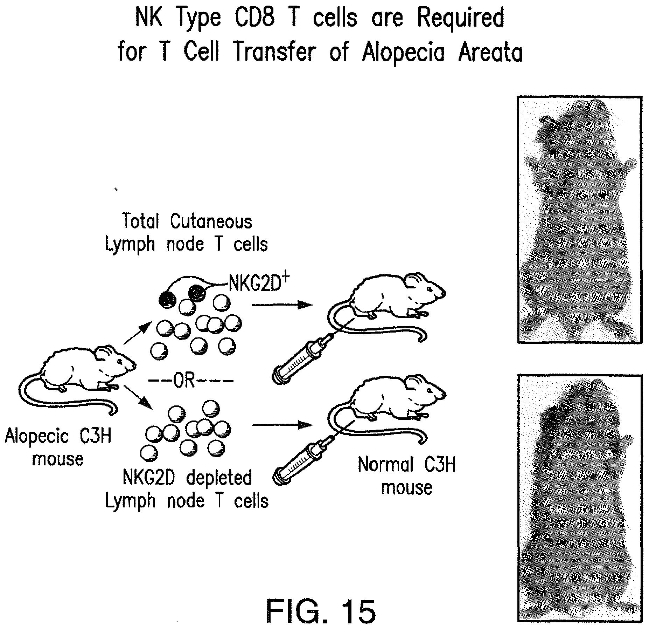

**FIG. 15** shows that NK Type CD8 T cells are required for T Cell transfer of Alopecia Areata.

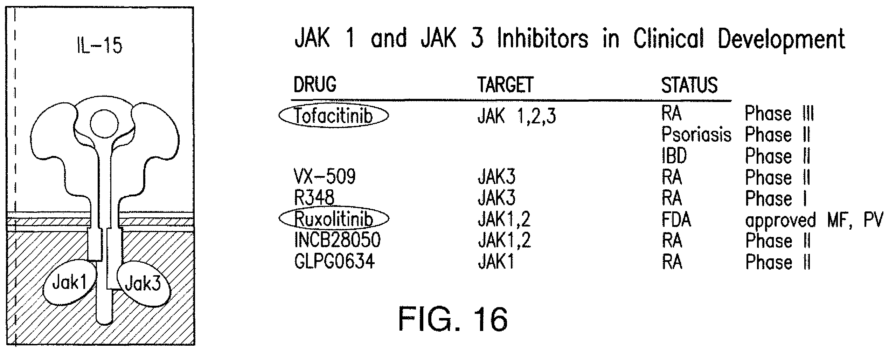

**FIG. 16** shows pre-clinical studies using systemic JAK inhibitors in the C3H-HeJ mouse model of alopecia areata (for example, *see* O'Shea, Immunity. 2012 Apr 20;36(4):542-50).

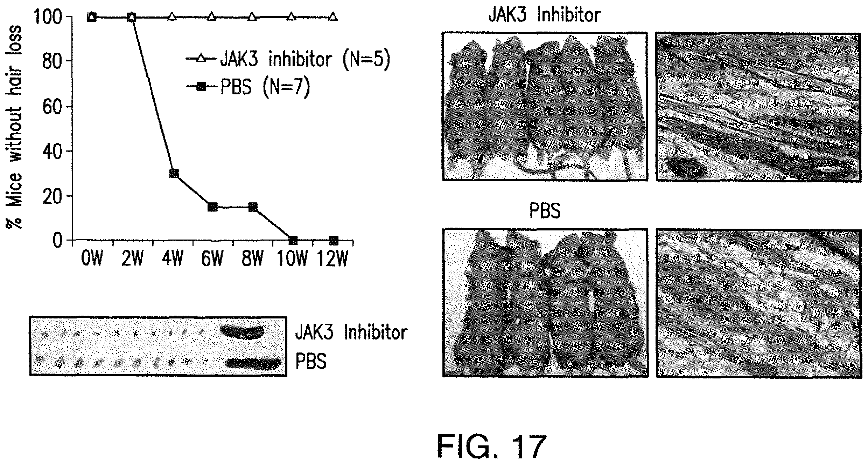

**FIG. 17** shows the results of pre-clinical studies with systemic delivery of JAK3 inhibitor (tofacitinib), and tofacitinib treatment prevents Alopecia Areata.

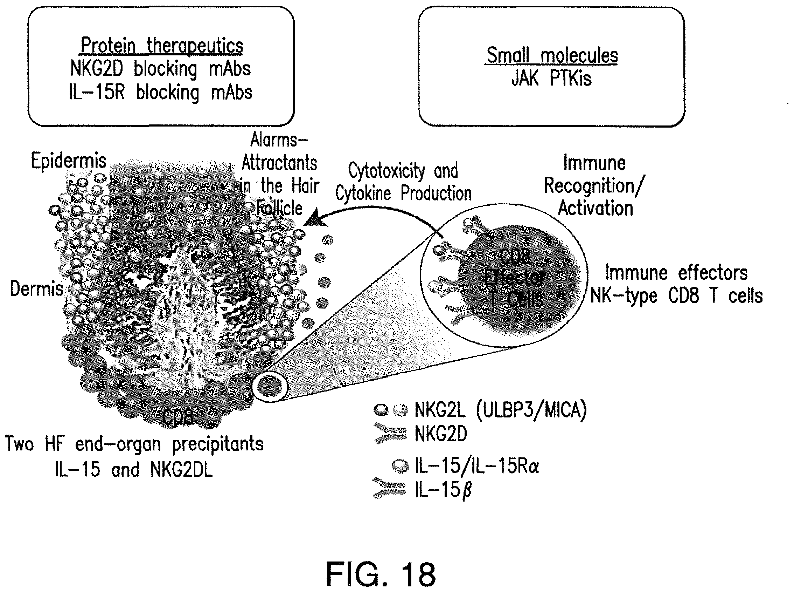

**FIG. 18** is a diagram depicting different examples of targeted therapies for alopecia areata. These topical therapies can be used to reverse the established disease.

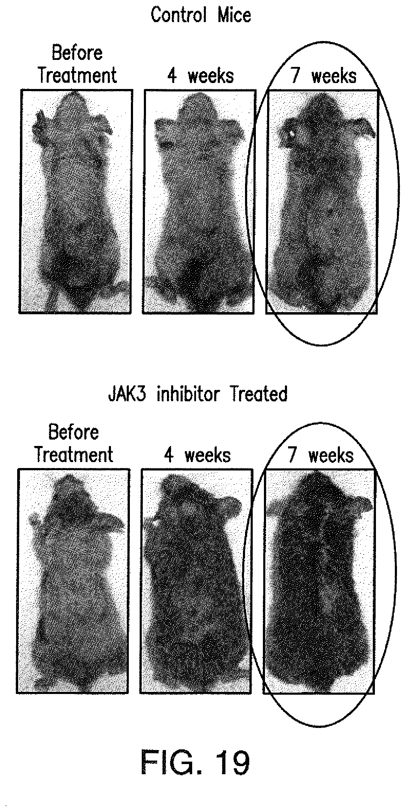

**FIG. 19** shows pre-clinical studies with topical delivery of JAK3 inhibitor (tofacitinib). Topical treatment using JAK3 inhibitor results in reversal of long-standing AA (2-3 months duration). Before treatment, all mice had hair loss for 2-3 months. Control mice were applied with cream daily. Mice treated with the JAK3 inhibitor, Tofacitnib, were treated daily with a topical cream containing 0.5% Tofacitnib.

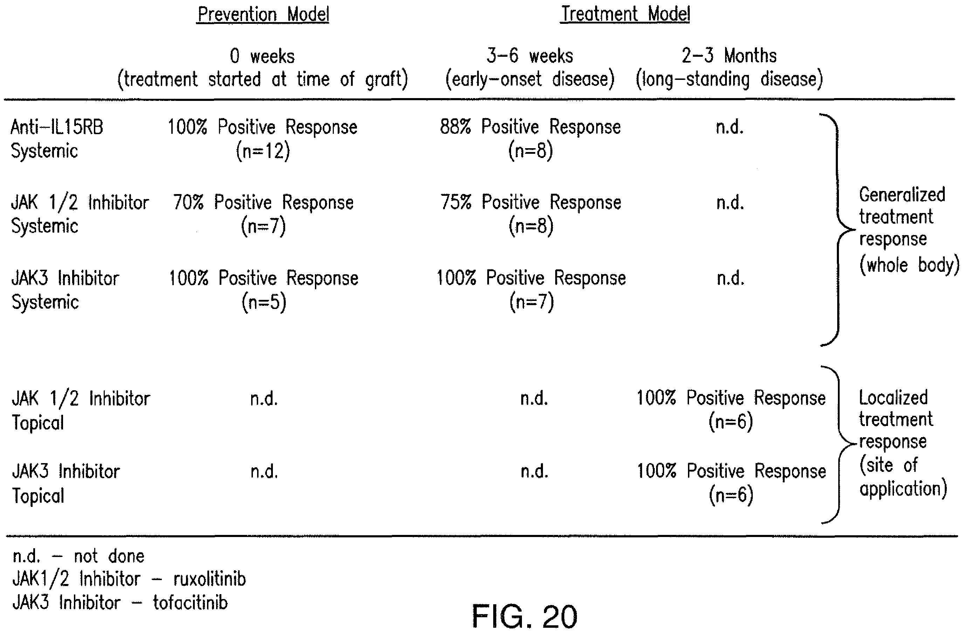

**FIG. 20** shows a summary of pre-clinical studies in alopecia C3H/HeJ mice.

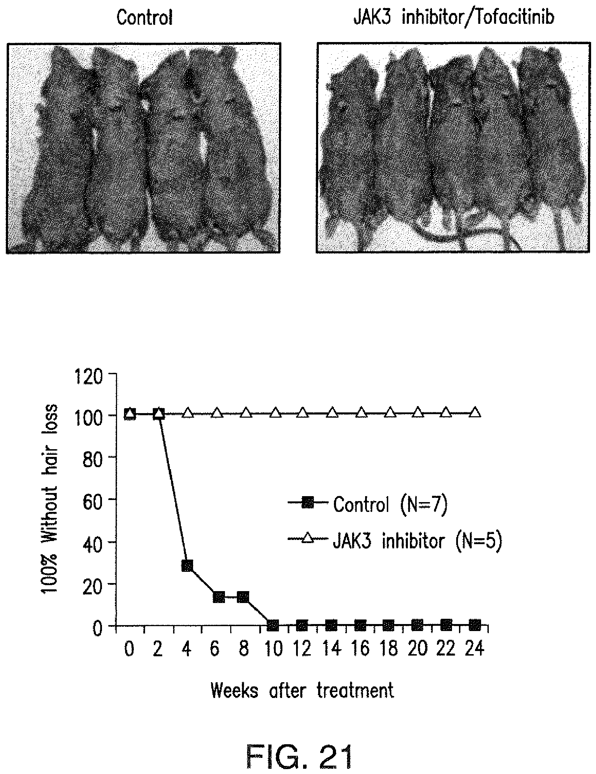

**FIG. 21** shows the prevention of AA with systemic treatment with a JAK3 inhibitor given at the time of skin grafting. All reagents are started at time of graft (prevention). JAK3 inhibitor (CP690550) was delivered using Alzet osmotic mini-pumps (28-day pumps, Model 2004) implanted subcutaneously, 10 mg/kg/day or vehicle (PEG300).
**FIG. 22** shows photomicrographs depicting that treatment with Jak3 inhibitor tofacitinib normalizes inflammatory infiltrate.
**FIG. 23A** shows the upregulation of NKG2DL in the hair follicle of C3H/HeJ alopecic mice. Immunofluorescence staining of NKG2D ligands (H60) in the hair follicle inner root sheath (marked by K71).
**FIG. 23B** shows the cellular phenotype and function of CD8⁺NKG2D⁺ cytotoxic T lymphocytes in AA mice. Immunofluorescence staining of CD8⁺NKG2D⁺ cells in hair follicles of C57BL/6, healthy C3H/HeJ and C3H/HeJ AA mice.
**FIG. 23C** shows the cellular phenotype and function of CD8⁺NKG2D⁺ cytotoxic T lymphocytes in AA mice. Striking cutaneous lymphadenopathy and cellularity in C3H/HeJ mice that developed AA.
**FIG. 23D** shows the cellular phenotype and function of CD8⁺NKG2D⁺ cytotoxic T lymphocytes in AA mice. Plots show the frequency of CD8⁺NKG2D⁺ T cells from skin and skin-draining lymph nodes in alopecic mice vs. ungrafted mice.
**FIG. 23E** shows the cellular phenotype and function of CD8⁺NKG2D⁺ cytotoxic T lymphocytes in AA mice. Plots shows the immunophenotype of CD8⁺NKG2D⁺ T cells in cutaneous lymph nodes in C3H/HeJ alopecic mice.
**FIG. 23F** shows the cellular phenotype and function of CD8⁺NKG2D⁺ cytotoxic T lymphocytes in AA mice. Graphs demonstrate that CD8⁺NKG2D⁺ T cells isolated from AA mice cutaneous LNs kill Rae-1 expressing dermal sheath cells grown from C3H/HeJ hair follicles in an NKG2D-dependent manner.
**FIG. 23G** shows the cellular phenotype and function of CD8⁺NKG2D⁺ cytotoxic T lymphocytes in AA mice. RNAseq was performed on CD8+NKG2D+ and CD8+NKG2D-T cells sort purified from AA cutaneous lymph nodes. Transcripts upregulated in CD8+NKG2D+ T cells were compared with CTL gene expression and NK cell gene expression in the literature^{8,9}, and overlapping gene signatures are displayed in this Venn Diagram.
**FIG. 23H** shows the cellular phenotype and function of CD8⁺NKG2D⁺ cytotoxic T lymphocytes in AA mice. C3H/HeJ mice were injected subcutaneously with 2×10⁶ cells from four different populations. Hair loss developed in recipients after either injection of total LN cells or CD8⁺NKG2D⁺ T cells alone (5 out of 5 mice per group), whereas mice receiving CD8⁺NKG2D⁻ T cells or LN cells depleted of NKG2D+ cells did not develop alopecia (0 out of 5 mice per group). ***p value=<0.001.

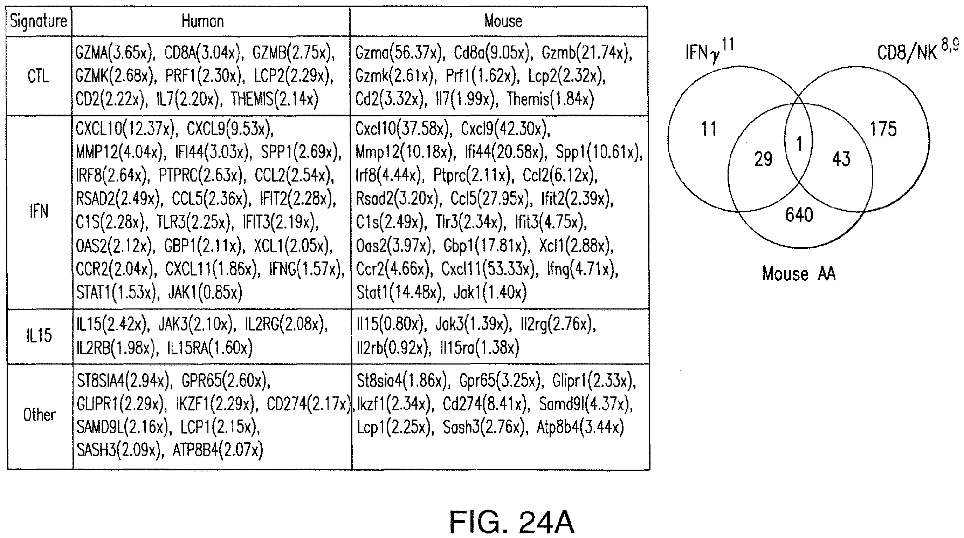

**FIG. 24A** shows transcriptional profiling of human and mouse AA skin that reveals evidence for IFNγ, cytotoxic T cells and the IL15 pathway as central to disease pathogenesis, inviting therapeutic targeting using Jak inhibitors. **FIG. 24A** is a Venn diagram showing overlap of gene list from whole alopecic C3H/HeJ mouse skin (compared with unaffected C3H/HeJ skin) with IFNγ-induced gene expression¹¹ and CD8 and NK cell gene signatures from ImmGen consortium publications^{8,9} (Top panel). Representative list of differentially expressed genes among human AA and C3H/HeJ AA reveals shared inflammatory pathways, in particular IFNγ pathway genes, genes representative of CD8 effectors, and a striking IL-15 pathway signature (Bottom panel).
**FIGS. 24B-D** show effect of treatment with a Jak3 inhibitor (JAK3i). **FIG. 24B****,** JAK3i (tofacitinib) inhibits IL-15-induced Stat3 activation in T cells. **FIG. 24C****,** JAK3i inhibits IL-15-induced LAK cell cytotoxic function. **FIG. 24D****,** JAK3i inhibits IL-15-induced LAK cell Granzyme B expression and IFNγ production.
**FIG. 25A** shows targeting CD8⁺NKG2D⁺ cytotoxic T lymphocytes with JAK3i prevents the onset of alopecia in grafted C3H/HeJ mice. C3H/HeJ grafted mice were treated systemically from the time of grafting. The onset of alopecia is inhibited by JAK3i, tofacitinib treatment. The durability of disease prevention was demonstrated by treatment withdrawal for an additional 12 weeks after treatment withdrawal.
**FIG. 25B** shows targeting CD8⁺NKG2D⁺ cytotoxic T lymphocytes with JAK3i prevents the onset of alopecia in grafted C3H/HeJ mice. C3H/HeJ grafted mice were treated systemically from the time of grafting. The onset of alopecia is inhibited by JAK3i, tofacitinib treatment.
**FIG. 25C** shows targeting CD8⁺NKG2D⁺ cytotoxic T lymphocytes with JAK3 prevents the onset of alopecia in grafted C3H/HeJ mice. The frequency of CD8⁺NKG2D⁺ T cells in skin and cutaneous lymph nodes of JAK3i treated mice were significantly decreased compared to control mice. ***p value=<0.001.
**FIG. 25D** shows targeting CD8⁺NKG2D⁺ cytotoxic T lymphocytes with JAK3i prevents the onset of alopecia in grafted C3H/HeJ mice. Immunohistochemical staining of skin biopsies showed that the expression of CD4, CD8, MHC class I and II in skin are significantly decreased in JAK3i treated mice compared to control mice.
**FIG. 25E** shows targeting CD8⁺NKG2D⁺ cytotoxic T lymphocytes with JAK3i prevents the onset of alopecia in grafted C3H/HeJ mice. ALADIN score (a summary statistic of expression of IFN signature and CTL signature genes) from JAK3i treated mice showed normalization. For gene expression studies, mice grafted with autologous health skin were included as sham-operated controls.
**FIG. 25F** shows targeting CD8⁺NKG2D⁺ cytotoxic T lymphocytes with JAK3i prevents the onset of alopecia in grafted C3H/HeJ mice. GEDI results of JAK3i treated mice showed reduction in CTL and IFN signatures.

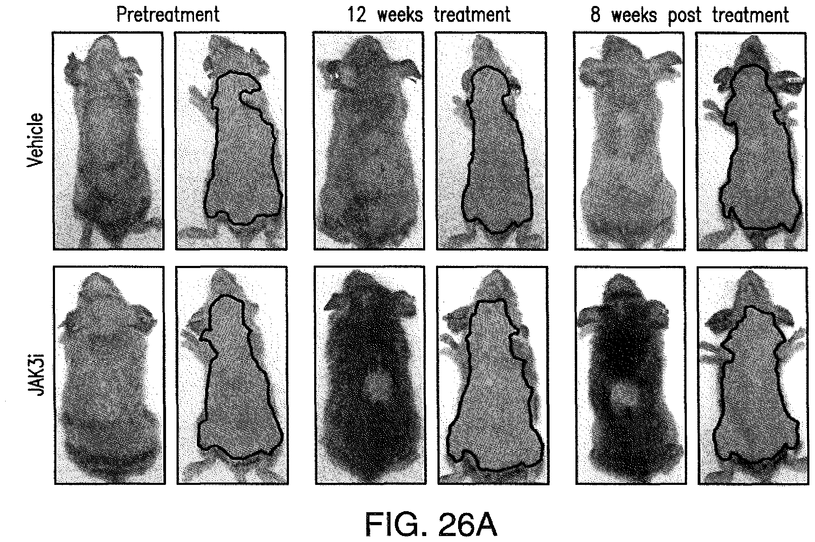

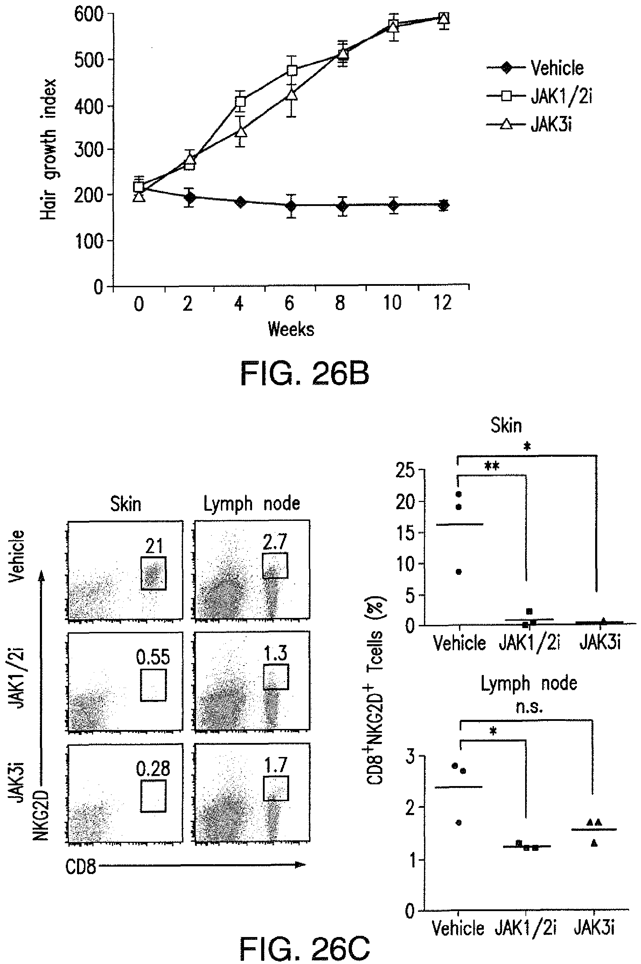

**FIG. 26A** shows reversal of established AA with topical small molecule inhibitor of the downstream effector kinase JAK3. Three-to-five per group of mice with long-standing AA (at least 12 weeks after grafting) were treated topically on the dorsal back with 0.5% JAK3i, tofacitinib or (top panels) vehicle alone (Aquaphor) by daily application for 12 weeks. A full coat of hair emerged in the JAK3i treated mice by 7 weeks of treatment and further developed by 12 weeks. The durability of hair regrowth was measured for an additional 8 weeks after treatment withdrawal with no evidence of disease recurrence.
**FIG. 26B** shows reversal of established AA with topical small molecule inhibitor of the downstream effector kinase JAK3. The graph depicts a time course of hair regrowth index shown as weeks after treatment.
**FIG. 26C** shows reversal of established AA with topical small molecule inhibitor of the downstream effector kinase, JAK3. The plots depict the frequency of CD8⁺NKG2D⁺ T cells in the skin of JAK3i-treated mice was significantly decreased compared to control mice. *p value=<0.05, **p value =<0.01, n.s.=not significant.
**FIG. 26D** shows reversal of established AA with topical small molecule inhibitor of the downstream effector kinase, JAK3. Immunohistochemical staining of skin biopsies shows treatment-related loss of expression of CD4, CD8, MHC class I and II markers.
**FIG. 26E** shows reversal of established AA with topical small molecule inhibitor of the downstream effector kinase, JAK3. The ALADIN score shows treatment-related loss of CTL and IFN signatures.
**FIG. 26F** shows reversal of established AA with topical small molecule inhibitor of the downstream effector kinase, JAK3. GEDI analysis shows territories of gene expression with treatment-related reversal of pathogenic signatures.
**FIG. 27A** shows that Rae-1 is upregulated in the AA HF. Immunostaining of lesional using a pan-Rae-1 antibody in C3H/HeJ alopecic mice, unaffected C3H/HeJ mice and C57B1.6 mice.
**FIG. 27B** shows that H60 and Rae-1 are overexpressed in AA. NKG2DL RNA expression in alopecic lesional skin from C3H/HeJ mice compared with non-lesional skin from unaffected C3H/HeJ mice. RT-PCR data from cDNA from 3 mice are shown and represented as relative fold-induction.
**FIG. 27C** shows that CD4 T cells are infrequent in AA lesional skin: Flow cytometric evaluation of lesional alopecic skin. Quantitation of CD4 and CD8 T cells as a percentage of total gated CD3⁺ or CD45⁺ cells.
**FIG. 27D** shows that effector memory immunophenotype of CD8⁺NKG2D⁺ T cells are similar in lesional skin and in the cutaneous draining lymph node. Gated CD8alpha⁺NKG2D⁺ T cells are displayed for CD8beta, NKG2D, NKG2A/C/E, CD44, CD103 and CD62L expression.
**FIG. 28** is a network map of differentially expressed upregulated genes in CD3+ CD8+NKG2D+ LN cells vs. CD8+NKG2D- cells. String.db was used to create a biological interaction score matrix with the differentially expressed genes. The network map was created using cytoscape; only biological interactions >0.75 were used. Nodes represent genes, and edges represent biological interactions as derived from string.db. Node size is proportional to fold change, and edge width is proportional to biological interaction.

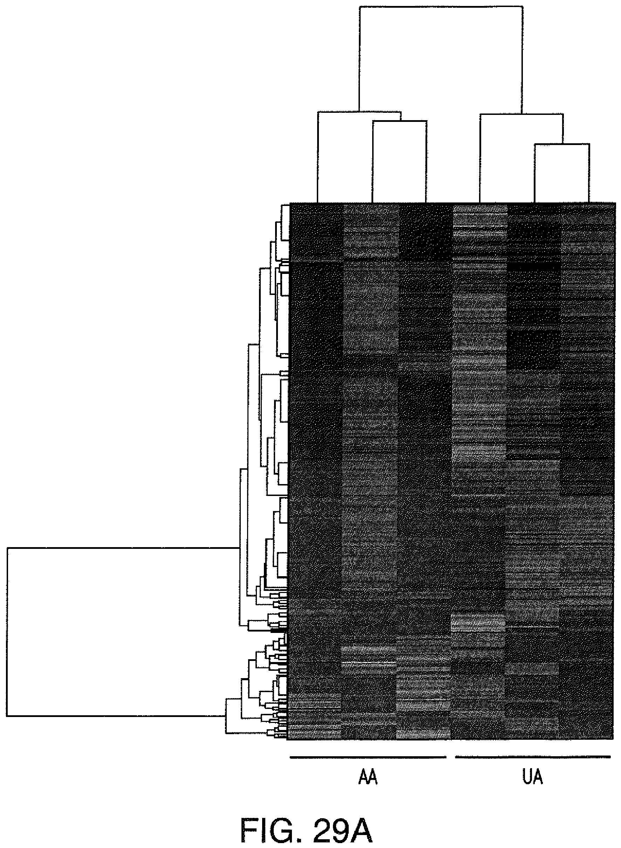

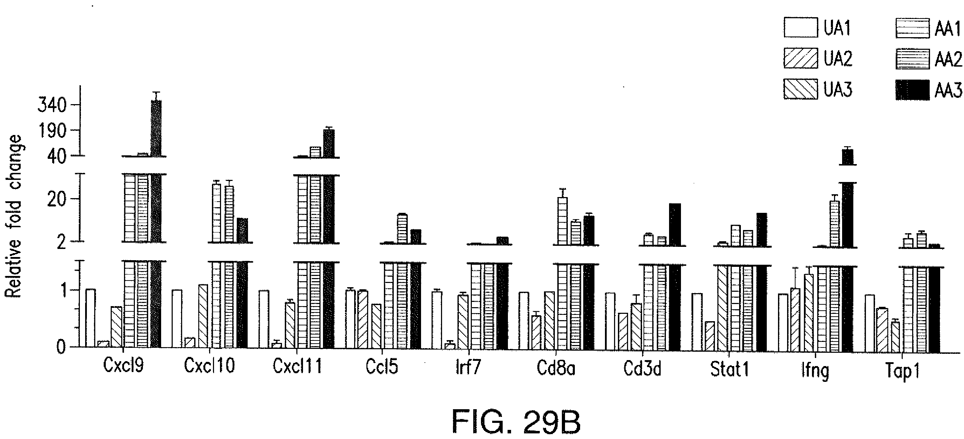

**FIG. 29** shows validation of mouse RNA expression studies. To determine the expression signature of C3H/HeJ mouse skin affected with alopecia areata, lesional skin was isolated from three affected female mice and three unaffected aged-matched controls. Total and small RNAs were isolated from whole skin and biotin-labeled cRNA was generated through *in vitro* transcription, followed by hybridization to the Affymetrix Mouse 430 2.0 Genechip. Data analysis was done as outlined in the Methods. **FIG. 29A** is a heatmap depicting the significantly and differentially expressed genes between C3H/HeJ affected and unaffected skin. **FIG. 29B** shows validation by qRT-PCR of several selected immune-related genes from this list whose expression levels are significantly upregulated in AA lesional skin compared to unaffected skin, where each bar represents the average fold change of three independent experiments. UA = unaffected; AA = affected.
**FIG. 30A** shows systemic treatment of AA mice with JAK3 inhibitor. C3H/HeJ mice with long-standing alopecia areata were treated with tofacitinib with Alzet osmotic mini-pumps (pumps, model 2004, Durect Corporation) implanted subcutaneously on the back of each mouse to deliver vehicle (poly(ethylene glycol) (PEG)300) or vehicle containing JAK3i tofacitinib (Abmole) at 15 mg/kg/day for 12 wks. Alopecia areata reversal was complete on both the back and belly.
**FIG. 30B** shows systemic treatment of AA mice with JAK3 inhibitor. Flow cytometric analysis of skin and cutaneous lymph node populations shows elimination of the CD8⁺NKG2D⁺ T cell population in treated mice (n=3 per group).
**FIG. 30C** shows systemic treatment of AA mice with JAK3 inhibitor. Immunostaining of skin from mice treated with tofactinib or placebo demonstrates elimination of CD8 infiltration and MHC I and II upregulation in tofacitinib treated mice.
**FIG. 31** depicts JAK-STAT signaling in normal hair follicle development. To analyze the status of the JAK-STAT signaling pathway during a normal hair cycle, mRNA was collected from skin of three mice at postnatal days 17, 23, 29, 33. Samples represent the telogen to anagen transition as evident in the photomicrographs of H&E stains from these time points. Day 17= early telogen; day 23 = late telogen; day 29 = early anagen; day 33 = full anagen. cDNA was made and probed on JAK-STAT qPCR arrays. JAK-STAT signaling is up in telogen and down in anagen (*see* **FIG. 32**).
**FIG. 32** is a heat map showing a hierarchical cluster analysis. mRNA isolated from mouse skin at each time point (D17, 23, 29, 33) were probed on JAK-STAT qPCR array. Red= induced, Green=repressed. Shown are all genes of the array. Several clusters of genes are evident that are upregulated in both telogen time points (d17 and d23), or upregulated in both anagen time points (d29 and d33).
**FIG. 33A** is a heat map showing a hierarchical cluster analysis. The figure shows averages of 3 biological replicates/ time point. The Control group: D17 (Telogen); Group 1: D23 (telogen); Group 2: D29 (anagen); and Group 3: D33 (anagen). JAK1 and 3 are upregulated in telogen group (red in control group and Group 1). Expression of JAK-STAT signaling pathway components is up in telogen and down in anagen.
**FIG. 33B** is a heat map showing a hierarchical cluster analysis. The figure shows averages of 3 biological replicates/ time point. The Control group: D17 (Telogen); Group 1: D23 (telogen); Group 2: D29 (anagen); and Group 3: D33 (anagen). Stat 1/2/3/5 are upregulated in telogen (red in control group and group 1). Expression of JAK-STAT signaling pathway components is up in telogen and down in anagen.
**FIG. 34** is a heat map (top) showing a cluster analysis. The Control group: D17 (Telogen); Group 1: D23 (telogen); Group 2: D29 (anagen); and Group 3: D33 (anagen). Potential ligands for JAK STAT or targets for JAK3i: OSM, IL6st (GP130), IL-4, IL-2Rg, CSF1R, and others upregulated in telogen samples.
**FIG. 35** shows summaries of genes upregulated in telogen as well as downregulated in telogen. The Control group: D17 (Telogen); Group 1: D23 (telogen); Group 2: D29 (anagen); and Group 3: D33 (anagen). CRP, a well-defined gene upregulated by JAK/STAT signaling, is strikingly increased in telogen.

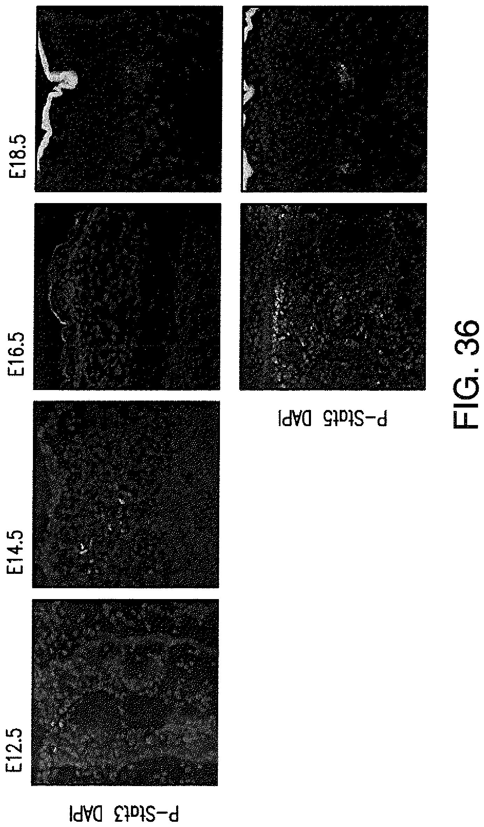

**FIG. 36** are photomicrographs showing JAK-STAT in hair follicle development: Embryonic development. Stat 3 and Stat 5 were identified to be differentially expressed between telogen and anagen. To examine the pattern of expression in embryonic development, the activated (phosphorylated) forms of these proteins were stained. As shown, P-Stat 3 is expressed in epithelial layers during early stages of development and then can be faintly seen in the dermal papilla. P-Stat5 expression appears later, at E16.5, in scattered dermal cells and becomes pronounced in the dermal condensate by E18.5.

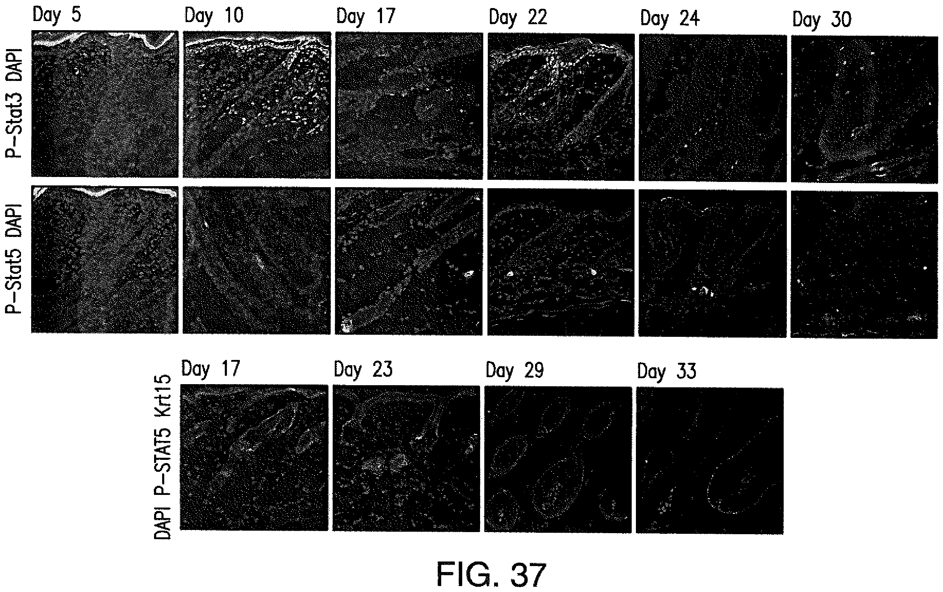

**FIG. 37** are photomicrographs showing JAK-STAT in hair follicle development: post-natal development. P-Stat3 is expressed in the basal layers of the epidermis and upper epithelial layers of the follicle during neonatal skin development. Expression can also be detected in the dermal papilla, most obviously during telogen (D17). P-Stat5 is highly expressed in the dermal papilla in all stage of the hair cycle. In telogen, expression of P-Stat5 maybe limited to a subset of DP cells, closest to the K15+ bulge (D17, bottom).

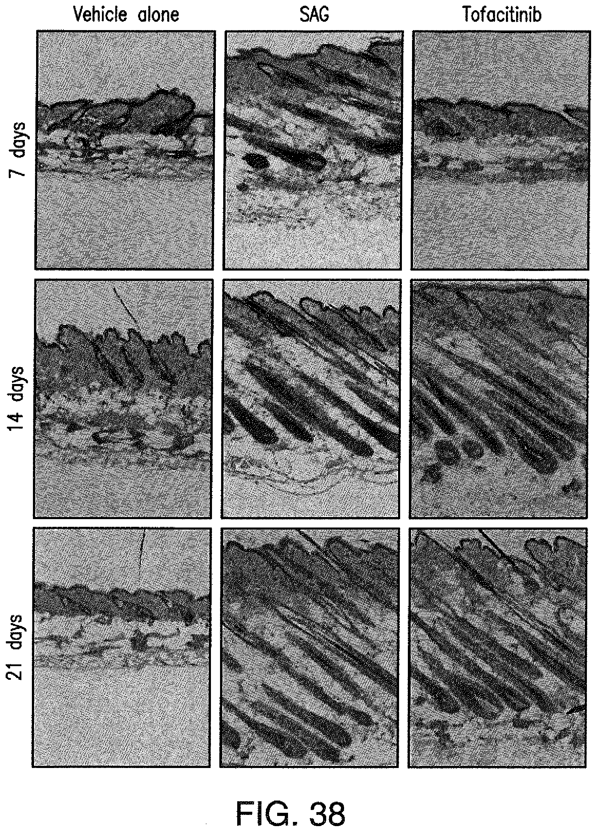

**FIG. 38** are photomicrographs showing the effect of a JAK-STAT inhibitor on the hair cycle of normal mice: Induction of anagen. Inhibition of the JAK-STAT pathway during telogen (by application of a JAK inhibitor) results in early onset of anagen. 7-8 weeks old animals in telogen were treated with controls or JAK STAT inhibitors. Negative (vehicle alone; Left Panel) and positive (SAG = sonic hedgehog agonist; Middle panel) controls show that DMSO treatment alone does not induce anagen, while treatment with sonic hedgehog agonist results in early (4-7 days post treatment) induction of anagen, as expected. Right: Treatment with the Jak 3 inhibitor Tofacitinib (10mg/mL) results in marked induction of anagen after 2 weeks or 3 weeks of treatment compared to vehicle treated mice which remain in telogen.
**FIG. 39** are photomicrographs showing effects of drug treatment on keratinocyte proliferation in vivo. Tofacitinib does not appear to cause hyperpoliferation of the epidermis. Top: Krt6, a marker of keratinocyte proliferation typically expressed in the inner root sheath of the follicle. Bottom: the effects of drug treatment on proliferation of the follicle. In tofacitinib treated skin, proliferation in the secondary germ occurs later, indicating that Tofacitinib can induce anagen.
**FIG. 40** are plots showing JAK-STAT in hair cycle: a drug treatment in mice. Quantification of number and thickness of hair follicles in skin treated with the Jak3 inhibitor, Tofacitinib, and a Jak1/2 inhibitor Ruxolitinib, as compared to plucked skin and vehicle-treated (DMSO). Both the numbers of follicles as well as the thickness of follicles is greater in drug treated skin than in vehicle treated mice.
**FIG. 41** are photomicrographs showing the effect of Jak inhibitors on human hair follicle morphogenesis: drug treatments of human fetal scalp. Human embryonic scalp at 20 weeks was obtained and treated in vitro with 30mg/mL of the Jak3 inhibitor, Tofacitinib, or vehicle alone. Skin was harvested and sectioned and assessed for hair follicle morphology. Hairs in treated skin appear more advanced in the first anagen of morphogenesis compared to DMSO treated scalp.
**FIG. 42** shows topical treatment with a JAK inhibitor. Seven-week-old normal C57BL/6 mice were shaved in telogen and treated with 1% Jak3 inhibitor (Tofacitinib; Right panel), 1.5mg/ml Isopropyl unoprostone (Latisse; Middle Right panel), 100uM SAG (shh agonist; Middle Left panel), or DMSO as vehicle (Left Panel) by daily application for 3 weeks. Effect is durable out to a little more than 1 month at the time the image was taken. Right half of each mouse was administered topical drug, left half of each mouse was treated with DMSO alone.
**FIG. 43** are photomicrographs of Ki67 staining (green) showing marked proliferation in the telogen hair follicle in Tofacitinib and SAG treated skin, compared to vehicle, indicative of the start of Anagen.

### DETAILED DESCRIPTION OF THE INVENTION

The invention provides for a Jak3 inhibitor for use in the treatment of a hair loss disorder (e.g., Alopecia Areata (AA), a common autoimmune form of hair loss). Clinical research in AA has lagged behind its more heavily investigated "sister" autoimmune diseases in which this gene has been implicated (e.g., rheumatoid arthritis (RA), type 1 diabetes mellitus (T1D), multiple sclerosis (MS)). The invention provides for therapeutics previously untested in AA, that can inform one about the clinical relevance of Jak3 related pathways in AA and related diseases.

### Abbreviations and Definitions

The singular forms "a", "an" and "the" include plural reference unless the context clearly dictates otherwise.

As used herein the term "about" is used herein to mean approximately, roughly, around, or in the region of. When the term "about" is used in conjunction with a numerical range, it modifies that range by extending the boundaries above and below the numerical values set forth. In general, the term "about" is used herein to modify a numerical value above and below the stated value by a variance of 20 percent up or down (higher or lower).

### Overview of the Integument and Hair Cells

The integument (or skin) is the largest organ of the body and is a highly complex organ covering the external surface of the body. It merges, at various body openings, with the mucous membranes of the alimentary and other canals. The integument performs a number of essential functions such as maintaining a constant internal environment via regulating body temperature and water loss; excretion by the sweat glands; but predominantly acts as a protective barrier against the action of physical, chemical and biologic agents on deeper tissues. Skin is elastic and except for a few areas such as the soles, palms, and ears, it is loosely attached to the underlying tissue. It also varies in thickness from 0.5 mm (0.02 inches) on the eyelids ("thin skin") to 4 mm (0.17 inches) or more on the palms and soles ("thick skin") (Ross MH, Histology: A text and atlas. 3rd edition, Williams and Wilkins, 1995: Chapter 14; Burkitt HG, et al, Wheater's Functional Histology. 3rd Edition, Churchill Livingstone, 1996: Chapter 9).

The skin is composed of two layers: a) the epidermis and b) the dermis. The epidermis is the outer layer, which is comparatively thin (0.1 mm). It is several cells thick and is composed of 5 layers: the stratum germinativum, stratum spinosum, stratum granulosum, stratum lucidum (which is limited to thick skin), and the stratum corneum. The outermost epidermal layer (the stratum corneum) consists of dead cells that are constantly shed from the surface and replaced from below by a single, basal layer of cells, called the stratum germinativum. The epidermis is composed predominantly of keratinocytes, which make up over 95% of the cell population. Keratinocytes of the basal layer (stratum germinativum) are constantly dividing, and daughter cells subsequently move upwards and outwards, where they undergo a period of differentiation, and are eventually sloughed off from the surface. The remaining cell population of the epidermis includes dendritic cells such as Langerhans cells and melanocytes. The epidermis is essentially cellular and non-vascular, containing little extracellular matrix except for the layer of collagen and other proteins beneath the basal layer of keratinocytes (Ross MH, Histology: A text and atlas. 3rd edition, Williams and Wilkins, 1995: Chapter 14; Burkitt HG, et al, Wheater's Functional Histology. 3rd Edition, Churchill Livingstone, 1996: Chapter 9).

The dermis is the inner layer of the skin and is composed of a network of collagenous extracellular material, blood vessels, nerves, and elastic fibers. Within the dermis are hair follicles with their associated sebaceous glands (collectively known as the pilosebaceous unit) and sweat glands. The interface between the epidermis and the dermis is extremely irregular and uneven, except in thin skin. Beneath the basal epidermal cells along the epidermal-dermal interface, the specialized extracellular matrix is organized into a distinct structure called the basement membrane (Ross MH, Histology: A text and atlas. 3rd edition, Williams and Wilkins, 1995: Chapter 14; Burkitt HG, et al, Wheater's Functional Histology. 3rd Edition, Churchill Livingstone, 1996: Chapter 9).

The mammalian hair fiber is composed of keratinized cells and develops from the hair follicle. The hair follicle is a peg of tissue derived from a downgrowth of the epidermis, which lies immediately underneath the skin's surface. The distal part of the hair follicle is in direct continuation with the external, cutaneous epidermis. Although a small structure, the hair follicle comprises a highly organized system of recognizably different layers arranged in concentric series. Active hair follicles extend down through the dermis, the hypodermis (which is a loose layer of connective tissue), and into the fat or adipose layer (Ross MH, Histology: A text and atlas. 3rd edition, Williams and Wilkins, 1995: Chapter 14; Burkitt HG, et al, Wheater's Functional Histology. 3rd Edition, Churchill Livingstone, 1996: Chapter 9).

At the base of an active hair follicle lies the hair bulb. The bulb consists of a body of dermal cells, known as the dermal papilla, contained in an inverted cup of epidermal cells known as the epidermal matrix. Irrespective of follicle type, the germinative epidermal cells at the very base of this epidermal matrix produce the hair fiber, together with several supportive epidermal layers. The lowermost dermal sheath is contiguous with the papilla basal stalk, from where the sheath curves externally around all of the hair matrix epidermal layers as a thin covering of tissue. The lowermost portion of the dermal sheath then continues as a sleeve or tube for the length of the follicle (Ross MH, Histology: A text and atlas. 3rd edition, Williams and Wilkins, 1995: Chapter 14; Burkitt HG, et al, Wheater's Functional Histology. 3rd Edition, Churchill Livingstone, 1996: Chapter 9).

Developing skin appendages, such as hair and feather follicles, rely on the interaction between the epidermis and the dermis, the two layers of the skin. In embryonic development, a sequential exchange of information between these two layers supports a complex series of morphogenetic processes, which results in the formation of adult follicle structures. However, in contrast to general skin dermal and epidermal cells, certain hair follicle cell populations, following maturity, retain their embryonic-type interactive, inductive, and biosynthetic behaviors. These properties can be derived from the very dynamic nature of the cyclical productive follicle, wherein repeated tissue remodeling necessitates a high level of dermal-epidermal interactive communication, which is vital for embryonic development and would be desirable in other forms of tissue reconstruction.

The hair fiber is produced at the base of an active follicle at a very rapid rate. For example, follicles produce hair fibers at a rate 0.4 mm per day in the human scalp and up to 1.5 mm per day in the rat vibrissa or whiskers, which means that cell proliferation in the follicle epidermis ranks amongst the fastest in adult tissues (Malkinson FD and JT Kearn, Int J Dermatol 1978, 17:536-551). Hair grows in cycles. The anagen phase is the growth phase, wherein up to 90% of the hair follicles said to be in anagen; catagen is the involuting or regressing phase which accounts for about 1-2% of the hair follicles; and telogen is the resting or quiescent phase of the cycle, which accounts for about 10-14% of the hair follicles. The cycle's length varies on different parts of the body.

Hair follicle formation and cycling is controlled by a balance of inhibitory and stimulatory signals. The signaling cues are potentiated by growth factors that are members of the TGFβ-BMP family. A prominent antagonist of the members of the TGFβ-BMP family is follistatin. Follistatin is a secreted protein that inhibits the action of various BMPs (such as BMP-2, -4, -7, and -11) and activins by binding to said proteins, and purportedly plays a role in the development of the hair follicle (Nakamura M, et al., FASEB J, 2003, 17(3):497-9; Patel K Intl J Biochem Cell Bio, 1998, 30:1087-93; Ueno N, et al., PNAS, 1987, 84:8282-86; Nakamura T, et al., Nature, 1990, 247:836-8; Iemura S, et al., PNAS, 1998, 77:649-52; Fainsod A, et al., Mech Dev, 1997, 63:39-50; Gamer LW, et al., Dev Biol, 1999, 208:222-32).

The deeply embedded end bulb, where local dermal-epidermal interactions drive active fiber growth, is the signaling center of the hair follicle comprising a cluster of mesenchymal cells, called the dermal papilla (DP). This same region is also central to the tissue remodeling and developmental changes involved in the hair fiber's or appendage's precise alternation between growth and regression phases. The DP, a key player in these activities, appears to orchestrate the complex program of differentiation that characterizes hair fiber formation from the primitive germinative epidermal cell source (Oliver RF, J Soc Cosmet Chem, 1971, 22:741-755; Oliver RF and CA Jahoda, Biology of Wool and Hair (eds Roger et al.), 1971, Cambridge University Press:51-67; Reynolds AJ and CA Jahoda, Development, 1992, 115:587-593; Reynolds AJ, et al., JInvest Dermatol, 1993, 101:634-38).

The lowermost dermal sheath (DS) arises below the basal stalk of the papilla, from where it curves outwards and upwards. This dermal sheath then externally encases the layers of the epidermal hair matrix as a thin layer of tissue and continues upward for the length of the follicle. The epidermally-derived outer root sheath (ORS) also continues for the length of the follicle, which lies immediately internal to the dermal sheath in between the two layers, and forms a specialized basement membrane termed the glassy membrane. The outer root sheath constitutes little more than an epidermal monolayer in the lower follicle, but becomes increasingly thickened as it approaches the surface. The inner root sheath (IRS) forms a mold for the developing hair shaft. It comprises three parts: the Henley layer, the Huxley layer, and the cuticle, with the cuticle being the innermost portion that touches the hair shaft. The IRS cuticle layer is a single cell thick and is located adjacent to the hair fiber. It closely interdigitates with the hair fiber cuticle layer. The Huxley layer can comprise up to four cell layers. The IRS Henley layer is the single cell layer that runs adjacent to the ORS layer (Ross MH, Histology: A text and atlas. 3rd edition, Williams and Wilkins, 1995: Chapter 14; Burkitt HG, et al, Wheater's Functional Histology. 3rd Edition, Churchill Livingstone, 1996: Chapter 9).

### Alopecia areata

Alopecia areata (AA) is one of the most prevalent autoimmune diseases, affecting approximately 4.6 million people in the US alone, including males and females across all ethnic groups, with a lifetime risk of 1.7% (1) In AA, autoimmunity develops against the hair follicle, resulting in non-scarring hair loss that can begin as patches, which can coalesce and progress to cover the entire scalp (alopecia totalis, AT) or eventually the entire body (alopecia universalis, AU). AA was first described by Cornelius Celsus in 30 A.D., using the term "ophiasis," which means "snake", due to the sinuous path of hair loss as it spread slowly across the scalp. Hippocrates first used the Greek word 'alopekia' (fox mange), the modern day term "alopecia areata" was first used by Sauvages in his Nosologica Medica, published in 1760 in Lyons, France.

Curiously, AA preferentially affects pigmented hair follicles in the anagen (growth) phase of the hair cycle, and when the hair regrows in patches of AA, it frequently grows back white or colorless. The phenomenon of 'sudden whitening of the hair' is therefore ascribed to AA with an acute onset, and has been documented throughout history as having affected several prominent individuals at times of profound grief, stress or fear (2). Examples include Shahjahan, who upon the death of his wife in 1631 experienced acute whitening of his hair, and in his grief built the Taj Mahal in her honor. Sir Thomas More, author of *Utopia,* who on the eve of his execution in 1535 was said to have become 'white in both beard and hair'. The sudden whitening of the hair is believed to result from an acute attack upon the pigmented hair follicles, leaving behind the white hairs unscathed.

Several clinical aspects of AA remain unexplained but can hold important clues toward understanding pathogenesis. AA attacks hairs only around the base of the hair follicles, which are surrounded by dense clusters of lymphocytes, resulting in the pathognomic 'swarm of bees' appearance on histology. Based on these observations, it is postulated that a signal(s) in the pigmented, anagen hair follicle is emitted which invokes an acute or chronic immune response against the lower end of the hair follicle, leading to hair cycle perturbation, acute hair shedding, hair shaft anomalies and hair breakage. Despite these dramatic perturbations in the hair follicle, there is no permanent organ destruction and the possibility of hair regrowth remains if immune privilege can be restored.

Throughout history, AA has been considered at times to be a neurological disease brought on by stress or anxiety, or as a result of an infectious agent, or even hormonal dysfunction. The concept of a genetically-determined autoimmune mechanism as the basis for AA emerged during the 20^{th} century from multiple lines of evidence. AA hair follicles exhibit an immune infiltrate with activated Th, Tc and NK cells (3, 4) and there is a shift from a suppressive (Th2) to an autoimmune (Th1) cytokine response. The humanized model of AA, which involves transfer of AA patient scalp onto immune-deficient SCID mice illustrates the autoimmune nature of the disease, since transfer of donor T-cells causes hair loss only when co-cultured with hair follicle or human melanoma homogenate (5, 6). Regulatory T cells which serve to maintain immune tolerance are observed in lower numbers in AA tissue (7), and transfer of these cells to C3H/HeJ mice leads to resistance to AA (8). Although AA has long been considered exclusively as a T-cell mediated disease, in recent years, an additional mechanism of disease has been postulated. The hair follicle is defined as one of a select few immune privileged sites in the body, characterized by the presence of extracellular matrix barriers to impede immune cell trafficking, lack of antigen presenting cells, and inhibition of NK cell activity via the local production of immunosuppressive factors and reduced levels of MHC class I expression (9). Thus, the notion of a 'collapse of immune privilege' has also been invoked as part of the mechanism by which AA can arise. Support for a genetic basis for AA comes from multiple lines of evidence, including the observed heritability in first degree relatives (10, 11), twin studies (12), and most recently, from the results of our family-based linkage studies (13).

### Treatment of Hair Loss Disorders

This application discloses the discovery that a known therapeutic, for example an inhibitor of a protein tyrosine kinase (PTK) involved in cytokine signaling, such as Jak 3, can be used for the treatment of hair loss disorders. Non-limiting examples of hair loss disorders include: androgenetic alopecia, Alopecia areata, telogen effluvium, alopecia areata, alopecia totalis, and alopecia universalis. For example, androgenetic alopecia (also called anrogenic alopecia in women) is a common form of *hair-loss* in both men and women resulting in hair thinning and baldness of the scalp. For example, alopecia areata (AA), typically begins with patches of hair-loss on the scalp or other parts of the body. If AA is not treated or is not responsive to the treatments, then baldness in the affected area can result (e.g., alopecia totalis). Alopecia totalis (AT) as well as alopecia universalis (AU) are severe forms of alopecia areata (AA). AU is the most severe form of alopecia areata. *See, e.g.,* Cho et al. (2012) J Korean Med Sci, 27: 799-802.

The application discloses a method of treating a hair-loss disorder in a mammalian subject in need thereof, the method comprising administering to the subject an inhibitor of a protein tyrosine kinase (PTK) involved in cytokine signaling. The Jak3 inhibitor may be an antibody that specifically binds to a Jak3 protein or a fragment thereof; an antisense RNA or antisense DNA that decreases expression of the gene that encodes the Jak3 protein; an antisense RNA or antisense DNA that decreases expression of the Jak3 protein; a siRNA that specifically targets the Jak3 gene; a small molecule; or a combination thereof. The inhibitor may be a Jak3 inhibitor. The inhibitor may be tofacitinib (CP690550). The small molecule may be Janex 1 (WHI-P131), PF-956980, WHI-P154, VX-509, JAK3 Inhibitor IV, NSC114792, or R348. The hair-loss disorder may comprise androgenetic alopecia, telogen effluvium, alopecia areata, tinea capitis, alopecia totalis, hypotrichosis, hereditary hypotrichosis simplex, or alopecia universalis.

The application discloses a method for inducing hair growth in a subject where the method comprises administering to the subject an effective amount of an inhibitor of a protein tyrosine kinase (PTK) involved in cytokine signaling. The Jak3 inhibitor may be an antibody that specifically binds to a Jak3 protein or a fragment thereof; an antisense RNA or antisense DNA that decreases expression of the gene that encodes the Jak3 protein; an antisense RNA or antisense DNA that decreases expression of the Jak3 protein; a siRNA that specifically targets the Jak3 gene; a small molecule; or a combination thereof. The inhibitor may be a Jak3 inhibitor. The inhibitor may be tofacitinib (CP690550). The small molecule may be Janex 1 (WHI-P131), PF-956980, WHI-P154, VX-509, JAK3 Inhibitor IV, NSC114792, or R348. The hair-loss disorder may comprise androgenetic alopecia, telogen effluvium, alopecia areata, tinea capitis, alopecia totalis, hypotrichosis, hereditary hypotrichosis simplex, or alopecia universalis.

The application also discloses a method of treating a hair-loss disorder in a mammalian subject in need thereof, the method comprising administering to the subject a Jak3 inhibitor. The inhibitor may be an antibody or antibody fragment that is directed to SEQ ID NO: 1. The hair-loss disorder may comprise androgenetic alopecia, telogen effluvium, alopecia areata, tinea capitis, alopecia totalis, hypotrichosis, hereditary hypotrichosis simplex, or alopecia universalis

The application also discloses a method for inducing hair growth in a subject where the method comprises administering to the subject an effective amount of a Jak3 inhibitor, thereby controlling hair growth in the subjectThe inhibitor may comprise an antibody that specifically binds to a protein comprising SEQ ID NO: 1. The subject may be afflicted with a hair-loss disorder. The hair-loss disorder may comprise androgenetic alopecia, telogen effluvium, alopecia areata, tinea capitis, alopecia totalis, hypotrichosis, hereditary hypotrichosis simplex, or alopecia universalis.

This application discloses the discovery that a number human genes have been identified as a cohort of genes involved in telogen-to-anagen transition of the hair cycle (e.g, Telogen-to-Anagen Hair Cycle (TAHC) gene). These genes were identified as being upregulated in the telogen phase of the hair cycle, and can be correlated with the presence of a hair loss disorder in a subject. These genes, now that they have been identified, can be used for a variety of useful methods; for example, they can be used to determine whether a subject has susceptibility to a hair-loss disorder, such as Alopecia Areata (AA). The genes identified as part of this telogen-to-anagen transition hair cycle cohort or group (i.e., "TAHC genes") include CSF1R (Gene ID Accession No. 1436), FCER2 (Gene ID Accession No. 2208), IFNGR1 (Gene ID Accession No. 3459), IL20 (Gene ID Accession No. 50604), OAS1 (Gene ID Accession No. 4938), PTPRC (Gene ID Accession No. 5788), CEBPD (Gene ID Accession No. 1052), CRP (Gene ID Accession No. 1401), IL2RA (Gene ID Accession No. 3559), IL4 (Gene ID Accession No. 3565), IL6ST (Gene ID Accession No. 3572), INSR (Gene ID Accession No. 3643), JAK3 (Gene ID Accession No. 3718), NR3C1 (Gene ID Accession No. 2908), OSM (Gene ID Accession No. 5008), PTPN11 (Gene ID Accession No. 5781), SOCS3 (Gene ID Accession No. 9021), STAT5A (Gene ID Accession No. 6776), STAT5B (Gene ID Accession No. 6777), CCND1 (Gene ID Accession No. 595), F2 (Gene ID Accession No. 2147), LRG1 (Gene ID Accession No. 116844), PRLR (Gene ID Accession No. 5618), MPL (Gene ID Accession No. 4352), and JUNB (Gene ID Accession No. 3726).

The application discloses a method for detecting the presence of or a predisposition to a hair-loss disorder in a human subject where the method comprises obtaining a biological sample from a human subject; and detecting whether or not there is an alteration in the level of expression of an mRNA or a protein encoded by a TAHC gene in the subject as compared to the level of expression in a subject not afflicted with a hair-loss disorder The detecting may comprise determining whether mRNA expression or protein expression of the TAHC gene is increased or decreased as compared to expression in a normal sample. The detecting may comprise determining in the sample whether expression of at least 2 TAHC proteins, at least 3 TAHC proteins, at least 4 TAHC proteins, at least 5 TAHC proteins, at least 6 TAHC proteins, at least 6 TAHC proteins, at least 7 TAHC proteins, or at least 8 TAHC proteins is increased or decreased as compared to expression in a normal sample. The detecting may comprise determining in the sample whether expression of at least 2 TAHC mRNAs, at least 3 TAHC mRNAs, at least 4 TAHC mRNAs, at least 5 TAHC mRNAs, at least 6 TAHC mRNAs, at least 6 TAHC mRNAs, at least 7 TAHC mRNAs, or at least 8 TAHC mRNAs is increased or decreased as compared to expression in a normal sample. In one embodiment, an increase in the expression of at least 2 TAHC genes, at least 3 TAHC genes, at least 4 TAHC genes, at least 5 TAHC genes, at least 6 TAHC genes, at least 7 TAHC genes, or at least 8 TAHC genes indicates a predisposition to or presence of a hair-loss disorder in the subject. In another embodiment, a decrease in the expression of at least 2 TAHC genes, at least 3 TAHC genes, at least 4 TAHC genes, at least 5 TAHC genes, at least 6 TAHC genes, at least 7 TAHC genes, or at least 8 TAHC genes indicates a predisposition to or presence of a hair-loss disorder in the subject. In one embodiment, the mRNA expression or protein expression level in the subject is about 5-fold increased, about 10-fold increased, about 15-fold increased, about 20-fold increased, about 25-fold increased, about 30-fold increased, about 35-fold increased, about 40-fold increased, about 45-fold increased, about 50-fold increased, about 55-fold increased, about 60-fold increased, about 65-fold increased, about 70-fold increased, about 75-fold increased, about 80-fold increased, about 85-fold increased, about 90-fold increased, about 95-fold increased, or is 100-fold increased, as compared to that in the normal sample. In some embodiments, the he mRNA expression or protein expression level in the subject is at least about 100-fold increased, at least about 200-fold increased, at least about 300-fold increased, at least about 400-fold increased, or is at least about 500-fold increased, as compared to that in the normal sample. In further embodiments, the mRNA expression or protein expression level of the TAHC gene in the subject is about 5-fold to about 70-fold increased, as compared to that in the normal sample. In other embodiments, the mRNA or protein expression level of the TAHC gene in the subject is about 5-fold to about 90-fold increased, as compared to that in the normal sample. In one embodiment, the mRNA expression or protein expression level in the subject is about 5-fold decreased, about 10-fold decreased, about 15-fold decreased, about 20-fold decreased, about 25-fold decreased, about 30-fold decreased, about 35-fold decreased, about 40-fold decreased, about 45-fold decreased, about 50-fold decreased, about 55-fold decreased, about 60-fold decreased, about 65-fold decreased, about 70-fold decreased, about 75-fold decreased, about 80-fold decreased, about 85-fold decreased, about 90-fold decreased, about 95-fold decreased, or is 100-fold decreased, as compared to that in the normal sample. In some embodiments, the mRNA expression or protein expression level in the subject is at least about 100-fold decreased, as compared to that in the normal sample. In some embodiments, the mRNA or protein expression level of the TAHC gene in the subject is about 5-fold to about 70-fold decreased, as compared to that in the normal sample. In yet other embodiments, the mRNA or protein expression level of the TAHC gene in the subject is about 5-fold to about 90-fold decreased, as compared to that in the normal sample. In further embodiments, the detecting comprises gene sequencing, selective hybridization, selective amplification, gene expression analysis, or a combination thereof. In another embodiment, the hair-loss disorder comprises androgenetic alopecia, alopecia areata, telogen effluvium, alopecia totalis, hypotrichosis, hereditary hypotrichosis simplex, or alopecia universalis. In one embodiment, the TAHC gene is CSF1R, FCER2, IFNGR1, IL20, OAS1, PTPRC, CEBPD, CRP, IL2RA, IL4, IL6ST, INSR, JAK3, NR3C1, OSM, PTPN11, SOCS3, STAT5A, STAT5B, CCND1, F2, LRG1, PRLR, MPL, or JUNB. In another embodiment, the TAHC gene is CRP.

### Diagnosis

The application discloses methods to diagnose whether or not a subject is susceptible to or has a hair loss disorder. The diagnostic methodsmay be based on monitoring the expression of TAHC genes, such as CSF1R, FCER2, IFNGR1, IL20, OAS1, PTPRC, CEBPD, CRP, IL2RA, IL4, IL6ST, INSR, JAK3, NR3C1, OSM, PTPN11, SOCS3, STAT5A, STAT5B, CCND1, F2, LRG1, PRLR, MPL, or JUNB, in a subject, for example whether they are increased or decreased as compared to a normal sample. As used herein, the term "diagnosis" includes the detection, typing, monitoring, dosing, comparison, at various stages, including early, pre-symptomatic stages, and late stages, in adults and children. Diagnosis can include the assessment of a predisposition or risk of development, the prognosis, or the characterization of a subject to define most appropriate treatment (pharmacogenetics).

The application discloses diagnostic methods to determine whether an individual is at risk of developing a hair-loss disorder, or suffers from a hair-loss disorder, wherein the disease results from an alteration in the expression of TAHC genes. A method of detecting the presence of or a predisposition to a hair-loss disorder in a subject is disclosed. The subject can be a human or a child thereof. The method can comprise detecting in a sample from the subject whether or not there is an alteration in the level of expression of a protein encoded by a TAHC gene in the subject as compared to the level of expression in a subject not afflicted with a hair-loss disorder. In one embodiment, the detecting can comprise determining whether mRNA expression of the TAHC is increased or decreased. For example, in a microarray assay, one can look for differential expression of a TAHC gene. Any expression of a TAHC gene that is either 2X higher or 2X lower than TAHC expression observed for a subject not afflicted with a hair-loss disorder (as indicated by a fluorescent read-out) is deemed not normal, and worthy of further investigation. The detecting can also comprise determining in the sample whether expression of at least 2 TAHC proteins, at least 3 TAHC proteins, at least 4 TAHC proteins, at least 5 TAHC proteins, at least 6 TAHC proteins, at least 6 TAHC proteins, at least 7 TAHC proteins, or at least 8 TAHC proteins is increased or decreased. The presence of such an alteration is indicative of the presence or predisposition to a hair-loss disorder.

The presence of an alteration in a TAHC gene in the sample is detected through the genotyping of a sample, for example via gene sequencing, selective hybridization, amplification, gene expression analysis, or a combination thereof. In one embodiment, the sample can comprise blood, serum, sputum, lacrimal secretions, semen, vaginal secretions, fetal tissue, skin tissue, epithelial tissue, muscle tissue, amniotic fluid, or a combination thereof.

The applciation discloses a diagnostic kit used to determine whether a sample from a subject exhibits increased expression of at least 2 or more TAHC genes. The kit may comprise a nucleic acid primer that specifically hybridizes to one or more TAHC genes. The application also discloses a diagnostic kit used to determine whether a sample from a subject exhibits a predisposition to a hair-loss disorder in a human subject. The TAHC gene may be CSF1R, FCER2, IFNGR1, IL20, OAS1, PTPRC, CEBPD, CRP, IL2RA, IL4, IL6ST, INSR, JAK3, NR3C1, OSM, PTPN11, SOCS3, STAT5A, STAT5B, CCND1, F2, LRG1, PRLR, MPL, or JUNB. The hair-loss disorder may comprise androgenetic alopecia, alopecia areata, telogen effluvium, alopecia totalis, hypotrichosis, hereditary hypotrichosis simplex, or alopecia universalis.

### DNA and Amino Acid Manipulation Methods and Purification Thereof

The practice of aspects of the present invention can employ, unless otherwise indicated, conventional techniques of cell biology, cell culture, molecular biology, transgenic biology, microbiology, recombinant DNA, and immunology, which are within the skill of the art. Such techniques are explained fully in the literature. *See,* e.g., Molecular Cloning A Laboratory Manual, 3rd Ed., ed. by Sambrook (2001), Fritsch and Maniatis (Cold Spring Harbor Laboratory Press: 1989); DNA Cloning. Volumes I and II (D. N. Glover ed., 1985); Oligonucleotide Synthesis (M. J. Gait ed., 1984); Mullis et al. U.S. Pat. No: 4,683,195; Nucleic Acid Hybridization (B. D. Hames & S. J. Higgins eds. 1984); Transcription and Translation (B. D. Hames & S. J. Higgins eds. 1984); Culture Of Animal Cells (R. I. Freshney, Alan R. Liss, Inc., 1987); Immobilized Cells and Enzymes (IRL Press, 1986); B. Perbal, A Practical Guide To Molecular Cloning (1984); the series, Methods In Enzymology (Academic Press, Inc., N.Y.), specifically, Methods In Enzymology, Vols. 154 and 155 (Wu et al. eds.); Gene Transfer Vectors For Mammalian Cells (J. H. Miller and M. P. Calos eds., 1987, Cold Spring Harbor Laboratory); Immunochemical Methods In Cell And Molecular Biology (Caner and Walker, eds., Academic Press, London, 1987); Handbook Of Experimental Immunology. Volumes I-IV (D. M. Weir and C. C. Blackwell, eds., 1986); Manipulating the Mouse Embryo, (Cold Spring Harbor Laboratory Press, Cold Spring Harbor, N.Y., 1986).

One skilled in the art can obtain a protein in several ways, which includeisolating the protein via biochemical means or expressing a nucleotide sequence encoding the protein of interest by genetic engineering methods.

A protein is encoded by a nucleic acid (including, for example, genomic DNA, complementary DNA (cDNA), synthetic DNA, as well as any form of corresponding RNA). For example, it can be encoded by a recombinant nucleic acid of a gene. The proteins of the invention can be obtained from various sources and can be produced according to various techniques known in the art. For example, a nucleic acid that encodes a protein can be obtained by screening DNA libraries, or by amplification from a natural source. A protein can be a fragment or portion thereof. The nucleic acids encoding a protein can be produced via recombinant DNA technology and such recombinant nucleic acids can be prepared by conventional techniques, including chemical synthesis, genetic engineering, enzymatic techniques, or a combination thereof. For example, a Jak 3 protein is the polypeptide encoded by the nucleic acid having the nucleotide sequence shown in SEQ ID NO: 2. An example of a Jak 3 polypeptide has the amino acid sequence shown in SEQ ID NO: 1.

The polypeptide sequence of human Jak 3 is depicted in SEQ ID NO: 1. The nucleotide sequence of human Jak 3 is shown in SEQ ID NO: 2. Sequence information related to Jak 3 is accessible in public databases by GenBank Accession numbers NP_000206 (for protein) and NM 000215 (for nucleic acid). JAK3 is a downstream signaling partner of the IL-2 receptor common gamma chain, which is shared with the IL-2, -4, -7, -9, -15, and - 21 receptors.

SEQ ID NO: 1 is the human wild type amino acid sequence corresponding to Jak3 (residues 1 - 1124):

1 
61 
121 
181 
241 
301 
361 
421 
481 
541 
601 
661 
721 
781 
841 
901 
961 
1021 
1081 

SEQ ID NO: 2 is the human wild type nucleotide sequence corresponding to Jak3 (nucleotides 1-5449), wherein the underscored bolded "ATG" denotes the beginning of the open reading frame:

1 cacacaggaa ggagccgagt gggactttcc tctcgctgcc tcccggctct gcccgccctt
61 cgaaagtcca gggtccctgc ccgctaggca agttgcactc atggcacctc caagtgaaga
121 gacgcccctg atccctcagc gttcatgcag cctcttgtcc acggaggctg gtgccctgca
181 tgtgctgctg cccgctcggg gccccggccc cccccagcgc ctatctttct cctttgggga
241 ccacttggct gaggacctgt gcgtgcaggc tgccaaggcc agcggcatcc tgcctgtgta
301 ccactccctc tttgctctgg ccacggagga cctgtcctgc tggttccccc cgagccacat
361 cttctccgtg gaggatgcca gcacccaagt cctgctgtac aggattcgct tttacttccc
421 caattggttt gggctggaga agtgccaccg cttcgggcta cgcaaggatt tggccagtgc
481 tatccttgac ctgccagtcc tggagcacct ctttgcccag caccgcagtg acctggtgag
541 tgggcgcctc cccgtgggcc tcagtctcaa ggagcagggt gagtgtctca gcctggccgt
601 gttggacctg gcccggatgg cgcgagagca ggcccagcgg ccgggagagc tgctgaagac
661 tgtcagctac aaggcctgcc tacccccaag cctgcgcgac ctgatccagg gcctgagctt
721 cgtgacgcgg aggcgtattc ggaggacggt gcgcagagcc ctgcgccgcg tggccgcctg
781 ccaggcagac cggcactcgc tcatggccaa gtacatcatg gacctggagc ggctggatcc
841 agccggggcc gccgagacct tccacgtggg cctccctggg gcccttggtg gccacgacgg
901 gctggggctg ctccgcgtgg ctggtgacgg cggcatcgcc tggacccagg gagaacagga
961 ggtcctccag cccttctgcg actttccaga aatcgtagac attagcatca agcaggcccc
1021 gcgcgttggc ccggccggag agcaccgcct ggtcactgtt accaggacag acaaccagat
1081 tttagaggcc gagttcccag ggctgcccga ggctctgtcg ttcgtggcgc tcgtggacgg
1141 ctacttccgg ctgaccacgg actcccagca cttcttctgc aaggaggtgg caccgccgag
1201 gctgctggag gaagtggccg agcagtgcca cggccccatc actctggact ttgccatcaa
1261 caagctcaag actgggggct cacgtcctgg ctcctatgtt ctccgccgca gcccccagga
1321 ctttgacagc ttcctcctca ctgtctgtgt ccagaacccc cttggtcctg attataaggg
1381 ctgcctcatc cggcgcagcc ccacaggaac cttccttctg gttggcctca gccgacccca
1441 cagcagtctt cgagagctcc tggcaacctg ctgggatggg gggctgcacg tagatggggt

1501 ggcagtgacc ctcacttcct gctgtatccc cagacccaaa gaaaagtcca acctgatcgt
1561 ggtccagaga ggtcacagcc cacccacatc atccttggtt cagccccaat cccaatacca
1621 gctgagtcag atgacatttc acaagatccc tgctgacagc ctggagtggc atgagaacct
1681 gggccatggg tccttcacca agatttaccg gggctgtcgc catgaggtgg tggatgggga
1741 ggcccgaaag acagaggtgc tgctgaaggt catggatgcc aagcacaaga actgcatgga
1801 gtcattcctg gaagcagcga gcttgatgag ccaagtgtcg taccggcatc tcgtgctgct
1861 ccacggcgtg tgcatggctg gagacagcac catggtgcag gaatttgtac acctgggggc
1921 catagacatg tatctgcgaa aacgtggcca cctggtgcca gccagctgga agctgcaggt
1981 ggtcaaacag ctggcctacg ccctcaacta tctggaggac aaaggcctgc cccatggcaa
2041 tgtctctgcc cggaaggtgc tcctggctcg ggagggggct gatgggagcc cgcccttcat
2101 caagctgagt gaccctgggg tcagccccgc tgtgttaagc ctggagatgc tcaccgacag
2161 gatcccctgg gtggcccccg agtgtctccg ggaggcgcag acacttagct tggaagctga
2221 caagtggggc ttcggcgcca cggtctggga agtgtttagt ggcgtcacca tgcccatcag
2281 tgccctggat cctgctaaga aactccaatt ttatgaggac cggcagcagc tgccggcccc
2341 caagtggaca gagctggccc tgctgattca acagtgcatg gcctatgagc cggtccagag
2401 gccctccttc cgagccgtca ttcgtgacct caatagcctc atctcttcag actatgagct
2461 cctctcagac cccacacctg gtgccctggc acctcgtgat gggctgtgga atggtgccca
2521 gctctatgcc tgccaagacc ccacgatctt cgaggagaga cacctcaagt acatctcaca
2581 gctgggcaag ggcaactttg gcagcgtgga gctgtgccgc tatgacccgc taggcgacaa
2641 tacaggtgcc ctggtggccg tgaaacagct gcagcacagc gggccagacc agcagaggga
2701 ctttcagcgg gagattcaga tcctcaaagc actgcacagt gatttcattg tcaagtatcg
2761 tggtgtcagc tatggcccgg gccgccagag cctgcggctg gtcatggagt acctgcccag
2821 cggctgcttg cgcgacttcc tgcagcggca ccgcgcgcgc ctcgatgcca gccgcctcct
2881 tctctattcc tcgcagatct gcaagggcat ggagtacctg ggctcccgcc gctgcgtgca
2941 ccgcgacctg gccgcccgaa acatcctcgt ggagagcgag gcacacgtca agatcgctga
3001 cttcggccta gctaagctgc tgccgcttga caaagactac tacgtggtcc gcgagccagg
3061 ccagagcccc attttctggt atgcccccga atccctctcg gacaacatct tctctcgcca
3121 gtcagacgtc tggagcttcg gggtcgtcct gtacgagctc ttcacctact gcgacaaaag
3181 ctgcagcccc tcggccgagt tcctgcggat gatgggatgt gagcgggatg tccccgccct
3241 ctgccgcctc ttggaactgc tggaggaggg ccagaggctg ccggcgcctc ctgcctgccc
3301 tgctgaggtt cacgagctca tgaagctgtg ctgggcccct agcccacagg accggccatc
3361 attcagcgcc ctgggccccc agctggacat gctgtggagc ggaagccggg ggtgtgagac
3421 tcatgccttc actgctcacc cagagggcaa acaccactcc ctgtcctttt catagctcct
3481 gcccgcagac ctctggatta ggtctctgtt gactggctgt gtgaccttag gcccggagct
3541 gcccctctct gggcctcaga ggccttatga gggtcctcta cttcaggaac acccccatga
3601 cattgcattt gggggggctc ccgtggcctg tagaatagcc tgtggccttt gcaatttgtt
3661 aaggttcaag acagatgggc atatgtgtca gtggggctct ctgagtcctg gcccaaagaa
3721 gcaaggaacc aaatttaaga ctctcgcatc ttcccaaccc cttaagccct ggccccctga
3781 gtttcctttt ctgtctctct ctttttattt tttttatttt tatttttatt tttgagacag
3841 agcctcgctc tgttacccag ggtggagtgc agtggtgcga tctcggctca gtgcaacctc
3901 tgcttcccag gttcaagcga ttctcctgcc tcagcctccc gagtagctgg gattacaggt
3961 gtgcaccacc acacccggct aatttttttt atttttaata gagatgaggt ttcaccatga
4021 tggccaggct gatctcgaac tcctaacctc aagtgatcct cccacctcag cctcccaaag
4081 tgttggaata ataggcatga gccactgcac ccaggctttt ttttttttaa atttattatt
4141 attattttta agagacagga tcttgctacg ttgcccaggc tggtcttgaa ctcctgggct
4201 acagtgatcc tcctgcctta tcctcctaaa tagctgggac tacagcacct agttttgagt
4261 ttcctgtctt atttccaatg gggacattca tgtagctttt tttttttttt tttttttgag
4321 acggagtctc gctctgtcgc ccaggctgga gtacagtggc gcaatctagg ctcactgcaa
4381 gctccgcctc ctgggttcac accattctct cgcctcagcc tcccaagtag ctgggactac
4441 aggcgcccgc caccacaccc ggctaatttt ttgtattttt agtagagacg gggtttcacc
4501 ttgttagcca ggatggtttc catctcctga cctcgtgatc tgcccgtctc ggcctcccaa
4561 agtgctggga ttacaggcat gagccactgc gcccggccct catgtagctt taaatgtatg
4621 atctgacttc tgctccccga tctctgtttc tctggaggaa gccaaggaca agagcagttg
4681 ctgtggctgg gactctgcct tttaggggag cccgtgtatc tctttgggat cctgaaaggg
4741 ggcaggaaag gctggggtcc cagtccaccc taatggtatc tgagtgtcct agggcttcag
4801 ttttcccacc tgtccaatgg gaccctttct gtcctcaccc tacaaggggc acaaagggat
4861 gacaccaaac ctggcaggaa cttttcacgc aatcaaggga aggaaaggca ttcctggcag
4921 agggaacagc atgccaagcg tgagaaggct cagagtaagg aggttaagag cccaagtatt
4981 ggagcctaca gttttgcccc ttccatgcag tgtgacagtg ggcaagttcc tttccctctc
5041 tgggtctcag ttctgtcccc tgcaaaatgg tcagagctta ccccttggct gtgcagggtc
5101 aactttctga ctggtgagag ggattctcat gcaggttaag cttctgctgc tcctcctcac

5161 ctgcaaagct tttctgccac ttttgcctcc ttggaaaact cttatccatc tctcaaaact
5221 ccagctacca catccttgca gccttccctc atataccccc actactactg tagccctgtc
5281 cttccctcca gccccactct ggccctgggg ctggggaagt gtctgtgtcc agctgtctcc
5341 cctgacctca gggttccttg ggggctgggc tgaggcctca gtacagaggg ggctctggaa
5401 atgtttgttg actgaataaa ggaattcagt ggaaaaaaaa aaaaaaaaa

Protein variants can include amino acid sequence modifications. For example, amino acid sequence modifications fall into one or more of three classes: substitutional, insertional or deletional variants. Insertions can include amino and/or carboxyl terminal fusions as well as intrasequence insertions of single or multiple amino acid residues. Insertions ordinarily will be smaller insertions than those of amino or carboxyl terminal fusions, for example, on the order of one to four residues. Deletions are characterized by the removal of one or more amino acid residues from the protein sequence. These variants ordinarily are prepared by site-specific mutagenesis of nucleotides in the DNA encoding the protein, thereby producing DNA encoding the variant, and thereafter expressing the DNA in recombinant cell culture.

Nucleic acid sequences comprising a gene, such as a Jak3 gene, that encodes a polypeptide can be synthesized, in whole or in part, using chemical methods known in the art. Alternatively, a polypeptide, such as Jak3, can be produced using chemical methods to synthesize its amino acid sequence, such as by direct peptide synthesis using solid-phase techniques. Protein synthesis can either be performed using manual techniques or by automation. Automated synthesis can be achieved, for example, using Applied Biosystems 431A Peptide Synthesizer (Perkin Elmer). Optionally, fragments of Jak3 polypeptides can be separately synthesized and combined using chemical methods to produce a full-length molecule.

As used herein, a "Jak3 molecule" can be a nucleic acid which encodes a polypeptide that exhibits Jak3 activity, or a polypeptide or peptidomimetic that exhibits Jak3 activity. For example, a Jak3 molecule can include the human Jak3 protein (e.g., having the amino acid sequence shown in SEQ ID NO: 1), or a variant thereof, such as a fragment thereof, that exhibits Jak3 activity. Jak3 activity can encompass signaling events by way of type I cytokine receptors (e.g., IL-2, IL-4, IL-7, IL-9, IL-15, IL-21) that use the common gamma chain (γc). For example, Jak3 activity can be a signal transduced in response to its activation via tyrosine phosphorylation by interleukin receptors.

The nucleic acid can be any type of nucleic acid, including genomic DNA, complementary DNA (cDNA), synthetic or semi-synthetic DNA, as well as any form of corresponding RNA. For example, a Jak3 molecule can comprise a recombinant nucleic acid encoding human Jak3 protein. In one embodiment, a Jak3 molecule can comprise a non-naturally occurring nucleic acid created artificially (such as by assembling, cutting, ligating or amplifying sequences). A Jak3 molecule can be double-stranded. A Jak3 molecule can be single-stranded. The Jak3 molecules of the invention can be obtained from various sources and can be produced according to various techniques known in the art. For example, a nucleic acid that is a Jak3 molecule can be obtained by screening DNA libraries, or by amplification from a natural source. The Jak3 molecules can be produced via recombinant DNA technology and such recombinant nucleic acids can be prepared by conventional techniques, including chemical synthesis, genetic engineering, enzymatic techniques, or a combination thereof. Non-limiting examples of a Jak3 molecule that is a nucleic acid, is the nucleic acid comprising SEQ ID NO: 2. Another example of a Jak3 molecule is a fragment of a nucleic acid comprising the sequence shown in SEQ ID NO: 2, wherein the fragment exhibits Jak3 activity. A Jak3 molecule of this invention also encompasses variants of the human nucleic acid encoding the Jak3 protein, or variants of the human Jak3 proteins that exhibit Jak3 activity. A Jak3 molecule can also include a fragment of the human Jak3 nucleic acid which encodes a polypeptide that exhibits Jak3 activity. A Jak3 molecule can encompass a fragment of the human Jak3 protein that exhibits Jak3 activity.

A Jak3 molecule can also encompass Jak3 ortholog genes, which are genes conserved among different biological species such as humans, dogs, cats, mice, and rats, that encode proteins (for example, homologs (including splice variants), mutants, and derivatives) having biologically equivalent functions as the human-derived protein (such as a Jak3 protein). Jak3 orthologs include any mammalian ortholog of Jak3 inclusive of the ortholog in humans and other primates, experimental mammals (such as mice, rats, hamsters and guinea pigs), mammals of commercial significance (such as horses, cows, camels, pigs and sheep), and also companion mammals (such as domestic animals, e.g., rabbits, ferrets, dogs, and cats).

The Jak 3 variants can comprise, for instance, naturally-occurring variants due to allelic variations between individuals (e.g., polymorphisms), mutated alleles related to alopecia areata, or alternative splicing forms. In one embodiment, a Jak3 molecule is a nucleic acid variant of the nucleic acid having the sequence shown in SEQ ID NO: 2, wherein the variant has a nucleotide sequence identity to SEQ ID NO: 2 of about 65%, about 75%, about 85%, about 90%, about 91%, about 92%, about 93%, about 94%, about 95%, about 96%, about 97%, about 98%, or about 99% with SEQ ID NO: 2. In one embodiment, a Jak3 molecule encompasses any portion of about 8 consecutive nucleotides of SEQ ID NO: 2. In one embodiment, the fragment can comprise about 15 nucleotides, about 20 nucleotides, or about 30 nucleotides of SEQ ID NO: 2. Fragments include all possible nucleotide lengths between about 8 and 100 nucleotides, for example, lengths between about 15 and 100, or between about 20 and 100.

The application further discloses nucleic acids that are complementary to a nucleic acid encoding a Jak3 protein. Such complementary nucleic acids can comprise nucleic acid sequences, which hybridize to a nucleic acid sequence encoding a Jak3 protein under stringent hybridization conditions. Non-limiting examples of stringent hybridization conditions include temperatures above 30°C, above 35°C, in excess of 42°C, and/or salinity of less than about 500 mM, or less than 200 mM. Hybridization conditions can be adjusted by the skilled artisan via modifying the temperature, salinity and/or the concentration of other reagents such as SDS or SSC.

|A Jak3 molecule may comprise a protein or polypeptide encoded by a Jak3 nucleic acid sequence, such as the sequence shown in SEQ ID NO: 1. In another embodiment, the polypeptide can be modified, such as by glycosylations and/or acetylations and/or chemical reaction or coupling, and can contain one or several non-natural or synthetic amino acids. An example of a Jak3 molecule is the polypeptide having the amino acid sequence shown in SEQ ID NO: 1. In another embodiment, a Jak3 molecule can be a fragment of a Jak3 protein. For example, the Jak3 molecule can encompass any portion of about 8 consecutive amino acids of SEQ ID NO: 1. The fragment can comprise about 10 amino acids, a least about 20 amino acids, about 30 amino acids, about 40 amino acids, a least about 50 amino acids, about 60 amino acids, or about 75 amino acids of SEQ ID NO: 1. Fragments include all possible amino acid lengths between about 8 and 100 about amino acids, for example, lengths between about 10 and 100 amino acids, between about 15 and 100 amino acids, between about 20 and 100 amino acids, between about 35 and 100 amino acids, between about 40 and 100 amino acids, between about 50 and 100 amino acids, between about 70 and 100 amino acids, between about 75 and 100 amino acids, or between about 80 and 100 amino acids.

The Jak3 molecule may include variants of the human Jak3 protein (comprising the amino acid sequence shown in SEQ ID NO: 1). Such variants can include those having at least from about 46% to about 50% identity to SEQ ID NO: 1, or having at least from about 50.1% to about 55% identity to SEQ ID NO: 1, or having at least from about 55.1% to about 60% identity to SEQ ID NO: 1, or having from about 60.1% to about 65% identity to SEQ ID NO: 1, or having from about 65.1% to about 70% identity to SEQ ID NO: 1, or having at least from about 70.1% to about 75% identity to SEQ ID NO: 1, or having at least from about 75.1% to about 80% identity to SEQ ID NO: 1, or having at least from about 80.1% to about 85% identity to SEQ ID NO: 1, or having at least from about 85.1% to about 90% identity to SEQ ID NO: 1, or having at least from about 90.1% to about 95% identity to SEQ ID NO: 1, or having at least from about 95.1% to about 97% identity to SEQ ID NO: 1, or having at least from about 97.1% to about 99% identity to SEQ ID NO: 1.

Techniques for making substitution mutations at predetermined sites in DNA having a known sequence are well known, for example M13 primer mutagenesis and PCR mutagenesis. Amino acid substitutions can be single residues, but can occur at a number of different locations at once. In one non-limiting embodiment, insertions can be on the order of about from 1 to about 10 amino acid residues, while deletions can range from about 1 to about 30 residues. Deletions or insertions can be made in adjacent pairs (for example, a deletion of about 2 residues or insertion of about 2 residues). Substitutions, deletions, insertions, or any combination thereof can be combined to arrive at a final construct. The mutations cannot place the sequence out of reading frame and should not create complementary regions that can produce secondary mRNA structure. Substitutional variants are those in which at least one residue has been removed and a different residue inserted in its place.

Substantial changes in function or immunological identity are made by selecting residues that differ more significantly in their effect on maintaining (a) the structure of the polypeptide backbone in the area of the substitution, for example as a sheet or helical conformation, (b) the charge or hydrophobicity of the molecule at the target site or (c) the bulk of the side chain. The substitutions that can produce the greatest changes in the protein properties will be those in which (a) a hydrophilic residue, e.g. seryl or threonyl, is substituted for (or by) a hydrophobic residue, e.g. leucyl, isoleucyl, phenylalanyl, valyl or alanyl; (b) a cysteine or proline is substituted for (or by) any other residue; (c) a residue having an electropositive side chain, e.g., lysyl, arginyl, or histidyl, is substituted for (or by) an electronegative residue, e.g., glutamyl or aspartyl; or (d) a residue having a bulky side chain, e.g., phenylalanine, is substituted for (or by) one not having a side chain, e.g., glycine, in this case, (e) by increasing the number of sites for sulfation and/or glycosylation.

There can be minor variations in the amino acid sequences of SEQ ID NO:1. The variations in the amino acid sequence can be when the sequence maintains about 30%, about 40%, about 50%, about 60%, about 70%, about 75%, about 80%, about 90%, about 95%, or about 99% identity to SEQ ID NO: 1. For example, conservative amino acid replacements can be utilized. Conservative replacements are those that take place within a family of amino acids that are related in their side chains, wherein the interchangeability of residues have similar side chains.

Genetically encoded amino acids are generally divided into families: (1) acidic amino acids are aspartate, glutamate; (2) basic amino acids are lysine, arginine, histidine; (3) non-polar amino acids are alanine, valine, leucine, isoleucine, proline, phenylalanine, methionine, tryptophan, and (4) uncharged polar amino acids are glycine, asparagine, glutamine, cysteine, serine, threonine, tyrosine. The hydrophilic amino acids include arginine, asparagine, aspartate, glutamine, glutamate, histidine, lysine, serine, and threonine. The hydrophobic amino acids include alanine, cysteine, isoleucine, leucine, methionine, phenylalanine, proline, tryptophan, tyrosine and valine. Other families of amino acids include (i) a group of amino acids having aliphatic-hydroxyl side chains, such as serine and threonine; (ii) a group of amino acids having amide-containing side chains, such as asparagine and glutamine; (iii) a group of amino acids having aliphatic side chains such as glycine, alanine, valine, leucine, and isoleucine; (iv) a group of amino acids having aromatic side chains, such as phenylalanine, tyrosine, and tryptophan; and (v) a group of amino acids having sulfur-containing side chains, such as cysteine and methionine. Useful conservative amino acids substitution groups are: valine-leucine-isoleucine, phenylalanine-tyrosine, lysine-arginine, alanine valine, glutamic-aspartic, and asparagine-glutamine.

For example, the replacement of one amino acid residue with another that is biologically and/or chemically similar is known to those skilled in the art as a conservative substitution. For example, a conservative substitution would be replacing one hydrophobic residue for another, or one polar residue for another. The substitutions include combinations such as, for example, Gly, Ala; Val, Ile, Leu; Asp, Glu; Asn, Gln; Ser, Thr; Lys, Arg; and Phe, Tyr. Substitutional or deletional mutagenesis can be employed to insert sites for N-glycosylation (Asn-X-Thr/Ser) or O-glycosylation (Ser or Thr). Deletions of cysteine or other labile residues also can be desirable. Deletions or substitutions of potential proteolysis sites, e.g. Arg, is accomplished for example by deleting one of the basic residues or substituting one by glutaminyl or histidyl residues.

The Jak3 molecule may encompass a peptidomimetic which exhibits Jak3 activity. A peptidomimetic is a small protein-like chain designed to mimic a peptide that can arise from modification of an existing peptide in order to protect that molecule from enzyme degradation and increase its stability, and/or alter the molecule's properties (e.g., modifications that change the molecule's stability or biological activity). These modifications involve changes to the peptide that cannot occur naturally (such as altered backbones and the incorporation of non-natural amino acids). Drug-like compounds can be developed from existing peptides. A peptidomimetic can be a peptide, partial peptide, or non-peptide molecule that mimics the tertiary binding structure or activity of a selected native peptide or protein functional domain (e.g., binding motif or active site). These peptide mimetics include recombinantly or chemically modified peptides.

A Jak3 molecule comprising SEQ ID NO: 1, variants of such, or fragments thereof, can be modified to produce peptide mimetics by replacement of one or more naturally occurring side chains of the 20 genetically encoded amino acids (or D amino acids) with other side chains. This can occur, for instance, with groups such as alkyl, lower alkyl, cyclic 4-, 5-, 6-, to 7-membered alkyl, amide, amide lower alkyl, amide di(lower alkyl), lower alkoxy, hydroxy, carboxy and the lower ester derivatives thereof, and with 4, 5-, 6-, to 7-membered heterocyclics. For example, proline analogs can be made in which the ring size of the proline residue is changed from 5 members to 4, 6, or 7 members. Cyclic groups can be saturated or unsaturated, and if unsaturated, can be aromatic or non-aromatic. Heterocyclic groups can contain one or more nitrogen, oxygen, and/or sulphur heteroatoms. Examples of such groups include the furazanyl, ifuryl, imidazolidinyl imidazolyl, imidazolinyl, isothiazolyl, isoxazolyl, morpholinyl (e.g. morpholino), oxazolyl, piperazinyl (e.g. 1-piperazinyl), piperidyl (e.g. 1-piperidyl, piperidino), pyranyl, pyrazinyl, pyrazolidinyl, pyrazolinyl, pyrazolyl, pyridazinyl, pyridyl, pyrimidinyl, pyrrolidinyl (e.g. 1-pyrrolidinyl), pyrrolinyl, pyrrolyl, thiadiazolyl, thiazolyl, thienyl, thiomorpholinyl (e.g. thiomorpholino), and triazolyl. These heterocyclic groups can be substituted or unsubstituted. Where a group is substituted, the substituent can be alkyl, alkoxy, halogen, oxygen, or substituted or unsubstituted phenyl. Peptidomimetics can also have amino acid residues that have been chemically modified by phosphorylation, sulfonation, biotinylation, or the addition or removal of other moieties. For example, peptidomimetics can be designed and directed to amino acid sequences encoded by a Jak3 molecule comprising SEQ ID NO: 1.

A variety of techniques are available for constructing peptide mimetics with the same or similar desired biological activity as the corresponding native but with more favorable activity than the peptide with respect to solubility, stability, and/or susceptibility to hydrolysis or proteolysis (*see,* e.g., Morgan & Gainor, Ann. Rep. Med. Chem. 24,243-252, 1989). Certain peptidomimetic compounds are based upon the amino acid sequence of the peptides of the invention. Peptidomimetic compounds can be synthetic compounds having a three-dimensional structure (*i.e.* a peptide motif) based upon the three-dimensional structure of a selected peptide. The peptide motif provides the peptidomimetic compound with the desired biological activity, wherein the binding activity of the mimetic compound is not substantially reduced, and is often the same as or greater than the activity of the native peptide on which the mimetic is modeled. Peptidomimetic compounds can have additional characteristics that enhance their therapeutic application, such as increased cell permeability, greater affinity and/or avidity and prolonged biological half-life. Peptidomimetic design strategies are readily available in the art (*see,* e.g., Ripka & Rich (1998) Curr. Op. Chem. Biol. 2:441-452; Hruby et al. (1997) Curr. Op. Chem. Biol. 1:114-119; Hruby & Balse, (2000) Curr. Med. Chem. 9:945-970).

### Cell Transfection

A eukaryotic expression vector can be used to transfect cells in order to produce proteins encoded by nucleotide sequences of the vector. Mammalian cells (such as isolated cells from the hair bulb; for example dermal sheath cells and dermal papilla cells) can contain an expression vector (for example, one that contains a gene encoding a Jak3 protein or polypeptide) via introducing the expression vector into an appropriate host cell via methods known in the art.

A host cell strain can be chosen for its ability to modulate the expression of the inserted sequences or to process the expressed polypeptide encoded by a gene, such as a Jak3 gene, in the desired fashion. Such modifications of the polypeptide includeacetylation, carboxylation, glycosylation, phosphorylation, lipidation, and acylation. Post-translational processing which cleaves a "prepro" form of the polypeptide also can be used to facilitate correct insertion, folding and/or function. Different host cells which have specific cellular machinery and characteristic mechanisms for post-translational activities (e.g., CHO, HeLa, MDCK, HEK293, and WI38), are available from the American Type Culture Collection (ATCC; 10801 University Boulevard, Manassas, Va. 20110-2209) and can be chosen to ensure the correct modification and processing of the foreign protein.

An exogenous nucleic acid can be introduced into a cell via a variety of techniques known in the art, such as lipofection, microinjection, calcium phosphate or calcium chloride precipitation, DEAE-dextran-mediated transfection, or electroporation. Electroporation is carried out at approximate voltage and capacitance to result in entry of the DNA construct(s) into cells of interest (such as cells of the end bulb of a hair follicle, for example dermal papilla cells or dermal sheath cells). Other transfection methods also include modified calcium phosphate precipitation, polybrene precipitation, liposome fusion, and receptor-mediated gene delivery.

Cells that will be genetically engineered can be primary and secondary cells obtained from various tissues, and include cell types which can be maintained and propagated in culture. Non-limiting examples of primary and secondary cells include epithelial cells (for example, dermal papilla cells, hair follicle cells, inner root sheath cells, outer root sheath cells, sebaceous gland cells, epidermal matrix cells), neural cells, endothelial cells, glial cells, fibroblasts, muscle cells (such as myoblasts) keratinocytes, formed elements of the blood (e.g., lymphocytes, bone marrow cells), and precursors of these somatic cell types.

Vertebrate tissue can be obtained by methods known to one skilled in the art, such a punch biopsy or other surgical methods of obtaining a tissue source of the primary cell type of interest. A punch biopsy or removal can be used to obtain a source of keratinocytes, fibroblasts, endothelial cells, or mesenchymal cells (for example, hair follicle cells or dermal papilla cells). Removal of a hair follicle can be used to obtain a source of fibroblasts, keratinocytes, endothelial cells, or mesenchymal cells (for example, hair follicle cells or dermal papilla cells). A mixture of primary cells can be obtained from the tissue, using methods readily practiced in the art, such as explanting or enzymatic digestion (for examples using enzymes such as pronase, trypsin, collagenase, elastase dispase, and chymotrypsin). Biopsy methods have also been described in United States Patent No. 7,419,661 and PCT application publication WO/2001/032840Primary cells can be acquired from the individual to whom the genetically engineered primary or secondary cells are administered. However, primary cells can also be obtained from a donor, other than the recipient, of the same species. The cells can also be obtained from another species (for example, rabbit, cat, mouse, rat, sheep, goat, dog, horse, cow, bird, or pig). Primary cells can also include cells from an isolated vertebrate tissue source grown attached to a tissue culture substrate (for example, flask or dish) or grown in a suspension; cells present in an explant derived from tissue; both of the aforementioned cell types plated for the first time; and cell culture suspensions derived from these plated cells. Secondary cells can be plated primary cells that are removed from the culture substrate and replated, or passaged, in addition to cells from the subsequent passages. Secondary cells can be passaged one or more times. These primary or secondary cells can contain expression vectors having a gene that encodes a protein of interest (for example, a Jak3 protein or polypeptide).

### Cell Culturing

Various culturing parameters can be used with respect to the host cell being cultured. Appropriate culture conditions for mammalian cells are well known in the art (Cleveland WL, et al., J Immunol Methods, 1983, 56(2): 221-234) or can be determined by the skilled artisan (see, for example, Animal Cell Culture: A Practical Approach 2nd Ed., Rickwood, D. and Hames, B. D., eds. (Oxford University Press: New York, 1992)). Cell culturing conditions can vary according to the type of host cell selected. Commercially available medium can be utilized. Non-limiting examples of medium include, for example, Minimal Essential Medium (MEM, Sigma, St. Louis, Mo.); Dulbecco's Modified Eagles Medium (DMEM, Sigma); Ham's F10 Medium (Sigma); HyClone cell culture medium (HyClone, Logan, Utah); RPMI-1640 Medium (Sigma); and chemically-defined (CD) media, which are formulated for various cell types, e.g., CD-CHO Medium (Invitrogen, Carlsbad, Calif.).

The cell culture media can be supplemented as necessary with supplementary components or ingredients, including optional components, in appropriate concentrations or amounts, as necessary or desired. Cell culture medium solutions provide at least one component from one or more of the following categories: (1) an energy source, usually in the form of a carbohydrate such as glucose; (2) all essential amino acids, and usually the basic set of twenty amino acids plus cysteine; (3) vitamins and/or other organic compounds required at low concentrations; (4) free fatty acids or lipids, for example linoleic acid; and (5) trace elements, where trace elements are defined as inorganic compounds or naturally occurring elements that can be required at very low concentrations, usually in the micromolar range.

The medium also can be supplemented electively with one or more components from any of the following categories: (1) salts, for example, magnesium, calcium, and phosphate; (2) hormones and other growth factors such as, serum, insulin, transferrin, and epidermal growth factor; (3) protein and tissue hydrolysates, for example peptone or peptone mixtures which can be obtained from purified gelatin, plant material, or animal byproducts; (4) nucleosides and bases such as, adenosine, thymidine, and hypoxanthine; (5) buffers, such as HEPES; (6) antibiotics, such as gentamycin or ampicillin; (7) cell protective agents, for example pluronic polyol; and (8) galactose. In one embodiment, soluble factors can be added to the culturing medium.

The mammalian cell culture that can be used with the present invention is prepared in a medium suitable for the type of cell being cultured. In one embodiment, the cell culture medium can be any one of those previously discussed (for example, MEM) that is supplemented with serum from a mammalian source (for example, fetal bovine serum (FBS)). In another embodiment, the medium can be a conditioned medium to sustain the growth of epithelial cells or cells obtained from the hair bulb of a hair follicle (such as dermal papilla cells or dermal sheath cells). For example, epithelial cells can be cultured according to Barnes and Mather in Animal Cell Culture Methods (Academic Press, 1998). In a further embodiment, epithelial cells or hair follicle cells can be transfected with DNA vectors containing genes that encode a polypeptide or protein of interest (for example, a Jak3 protein or polypeptide). In other embodiments of the invention, cells are grown in a suspension culture (for example, a three-dimensional culture such as a hanging drop culture) in the presence of an effective amount of enzyme, wherein the enzyme substrate is an extracellular matrix molecule in the suspension culture. For example, the enzyme can be a hyaluronidase. Epithelial cells or hair follicle cells can be cultivated according to methods practiced in the art, for example, as those described in U.S. Patent No. 7,785,876, or as described by Harris in Handbook in Practical Animal Cell Biology: Epithelial Cell Culture (Cambridge Univ. Press, Great Britain; 1996; see Chapter 8).

A suspension culture is a type of culture wherein cells, or aggregates of cells (such as aggregates of DP cells), multiply while suspended in liquid medium. A suspension culture comprising mammalian cells can be used for the maintenance of cell types that do not adhere or to enable cells to manifest specific cellular characteristics that are not seen in the adherent form. Some types of suspension cultures can include three-dimensional cultures or a hanging drop culture. A hanging-drop culture is a culture in which the material to be cultivated is inoculated into a drop of fluid attached to a flat surface (such as a coverglass, glass slide, Petri dish, flask, and the like), and can be inverted over a hollow surface. Cells in a hanging drop can aggregate toward the hanging center of a drop as a result of gravity. However, according to the methods of the invention, cells cultured in the presence of a protein that degrades the extracellular matrix (such as collagenase, chondroitinase, hyaluronidase, and the like) will become more compact and aggregated within the hanging drop culture, for degradation of the ECM will allow cells to become closer in proximity to one another since less of the ECM will be present. *See also* U.S. Patent Publication No. US 2010-0303767 A1.

Cells obtained from the hair bulb of a hair follicle (such as dermal papilla cells or dermal sheath cells) can be cultured as a single, homogenous population (for example, comprising DP cells) in a hanging drop culture so as to generate an aggregate of DP cells. Cells can also be cultured as a heterogeneous population (for example, comprising DP and DS cells) in a hanging drop culture so as to generate a chimeric aggregate of DP and DS cells. Epithelial cells can be cultured as a monolayer to confluency as practiced in the art. Such culturing methods can be carried out essentially according to methods described in Chapter 8 of the Handbook in Practical Animal Cell Biology: Epithelial Cell Culture (Cambridge Univ. Press, Great Britain; 1996); Underhill CB, J Invest Dermatol, 1993, 101(6):820-6); in Armstrong and Armstrong, (1990) J Cell Biol 110:1439-55; or in Animal Cell Culture Methods (Academic Press, 1998.

Three-dimensional cultures can be formed from agar (such as Gey's Agar), hydrogels (such as matrigel, agarose, and the like; Lee et al., (2004) Biomaterials 25: 2461-2466) or polymers that are cross-linked. These polymers can comprise natural polymers and their derivatives, synthetic polymers and their derivatives, or a combination thereof. Natural polymers can be anionic polymers, cationic polymers, amphipathic polymers, or neutral polymers. Non-limiting examples of anionic polymers can include hyaluronic acid, alginic acid (alginate), carageenan, chondroitin sulfate, dextran sulfate, and pectin. Some examples of cationic polymers, include chitosan or polylysine. (Peppas et al., (2006) Adv Mater. 18: 1345-60; Hoffman, A. S., (2002) Adv DrugDeliv Rev. 43: 3-12; Hoffman, A. S., (2001) Ann NY Acad Sci 944: 62-73). Examples of amphipathic polymers can includecollagen, gelatin, fibrin, and carboxymethyl chitin. Non-limiting examples of neutral polymers can include dextran, agarose, or pullulan. (Peppas et al., (2006) Adv Mater. 18: 1345-60; Hoffman, A. S., (2002) Adv Drug Deliv Rev. 43: 3-12; Hoffman, A. S., (2001) Ann NY Acad Sci 944: 62-73).

Cells suitable for culturing according to methods of the invention can harbor introduced expression vectors, such as plasmids. The expression vector constructs can be introduced via transformation, microinjection, transfection, lipofection, electroporation, or infection. The expression vectors can contain coding sequences, or portions thereof, encoding the proteins for expression and production. Expression vectors containing sequences encoding the produced proteins and polypeptides, as well as the appropriate transcriptional and translational control elements, can be generated using methods well known to and practiced by those skilled in the art. These methods include synthetic techniques, *in vitro* recombinant DNA techniques, and *in vivo* genetic recombination which are described in J. Sambrook et al., 2001, Molecular Cloning, A Laboratory Manual, Cold Spring Harbor Press, Plainview, N.Y. and in F. M. Ausubel et al., 1989, Current Protocols in Molecular Biology, John Wiley & Sons, New York, N.Y.

### Jak3 Modulating Compounds

As used herein, a "Jak3 modulating compound" refers to a compound that interacts with a Jak3 gene or a Jak3 protein or polypeptide and modulates its activity and/or its expression. The compound can either increase the activity or expression of a protein encoded by a Jak3 gene. Conversely, the compound can decrease the activity or expression of a protein encoded by a Jak3 gene. The compound can be a Jak3 agonist or a Jak3 antagonist (e.g., a Jak3 inhibitor). Some non-limiting examples of Jak3 modulating compounds include peptides (such as peptide fragments comprising a polypeptide encoded by a Jak3 gene, or antibodies or fragments thereof), small molecules, and nucleic acids (such as siRNA or antisense RNA specific for a nucleic acid comprising a Jak3 gene). Agonists of a Jak3 protein can be molecules which, when bound to a Jak3 protein, increase or prolong the activity of the Jak3 protein. Jak3 agonists include,proteins, nucleic acids, small molecules, or any other molecule which activates a Jak3 protein. Antagonists of a Jak3 protein can be molecules which, when bound to a Jak3 protein decrease the amount or the duration of the activity of the Jak3 protein. Antagonists include proteins, nucleic acids, antibodies, small molecules, or any other molecule which decrease the activity of a Jak3 protein.

The term "modulate," as it appears herein, refers to a change in the activity or expression of a gene or protein of Jak3. For example, modulation can cause an increase or a decrease in protein activity, binding characteristics, or any other biological, functional, or immunological properties of a Jak3 protein.

A Jak3 modulating compound can be a peptide fragment of a Jak3 protein that binds to the protein. For example, the Jak3 polypeptide can encompass any portion of about 8 consecutive amino acids of SEQ ID NO: 1. The fragment can comprise about 10 consecutive amino acids, about 20 consecutive amino acids, about 30 consecutive amino acids, about 40 consecutive amino acids, about 50 consecutive amino acids, about 60 consecutive amino acids, or about 75 consecutive amino acids of SEQ ID NO: 1. Fragments include all possible amino acid lengths between and including about 8 and about 100 amino acids, for example, lengths between about 10 and about 100 amino acids, between about 15 and about 100 amino acids, between about 20 and about 100 amino acids, between about 35 and about 100 amino acids, between about 40 and about 100 amino acids, between about 50 and about 100 amino acids, between about 70 and about 100 amino acids, between about 75 and about 100 amino acids, or between about 80 and about 100 amino acids. These peptide fragments can be obtained commercially or synthesized via liquid phase or solid phase synthesis methods (Atherton et al., (1989) Solid Phase Peptide Synthesis: a Practical Approach. IRL Press, Oxford, England). The Jak3 peptide fragments can be isolated from a natural source, genetically engineered, or chemically prepared. These methods are well known in the art.

A Jak3 modulating compound can be a protein, such as an antibody (monoclonal, polyclonal, humanized, chimeric, or fully human), or a binding fragment thereof, directed against a polypeptide encoded by a Jak3 gene. An antibody fragment can be a form of an antibody other than the full-length form and includes portions or components that exist within full-length antibodies, in addition to antibody fragments that have been engineered. Antibody fragments can includesingle chain Fv (scFv), diabodies, Fv, and (Fab')₂, triabodies, Fc, Fab, CDR1, CDR2, CDR3, combinations of CDR's, variable regions, tetrabodies, bifunctional hybrid antibodies, framework regions, constant regions, and the like (*see,* Maynard et al., (2000) Ann. Rev. Biomed. Eng. 2:339-76; Hudson (1998) Curr. Opin. Biotechnol. 9:395-402). Antibodies can be obtained commercially, custom generated, or synthesized against an antigen of interest according to methods established in the art (*see* Roland E. Kontermann and Stefan Dübel (editors), Antibody Engineering. Vol. I & II, (2010) 2nd ed., Springer; Antony S. Dimitrov (editor), Therapeutic Antibodies: Methods and Protocols (Methods in Molecular Biology), (2009), Humana Press; Benny Lo (editor) Antibody Engineering: Methods and Protocols (Methods in Molecular Biology), (2004) Humana Press. For example, antibodies directed to Jak3 can be obtained commercially from Abcam, Santa Cruz Biotechnology, Abgent, R&D Systems, Novus Biologicals, etc. Human antibodies directed to either Jak3 (such as monoclonal, humanized, or chimeric antibodies) can be useful antibody therapeutics for use in humans. In one embodiment, an antibody or binding fragment thereof is directed against SEQ ID NO: 1.

Inhibition of RNA encoding a polypeptide encoded by a Jak3 gene can effectively modulate the expression of a Jak3 gene from which the RNA is transcribed. Inhibitors are selected from the group comprising: siRNA; interfering RNA or RNAi; dsRNA; RNA Polymerase III transcribed DNAs; ribozymes; and antisense nucleic acids, which can be RNA, DNA, or an artificial nucleic acid.

Antisense oligonucleotides, including antisense DNA, RNA, and DNA/RNA molecules, act to directly block the translation of mRNA by binding to targeted mRNA and preventing protein translation. For example, antisense oligonucleotides of about 15 bases and complementary to unique regions of the DNA sequence encoding a polypeptide encoded by a Jak3 gene can be synthesized, e.g., by conventional phosphodiester techniques (Dallas et al., (2006) Med. Sci. Monit. 12(4):RA67-74; Kalota et al., (2006) Handb. Exp. Pharmacol. 173:173-96; Lutzelburger et al., (2006) Handb. Exp. Pharmacol. 173:243-59). Antisense nucleotide sequences include: morpholinos, 2'-O-methyl polynucleotides, DNA, RNA and the like. In one embodiment, the antisense oligonucleotide for Jak3 comprises TGCCATGAGTGCAACTTGCCTAGC (SEQ ID NO: 3).

siRNA comprises a double stranded structure containing from about 15 to about 50 base pairs, for example from about 21 to about 25 base pairs, and having a nucleotide sequence identical or nearly identical to an expressed target gene or RNA within the cell. The siRNA comprise a sense RNA strand and a complementary antisense RNA strand annealed together by standard Watson-Crick base-pairing interactions. The sense strand comprises a nucleic acid sequence which is substantially identical to a nucleic acid sequence contained within the target miRNA molecule. "Substantially identical" to a target sequence contained within the target mRNA refers to a nucleic acid sequence that differs from the target sequence by about 3% or less. The sense and antisense strands of the siRNA can comprise two complementary, single-stranded RNA molecules, or can comprise a single molecule in which two complementary portions are base-paired and are covalently linked by a single-stranded "hairpin" area. See also, McMnaus and Sharp (2002) Nat Rev Genetics, 3:737-47, and Sen and Blau (2006) FASEB J., 20:1293-99.

The siRNA can be altered RNA that differs from naturally-occurring RNA by the addition, deletion, substitution and/or alteration of one or more nucleotides. Such alterations can include addition of non-nucleotide material, such as to the end(s) of the siRNA or to one or more internal nucleotides of the siRNA, or modifications that make the siRNA resistant to nuclease digestion, or the substitution of one or more nucleotides in the siRNA with deoxyribo-nucleotides. One or both strands of the siRNA can also comprise a 3' overhang. As used herein, a 3' overhang refers to at least one unpaired nucleotide extending from the 3'-end of a duplexed RNA strand. For example, the siRNA can comprise at least one 3' overhang of from 1 to about 6 nucleotides (which includes ribonucleotides or deoxyribonucleotides) in length, or from 1 to about 5 nucleotides in length, or from 1 to about 4 nucleotides in length, or from about 2 to about 4 nucleotides in length. For example, each strand of the siRNA can comprise 3' overhangs of dithymidylic acid ("TT") or diuridylic acid ("uu").

siRNA can be produced chemically or biologically, or can be expressed from a recombinant plasmid or viral vector (for example, see U.S. Patent No. 7,294,504 and U.S. Patent No. 7,422,896. Exemplary methods for producing and testing dsRNA or siRNA molecules are described in U.S. Patent Application Publication No. 2002/0173478 to Gewirtz, U.S. Patent No. 8,071,559 to Hannon et al., U.S. Patent No. 7,674,895 to Reich et al., and in U.S. Patent No. 7,148,342 to Tolentino et al.

In one embodiment, an siRNA directed to a human nucleic acid sequence comprising a Jak3 gene can be generated against any one of SEQ ID NOS: 2. In another embodiment, an siRNA directed to a human nucleic acid sequence comprising a Jak3 gene can comprise any one of the sequences listed in Table 1.

In another embodiment, the siRNA directed to Jak3 is listed in Table 1.

**Table 1. siRNA SEQUENCES for Jak3**

| SEQ ID NO: | |
|---|---|
| 4 | TCAACTATCTGGAGGACAA |
| 5 | AGACAGAGGTGCTGCTGAA |
| 6 | GGTCCTTCACCAAGATTTA |
| 7 | CCTGGATCCTGCTAAGAAA |
| 8 | CGATCTTCGAGGAGAGACA |
| 9 | GGACAGACAACCAGATTTT |
| 10 | GGAAGCTGCAGGTGGTCAA |
| 11 | CCCAATACCAGCTGAGTCA |

RNA polymerase III transcribed DNAs contain promoters, such as the U6 promoter. These DNAs can be transcribed to produce small hairpin RNAs in the cell that can function as siRNA or linear RNAs that can function as antisense RNA. The Jak3 modulating compound can contain ribonucleotides, deoxyribonucleotides, synthetic nucleotides, or any suitable combination such that the target RNA and/or gene is inhibited. In addition, these forms of nucleic acid can be single, double, triple, or quadruple stranded. (see for example Bass (2001) Nature, 411:428-429; Elbashir et al., (2001) Nature, 411:494 498; U.S. Patent No. 6,509,154; and PCT Publication Nos. WO 00/044895, WO 01/036646, WO 99/032619, WO 00/01846, WO 01/029058, WO 00/44914).

A Jak3 modulating compound can be a small molecule that binds to a Jak3 protein and disrupts its function, or conversely, enhances its function. Small molecules are a diverse group of synthetic and natural substances generally having low molecular weights. They can be isolated from natural sources (for example, plants, fungi, microbes and the like), are obtained commercially and/or available as libraries or collections, or synthesized. Candidate small molecules that modulate a Jak3 protein can be identified via *in silico* screening or high-through-put (HTP) screening of combinatorial libraries according to methods established in the art (e.g., see Potyrailo et al., (2011) ACS Comb Sci. 13(6):579-633; Mensch et al., (2009) J Pharm Sci. 98(12):4429-68; Schnur (2008) Curr Opin Drug Discov Devel. 11(3):375-80; and Jhoti (2007) Ernst Schering Found Symp Proc. (3): 169-85. Most conventional pharmaceuticals, such as aspirin, penicillin, and many chemotherapeutics, are small molecules, can be obtained commercially, can be chemically synthesized, or can be obtained from random or combinatorial libraries as described below (*see,* e.g., Werner et al., (2006) Brief Funct. Genomic Proteomic 5(1):32-6).

JAK3 inhibitors are currently in clinical trials in humans for the treatment of acute kidney transplant rejection and rheumatoid arthritis. Non-limiting examples of Jak3 inhibitors include: **Janex 1 (WHI-P131)** (Changelian et al., (2008) Blood, 111(4):2155-7); Uckun ey al., (1999) Clin Cancer Res. 5(10):2954-62; Uckun et al., (2010) Arzneimittelforschung. 60(4):218-25), **PF-956980** (Sigma Product # PZ0151 (St. Louis, MO, http://www.sigmaaldrich.com/catalog/product/sigma/pz0151 ?lang=en*&*region=US)); Changelian et al., (2008) Blood, 111(4):2155-7), **WHI-P154** (Calbiochem Product # 420104-5MG (San Diego, CA, http://www.emdbiosciences.com)); Changelian et al., (2008) Blood, 111(4):2155-7), **VX-509** (oral from Vertex Pharmaceuticals, Cambridge MA; Fleischmann et al. (2011) Arthritis Rheum, 63:LB3; Fleischmann et al., (2012) Curr Opin Rheumatol. Feb 18, PMID: 22357358), **JAK3 Inhibitor IV (ZM-39923)** Calbiochem Product # 420121-10MG (San Diego, CA, http://www.emdbiosciences.com, WO1998022103), NSC114792 (Kim et al., (2010) Mol Cancer. 2010, 9:36), **tofacitinib (CP690550)** (Changelian et al., (2008) Blood, 111(4):2155-7; Vijayakrishnan et al. (2011) Trends Pharmacol Sci. 32(1):25-34; Fleischmann et al., (2012) Curr Opin Rheumatol. Feb 18, PMID: 22357358), and **R348** (topical and oral from Rigel Pharmaceuticals, San Francisco CA; Deuse et al., (2008) Transplantation. 85(6):885-92; Vijayakrishnan et al. (2011) Trends Pharmacol Sci. 32(1):25-34).

Structures of JAK3 inhibitors useful for the invention include a) Janex 1, oral and topical; b) PF-956980, i.v. infusion; c) WHI-P154; d) ZM-39923; e) NSC114792; f) tofacitinib (CP690550), oral.
a) 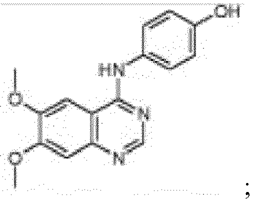
b) 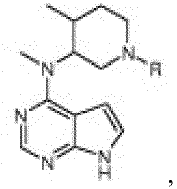 for example, 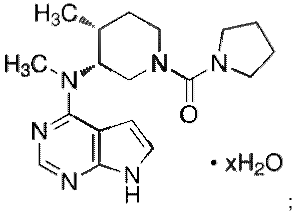
c) 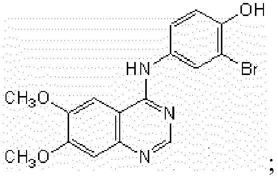
d) 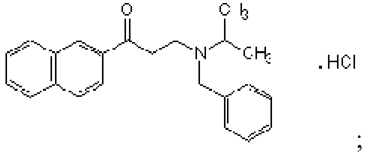
e) 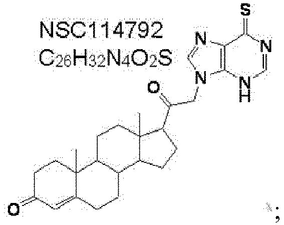
f)

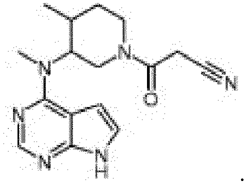

Non-limiting examples of JAK inhibtors (for example, Type I and Type II Jak Inhibitors) are discussed in O'Shea and Plenge (Immunity, 2012 Apr 20;36(4):542-50), LaFave and Levine (Trends Pharmacol Sci. 2012 Nov;33(11):574-82), Kontzias et al, (Curr Opin Pharmacol. 2012 Aug;12(4):464-70), Norman (Expert Opin Ther Pat. 2012 Oct;22(10):1233-49), and Wilson (Expert Opin Ther Pat. 2010 May;20(5):609-23).

Knowledge of the primary sequence of a molecule of interest, such as a polypeptide encoded by a Jak3 gene, and the similarity of that sequence with proteins of known function, can provide information as to the inhibitors or antagonists of the protein of interest in addition to agonists. Identification and screening of agonists and antagonists is further facilitated by determining structural features of the protein, e.g., using X-ray crystallography, neutron diffraction, nuclear magnetic resonance spectrometry, and other techniques for structure determination. These techniques provide for the rational design or identification of agonists and antagonists.

Test compounds, such as Jak3 modulating compounds, can be screened from large libraries of synthetic or natural compounds (*see* Alicea-Velázquez and Boggon (2011) Curr Drug Targets, 12(4):546-55; Wang et al., (2007) Curr Med Chem, 14(2):133-55; Mannhold (2006) Curr Top Med Chem, 6 (10):1031-47; and Hensen (2006) Curr Med Chem 13(4):361-76). Numerous means are currently used for random and directed synthesis of saccharide, peptide, and nucleic acid based compounds. Synthetic compound libraries are commercially available from Maybridge Chemical Co. (Trevillet, Cornwall, UK), AMRI (Albany, NY), ChemBridge (San Diego, CA), and MicroSource (Gaylordsville, CT). A rare chemical library is available from Aldrich (Milwaukee, Wis.). Alternatively, libraries of natural compounds in the form of bacterial, fungal, plant and animal extracts are available from e.g. Pan Laboratories (Bothell, Wash.) or MycoSearch (N.C.), or are readily producible. Additionally, natural and synthetically produced libraries and compounds are readily modified through conventional chemical, physical, and biochemical means (Blondelle et al., (1996) Tib Tech 14:60).

Methods for preparing libraries of molecules are well known in the art and many libraries are commercially available. Libraries of interest in the invention include peptide libraries, randomized oligonucleotide libraries, synthetic organic combinatorial libraries, and the like. Degenerate peptide libraries can be readily prepared in solution, in immobilized form as bacterial flagella peptide display libraries or as phage display libraries. Peptide ligands can be selected from combinatorial libraries of peptides containing at least one amino acid. Libraries can be synthesized of peptoids and non-peptide synthetic moieties. Such libraries can further be synthesized which contain non-peptide synthetic moieties, which are less subject to enzymatic degradation compared to their naturally-occurring counterparts. For example, libraries can also includepeptide-on-plasmid libraries, synthetic small molecule libraries, aptamer libraries, in vitro translation-based libraries, polysome libraries, synthetic peptide libraries, neurotransmitter libraries, and chemical libraries.

Examples of chemically synthesized libraries are described in Fodor et al., (1991) Science 251:767-773; Houghten et al., (1991) Nature 354:84-86; Lam et al., (1991) Nature 354:82-84; Medynski, (1994) BioTechnology 12:709-710; Gallop et al., (1994) J. Medicinal Chemistry 37(9):1233-1251; Ohlmeyer et al., (1993) Proc. Natl. Acad. Sci. USA 90:10922-10926; Erb et al., (1994) Proc. Natl. Acad. Sci. USA 91:11422-11426; Houghten et al., (1992) Biotechniques 13:412; Jayawickreme et al., (1994) Proc. Natl. Acad. Sci. USA 91:1614-1618; Salmon et al., (1993) Proc. Natl. Acad. Sci. USA 90:11708-11712; PCT Publication No. WO 93/020242, dated Oct. 14, 1993; and Brenner et al., (1992) Proc. Natl. Acad. Sci. USA 89:5381-5383.

Examples of phage display libraries are described in Scott et al., (1990) Science 249:386-390; Devlin et al., (1990) Science, 249:404-406; Christian, et al., (1992) J. Mol. Biol. 227:711-718; Lenstra, (1992) J. Immunol. Meth. 152:149-157; Kay et al., (1993) Gene 128:59-65; and PCT Publication No. WO 94/018318.

*In vitro* translation-based libraries include those described in PCT Publication No. WO 91/005058; and Mattheakis et al., (1994) Proc. Natl. Acad. Sci. USA 91:9022-9026.

As used herein, the term "ligand source" can be any compound library described herein, or tissue extract prepared from various organs in an organism's system, that can be used to screen for compounds that would act as an agonist or antagonist of a Jak3 protein. Screening compound libraries listed herein (also *see* U.S. Patent No. 7,884,189), in combination with *in vivo* animal studies, functional and signaling assays described below can be used to identify Jak3 modulating compounds that regulate hair growth or treat hair loss disorders.

Screening the libraries can be accomplished by any variety of commonly known methods. See, for example, the following references, which disclose screening of peptide libraries: Parmley and Smith, (1989) Adv. Exp. Med. Biol. 251:215-218; Scott and Smith, (1990) Science 249:386-390; Fowlkes et al., (1992) BioTechniques 13:422-427; Oldenburg et al., (1992) Proc. Natl. Acad. Sci. USA 89:5393-5397; Yu et al., (1994) Cell 76:933-945; Staudt et al., (1988) Science 241:577-580; Bock et al., (1992) Nature 355:564-566; Tuerk et al., (1992) Proc. Natl. Acad. Sci. USA 89:6988-6992; Ellington et al., (1992) Nature 355:850-852; U.S. Patent Nos. 5,096,815, 5,223,409, and 5,198,346, all to Ladner et al.; Rebar et al., (1993) Science 263:671-673; and PCT Publication No. WO 94/018318.

Small molecule combinatorial libraries can also be generated and screened. A combinatorial library of small organic compounds is a collection of closely related analogs that differ from each other in one or more points of diversity and are synthesized by organic techniques using multi-step processes. Combinatorial libraries include a vast number of small organic compounds. One type of combinatorial library is prepared by means of parallel synthesis methods to produce a compound array. A compound array can be a collection of compounds identifiable by their spatial addresses in Cartesian coordinates and arranged such that each compound has a common molecular core and one or more variable structural diversity elements. The compounds in such a compound array are produced in parallel in separate reaction vessels, with each compound identified and tracked by its spatial address. Examples of parallel synthesis mixtures and parallel synthesis methods are provided in U.S. Ser. No. 08/177,497, filed Jan. 5, 1994 and its corresponding PCT Publication No. WO 95/018972, as well as U.S. Pat. No. 5,712,171 and its corresponding PCT Publication No. WO 96/022529.

In one non-limiting example, non-peptide libraries, such as a benzodiazepine library (see e.g., Bunin et al., (1994) Proc. Natl. Acad. Sci. USA 91:4708-4712), can be screened. Peptoid libraries, such as that described by Simon et al., (1992) Proc. Natl. Acad. Sci. USA 89:9367-9371, can also be used. Another example of a library that can be used, in which the amide functionalities in peptides have been permethylated to generate a chemically transformed combinatorial library, is described by Ostresh et al. (1994), Proc. Natl. Acad. Sci. USA 91:11138-42.

Computer modeling and searching technologies permit the identification of compounds, or the improvement of already identified compounds, that can modulate the expression or activity of a Jak3 protein. Having identified such a compound or composition, the active sites or regions of a Jak3 protein can be subsequently identified via examining the sites to which the compounds bind. These sites can be ligand binding sites and can be identified using methods known in the art including, for example, from the amino acid sequences of peptides, from the nucleotide sequences of nucleic acids, or from study of complexes of the relevant compound or composition with its natural ligand. In the latter case, chemical or X-ray crystallographic methods can be used to find the active site by finding where on the factor the complexed ligand is found.

The three dimensional geometric structure of a site, for example that of a polypeptide encoded by a Jak3 gene, can be determined by known methods in the art, such as X-ray crystallography, which can determine a complete molecular structure. Solid or liquid phase NMR can be used to determine certain intramolecular distances. Any other experimental method of structure determination can be used to obtain partial or complete geometric structures. The geometric structures can be measured with a complexed ligand, natural or artificial, which can increase the accuracy of the active site structure determined.

Other methods for preparing or identifying peptides that bind to a target are known in the art. Molecular imprinting, for instance, can be used for the de novo construction of macromolecular structures such as peptides that bind to a molecule. See, for example, Kenneth J. Shea (1994) Molecular Imprinting of Synthetic Network Polymers: The De Novo synthesis of Macromolecular Binding and Catalytic Sites, TRIP 2(5); Mosbach, (1994) Trends in Biochem. Sci., 19(9); and Wulff, G., in Polymeric Reagents and Catalysts (Ford, W. T., Ed.) ACS Symposium Series No. 308, pp 186-230, American Chemical Society (1986). One method for preparing mimics of a Jak3 modulating compound involves the steps of: (i) polymerization of functional monomers around a known substrate (the template) that exhibits a desired activity; (ii) removal of the template molecule; and then (iii) polymerization of a second class of monomers in, the void left by the template, to provide a new molecule which exhibits one or more desired properties which are similar to that of the template. In addition to preparing peptides in this manner other binding molecules such as polysaccharides, nucleosides, drugs, nucleoproteins, lipoproteins, carbohydrates, glycoproteins, steroids, lipids, and other biologically active materials can also be prepared. This method is useful for designing a wide variety of biological mimics that are more stable than their natural counterparts, because they are prepared by the free radical polymerization of functional monomers, resulting in a compound with a non-biodegradable backbone. Other methods for designing such molecules include for example drug design based on structure activity relationships, which require the synthesis and evaluation of a number of compounds and molecular modeling.

### Screening Assays

A Jak3 modulating compound can be a compound that affects the activity and/or expression of a Jak3 protein *in vivo* and/or *in vitro.* Jak3 modulating compounds can be agonists and antagonists of a Jak3 protein, and can be compounds that exert their effect on the activity of a Jak3 protein via the expression, via post-translational modifications, or by other means.

Test compounds or agents which bind to a Jak3 protein, and/or have a stimulatory or inhibitory effect on the activity or the expression of a Jak3 protein, can be identified by two types of assays: (a) cell-based assays which utilize cells expressing a Jak3 protein or a variant thereof on the cell surface; or (b) cell-free assays, which can make use of isolated Jak3 proteins. These assays can employ a biologically active fragment of a Jak3 protein, full-length proteins, or a fusion protein which includes all or a portion of a polypeptide encoded by a Jak3 gene. A Jak3 protein can be obtained from any suitable mammalian species (e.g., human, rat, chick, xenopus, equine, bovine or murine). The assay can be a binding assay comprising direct or indirect measurement of the binding of a test compound. The assay can also be an activity assay comprising direct or indirect measurement of the activity of a Jak3 protein. The assay can also be an expression assay comprising direct or indirect measurement of the expression of Jak3 mRNA nucleic acid sequences or a protein encoded by a Jak3 gene. The various screening assays can be combined with an *in vivo* assay comprising measuring the effect of the test compound on the symptoms of a hair loss disorder or disease in a subject (for example, androgenetic alopecia, alopecia areata, alopecia totalis, or alopecia universalis), or even hypotrichosis.

An *in vivo* assay can also comprise assessing the effect of a test compound on regulating hair growth in known mammalian models that display defective or aberrant hair growth phenotypes or mammals that contain mutations in the open reading frame (ORF) of nucleic acid sequences comprising a Jak3 gene that affects hair growth regulation or hair density. Controlling hair growth can comprise an induction of hair growth or density in the subject. Here, the compound's effect in regulating hair growth can be observed either visually via examining the organism's physical hair growth or loss, or by assessing protein or mRNA expression using methods known in the art.

Assays for screening test compounds that bind to or modulate the activity of a Jak3 protein can also be carried out. The test compound can be obtained by any suitable means, such as from conventional compound libraries. Determining the ability of the test compound to bind to a membrane-bound form of the Jak3 protein can be accomplished via coupling the test compound with a radioisotope or enzymatic label such that binding of the test compound to the cell expressing a Jak3 protein can be measured by detecting the labeled compound in a complex. For example, the test compound can be labeled with ³H, ¹⁴C, ³⁵S, or ¹²⁵I, either directly or indirectly, and the radioisotope can be subsequently detected by direct counting of radioemmission or by scintillation counting. Alternatively, the test compound can be enzymatically labeled with, for example, horseradish peroxidase, alkaline phosphatase, or luciferase, and the enzymatic label detected by determination of conversion of an appropriate substrate to product.

Cell-based assays can comprise contacting a cell expressing Jak3 with a test agent and determining the ability of the test agent to modulate (such as increase or decrease) the activity or the expression of the membrane-bound molecule. Determining the ability of the test agent to modulate the activity of the membrane-bound Jak3 molecule can be accomplished by any method suitable for measuring the activity of such a molecule, such as monitoring downstream signaling events (e.g., Thoma et al. (2011) J Med Chem. 54(1):284-8; Flanagan (2010) J Med Chem. 53(24):8468-84; Lin et al., (2010) Arthritis Rheum. 62(8):2283-93; Kim et al., (2010) Mol Cancer. 9:36; Malabarba et al. (1995) J Biol Chem. 270(16):9630-7; JAK3 Kinase Enzyme Systems (Product Nos. V9441 and V3701, available from Promega (http://www.promega.com/products/cell-signaling/protein--kinases-and-kinase--assays/nonreceptor-tyrosine-kinase-enzyme-systems/jak3-kinase-enzyme-system/),.

A Jak3 protein or the target of a Jak3 protein can be immobilized to facilitate the separation of complexed from uncomplexed forms of one or both of the proteins. Binding of a test compound to a Jak3 protein or a variant thereof, or interaction of a Jak3 protein with a target molecule in the presence and absence of a test compound, can be accomplished in any vessel suitable for containing the reactants. Examples of such vessels include microtiter plates, test tubes, and micro-centrifuge tubes. In one embodiment, a fusion protein can be provided which adds a domain that allows one or both of the proteins to be bound to a matrix (for example, glutathione-S-transferase (GST) fusion proteins or glutathione-S-transferase fusion proteins can be adsorbed onto glutathione sepharose beads (Sigma Chemical; St. Louis, Mo.) or glutathione derivatized microtiter plates).

A Jak3 protein, or a variant thereof, can also be immobilized via being bound to a solid support. Non-limiting examples of suitable solid supports include glass or plastic slides, tissue culture plates, microtiter wells, tubes, silicon chips, or particles such as beads (includinglatex, polystyrene, or glass beads). Any method known in the art can be used to attach a polypeptide (or polynucleotide) corresponding to Jak3 or a variant thereof, or test compound to a solid support, including use of covalent and non-covalent linkages, or passive absorption.

The expression of a Jak3 protein can also be monitored. For example, regulators of the expression of a Jak3 protein can be identified via contacting a cell with a test compound and determining the expression of a protein encoded by a Jak3 gene or Jak3 mRNA nucleic acid sequences in the cell. The expression level of a protein encoded by a Jak3 gene or Jak3 mRNA nucleic acid sequences in the cell in the presence of the test compound is compared to the protein or mRNA expression level in the absence of the test compound. The test compound can then be identified as a regulator of the expression of a Jak3 protein based on this comparison. For example, when expression of a protein encoded by a Jak3 gene or Jak3 mRNA nucleic acid sequences in the cell is statistically or significantly greater in the presence of the test compound than in its absence, the test compound is identified as a stimulator/enhancer of expression of a protein encoded by a Jak3 gene or Jak3 mRNA nucleic acid sequences in the cell. The test compound can be said to be a Jak3 modulating compound (such as an agonist).

Alternatively, when expression of a protein encoded by a Jak3 gene or Jak3 mRNA nucleic acid sequences in the cell is statistically or significantly less in the presence of the test compound than in its absence, the compound is identified as an inhibitor of the expression of a protein encoded by a Jak3 gene or Jak3 mRNA nucleic acid sequences in the cell. The test compound can also be said to be a Jak3 modulating compound (such as an antagonist). The expression level of a protein encoded by a Jak3 gene or Jak3 mRNA nucleic acid sequences in the cell in cells can be determined by methods previously described.

For binding assays, the test compound can be a small molecule which binds to and occupies the binding site of a polypeptide encoded by a Jak3 gene, or a variant thereof. This can make the ligand binding site inaccessible to substrate such that normal biological activity is prevented. Examples of such small molecules include, , small peptides or peptide-like molecules. In binding assays, either the test compound or a polypeptide encoded by a Jak3 gene can comprise a detectable label, such as a fluorescent, radioisotopic, chemiluminescent, or enzymatic label (for example, alkaline phosphatase, horseradish peroxidase, or luciferase). Detection of a test compound which is bound to a polypeptide encoded by a Jak3 gene can then be determined via direct counting of radioemmission, by scintillation counting, or by determining conversion of an appropriate substrate to a detectable product.

Determining the ability of a test compound to bind to a Jak3 protein also can be accomplished using real-time Biamolecular Interaction Analysis (BIA) (McConnell et al., 1992, Science 257, 1906-1912; Sjolander, Urbaniczky, 1991, Anal. Chem. 63, 2338-2345). BIA is a technology for studying biospecific interactions in real time, without labeling any of the interactants (for example, BIA-core™). Changes in the optical phenomenon surface plasmon resonance (SPR) can be used as an indication of real-time reactions between biological molecules.

To identify other proteins which bind to or interact with a Jak3 protein and modulate its activity, a polypeptide encoded by a Jak3 gene can be used as a bait protein in a two-hybrid assay or three-hybrid assay (Szabo et al., 1995, Curr. Opin. Struct. Biol. 5, 699-705; U.S. Pat. No. 5,283,317), according to methods practiced in the art. The two-hybrid system is based on the modular nature of most transcription factors, which consist of separable DNA-binding and activation domains.

Functional Assays. Test compounds can be tested for the ability to increase or decrease the activity of a Jak3 protein, or a variant thereof. Activity can be measured after contacting a purified Jak3 protein, a cell membrane preparation, or an intact cell with a test compound. A test compound that decreases the activity of a Jak3 protein by about 10%, about 20%, about 30%, about 40%, about 50%, about 60%, about 70%, about 75%, about 80%, about 90%, about 95% or 100% is identified as a potential agent for decreasing the activity of a Jak3 protein, for example an antagonist. A test compound that increases the activity of a Jak3 protein by about 10%, about 20%, about 30%, about 40%, about 50%, about 60%, about 70%, about 75%, about 80%, about 90%, about 95% or 100% is identified as a potential agent for increasing the activity of a Jak3 protein, for example an agonist.

### Treatment and Prevention

The application also discloses a method for treating or preventing a hair-loss disorder in a subject. The method comprises detecting the presence of an alteration in a Jak3 gene in a sample from the subject, the presence of the alteration being indicative of a hair-loss disorder, or the predisposition to a hair-loss disorder, and, administering to the subject in need a therapeutic treatment against a hair-loss disorder. The therapeutic treatment can be a drug administration (for example, a pharmaceutical composition comprising a siRNA directed to a Jak3 nucleic acid). The therapeutic molecule to be administered may comprise a polypeptide encoded by a Jak3 gene, comprising about 75%, about 80%, about 85%, about 90%, about 93%, about 95%, about 97%, about 98%, about 99%, or 100% of the amino acid sequence of SEQ ID NO: 1, and exhibits the function of decreasing expression of a protein encoded by a Jak3 gene. This can restore the capacity to initiate hair growth in cells derived from hair follicles or skin. The therapeutic molecule intended to be administered may comprise a nucleic acid sequence comprising a Jak3 gene that encodes a polypeptide, comprising about 75%, about 80%, about 85%, about 90%, about 93%, about 95%, about 97%, about 98%, about 99%, or 100% of the nucleic acid sequence of SEQ ID NO: 2, and encodes a polypeptide with the function of decreasing expression of a protein encoded by a Jak3 gene, thus restoring the capacity to initiate hair growth in cells derived from hair follicle cells or skin.

The presence of an alteration in a gene encoding a Jak3 molecule in the sample can be detected through the genotyping of a sample, for example via gene sequencing, selective hybridization, amplification, gene expression analysis, or a combination thereof. The sample can comprise blood, plasma, serum, sputum, lacrimal secretions, semen, vaginal secretions, skin tissue, muscle tissue, amniotic fluid, or a combination thereof.

The alteration can be determined at the level of the DNA, RNA, or polypeptide. Optionally, detection can be determined by performing an oligonucleotide ligation assay, a confirmation based assay, a hybridization assay, a sequencing assay, an allele-specific amplification assay, a microsequencing assay, a melting curve analysis, a denaturing high performance liquid chromatography (DHPLC) assay (for example, *see* Jones et al, (2000) Hum Genet., 106(6):663-8), or a combination thereof. The detection may be performed by sequencing all or part of a Jak3 gene or by selective hybridization or amplification of all or part of a Jak3 gene. A Jak3 gene specific amplification can be carried out before the alteration identification step.

An alteration in a chromosome region occupied by a Jak3 gene can be any form of mutation(s), deletion(s), rearrangement(s) and/or insertions in the coding and/or non-coding region of the locus, alone or in various combination(s). Mutations can include point mutations. Insertions can encompass the addition of one or several residues in a coding or non-coding portion of the gene locus. Insertions can comprise an addition of between 1 and 50 base pairs in the gene locus. Deletions can encompass any region of one, two or more residues in a coding or non-coding portion of the gene locus, such as from two residues up to the entire gene or locus. Deletions can affect smaller regions, such as domains (introns) or repeated sequences or fragments of less than about 50 consecutive base pairs, although larger deletions can occur as well. Rearrangement includes inversion of sequences. The alteration in a chromosome region occupied by a Jak3 gene can result in amino acid substitutions, RNA splicing or processing, product instability, the creation of stop codons, frame-shift mutations, and/or truncated polypeptide production. The alteration can result in the production of a polypeptide encoded by a Jak3 gene with altered function, stability, targeting or structure. The alteration can also cause a reduction, or even an increase in protein expression. In one embodiment, the alteration in the chromosome region occupied by a Jak3 gene can comprise a point mutation, a deletion, or an insertion in a Jak3 gene or corresponding expression product. In another embodiment, the alteration can be a deletion or partial deletion of a Jak3 gene. The alteration can be determined at the level of the DNA, RNA, or polypeptide.

The method can comprise detecting the presence of altered RNA expression. Altered RNA expression includes the presence of an altered RNA sequence, the presence of an altered RNA splicing or processing, or the presence of an altered quantity of RNA. These can be detected by various techniques known in the art, including sequencing all or part of the RNA or by selective hybridization or selective amplification of all or part of the RNA. The method can comprise detecting the presence of altered expression of a polypeptide encoded by a Jak3 gene. Altered polypeptide expression includes the presence of an altered polypeptide sequence, the presence of an altered quantity of polypeptide, or the presence of an altered tissue distribution. These can be detected by various techniques known in the art, including by sequencing and/or binding to specific ligands (such as antibodies, e.g., directed to Jak3).

Various techniques known in the art can be used to detect or quantify altered gene or RNA expression or nucleic acid sequences, which includehybridization, sequencing, amplification, and/or binding to specific ligands (such as antibodies). Other suitable methods include allele-specific oligonucleotide (ASO), oligonucleotide ligation, allele-specific amplification, Southern blot (for DNAs), Northern blot (for RNAs), single-stranded conformation analysis (SSCA),), pulsed-field gel electrophoresis (PFGE), isoelectric focusing, fluorescent in situ hybridization (FISH), gel migration, clamped denaturing gel electrophoresis, denaturing HPLC, melting curve analysis, heteroduplex analysis, RNase protection, chemical or enzymatic mismatch cleavage, ELISA, radio-immunoassays (RIA) and immuno-enzymatic assays (IEMA). In one embodiment, the detecting comprises using a northern blot; real time PCR and primers directed to SEQ ID NO: 2; a ribonuclease protection assay; a hybridization, amplification, or sequencing technique to distinguish SEQ ID NO: 2; or a combination thereof.

Some of these approaches (such as SSCA and constant gradient gel electrophoresis (CGGE)) are based on a change in electrophoretic mobility of the nucleic acids, as a result of the presence of an altered sequence. According to these techniques, the altered sequence is visualized by a shift in mobility on gels. The fragments can then be sequenced to confirm the alteration. Some other approaches are based on specific hybridization between nucleic acids from the subject and a probe specific for wild type or altered gene or RNA. The probe can be in suspension or immobilized on a substrate. The probe can be labeled to facilitate detection of hybrids. Some of these approaches are suited for assessing a polypeptide sequence or expression level, such as Northern blot, ELISA, and RIA. These latter require the use of a ligand specific for the polypeptide, for example, the use of a specific antibody. the antibody may be directed to a Jak3 molecule comprising SEQ ID NO: 1.

Sequencing. Sequencing can be carried out using techniques well known in the art, using automatic sequencers. The sequencing can be performed on the complete Jak3 gene or on specific domains thereof, such as those known or suspected to carry deleterious mutations or other alterations.

Amplification. Amplification is based on the formation of specific hybrids between complementary nucleic acid sequences that serve to initiate nucleic acid reproduction. Amplification can be performed according to various techniques known in the art, such as by polymerase chain reaction (PCR), ligase chain reaction (LCR), strand displacement amplification (SDA) and nucleic acid sequence based amplification (NASBA). These techniques can be performed using commercially available reagents and protocols. Useful techniques in the art encompass real-time PCR, allele-specific PCR, or PCR based single-strand conformational polymorphism (SSCP). Amplification usually requires the use of specific nucleic acid primers, to initiate the reaction. Nucleic acid primers useful for amplifying sequences from a Jak3 gene or locus are able to specifically hybridize with a portion of a Jak3 gene locus that flank a target region of the locus, wherein the target region is altered in certain subjects having a hair-loss disorder. In one embodiment, amplification can comprise using forward and reverse PCR primers comprising nucleotide sequences of SEQ ID NO: 2. Non-limiting amplification methods include, e.g., polymerase chain reaction, PCR (PCR Protocols. A Guide To Methods And Applications, ed. Innis, Academic Press, N.Y., 1990 and PCR Strategies, 1995, ed. Innis, Academic Press, Inc., N.Y.); ligase chain reaction (LCR) (Wu (1989) Genomics 4:560; Landegren (1988) Science 241:1077; Barringer (1990) Gene 89:117); transcription amplification (Kwoh (1989) PNAS 86:1173); and, self-sustained sequence replication (Guatelli (1990) PNAS 87:1874); Q Beta replicase amplification (Smith (1997) J. Clin. Microbiol. 35:1477-1491), automated Q-beta replicase amplification assay (Burg (1996) Mol. Cell. Probes 10:257-271) and other RNA polymerase mediated techniques (e.g., NASBA, Cangene, Mississauga, Ontario; *see also* Berger (1987) Methods Enzymol. 152:307-316; U.S. Pat. Nos. 4,683,195 and 4,683,202; and Sooknanan (1995) Biotechnology 13:563-564). The application discloses a nucleic acid primer, wherein the primer can be complementary to and hybridize specifically to a portion of a Jak3 coding sequence (e.g., gene or RNA) altered in certain subjects having a hair-loss disorder. Primers can be specific for altered sequences in a Jak3 gene or RNA. By using such primers, the detection of an amplification product indicates the presence of an alteration in a Jak3 gene or the absence of such gene. Primers can also be used to identify single nucleotide polymorphisms (SNPs) located in or around a Jak3 gene locus; SNPs can comprise a single nucleotide change, or a cluster of SNPs in and around a Jak3 gene. Examples of primers of this application can be single-stranded nucleic acid molecules of about 5 to 60 nucleotides in length, or about 8 to about 25 nucleotides in length. The sequence can be derived directly from the sequence of a Jak3 gene. Perfect complementarity is useful to ensure high specificity; however, certain mismatch can be tolerated. For example, a nucleic acid primer or a pair of nucleic acid primers as described above can be used in a method for detecting the presence of or a predisposition to a hair-loss disorder in a subject.

Selective Hybridization. Hybridization detection methods are based on the formation of specific hybrids between complementary nucleic acid sequences that serve to detect nucleic acid sequence alteration(s). A detection technique involves the use of a nucleic acid probe specific for wild type or altered gene or RNA, followed by the detection of the presence of a hybrid. The probe can be in suspension or immobilized on a substrate or support (for example, as in nucleic acid array or chips technologies). The probe can be labeled to facilitate detection of hybrids. For example, a sample from the subject can be contacted with a nucleic acid probe specific for a wild type Jak3 gene or an altered Jak3 gene, and the formation of a hybrid can be subsequently assessed. In one embodiment, the method comprises contacting simultaneously the sample with a set of probes that are specific, respectively, for a wild type Jak3 gene and for various altered forms thereof. Thus, it is possible to detect directly the presence of various forms of alterations in a Jak3 gene in the sample. Also, various samples from various subjects can be treated in parallel.

According to the application, a probe can be a polynucleotide sequence which is complementary to and can specifically hybridize with a (target portion of a) Jak3 gene or RNA, and that is suitable for detecting polynucleotide polymorphisms associated with alleles of a Jak3 gene (or genes) which predispose to or are associated with a hair-loss disorder. Useful probes are those that are complementary to a Jak3 gene, RNA, or target portion thereof. Probes can comprise single-stranded nucleic acids of between 8 to 1000 nucleotides in length, for instance between 10 and 800, between 15 and 700, or between 20 and 500. Longer probes can be used as well. A useful probe of the application is a single stranded nucleic acid molecule of between 8 to 500 nucleotides in length, which can specifically hybridize to a region of a Jak3 gene or RNA that carries an alteration. For example, the probe can be directed to a chromosome region occupied by a Jak3 gene.

The sequence of the probes can be derived from the sequences of a Jak3 gene and RNA as provided herein. Nucleotide substitutions can be performed, as well as chemical modifications of the probe. Such chemical modifications can be accomplished to increase the stability of hybrids (e.g., intercalating groups) or to label the probe. Some examples of labels include, without limitation, radioactivity, fluorescence, luminescence, and enzymatic labeling.

A guide to the hybridization of nucleic acids is found in e.g., Sambrook, ed., Molecular Cloning: A Laboratory Manual (3rd Ed.), Vols. 1-3, Cold Spring Harbor Laboratory, 1989; Current Protocols In Molecular Biology, Ausubel, ed. John Wiley & Sons, Inc., New York, 2001; Laboratory Techniques In Biochemistry And Molecular Biology: Hybridization With Nucleic Acid Probes. Part I. Theory and Nucleic Acid Preparation, Tijssen, ed. Elsevier, N.Y., 1993.

### Specific Ligand Binding

As indicated herein, alteration in a chromosome region occupied by a Jak3 gene or alteration in expression of a Jak3 gene, can also be detected by screening for alteration(s) in a sequence or expression level of a polypeptide encoded by a Jak3 gene. Different types of ligands can be used, such as specific antibodies. In one embodiment, the sample is contacted with an antibody specific for a polypeptide encoded by a Jak3 gene and the formation of an immune complex is subsequently determined. Various methods for detecting an immune complex can be used, such as ELISA, radioimmunoassays (RIA) and immuno-enzymatic assays (IEMA). In one embodiment, levels are measured by ELISA using an antibody directed to SEQ ID NO: 1; western blot using an antibody directed to SEQ ID NO: 1; mass spectroscopy, isoelectric focusing, or electrophoresis-based techniques targeting epitopes of SEQ ID NO: 1; or a combination thereof.

For example, an antibody can be a polyclonal antibody, a monoclonal antibody, as well as fragments or derivatives thereof having substantially the same antigen specificity. Fragments include Fab, Fab'2, or CDR regions. Derivatives include single-chain antibodies, humanized antibodies, or poly-functional antibodies. An antibody specific for a polypeptide encoded by a Jak3 gene can be an antibody that selectively binds such a polypeptide, namely, an antibody raised against a polypeptide encoded by a Jak3 gene or an epitope-containing fragment thereof. Although non-specific binding towards other antigens can occur, binding to the target polypeptide occurs with a higher affinity and can be reliably discriminated from non-specific binding. The method can comprise contacting a sample from the subject with an antibody specific for a wild type or an altered form of a polypeptide encoded by a Jak3 gene, and determining the presence of an immune complex. Optionally, the sample can be contacted to a support coated with antibody specific for the wild type or altered form of a polypeptide encoded by a Jak3 gene. The sample can be contacted simultaneously, or in parallel, or sequentially, with various antibodies specific for different forms of a polypeptide encoded by a Jak3 gene, such as a wild type and various altered forms thereof.

### Gene Therapy and Protein Replacement Methods

Delivery of nucleic acids into viable cells can be effected ex vivo, in situ, or in vivo by use of vectors, such as viral vectors (e.g., lentivirus, adenovirus, adeno-associated virus, or a retrovirus), or ex vivo by use of physical DNA transfer methods (e.g., liposomes or chemical treatments). Non-limiting techniques suitable for the transfer of nucleic acid into mammalian cells *in vitro* include the use of liposomes, electroporation, microinjection, cell fusion, DEAE-dextran, and the calcium phosphate precipitation method (*See,* for example, Anderson, Nature, 392(6679): 25 (1998)). Introduction of a nucleic acid or a gene encoding a polypeptide of the invention can also be accomplished with extrachromosomal substrates (transient expression) or artificial chromosomes (stable expression). Cells can also be cultured ex vivo in the presence of therapeutic compositions of the present invention in order to proliferate or to produce a desired effect on or activity in such cells. Treated cells can then be introduced in vivo for therapeutic purposes.

Nucleic acids can be inserted into vectors and used as gene therapy vectors. A number of viruses have been used as gene transfer vectors, including papovaviruses, e.g., SV40 (Madzak et al., (1992) J Gen Virol. 73(Pt 6):1533-6), adenovirus (Berkner (1992) Curr Top Microbiol Immunol. 158:39-66; Berkner (1988) Biotechniques, 6(7):616-29; Gorziglia and Kapikian (1992) J Virol. 66(7):4407-12; Quantin et al., (1992) Proc Natl Acad Sci U S A. 89(7):2581-4; Rosenfeld et al., (1992) Cell. 68(1):143-55; Wilkinson et al., (1992) Nucleic Acids Res. 20(9):2233-9; Stratford-Perricaudet et al., (1990) Hum Gene Ther. 1(3):241-56), vaccinia virus (Moss (1992) Curr Opin Biotechnol. 3(5):518-22), adeno-associated virus (Muzyczka, (1992) Curr Top Microbiol Immunol. 158:97-129; Ohi et al., (1990) Gene. 89(2):279-82), herpesviruses including HSV and EBV (Margolskee (1992) Curr Top Microbiol Immunol. 158:67-95; Johnson et al., (1992) Brain Res Mol Brain Res. 12(1-3):95-102; Fink et al., (1992) Hum Gene Ther. 3(1):11-9; Breakefield and Geller (1987) Mol Neurobiol. 1(4):339-71; Freese et al., (1990) Biochem Pharmacol. 40(10):2189-99), and retroviruses of avian (Bandyopadhyay and Temin (1984) Mol Cell Biol. 4(4):749-54; Petropoulos et al., (1992) J Virol. 66(6):3391-7), murine (Miller et al. (1992) Mol Cell Biol. 12(7):3262-72; Miller et al., (1985) J Virol. 55(3):521-6; Sorge et al., (1984) Mol Cell Biol. 4(9):1730-7; Mann and Baltimore (1985) J Virol. 54(2):401-7; Miller et al., (1988) J Virol. 62(11):4337-45), and human origin (Shimada et al., (1991) J Clin Invest. 88(3):1043-7; Helseth et al., (1990) J Virol. 64(12):6314-8; Page et al., (1990) J Virol. 64(11):5270-6; Buchschacher and Panganiban (1992) J Virol. 66(5):2731-9).

Non-limiting examples of *in vivo* gene transfer techniques include transfection with viral (e.g., retroviral) vectors (*see* U.S. Pat. No. 5,252,479) and viral coat protein-liposome mediated transfection (Dzau et al., (1993) Trends in Biotechnology 11:205-210). For example, naked DNA vaccines are generally known in the art; *see* Brower, (1998) Nature Biotechnology, 16:1304-1305. Gene therapy vectors can be delivered to a subject by, for example, intravenous injection, local administration (*see,* e.g., U.S. Pat. No. 5,328,470) or by stereotactic injection (*see,* e.g., Chen, et al., (1994) Proc. Natl. Acad. Sci. USA 91:3054-3057). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, e.g., retroviral vectors, the pharmaceutical preparation can include one or more cells that produce the gene delivery system.

For reviews of gene therapy protocols and methods *see* Anderson et al. (1992) Science 256:808-813; U.S. Pat. Nos. 5,252,479, 5,747,469, 6,017,524, 6,143,290, 6,410,010 6,511,847 8,398,968; and 8,404,653; and U.S. Application Publication Nos. 2002/0077313 and 2002/00069. For an example of gene therapy treatment in humans see Porter et al., NEJM 2011 365:725-733 and Kalos et al. Sci. Transl. Med. 2011. 201 3(95):95. For additional reviews of gene therapy technology, see Friedmann (1989) Science, 244:1275-1281; Verma, Scientific American: 68-84 (1990); Miller (1992) Nature, 357: 455-460; Kikuchi et al. (2008) J Dermatol Sci. 50(2):87-98; Isaka et al. (2007) Expert Opin Drug Deliv. 4(5):561-71; Jager et al.(2007) Curr Gene Ther. 7(4):272-83; Waehler et al.(2007) Nat Rev Genet. 8(8):573-87; Jensen et al. (2007) Ann Med. 39(2):108-15; Herweijer et al. (2007) Gene Ther. 14(2):99-107; Eliyahu et al. (2005) Molecules 10(1):34-64; and Altaras et al. (2005) Adv Biochem Eng Biotechnol. 99:193-260.

### Protein Delivery Methods

Protein replacement therapy can increase the amount of protein by exogenously introducing wild-type or biologically functional protein by way of infusion. A replacement polypeptide can be synthesized according to known chemical techniques or can be produced and purified via known molecular biological techniques. Protein replacement therapy has been developed for various disorders. For example, a wild-type protein can be purified from a recombinant cellular expression system (e.g., mammalian cells or insect cells, *see* U.S. Pat. No. 5,580,757 to Desnick et al.; U.S. Pat. Nos. 6,395,884 and 6,458,574 to Selden et al.; U.S. Pat. No. 6,461,609 to Calhoun et al.; U.S. Pat. No. 6,210,666 to Miyamura et al.; U.S. Pat. No. 6,083,725 to Selden et al.; U.S. Pat. No. 6,451,600 to Rasmussen et al.; U.S. Pat. No. 5,236,838 to Rasmussen et al. and U.S. Pat. No. 5,879,680 to Ginns et al.), human placenta, or animal milk (*see* U.S. Pat. No. 6,188,045 to Reuser et al.), or other sources known in the art. After the infusion, the exogenous protein can be taken up by tissues through non-specific or receptor-mediated mechanism.

A polypeptide encoded by a Jak3 gene or a Jak3 modulating compound can also be delivered in a controlled release system. For example, the polypeptide and molecule can be administered using intravenous infusion, an implantable osmotic pump, a transdermal patch, liposomes, or other modes of administration. A pump can be used (*see* Sefton (1987) Biomed. Eng. 14:201; Buchwald et al. (1980) Surgery 88:507; Saudek et al. (1989) N. Engl. J. Med. 321:574). Polymeric materials can be used (*see* Medical Applications of Controlled Release, Langer and Wise (eds.), CRC Pres., Boca Raton, Fla. (1974); Controlled Drug Bioavailability. Drug Product Design and Performance, Smolen and Ball (eds.), Wiley, New York (1984); Ranger and Peppas, (1983) J. Macromol. Sci. Rev. Macromol. Chem. 23:61; *see also* Levy et al. (1985) Science 228:190; During et al. (1989) Ann. Neurol. 25:351; Howard et al. (1989) J. Neurosurg. 71:105). A controlled release system can be placed in proximity of the therapeutic target thus requiring only a fraction of the systemic dose (*see,* e.g., Goodson, in Medical Applications of Controlled Release, supra, vol. 2, pp. 115-138 (1984)). Other controlled release systems are discussed in the review by Langer (Science (1990) 249:1527-1533).

### Inhibitors

Cytokines are produced to activate neighboring cells to communicate danger signals to one another and spread and amplify the inflammatory response. Over the years, it was learned how to both neutralize these cytokines with blocking antibodies and inhibit their signaling in responding cells by small molecule protein tyrosine kinase inhibitors FDA approved drugs exist for both approaches. Whenever possible, topical small molecule formulations that should improve efficacy will be pursued while limiting systemic toxicity (improved therapeutic indexes) encouraging clinical evaluation in AA of other small molecule inhibitors in the biopharmaceutical pipeline.

To block signaling of cytokine receptors, topical and/or oral JAK3 inhibitors as described herein (e.g., tofacitinib (CP690550), R348 and VX-509), which have demonstrated preliminary safety and efficacy in patients with RA, can be used.

The inhibitors can comprise peptides (such as antibodies or fragments thereof), small molecules, nucleic acids (such as siRNA or antisense RNA), or other agents) that can bind to a polypeptide molecule encoded by a gene of interest and/or molecules that have an inhibitory effect on the biological activity of a protein of interest or its expression.

As used herein, a "Jak 3 inhibitor" can be a compound that interacts with a Jak 3 gene, or a Jak 3 protein or polypeptide, and inhibits its activity and/or its expression. The compound can decrease the activity or expression of a protein encoded by Jak 3. A Jak3 inhibitor can be a Jak3 modulating compound.

According to an aspect of the invention, there is provided a Jak3 inhibitor selected from the group consisting of: decernotinib, tofacitinib; JAK3 inhibitor IV (ZM-39923); NSC114792; PF-956980; an antisense RNA or antisense DNA that is specific for a nucleic acid encoding a JAK3 polypeptide; and a siRNA that specifically targets the Jak3 gene for use in the treatment of a hair-loss disorder selected from the group consisting of alopecia areata and androgenetic alopecia.

A Jak 3 inhibitor can be a peptide fragment that binds a protein comprising SEQ ID NO: 1. For example, the fragment can encompass any portion of about 8 consecutive amino acids of SEQ ID NO: 1. The fragment can comprise about 10 consecutive amino acids, about 20 consecutive amino acids, about 30 consecutive amino acids, about 40 consecutive amino acids, about 50 consecutive amino acids, about 60 consecutive amino acids, or about 75 consecutive amino acids of SEQ ID NO: 1. Fragments include all possible amino acid lengths between and including about 8 and about 100 amino acids, for example, lengths between about 10 and about 100 amino acids, between about 15 and about 100 amino acids, between about 20 and about 100 amino acids, between about 35 and about 100 amino acids, between about 40 and about 100 amino acids, between about 50 and about 100 amino acids, between about 70 and about 100 amino acids, between about 75 and about 100 amino acids, or between about 80 and about 100 amino acids. These peptide fragments can be obtained commercially or synthesized via liquid phase or solid phase synthesis methods (Atherton et al., (1989) Solid Phase Peptide Synthesis: a Practical Approach. IRL Press, Oxford, England).

An inhibitor of the application can be a protein, such as an antibody (monoclonal, polyclonal, humanized, chimeric, or fully human), or a binding fragment thereof, directed against a polypeptide encoded by SEQ ID NO: 1. An antibody fragment can be a form of an antibody other than the full-length form and includes portions or components that exist within full-length antibodies, in addition to antibody fragments that have been engineered. Antibody fragments can includesingle chain Fv (scFv), diabodies, Fv, and (Fab')₂, triabodies, Fc, Fab, CDR1, CDR2, CDR3, combinations of CDR's, variable regions, tetrabodies, bifunctional hybrid antibodies, framework regions, constant regions, and the like (*see,* Maynard et al., (2000) Ann. Rev. Biomed. Eng. 2:339-76; Hudson (1998) Curr. Opin. Biotechnol. 9:395-402). Antibodies can be obtained commercially, custom generated, or synthesized against an antigen of interest according to methods established in the art (*see* Roland E. Kontermann and Stefan Dübel (editors), Antibody Engineering. Vol. I & II, (2010) 2nd ed., Springer; Antony S. Dimitrov (editor), Therapeutic Antibodies: Methods and Protocols (Methods in Molecular Biology), (2009), Humana Press; Benny Lo (editor) Antibody Engineering: Methods and Protocols (Methods in Molecular Biology), (2004) Humana Press.

An inhibitor of the application can also be a small molecule that binds to a protein and disrupts its function. Small molecules are a diverse group of synthetic and natural substances generally having low molecular weights. They can be isolated from natural sources (for example, plants, fungi, microbes and the like), are obtained commercially and/or available as libraries or collections, or synthesized. Candidate small molecules that modulate a protein can be identified via *in silico* screening or high-through-put (HTP) screening of combinatorial libraries. Most conventional pharmaceuticals, such as aspirin, penicillin, and many chemotherapeutics, are small molecules, can be obtained commercially, can be chemically synthesized, or can be obtained from random or combinatorial libraries (Werner et al., (2006) Brief Funct. Genomic Proteomic 5(1):32-6). The agent may be a small molecule that binds, interacts, or associates with a target protein or RNA. Such a small molecule can be an organic molecule that, when the target is an intracellular target, is capable of penetrating the lipid bilayer of a cell to interact with the target. Small molecules includetoxins, chelating agents, metals, and metalloid compounds. Small molecules can be attached or conjugated to a targeting agent so as to specifically guide the small molecule to a particular cell.

Jak3 proteins and Jak3 modulating compounds of the application are intended to be administered to the subject once (e.g., as a single injection or deposition). Alternatively, Jak3 proteins and Jak3 modulating compounds are intended to be administered once or twice daily to a subject in need thereof for a period of from about two to about twenty-eight days, or from about seven to about ten days. Jak3 proteins and Jak3 modulating compounds are also intended to be administered once or twice daily to a subject for a period of 1, 2, 3, 4, 5, 6, 7, 8, 9, 10, 11, 12 times per year, or a combination thereof.

Furthermore, Jak3 proteins and Jak3 modulating compounds of the application can be co-administrated with another therapeutic. In some embodiments, the Jak3 modulating compound (e.g., a Jak3 inhibitor) can be co-adminstered with a Jak1/Jak2 inhibitor (e.g., a Jak1/Jak2 small molecule inhibitor, a therapeutic antibody directed to Jak1, a therapeutic antibody directed to Jak2, an siRNA directed to Jak1, or an siRNA directed to Jak2). Non-limiting examples of Jak1/Jak2 small molecule inhibitors include: **AG490** (Caceres-Cortes, (2008) Anticancer Agents Med Chem. 8(7):717-22); **CYT387** (Pardanani et al., (2009) Leukemia 23(8):1441-5; Monaghan et al., (2011) Leukemia. 25(12):1891-9); **SB1518** (William et al., (2011) J Med Chem. 54(13):4638-58; Hart et al., (2011) Leukemia. 25(11):1751-9); **LY3009104** (Baricitinib; INCB28050) (Incyte and Lilly); **TG101348** ( Wernig et al., (2008) Cancer Cell. 13(4):311-20; Pardanani et al., (2011) J Clin Oncol. 29(7):789-96); **INCB018424** (Incyte; Shi et al., (2011) J Clin Pharmacol. 51(12):1644-54); **CEP-701** (Cephalon; Hexner et al., (2008) Blood. 111(12):5663-71); **GLPG0634** (Galapagos; *see* http://www.glpg.com/index.php/randd/pipeline/glpg0634-ra/); **CYT387** (YM Biosciences; Pardanani (2009) Leukemia. 23(8):1441-5); **AZD1480** (Selleckchem, Houston TX; Scuto et al., (2011) Leukemia, 25(3):538-50); and **BMS-911543** (Purandare et al., (2012) Leukemia. 26(2):280-8. In one embodiment, inhibition of JAK1 also provides a mode of inhibiting JAK3 (e.g., *see* Haan et al., (2011) Chem Biol. 18(3):314-23).

JAK1/2 small molecule inhibitors in clinical development include a) INCB018424, topical and oral; 5 nM activity (Incyte); b) CEP-701 (Cephalon); and c) TG101348.
a) 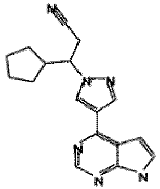
b) 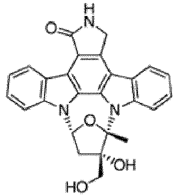
c)

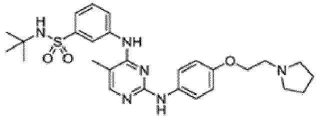

Where a dosage regimen comprises multiple administrations, the effective amount of the Jak3 proteins and Jak3 modulating compounds intended to be administered to the subject can comprise the total amount of gene product administered over the entire dosage regimen.

Jak3 proteins and Jak3 modulating compounds (e.g., a Jak3 inhibitor) are intended to be administered to a subject by any means suitable for delivering the Jak3 proteins and Jak3 modulating compounds to cells of the subject, such as the dermis, epidermis, dermal papilla cells, or hair follicle cells. For example, Jak3 proteins and Jak3 modulating compounds are intended to be administered by methods suitable to transfect cells. Transfection methods for eukaryotic cells are well known in the art, and include direct injection of the nucleic acid into the nucleus or pronucleus of a cell; electroporation; liposome transfer or transfer mediated by lipophilic materials; receptor mediated nucleic acid delivery, bioballistic or particle acceleration; calcium phosphate precipitation, and transfection mediated by viral vectors.

For example, the Jak3 protein or Jak3 modulating compound (e.g., a Jak3 siRNA) may be intended to be administered to the subject either as RNA, in conjunction with a delivery reagent, or as a nucleic acid (e.g., a recombinant plasmid or viral vector) comprising sequences which expresses the gene product. Suitable delivery reagents for administration of Jak3 or Jak3 globulin modulating compound include the Mirus Transit TKO lipophilic reagent; lipofectin; lipofectamine; cellfectin; or polycations (e.g., polylysine), or liposomes.

A therapeutically effective dose of Jak3 or a Jak3 modulating compound can depend upon a number of factors known to those or ordinary skill in the art. The dose(s) of Jak3 or a Jak3 modulating compound can vary, for example, depending upon the identity, size, and condition of the subject or sample being treated, further depending upon the route by which the composition is to be administered, if applicable, and the effect which the practitioner desires the Jak3 or a Jak3 modulating compound to have upon the nucleic acid or polypeptide of the invention. These amounts can be readily determined by a skilled artisan.

The compositions of this application can be formulated and administered to reduce the symptoms associated with a hair-loss disorder by any means that produces contact of the active ingredient with the agent's site of action in the body of a subject, such as a human or animal (e.g., a dog, cat, or horse). They can be administered by any conventional means available for use in conjunction with pharmaceuticals, either as individual therapeutic active ingredients or in a combination of therapeutic active ingredients. They can be administered alone, but are generally administered with a pharmaceutical carrier selected on the basis of the chosen route of administration and standard pharmaceutical practice.

A therapeutically effective dose of Jak3 modulating compounds can depend upon a number of factors known to those or ordinary skill in the art. The dose(s) of the Jak3 modulating compounds can vary, for example, depending upon the identity, size, and condition of the subject or sample being treated, further depending upon the route by which the composition is to be administered, if applicable, and the effect which the practitioner desires the Jak3 modulating compounds to have upon the nucleic acid or polypeptide of the invention. These amounts can be readily determined by a skilled artisan. Any of the therapeutic applications described herein can be applied to any subject in need of such therapy, including, for example, a mammal such as a dog, a cat, a cow, a horse, a rabbit, a monkey, a pig, a sheep, a goat, or a human.

Pharmaceutical compositions for use in accordance with the invention can be formulated in conventional manner using one or more physiologically acceptable carriers or excipients. The therapeutic compositions of the invention can be formulated for a variety of routes of administration, including systemic and topical or localized administration. Techniques and formulations generally can be found in Remington's The Science and Practice of Pharmacy, 20th ed. Lippincott Williams & Wilkins., Philadelphia, PA (2000. For systemic administration, an injection is useful, including intramuscular, intravenous, intraperitoneal, and subcutaneous. For injection, the therapeutic compositions of the invention can be formulated in liquid solutions, for example in physiologically compatible buffers such as Hank's solution or Ringer's solution. In addition, the therapeutic compositions can be formulated in solid form and redissolved or suspended immediately prior to use. Lyophilized forms are also included. Pharmaceutical compositions of the present invention are characterized as being at least sterile and pyrogen-free. These pharmaceutical formulations include formulations for human and veterinary use.

According to the invention, a pharmaceutically acceptable carrier can comprise any and all solvents, dispersion media, coatings, antibacterial and antifungal agents, isotonic and absorption delaying agents, and the like, compatible with pharmaceutical administration. The use of such media and agents for pharmaceutically active substances is well known in the art. Any conventional media or agent that is compatible with the active compound can be used. Supplementary active compounds can also be incorporated into the compositions.

The application also discloses a kit that comprises a pharmaceutically acceptable carrier and a Jak3 modulating compound identified using the screening assays of the invention packaged with instructions for use. For modulators that are antagonists of the activity of a Jak3 protein, or which reduce the expression of a Jak3 protein, the instructions would specify use of the pharmaceutical composition for promoting the loss of hair on the body surface of a mammal (for example, arms, legs, bikini area, face).

For Jak3 modulating compounds that are agonists of the activity of a Jak3 protein or increase the expression of one or more proteins encoded by Jak3 genes, the instructions would specify use of the pharmaceutical composition for regulating hair growth. In one embodiment, the instructions would specify use of the pharmaceutical composition for the treatment of hair loss disorders.

A pharmaceutical composition containing a Jak3 modulating compound can be administered in conjunction with a pharmaceutically acceptable carrier, for any of the therapeutic effects discussed herein. Such pharmaceutical compositions can comprise, for example antibodies directed to polypeptides encoded by genes comprising a Jak3 gene, or variants thereof, or agonists and antagonists of a polypeptide encoded by a Jak3 gene. The compositions can be administered alone or in combination with at least one other agent, such as a stabilizing compound, which can be administered in any sterile, biocompatible pharmaceutical carrier including saline, buffered saline, dextrose, and water. The compositions can be administered to a patient alone, or in combination with other agents, drugs or hormones.

Sterile injectable solutions can be prepared by incorporating the Jak3 modulating compound (e.g., a polypeptide or antibody) in the required amount in an appropriate solvent with one or a combination of ingredients enumerated herein, as required, followed by filtered sterilization. Generally, dispersions are prepared by incorporating the active compound into a sterile vehicle which contains a basic dispersion medium and the required other ingredients from those enumerated herein. In the case of sterile powders for the preparation of sterile injectable solutions, examples of useful preparation methods are vacuum drying and freeze-drying which yields a powder of the active ingredient plus any additional desired ingredient from a previously sterile-filtered solution thereof.

In some embodiments, the Jak3 inhibitor can be applied via transdermal delivery systems, which slowly releases the active compound for percutaneous absorption. Permeation enhancers can be used to facilitate transdermal penetration of the active factors in the conditioned media. Transdermal patches are described in for example, U.S. Pat. No. 5,407,713; U.S. Pat. No. 5,352,456; U.S. Pat. No. 5,332,213; U.S. Pat. No. 5,336,168; U.S. Pat. No. 5,290,561; U.S. Pat. No. 5,254,346; U.S. Pat. No. 5,164,189; U.S. Pat. No. 5,163,899; U.S. Pat. No. 5,088,977; U.S. Pat. No. 5,087,240; U.S. Pat. No. 5,008,110; and U.S. Pat. No. 4,921,475.

Various routes of administration and various sites of cell implantation can be utilized, such as, subcutaneous or intramuscular, in order to introduce the aggregated population of cells into a site of preference. Once implanted in a subject (such as a mouse, rat, or human), the aggregated cells can then stimulate the formation of a hair follicle and the subsequent growth of a hair structure at the site of introduction. In another embodiment, transfected cells (for example, cells expressing a protein encoded by a Jak3 gene are implanted in a subject to promote the formation of hair follicles within the subject. In further embodiments, the transfected cells are cells derived from the end bulb of a hair follicle (such as dermal papilla cells or dermal sheath cells). Aggregated cells (for example, cells grown in a hanging drop culture) or transfected cells (for example, cells produced as described herein) maintained for 1 or more passages can be introduced (or implanted) into a subject (such as a rat, mouse, dog, cat, human, and the like).

Subcutaneous administration can refer to administration just beneath the skin (i.e., beneath the dermis). Generally, the subcutaneous tissue is a layer of fat and connective tissue that houses larger blood vessels and nerves. The size of this layer varies throughout the body and from person to person. The interface between the subcutaneous and muscle layers can be encompassed by subcutaneous administration.

This mode of administration can be feasible where the subcutaneous layer is sufficiently thin so that the factors present in the compositions can migrate or diffuse from the locus of administration and contact the hair follicle cells responsible for hair formation. Thus, where intradermal administration is utilized, the bolus of composition administered is localized proximate to the subcutaneous layer.

Administration of the cell aggregates (such as DP or DS aggregates) is not restricted to a single route, but can encompass administration by multiple routes. For instance, exemplary administrations by multiple routes include, among others, a combination of intradermal and intramuscular administration, or intradermal and subcutaneous administration. Multiple administrations can be sequential or concurrent. Other modes of application by multiple routes will be apparent to the skilled artisan.

In other embodiments, this implantation method will be a one-time treatment for some subjects. In further embodiments of the invention, multiple cell therapy implantations will be required. In some embodiments, the cells used for implantation will generally be subject-specific genetically engineered cells. In another embodiment, cells obtained from a different species or another individual of the same species can be used. Thus, using such cells can require administering an immunosuppressant to prevent rejection of the implanted cells. Such methods have also been described in United States Patent No. 7,419,661 and PCT application publication WO 2001/32840.

In one embodiment, an inhibitor of the invention can be incorporated into pharmaceutical compositions suitable for administration, for example the inhibitor and a pharmaceutically acceptable carrier.

According to the invention, a pharmaceutically acceptable carrier can comprise any and all solvents, dispersion media, coatings, antibacterial and antifungal agents, isotonic and absorption delaying agents, and the like, compatible with pharmaceutical administration. The use of such media and agents for pharmaceutically active substances is well known in the art. Any conventional media or agent that is compatible with the active compound can be used. Supplementary active compounds can also be incorporated into the compositions.

A pharmaceutical composition of the invention can be administered in conjunction with a pharmaceutically acceptable carrier, for any of the therapeutic effects discussed herein. Such pharmaceutical compositions can comprise, for example antibodies directed to polypeptides. The compositions can be administered alone or in combination with at least one other agent, such as a stabilizing compound, which can be administered in any sterile, biocompatible pharmaceutical carrier including saline, buffered saline, dextrose, and water. The compositions can be administered to a patient alone, or in combination with other agents, drugs or hormones.

A pharmaceutical composition of the invention is formulated to be compatible with its intended route of administration. Examples of routes of administration include parenteral, e.g., intravenous, intradermal, subcutaneous, oral (e.g., inhalation or ingestion), transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersions. For intravenous administration, suitable carriers include physiological saline, bacteriostatic water, Cremophor EM™ (BASF, Parsippany, N.J.) or phosphate buffered saline (PBS). In all cases, the composition must be sterile and should be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, a pharmaceutically acceptable polyol like glycerol, propylene glycol, liquid polyetheylene glycol, and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it can be useful to include isotonic agents, for example, sugars, polyalcohols such as mannitol, sorbitol, sodium chloride in the composition. Prolonged absorption of the injectable compositions can be brought about by including in the composition an agent which delays absorption, for example, aluminum monostearate and gelatin.

Sterile injectable solutions can be prepared by incorporating the inhibitor (e.g., a polypeptide or antibody or small molecule) or agonist of the invention in the required amount in an appropriate solvent with one or a combination of ingredients enumerated herein, as required, followed by filtered sterilization. Generally, dispersions are prepared by incorporating the active compound into a sterile vehicle which contains a basic dispersion medium and the required other ingredients from those enumerated herein. In the case of sterile powders for the preparation of sterile injectable solutions, examples of useful preparation methods are vacuum drying and freeze-drying which yields a powder of the active ingredient plus any additional desired ingredient from a previously sterile-filtered solution thereof.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier and subsequently swallowed.

Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or sterotes; a glidant such as colloidal silicon dioxide; a sweetening agent such as sucrose or saccharin; or a flavoring agent such as peppermint, methyl salicylate, or orange flavoring.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The dosage administered can be a therapeutically effective amount of the composition sufficient to result in amelioration of symptoms of alopecia areata, and can vary depending upon known factors such as the pharmacodynamic characteristics of the active ingredient and its mode and route of administration; age, sex, health and weight of the recipient; nature and extent of symptoms; kind of concurrent treatment, frequency of treatment and the effect desired.

In some embodiments, the Jak3 inhibitor is intended to be administered to the subject in an amount of about 0.0001 µg/kg body weight, about 0.00025 µg/kg body weight, about 0.0005 µg/kg body weight, about 0.00075 µg/kg body weight, about 0.001 µg/kg body weight, about 0.0025 µg/kg body weight, about 0.005 µg/kg body weight, about 0.0075 µg/kg body weight, about 0.01 µg/kg body weight, about 0.025 µg/kg body weight, about 0.05 µg/kg body weight, about 0.075 µg/kg body weight, about 0.1 µg/kg body weight, about 0.25 µg/kg body weight, about 0.5 µg/kg body weight, about 0.75 µg/kg body weight, about 1 µg/kg body weight, about 5 µg/kg body weight, about 10 µg/kg body weight, about 25 µg/kg body weight, about 50 µg/kg body weight, about 75 µg/kg body weight, about 100 µg/kg body weight, about 150 µg/kg body weight, about 200 µg/kg body weight, about 250 µg/kg body weight, about 300 µg/kg body weight, about 350 µg/kg body weight, about 400 µg/kg body weight, about 450 µg/kg body weight, about 500 µg/kg body weight, about 550 µg/kg body weight, about 600 µg/kg body weight, about 650 µg/kg body weight, about 700 µg/kg body weight, about 750 µg/kg body weight, about 800 µg/kg body weight, about 850 µg/kg body weight, about 900 µg/kg body weight, about 950 µg/kg body weight, about 1000 µg/kg body weight, about 2000 µg/kg body weight, about 3000 µg/kg body weight, about 4000 µg/kg body weight, about 5000 µg/kg body weight, about 6000 µg/kg body weight, about 7000 µg/kg body weight, about 8000 µg/kg body weight, about 9500 µg/kg body weight, or about 10,000 µg/kg body weight.

In some embodiments, the JAK3 is intended to be administered to the subject in an amount of about 1 mg/kg body weight, about 1.5 mg/kg body weight, about 2 mg/kg body weight, about 2.5 mg/kg body weight, about 3 mg/kg body weight, about 3.5 mg/kg body weight, about 4 mg/kg body weight, about 4.5 mg/kg body weight, about 5 mg/kg body weight, about 5.5 mg/kg body weight, about 6 mg/kg body weight, about 6.5 mg/kg body weight, about 7 mg/kg body weight, about 7.5 mg/kg body weight, about 8 mg/kg body weight, about 9.5 mg/kg body weight, about 10 mg/kg body weight, about 10.5 mg/kg body weight, about 11.0 mg/kg body weight, about 11.5 mg/kg body weight, about 12 mg/kg body weight, about 12.5 mg/kg body weight, about 13 mg/kg body weight, about 13.5 mg/kg body weight, about 14 mg/kg body weight, about 14.5 mg/kg body weight, about 15 mg/kg body weight, about 15.5 mg/kg body weight, about 16 mg/kg body weight, about 16.5 mg/kg body weight, about 17 mg/kg body weight, about 17.5 mg/kg body weight, about 18 mg/kg body weight, about 19.5 mg/kg body weight, about 20 mg/kg body weight, about 21.5 mg/kg body weight, about 22 mg/kg body weight, about 22.5 mg/kg body weight, about 23 mg/kg body weight, about 23.5 mg/kg body weight, about 24 mg/kg body weight, about 24.5 mg/kg body weight, about 25 mg/kg body weight, about 25.5 mg/kg body weight, about 26 mg/kg body weight, about 26.5 mg/kg body weight, about 27 mg/kg body weight, about 27.5 mg/kg body weight, about 28 mg/kg body weight, about 29.5 mg/kg body weight, or about 30 mg/kg body weight.

In other embodiments, the Jak3 inhibitor is intended to be administered to the subject in a pharmaceutical composition selected from solution, ointment, salve, gel and cream. In some embodiments, the Jak3 inhibitor is contained in a topical pharmaceutical composition at a concentration selected from about 0.1%, about 0.2%, about 0.3%, about 0.4%, about 0.5%, about 0.6%, about 0.7%, about 0.8%, about 0.9%, about 1.0%, about 1.1%, about 1.2%, about 1.3%, about 1.4%, about 1.5%, about 1.6%, about 1.7%, about 1.8%, about 1.9%, about 2.0%, about 2.1%, about 2.2%, about 2.3%, about 2.4%, about 2.5%, about 2.6%, about 2.7%, about 2.8%, about 2.9%, or about 3.0%.,about 3.5%, about 4%, about 4.5%, about 5%, about 5.5%, about 6%, about 6.5%, about 7%, about 7.5%, about 8%, about 8.5%, about 9%, about 9.5%, or of about 10%.

The Jak3 modulating compound (e.g., a compound described herein that is directed to Jak3, such as a small molecule JAK3 inhibitor), can be co-administered with a second JAK inhibitor (such as a JAK1/2 inhibitor). In one embodiment, the JAK1/2 inhibitor can be administered as a topical cream. In some embodiments, the effective amount of the administered small molecule JAK1/2 inhibitor is present at a concentration of about 0.1%, about 0.2%, about 0.3%, about 0.4%, about 0.5%, about 0.6%, about 0.7%, about 0.8%, about 0.9%, about 1.0%, about 1.1%, about 1.2%, about 1.3%, about 1.4%, about 1.5%, about 1.6%, about 1.7%, about 1.8%, about 1.9%, about 2.0%, about 2.1%, about 2.2%, about 2.3%, about 2.4%, about 2.5%, about 2.6%, about 2.7%, about 2.8%, about 2.9%, or about 3.0%. In some embodiments, the effective amount of the administered JAK1/2 inhibitor is present at a concentration of about 3.5%, about 4%, about 4.5%, about 5%, about 5.5%, about 6%, about 6.5%, about 7%, about 7.5%, about 8%, about 8.5%, about 9%, about 9.5%, or of about 10%.

Toxicity and therapeutic efficacy of therapeutic compositions of the present invention can be determined by standard pharmaceutical procedures in cell cultures or experimental animals, e.g., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. Therapeutic agents that exhibit large therapeutic indices are useful. Therapeutic compositions that exhibit some toxic side effects can be used.

Unless otherwise defined, all technical and scientific terms used herein have the same meaning as commonly understood by one of ordinary skill in the art to which this invention belongs. Exemplary methods and materials are described below, although methods and materials similar or equivalent to those described herein can also be used in the practice or testing of the present invention.

### EXAMPLES

Examples are provided below to facilitate a more complete understanding of the invention. The following examples illustrate the exemplary modes of making and practicing the invention. However, the scope of the invention is not limited to specific embodiments disclosed in these Examples, which are for purposes of illustration only, since alternative methods can be utilized to obtain similar results.

### EXAMPLE 1 - Mouse Transplant Model For Alopecia Areata

Alopecia areata (AA) is one of the most prevalent autoimmune diseases and manifests as nonscarring hair loss. The course of the disease is unpredictable, and there are currently no consistently efficacious treatments available. Although research into disease pathogenesis and the development of targeted therapies are lacking, the creation of a mouse transplant model with a high incidence of disease has opened up new avenues of preclinical experimentation.

Several lines of evidence support the potential efficacy of JAK3 inhibitors in the treatment of AA: (1), a recently completed genome-wide association study of AA identified a number of gene-associated loci with immunological relevance, including those for IL-2/IL-2RA and IL-21; (2) initial findings in a preclinical mouse model suggested disruption of IL-15/IL-15R signaling ameliorates the disease; and (3) data indicate that T cells, dependent on IL-7 and IL-15 for survival, mediate the disease in the mouse model and in humans.

We therefore assessed the efficacy of JAK3 inhibition on the development of alopecia in a preclinical mouse model. Alopecic C3H/HeJ skin grafts were transplanted onto unaffected C3H/HeJ mouse recipients and subsequently treated with a JAK3 inhibitor administered by osmotic pump, vehicle administered by osmotic pump, or PBS injections. While 4/4 PBS-treated and 2/2 vehicle-pump treated mice developed alopecia by six weeks following transplantation, 0/5 mice treated with the JAK3 inhibitor developed alopecia. Microarray analysis of skin, immunohistochemical stains of skin for T cell infiltrate, and serum marker assessments demonstrated decreased inflammatory profiles in mice treated with JAK3 inhibitors. In total, our findings suggest JAK3 inhibitors may be an effective treatment in patients with AA.

### EXAMPLE 2 - Targeting the Immune Effector Response in AA

Specific autoimmune mechanisms underlying Alopecia Areata (AA) have remained obscure and therefore clinical investigation of AA has historically lagged behind other autoimmune diseases.

### Identify effective, clinically relevant, therapies in mouse models of alopecia areata.

In both human AA and in the AA mouse model, local hair follicle (HF) IL-15/NKG2DL upregulation was identified as key inflammatory signals that recruit/activate CD8' T cells, likely the critical AA immune effectors responsible for IFN-gamma (γ) production and hair follicle (HF) cytotoxicity. These observations provided the rationale for the studies to therapeutically target the IL-15/NKG2D.

It has been shown that the IL-15 pathway can be blocked using JAK3 protein tyrosine kinase inhibitors (PTKi).

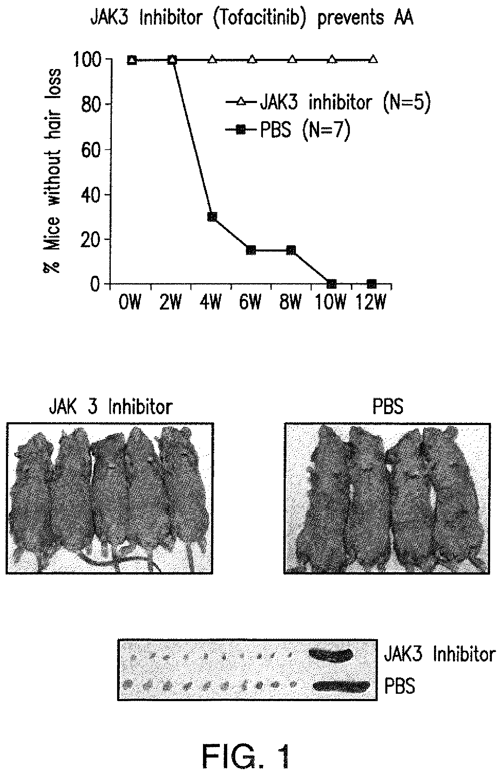

Small molecule PTKi prevents AA. 5 mice were treated systemically with tofacitinib (CP-69055; 10 mg/kg/day within an Alzet pump). Tofacitinib is a JAK3 inhibitor known to inhibit IL-15 signaling in human whole blood assays. None of the treated mice developed alopecia areata or cutaneous lymphadenopathy, whereas untreated mice manifested both AA and associated cutaneous lymphadenopathy (**Figure 1**). Topical delivery approaches will be pursued for AA reversal in the mouse model that has the major benefit of an improved therapeutic index for clinical development.

### Identify disease-associated AA biomarkers and their reversal with effective therapy in C3H mice.

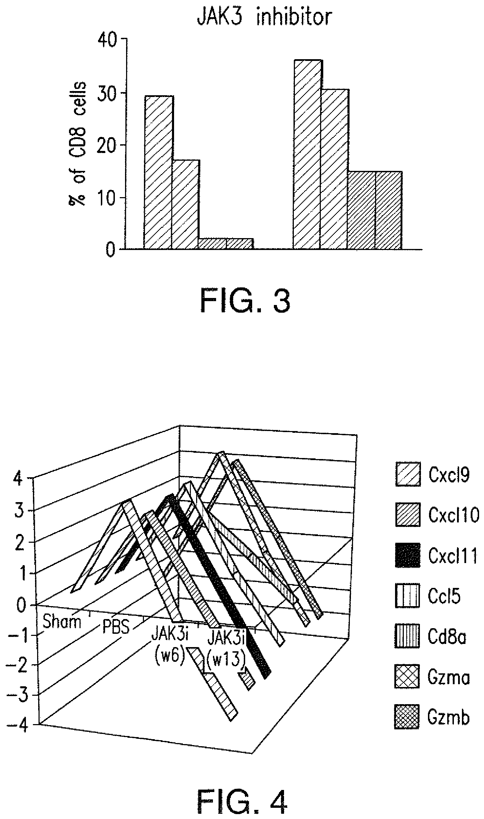

Cellular, inflammatory, and molecular biomarkers have been assessed in treated mice. Targeted therapy with tofacitinib eliminated the pathogenic CD8⁺NKG2D^{hi} cells from both the skin and the cutaneous draining lymph nodes and moreover down-regulated inflammatory biomarkers in the skin, including MHC molecules and the AA-associated IFN signature (**Figure 2**). HF NKG2DL expression was reduced although not completely eliminated (e.g. H60 expression by immunostain) (**Figures 2** and **3**), indicating H60 upregulation as a proximal event.

Transcriptional profiling analysis of treated vs. untreated skin shows abrogation of the cutaneous IFN-signature with treatment. These murine transcriptional profiles will be integrated computationally with transcriptional profiles obtained from human skin biopsies from untreated alopecic subjects to identify candidate dynamic human biomarkers that are associated with patchy stable disease vs. progressive disease vs. alopecia universalis.

Interpretation of PTKi targeted approaches. PTKi's vary greatly in their selectivity of their target kinases. For tofacitinib, JAK sensitivity is greater for JAK3>JAK1>>JAK2 (IC₅₀ is 28-50 nM, 140-180 nM, 1000-5000 nM, in whole blood assays, respectively). In the treated mice, tofacitinib serum levels would be 30-40 nM, and potential impacts will be assessed on both JAK1 and 3 activity. For ruxolitinib (a JAK1/2 inhibitor), sensitivity is JAK2>JAK1>>JAK3 (IC₅₀ is 3.3 nM, 2.8 nM, and 428 nM for in vitro kinase inhibition, respectively). Pharmacodynamic JAK inhibition in the mouse is likely to be transient since ruxolitinib dosing is once daily and the half-life in rodents is 3-6 hours.

For example, a Jak3 inhibitor can exert selective inhibition activity of its JAK3 target at about 10 nM, about 15 nM, about 20 nM, about 25 nM, about 30 nM, about 35 nM, about 40 nM, about 45 nM, about 50 nM, or at about 55 nM. A Jak3 inhibitor can also exert selective inhibition activity of its JAK3 target at about 60 nM, about 65 nM, about 70 nM, about 75 nM, about 80 nM, about 85 nM, about 90 nM, about 95 nM, about 100 nM, about 105 nM, about 110 nM, about 115 nM, about 120 nM, about 125 nM, about 130 nM, or at about 135 nM.

For example, a Jak3 inhibitor can also exert inhibition activity of its JAK3 target, while also able to promiscuously exert inhibition activity of a JAK1 target, at about 140 nM, about 145 nM, about 150 nM, about 155 nM, about 160 nM, about 165 nM, about 170 nM, about 175 nM, about 180 nM, about 185 nM, about 190 nM, about 195 nM, about 200 nM, about 205 nM, about 210 nM, about 215 nM, about 220 nM, or at about 225 nM.

For example, a Jak3 inhibitor can also exert inhibition activity of its JAK3 target at about 250 nM, about 300 nM, about 350 nM, about 400 nM, about 450 nM, about 500 nM, about 650 nM, about 700 nM, about 750 nM, about 800 nM, about 850 nM, about 900 nM, or at about 950 nM.

For example, a Jak3 inhibitor can also exert inhibition activity of its JAK3 target, while also able to promiscuously exert inhibition activity of a JAK2 target, at about 1000 nM, about 1250 nM, about 1500 nM, about 1750 nM, about 2000 nM, about 2250 nM, about 2500 nM, about 2750 nM, about 3000 nM, about 3250 nM, about 3500 nM, about 3750 nM, about 4000 nM, about 4250 nM, about 4500 nM, about 4750 nM, about 5000 nM, about 5250 nM, about 5500 nM, about 5750 nM, about 6000 nM, about 6250 nM, about 6500 nM, about 6750 nM, about 7000 nM, about 7250 nM, about 7500 nM, about 7750 nM, or at about 8000 nM.

For example, a Jak1/2 inhibitor can exert selective inhibition activity of its JAK1 and/or JAK2 target at about 0.5 nM, about 1.0 nM, about 1.5 nM, about 2.0 nM, about 2.5 nM, about 3.0 nM, about 3.5 nM, about 4.0 nM, about 4.5 nM, about 5.0 nM, about 5.5 nM, 6.0 nM, about 6.5 nM, about 7.0 nM, about 7.5 nM, about 8.0 nM, about 8.5 nM, about 9.0 nM, about 9.5 nM, or about 10.0 nM. For example, a Jak1/2 inhibitor can exert selective inhibition activity of its JAK1 and/or JAK2 target at about 15 nM, about 20 nM, about 25 nM, about 30 nM, about 35 nM, about 40 nM, about 45 nM, about 50 nM, about 55 nM, about 60 nM, about 65 nM, about 70 nM, about 75 nM, about 80 nM, about 85 nM, about 90 nM, about 95 nM, about 100 nM, about 150 nM, about 200 nM, about 250 nM, about 300 nM, about 350 nM, or at about 400 nM.

For example, a Jak1/2 inhibitor can also exert inhibition activity of its JAK1 and/or JAK2 target, while also able to promiscuously exert inhibition activity of a JAK3 target, at about 425 nM, about 450 nM, about 475 nM, about 500 nM, about 525 nM, about 550 nM, about 575 nM, about 600 nM, about 625 nM, about 650 nM, about 675 nM, about 700 nM, about 725 nM, about 750 nM, about 775 nM, about 800 nM, about 825 nM, about 850 nM, about 875 nM, about 900 nM, about 925 nM, about 950 nM, about 975 nM, or at about 1000 nM.

To activate a Jak protein target in the skin, the skin should be penetrated at about 0.5 mm, about 1mm, about 1.5 mm, about 2mm, about 2.5 mm, about 3 mm, about 3.5 mm, or at about 4 mm. Depending on the depth of penetration, the concentration of a JAK inhibitor, such as a JAK1/2 inhibitor or a JAK3 inhibitor, can range from about 25 nM to about 5000 nM. At the higher concentration ranges, one JAK inhibitor can elicit an inhibitory effect not only of its target but also of other JAK proteins.

### Integration of Human and Mouse Gene Expression Profiles.

The ribbon plots shown in **Figure 4** depict microarray data of normalized skin expression profiles of select genes from mice treated with 13 weeks of the JAK3 inhibitor CP690550 and PBS control. Briefly, after 13 weeks of treatment with the JAK3 inhibitor, skin was harvested and subjected to microarray analysis and compared with that of mice treated with PBS. The data show treatment-related elimination of the IFN-response chemokines and markers of CD8 cytotoxic effectors. Expression arrays from mouse and human will be integrated to compare expression profiles and eventually response to treatment

### EXAMPLE 3 - Systemic treatment with JAK3i

"NK-type" CD8αβ+ T cells are massively expanded in alopecic skin and draining LNs, and are required for T cell mediated transfer implicating this cytotoxic cell subset as the likely pathogenic effectors.

IL-15 is a key cytokine responsible for inducing CD8 T effectors in vitro. Moreover, IL-15 is produced by intestinal epithelial cells and is a known precipitant of CD8 cytotoxicity in celiac disease.

Alopecia areata is marked by IL-15/IL-15Ra upregulation in the human and murine hair follicle and implicates this cytokine as an end-organ trigger of CD8-mediated autoreactivity in AA.

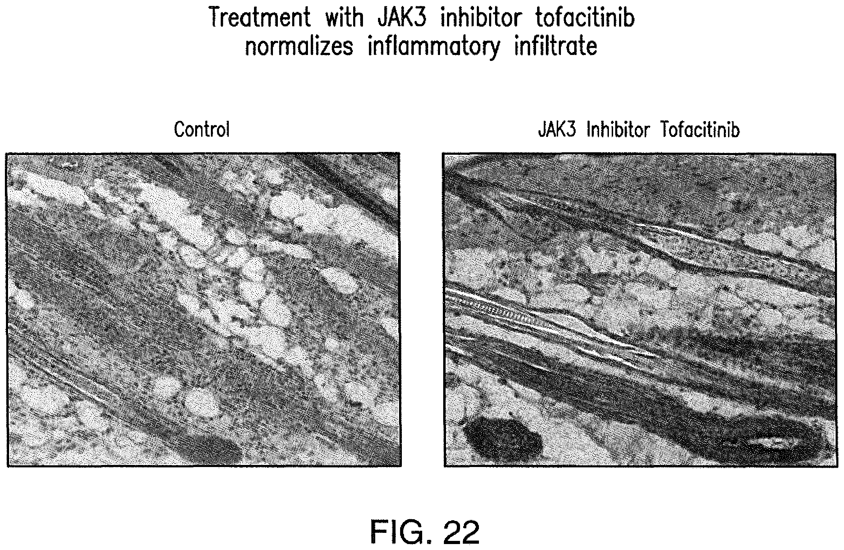

Systemic IL-15 blockade with a small molecule JAK3 PTKi effectively prevented alopecia areata, eliminating expansion of the pathogenic NK-type CD8 population and ablating the inflammatory signature in the skin. **FIG. 22** shows reversal of established disease with topical therapies.

### EXAMPLE 4 - Cellular Identity of Cytotoxic T Lymphocytes in Alopecia Areata Defines a Therapeutic Strategy

Alopecia areata (AA) is a common autoimmune disease resulting in an immune attack on the hair follicle¹. Although T cells have been implicated in the disease process, the pathways underlying their activation had not been determined². Prior to the GWAS study, the genetic basis of AA was largely undefined. Unexpectedly, a region of strong association was identified within the ULBP gene cluster on chromosome 6q25.1, encoding activating ligands of the natural killer cell receptor, NKG2D, which had never before been implicated in an autoimmune disease. Guided by the GWAS studies implicating NKG2D ligands (NKG2DL)³, here 'NK-type' CD8+ T cells were identified as the dominant effectors, which are both necessary and sufficient for disease induction. Global transcriptional profiling of mouse and human AA skin revealed striking signatures indicative of a robust IFNγ response and the presence of a cytotoxic T cell infiltrate.

Using the graft model of C3H/HeJ mouse skin to transfer AA, disease prevention can be recapitulated when treating at the time of grafting, as well as reversal of established disease by allowing grafted mice to first lose their hair. Systemically-administered pharmacological inhibitors of the JAK3 protein tyrosine kinases eliminated the IFN-signature and prevented the development of AA, and topical administration reversed established disease. Notably, these effects were durable up to 3 months after cessation of therapy. These findings illustrate the power of GWAS studies in uncovering new disease mechanisms, which have rapidly translated into new therapeutic opportunities in AA.

AA is a T cell mediated autoimmune disease characterized by hair loss and, histologically, by infiltrating T cells surrounding the hair follicle bulb, the so-called "swarm of bees^{1,2}. Previous studies have shown that transfer of total T cells (but not B cells or sera), can confer the disease in human xenograft models as well as the C3H/HeJ mouse model^{4,5}. However, the identity of the specific pathogenic T cell subsets in either human or mouse AA has not yet been defined.

Guided by a previous GWAS study, which identified ULBP3/6 as the most highly significant non-HLA risk locus in AA (p=2x10⁻²)³, a focus has been centered on establishing NKG2DLs as potential "danger signals" in the disease process. Upregulation of ULBP3 has previously shown in human AA hair follicles, associated with a dense infiltrate of CD8⁺NKG2D⁺ T cells³. Here, the role of these cells in AA immunopathogenesis, as well as a target for potential therapeutic intervention, was investigated.

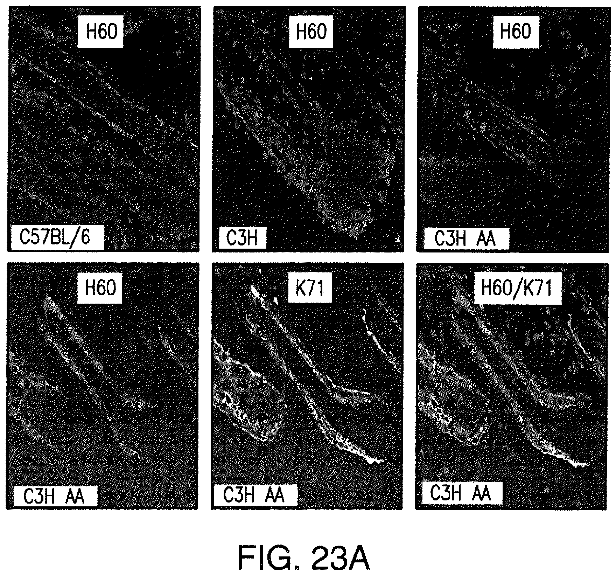

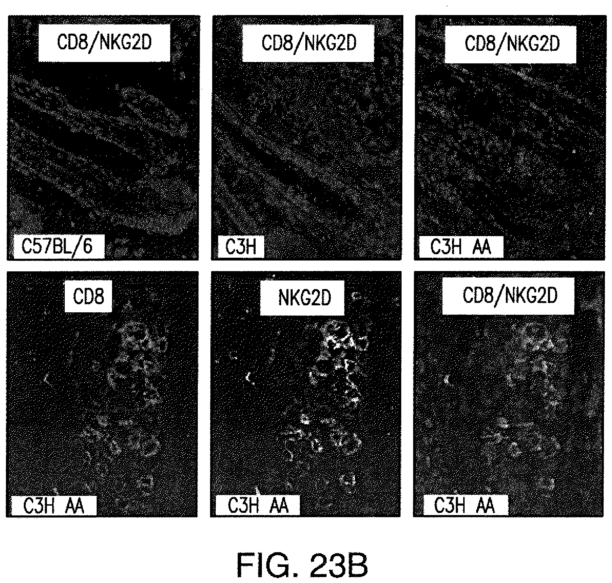

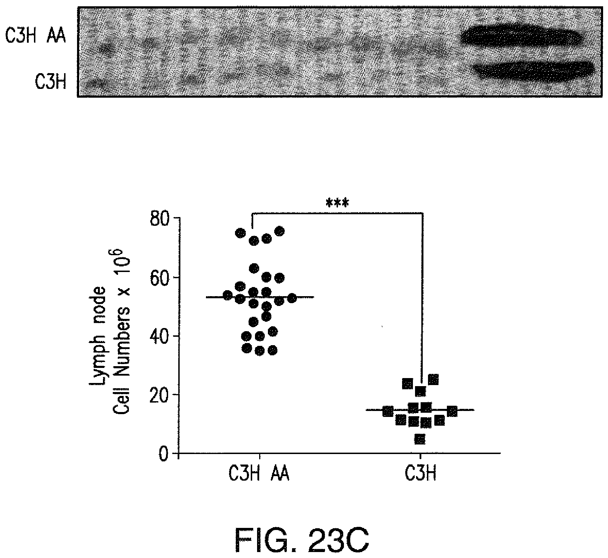

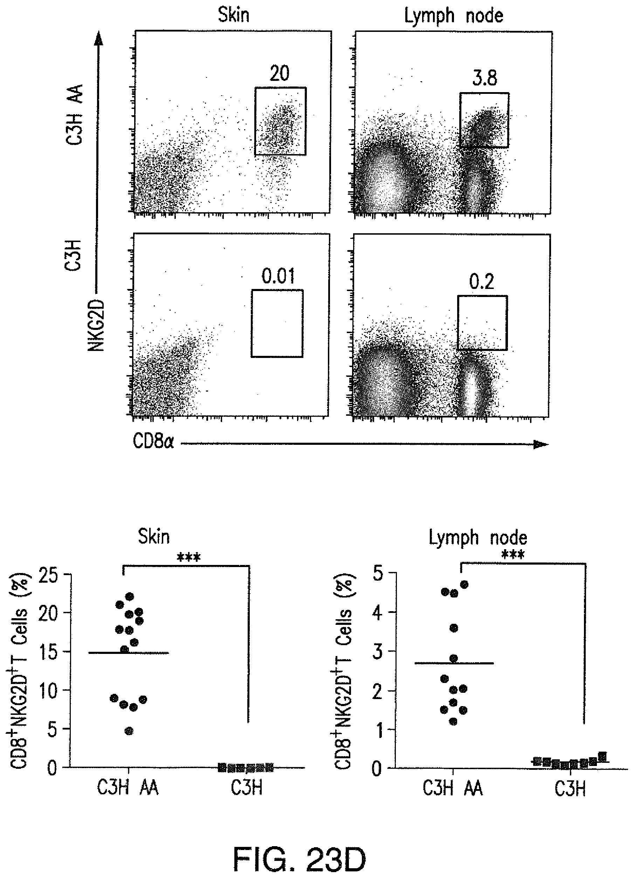

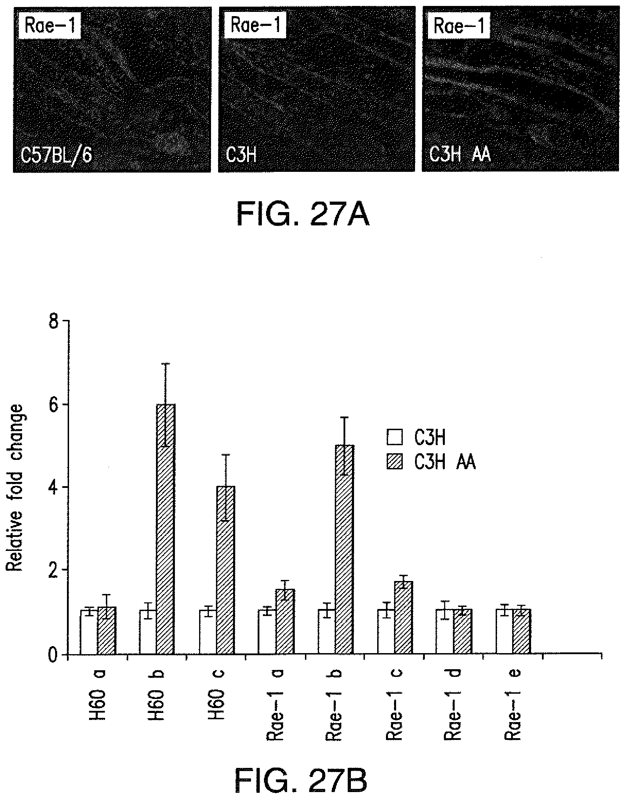

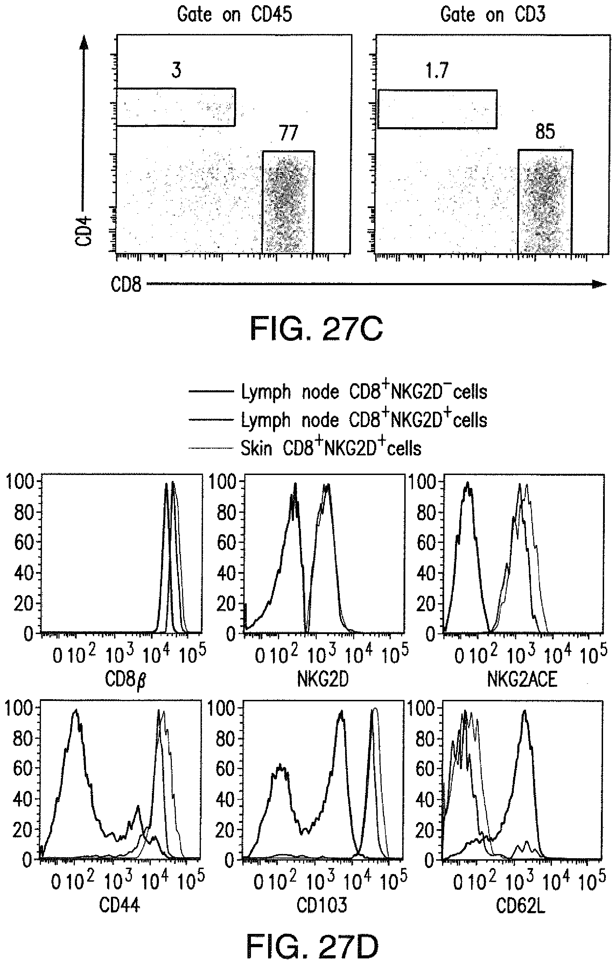

To determine the mechanistic importance of these cells in pathogenesis, the C3H/HeJ mouse model of AA was used, in which spontaneous disease develops with ∼15% incidence between 6 and 12 months of age⁶, and the graft-transfer C3H model was used, in which grafts from affected animals can transfer disease to 100% of C3H/HeJ recipients in 8-12 weeks time⁷. Lesional skin biopsies revealed that CD8⁺NKG2D⁺ T cells infiltrate the epithelial layers of the hair follicle that overexpress the NKG2DLs H60 and Rae-1, analogous to the situation in human AA (**FIG. 23A-B** and **FIG. 27A-B**). Flow cytometric examination of the CD45⁺ leukocyte population in the skin identified a striking expansion of CD8⁺NKG2D⁺ T cells (**FIG. 23D**). Other cell types, including CD4⁺ T cells were present in much smaller numbers (**FIG. 27C**). C3H/HeJ mice exhibit striking cutaneous lymphadenopathy (**FIG. 23C**) with increased total cellularity and, as noted in the skin, a dramatic expansion of a CD8⁺NKG2D⁺ cell population not present in disease-free C3H/HeJ mice (**FIG**. **23C-D**).

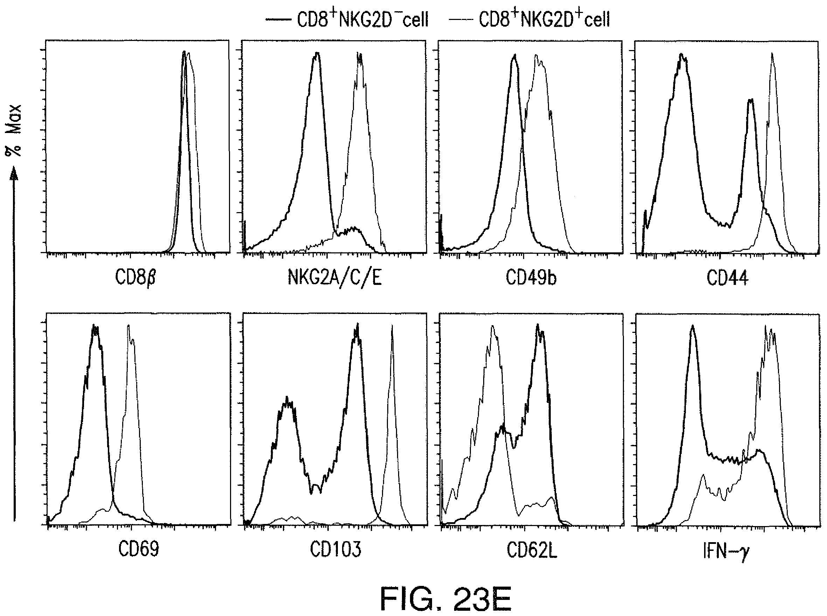

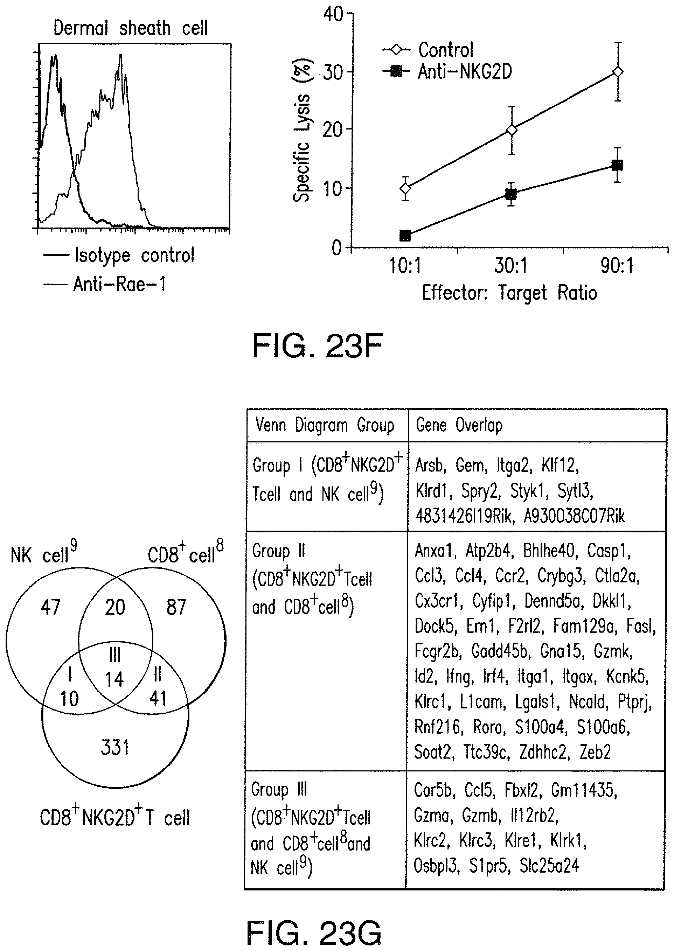

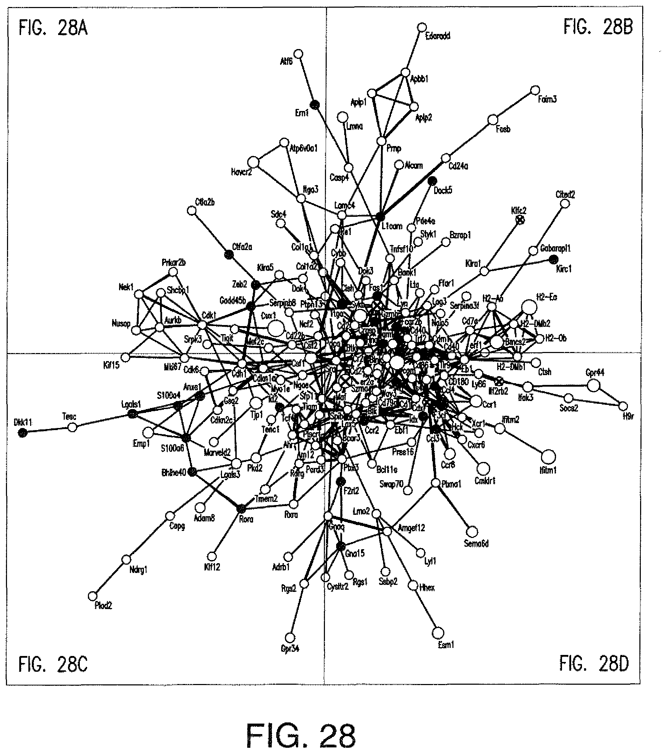

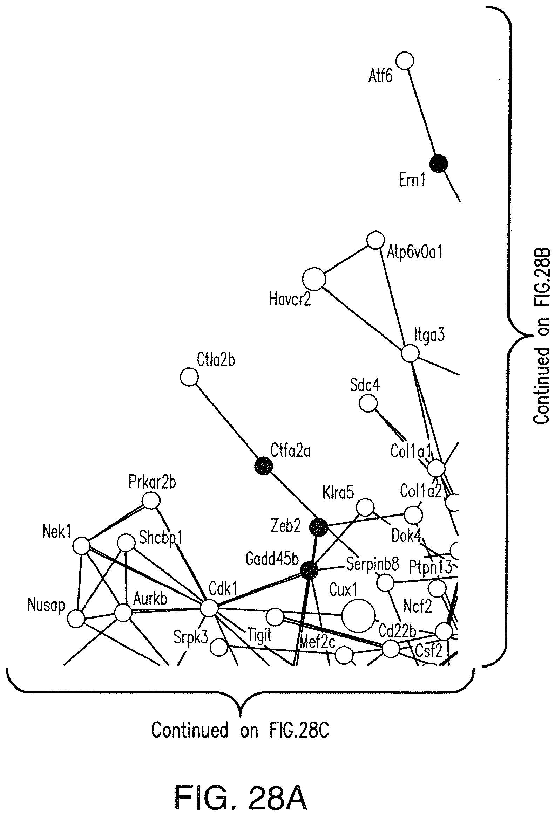

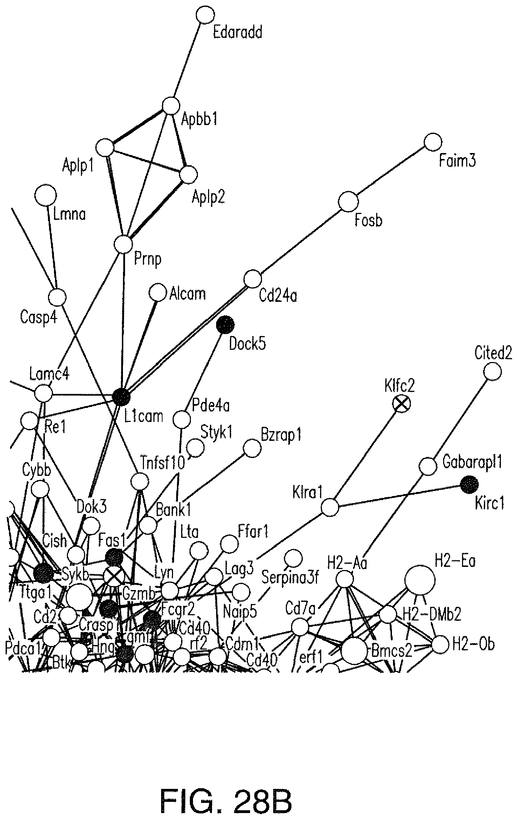

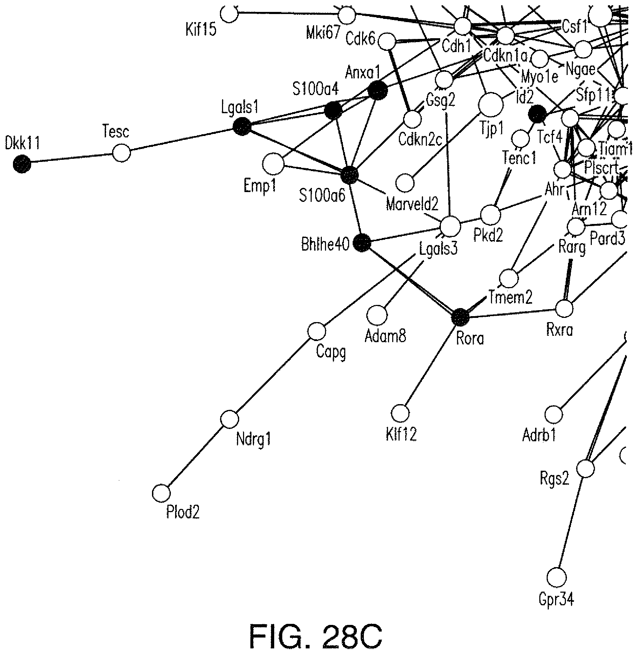

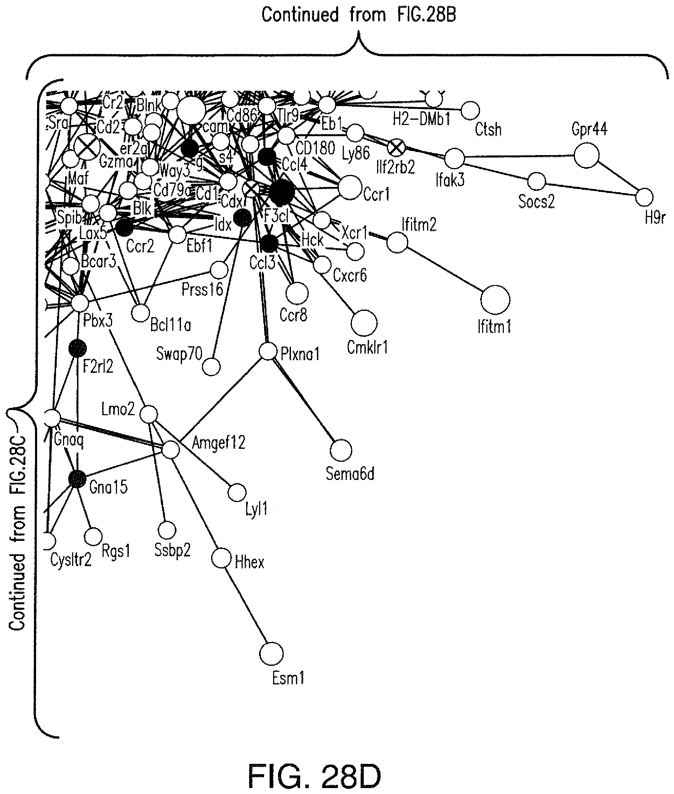

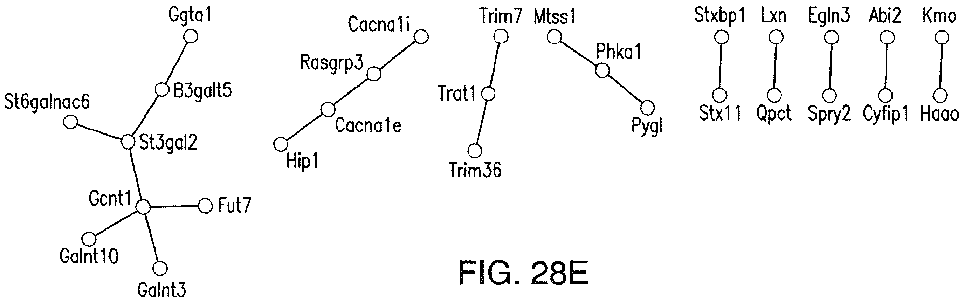

The immunophenotype of the skin-infiltrating CD8⁺ T cells were similar to the CD8⁺NKG2D⁺ population found in the cutaneous lymph nodes: CD8ab⁺ effector memory T cells (CDS^{hi} CD44^{hi} CD62L^{low} CD103⁺) bearing several NK immunoreceptors, including CD49b and NKG2A/C/E (**FIG. 23E** and **FIG. 27D**). These 'NK-type' CD8⁺ T cells expressed high levels of IFNg and exhibited NKG2D-dependent cytotoxicity against *ex vivo* expanded syngeneic dermal sheath target cells (**FIG. 23F**). Gene expression analysis of the CD8⁺NKG2D⁺ T cells isolated from alopecic C3H/HeJ lymph node cells using RNAseq demonstrated a transcriptional profile characteristic of effector CTLs as defined by the ImmGen Consortium⁸⁻¹⁰ and identified several additional "NK-specific" transcripts (**FIG. 23G** and **FIG. 28** and **Table 2**).

**Table 2.** Differentially expressed genes in CD8+NKG2D+ memory T cells vs. CD8⁺NKG2D⁻ memory T cells isolated from cutaneous lymph nodes obtained from alopecic mice. RNAseq was performed on cRNA made isolated from flow-sorted cells (BD influx) from total cutaneous lymph nodes from two alopecic mice.

| **GeneSymbol** | **FC** |
|---|---|
| Gstm5 | 63.99 |
| Gzma | 52.13 |
| Ifitm1 | 44.81 |
| Cx3cr1 | 38.24 |
| Sprn | 35.20 |
| Tjpl | 34.76 |
| Igsf5 | 32.13 |
| Tmem132e | 31.71 |
| Cmklr1 | 30.91 |
| 5430421N21Rik | 30.69 |
| Csf1 | 29.97 |
| Olfr60 | 28.79 |
| 4933431E20Rik | 27.87 |
| Metrnl | 27.44 |
| Epdr1 | 27.43 |
| Cd244 | 27.22 |
| Gpr141 | 26.45 |
| Ccr1 | 26.17 |
| Ccr8 | 23.75 |
| Pkd2 | 23.23 |
| Lgals3 | 22.66 |
| Esm1 | 21.59 |
| AI661453 | 20.69 |
| Sema6d | 20.58 |
| Tmem171 | 19.13 |
| Havcr2 | 19.01 |
| Rasgrf1 | 18.32 |
| Bean1 | 18.25 |
| Adora3 | 18.13 |
| Itga1 | 17.66 |
| Tpbg | 17.52 |
| Gzmb | 17.43 |
| Gpr44 | 17.11 |
| Stk32c | 16.81 |
| Bcar1 | 16.72 |
| Cntn1 | 16.70 |
| Slc38a8 | 16.65 |
| Adam8 | 16.15 |
| Usp44 | 15.85 |
| B4galnt4 | 15.50 |
| Cass4 | 14.94 |
| Galr3 | 14.86 |
| Ifitm2 | 14.77 |
| Rimbp2 | 14.64 |
| Fam171b | 14.02 |
| Srxn1 | 13.78 |
| Ptger3 | 13.70 |
| Lrat | 13.48 |
| Trpm6 | 13.43 |
| Lrrn2 | 13.17 |
| Arnt2 | 13.06 |
| Yap1 | 13.04 |
| Anxa1 | 12.85 |
| Gpr56 | 12.78 |
| Rail4 | 12.59 |
| Lypd3 | 12.52 |
| Gm5127 | 12.23 |
| Pdgfrb | 12.13 |
| Tktl1 | 11.87 |
| Gzmk | 11.63 |
| Olfr252 | 11.61 |
| AF529169 | 11.57 |
| Olfr523 | 11.48 |
| Selm | 11.43 |
| Sema4c | 11.25 |
| Ppp2r2c | 11.12 |
| Fxyd4 | 11.09 |
| Sept5 | 11.07 |
| Styk1 | 11.04 |
| Myadm | 10.84 |
| Wnt10b | 10.65 |
| Fgl2 | 10.61 |
| Gcnt4 | 10.59 |
| Emp1 | 10.44 |
| Cacng8 | 10.38 |
| Car5b | 10.36 |
| Ly6g5b | 10.32 |
| Lmna | 10.16 |
| Vipr2 | 10.08 |
| Ptpn5 | 10.01 |
| Plekhf1 | 9.92 |
| Il13 | 9.87 |
| 9430020K01Rik | 9.87 |
| Klrk1 | 9.80 |
| Itsn1 | 9.65 |
| Ret | 9.62 |
| Itgae | 9.58 |
| Col6a3 | 9.50 |
| Nbea | 9.41 |
| Rab27b | 9.41 |
| Clec12a | 9.36 |
| Mgat3 | 9.35 |
| Zfp57 | 9.30 |
| S100a4 | 9.27 |
| AI414108 | 9.22 |
| Trim58 | 9.19 |
| Irak3 | 9.18 |
| Gpr34 | 9.15 |
| Mt3 | 9.04 |
| AW555464 | 9.02 |
| Perp | 9.00 |
| Ace | 8.94 |
| Tmeff2 | 8.92 |
| Slc35f3 | 8.81 |
| Dyrk3 | 8.81 |
| Nxnl2 | 8.72 |
| Galnt3 | 8.67 |
| Cish | 8.63 |
| Vash1 | 8.58 |
| Klre1 | 8.58 |
| Dmrt2 | 8.56 |
| Wnt9a | 8.48 |
| Dusp14 | 8.43 |
| Dmd | 8.29 |
| St6galnac2 | 8.26 |
| Slc41a2 | 8.26 |
| Cdh1 | 8.22 |
| Tead1 | 8.09 |
| Msc | 8.05 |
| Ttc39c | 8.01 |
| Slc27a6 | 7.94 |
| Hlx | 7.85 |
| Mid2 | 7.73 |
| Plod2 | 7.70 |
| Prkcc | 7.68 |
| Ptgs1 | 7.63 |
| 6430571L13Rik | 7.60 |
| Specc1 | 7.55 |
| Prss16 | 7.53 |
| Fam129b | 7.46 |
| Spns3 | 7.49 |
| Lonrf3 | 7.45 |
| Fat4 | 7.41 |
| Nphs2 | 7.39 |
| Cpd | 7.34 |
| Cdkn2a | 7.26 |
| Ill7rd | 7.14 |
| Nebl | 7.12 |
| Src | 7.10 |
| Scnlb | 7.07 |
| Sccpdh | 7.04 |
| Igsf9b | 7.03 |
| Crispld2 | 7.02 |
| Plscr4 | 7.01 |
| Tmigd1 | 6.96 |
| Camk2n1 | 6.95 |
| Trat1 | 6.87 |
| Mtap6 | 6.85 |
| Qpct | 6.85 |
| Thsd7b | 6.82 |
| Tceal3 | 6.76 |
| Nlrp12 | 6.75 |
| 2010002N04Rik | 6.70 |
| Lat2 | 6.68 |
| Susd2 | 6.61 |
| Ptprj | 6.61 |
| P2ry14 | 6.56 |
| Csgagnact1 | 6.55 |
| Klri2 | 6.54 |
| 1700001C19Rik | 6.48 |
| Spp1 | 6.43 |
| Hlf | 6.41 |
| Gpx8 | 6.39 |
| a | 6.37 |
| Rtn4r | 6.31 |
| Lamc1 | 6.25 |
| Kndc1 | 6.23 |
| Slc22a15 | 6.19 |
| Id2 | 6.15 |
| Tnfsf13b | 6.14 |
| Elovl4 | 6.13 |
| 5830411N06Rik | 6.04 |
| Plxnd1 | 6.00 |
| Vstm2a | 5.98 |
| Rora | 5.96 |
| Il2 | 5.93 |
| Klrc1 | 5.92 |
| Tesc | 5.86 |
| Cd401g | 5.84 |
| Glis2 | 5.84 |
| Mt1 | 5.83 |
| Mdfic | 5.82 |
| P2ry2 | 5.73 |
| Hpse | 5.72 |
| Ttc16 | 5.70 |
| Kit1 | 5.56 |
| Arhgef25 | 5.65 |
| Foxf2 | 5.62 |
| Kir3dl2 | 5.56 |
| Unc79 | 5.52 |
| Aim11 | 5.47 |
| Rgag4 | 5.44 |
| Pugl | 5.41 |
| Efcab6 | 5.39 |
| Htra3 | 5.39 |
| Card10 | 5.39 |
| Nek6 | 5.38 |
| 6720401G13Rik | 5.36 |
| Fam131b | 5.36 |
| Grb14 | 5.34 |
| Pde1c | 5.33 |
| Pld1 | 5.31 |
| Gipc2 | 5.30 |
| 8430427H17Rik | 5.30 |
| Gabarapl1 | 5.29 |
| Olfr527 | 5.28 |
| Tenc1 | 5.27 |
| Snx7 | 5.27 |
| Rgs8 | 5.25 |
| Slc16a7 | 5.24 |
| Alcam | 5.23 |
| Tmbim1 | 5.23 |
| Nefl | 5.22 |
| Pdcd1 | 5.21 |
| 2810030E01Rik | 5.21 |
| Abhd3 | 5.19 |
| Clnk | 5.18 |
| Nlrplb | 5.14 |
| Cav2 | 5.13 |
| Aff2 | 5.12 |
| Kbtbd13 | 5.10 |
| Ppap2a | 5.07 |
| Gm14718 | 5.06 |
| Pde4a | 5.03 |
| Cnga2 | 4.95 |
| Acvrl1 | 4.92 |
| Mapkapk3 | 4.92 |
| Ccr2 | 4.87 |
| Hip1 | 4.84 |
| Pkp2 | 4.83 |
| Nap1l3 | 4.83 |
| Gorasp1 | 4.82 |
| Zfp651 | 4.82 |
| Hmga2-ps 1 | 4.81 |
| Tceal1 | 4.81 |
| Atp1a2 | 4.81 |
| 2200002K05Rik | 4.81 |
| Mmp2 | 4.77 |
| Ankrd35 | 4.77 |
| Klrc2 | 4.77 |
| Ptgfrn | 4.74 |
| Ildr1 | 4.66 |
| Ptpn3 | 4.64 |
| 5031425F14Rik | 4.62 |
| 2510009E07Rik | 4.58 |
| Emilin2 | 4.58 |
| 2900026A02Rik | 4.57 |
| Cd101 | 4.54 |
| 9030425E11Rik | 4.52 |
| 1500009L16Rik | 4.50 |
| Cdh17 | 4.48 |
| Clec5a | 4.48 |
| Dnahc9 | 4.47 |
| Gpr25 | 4.45 |
| Ifng | 4.44 |
| Socs2 | 4.42 |
| Pard3 | 4.42 |
| Nhsl2 | 4.41 |
| Layn | 4.40 |
| Csf2 | 4.35 |
| Crybb3 | 4.30 |
| Cxcr6 | 4.30 |
| Zeb2 | 4.27 |
| Prkar2b | 4.27 |
| Fam70a | 4.26 |
| Gdpd5 | 4.23 |
| Gm8773 | 4.22 |
| Fbn1 | 4.20 |
| Itgax | 4.20 |
| Rbpj | 4.17 |
| Cxcl13 | 4.15 |
| Tmem2 | 4.14 |
| Tmem205 | 4.14 |
| Klrc3 | 4.13 |
| Gcnt1 | 4.13 |
| Zc3h12c | 4.12 |
| Mir1199 | 4.09 |
| Ermn | 4.08 |
| Agap1 | 4.07 |
| 1810033B17Rik | 4.07 |
| Klf8 | 4.06 |
| 4831426I19Rik | 4.06 |
| Nin1 | 4.06 |
| Fbxo44 | 4.04 |
| Rab39b | 4.03 |
| Tspan2 | 4.02 |
| Adrb1 | 4.00 |
| Spns2 | 3.98 |
| Osbpl3 | 3.97 |
| Rapsn | 3.96 |
| Dhrs9 | 3.94 |
| Mboat2 | 3.90 |
| Pparg | 3.90 |
| Clip4 | 3.89 |
| Apol9a | 3.88 |
| Marveld2 | 3.86 |
| Capn5 | 3.86 |
| Ranbp17 | 3.86 |
| Ncam1 | 3.85 |
| Ctnnd2 | 3.85 |
| Slc25a24 | 3.85 |
| Palm | 3.84 |
| Fcer1g | 3.79 |
| Gzmc | 3.78 |
| Prdm1 | 3.78 |
| Scn8a | 3.75 |
| Fkbp9 | 3.72 |
| Gstt1 | 3.71 |
| Adssl1 | 3.71 |
| Prrg4 | 3.71 |
| Kir3dl1 | 3.70 |
| Ssbp2 | 3.67 |
| Swap70 | 3.67 |
| Trp73 | 3.67 |
| Il12rb2 | 3.66 |
| Phka1 | 3.66 |
| Xcr1 | 3.65 |
| Stau2 | 3.64 |
| 9130019P16Rik | 3.63 |
| Clec7a | 3.63 |
| 6430531B16Rik | 3.63 |
| Marco | 3.62 |
| Sulf2 | 3.61 |
| Reep1 | 3.61 |
| Anxa4 | 3.61 |
| Procr | 3.60 |
| Slc40a1 | 3.60 |
| Tnfrsf8 | 3.59 |
| Matk | 3.56 |
| Cysltr2 | 3.56 |
| Smtn | 3.55 |
| Rasl11b | 3.54 |
| Amph | 3.52 |
| Cysltr1 | 3.52 |
| Klri1 | 3.52 |
| B4alt2 | 3.51 |
| Mlf1 | 3.49 |
| B4galt4 | 3.49 |
| Sle4a11 | 3.47 |
| Htra1 | 3.47 |
| Ebf1 | 3.44 |
| Mpp2 | 3.44 |
| Ical | 3.44 |
| Tigit | 3.43 |
| Lzts1 | 3.43 |
| Epha3 | 3.43 |
| Mrgpre | 3.43 |
| Vdr | 3.42 |
| Npnt | 3.41 |
| D630039A03Rik | 3.40 |
| Rapgef3 | 3.37 |
| Cnksr1 | 3.37 |
| Napsa | 3.37 |
| E030011005Rik | 3.37 |
| 1700019E19Rik | 3.37 |
| Ncald | 3.37 |
| Ccl8 | 3.36 |
| Dhrs3 | 3.36 |
| Ppap2b | 3.36 |
| Gas2l1 | 3.36 |
| St3gal2 | 3.35 |
| Phactr2 | 3.34 |
| Rasd1 | 3.34 |
| Mafa | 3.33 |
| Olfr433 | 3.33 |
| L1cam | 3.31 |
| Pik3r6 | 3.31 |
| C1qa | 3.31 |
| Fgd1 | 3.30 |
| Gm17745 | 3.30 |
| Apol9b | 3.30 |
| Cmtm7 | 3.29 |
| Bicd1 | 3.29 |
| Rgs2 | 3.29 |
| Nos1ap | 3.29 |
| Bcl2a1d | 3.29 |
| Eif4e3 | 3.28 |
| Sgtb | 3.28 |
| Fut7 | 3.28 |
| Kif19a | 3.27 |
| Mtmr7 | 3.27 |
| Fam114a1 | 3.27 |
| Sdc4 | 3.27 |
| Farp1 | 3.26 |
| Fbxl21 | 3.25 |
| Ccrl2 | 3.25 |
| H1fx | 3.24 |
| Eif2c4 | 3.24 |
| Ube216 | 3.23 |
| Gpr68 | 3.23 |
| 4930544D05Rik | 3.23 |
| Hrh4 | 3.22 |
| Ryr1 | 3.22 |
| 9030418K01Rik | 3.22 |
| Acot1 1 | 3.22 |
| Pon3 | 3.22 |
| Serpine2 | 3.20 |
| Ly6a | 3.20 |
| Lrrn3 | 3.19 |
| B3galt5 | 3.19 |
| Ankrd29 | 3.19 |
| Cel1 | 3.19 |
| Serpina3f | 3.19 |
| St14 | 3.15 |
| Dscam | 3.14 |
| Fam129a | 3.14 |
| Med121 | 3.14 |
| 1300014I06Rik | 3.13 |
| Rnf216 | 3.13 |
| St3gal5 | 3.13 |
| S1e35e4 | 3.12 |
| Rab3il1 | 3.12 |
| Tmem176a | 3.12 |
| Syngr3 | 3.12 |
| Il9r | 3.12 |
| Lrrk2 | 3.11 |
| Zfp532 | 3.11 |
| Ache | 3.10 |
| Wipf3 | 3.09 |
| Cdkn1a | 3.09 |
| Ccdc136 | 3.08 |
| Epn2 | 3.07 |
| Fam167a | 3.07 |
| Syp | 3.07 |
| Spry2 | 3.06 |
| Tiam1 | 3.06 |
| Gnaq | 3.05 |
| Armcx6 | 3.05 |
| Raph1 | 3.05 |
| F730043M19Rik | 3.05 |
| Lag3 | 3.05 |
| Wscd1 | 3.04 |
| Dkkl1 | 3.04 |
| Zfp839 | 3.04 |
| Armcx1 | 3.03 |
| Zkscan16 | 3.03 |
| 1700009P17Rik | 3.02 |
| Nkd1 | 3.01 |
| Cdkn2b | 3.01 |
| Igfbp6 | 3.01 |
| Dlg2 | 3.01 |
| Plscr1 | 3.00 |
| Cacnali | 3.00 |
| Ckm | 3.00 |
| Kcnmb4 | 3.00 |
| Arhgap8 | 2.99 |
| Gspt2 | 2.99 |
| Foxd2 | 2.98 |
| Vat11 | 2.98 |
| Bfsp2 | 2.98 |
| Prrt2 | 2.98 |
| Wbscr17 | 2.98 |
| Car13 | 2.98 |
| Muc20 | 2.97 |
| Bzrap1 | 2.96 |
| Frem2 | 2.96 |
| Fcer2a | 2.96 |
| Chn2 | 2.95 |
| Blk | 2.95 |
| Kcnk5 | 2.95 |
| Mpzl1 | 2.95 |
| Aplp1 | 2.95 |
| Tlr4 | 2.95 |
| H2-DMb2 | 2.95 |
| Lyn | 2.94 |
| Meis2 | 2.94 |
| Camk1 | 2.93 |
| Pacsin3 | 2.93 |
| Tub | 2.93 |
| Cebpd | 2.93 |
| Gm5111 | 2.92 |
| Fasl | 2.92 |
| Lpcat2 | 2.91 |
| Mybpc2 | 2.90 |
| Sytl3 | 2.90 |
| H2-Eb2 | 2.90 |
| Hdac11 | 2.89 |
| Slc43a3 | 2.89 |
| Klf12 | 2.89 |
| Blnk | 2.89 |
| Atp2b4 | 2.88 |
| Faim3 | 2.88 |
| C1qc | 2.88 |
| Epb4.113 | 2.87 |
| Ms4a1 | 2.87 |
| Prr51 | 2.86 |
| Ferla | 2.86 |
| Ell3 | 2.86 |
| Prdx4 | 2.85 |
| Adcy9 | 2.85 |
| Pbx3 | 2.85 |
| Chmp4c | 2.84 |
| Osbpl1a | 2.84 |
| Apbb1 | 2.84 |
| Cd79a | 2.84 |
| Slc6a8 | 2.83 |
| Zcchc14 | 2.83 |
| 5730416F02Rik | 2.83 |
| Gm9199 | 2.82 |
| Ern1 | 2.82 |
| Lxn | 2.82 |
| Armc2 | 2.82 |
| Cr2 | 2.82 |
| Ptpn13 | 2.81 |
| Bcl11a | 2.81 |
| Scd1 | 2.81 |
| Chst3 | 2.80 |
| Gcnt2 | 2.80 |
| Lyl1 | 2.80 |
| Itga2 | 2.79 |
| Scn4a | 2.78 |
| Ccl5 | 2.78 |
| March1 | 2.76 |
| Siglecg | 2.76 |
| Islr | 2.76 |
| Spib | 2.76 |
| Ndrg1 | 2.75 |
| Tex15 | 2.75 |
| Rgs1 | 2.74 |
| 5031414D18Rik | 2.74 |
| Frmd4b | 2.74 |
| Ankrd6 | 2.74 |
| Bcar3 | 2.74 |
| Rhbdf1 | 2.72 |
| Slco4a1 | 2.72 |
| Capg | 2.71 |
| H2-DMb1 | 2.71 |
| Hhex | 2.71 |
| Postn | 2.71 |
| Myole | 2.71 |
| Ill5 | 2.70 |
| Soat2 | 2.69 |
| Casp1 | 2.69 |
| Itga3 | 2.69 |
| Rtnl | 2.68 |
| Sema6b | 2.68 |
| Ciita | 2.68 |
| Haao | 2.68 |
| Atf6 | 2.68 |
| Kcng1 | 2.67 |
| Ptk7 | 2.67 |
| Kdelc2 | 2.67 |
| Cd180 | 2.67 |
| Rhbd13 | 2.66 |
| Cd86 | 2.66 |
| Naip5 | 2.66 |
| Slc15a3 | 2.65 |
| BC013712 | 2.65 |
| Cd74 | 2.64 |
| C77080 | 2.64 |
| Fam43a | 2.63 |
| S100a6 | 2.63 |
| Lmo2 | 2.63 |
| Col8a2 | 2.63 |
| Celf5 | 2.63 |
| Lhfpl4 | 2.63 |
| Col1a1 | 2.62 |
| Ppap2c | 2.62 |
| Ccl3 | 2.62 |
| Rasgrp3 | 2.62 |
| Klhl30 | 2.62 |
| Clu | 2.62 |
| Cdl9 | 2.61 |
| Syk | 2.61 |
| Cpne7 | 2.61 |
| Rasgef1b | 2.61 |
| Gml1435 | 2.60 |
| Vpreb3 | 2.60 |
| Lhx2 | 2.58 |
| Cybb | 2.58 |
| Mapk8ip1 | 2.58 |
| Hck | 2.57 |
| 1110046J04Rik | 2.57 |
| Fam59a | 2.57 |
| Stx11 | 2.56 |
| Btk | 2.56 |
| H1f0 | 2.56 |
| Vav3 | 2.56 |
| F2rl2 | 2.55 |
| Hspa2 | 2.55 |
| Slc29a4 | 2.55 |
| Tubb4a | 2.54 |
| Arsb | 2.54 |
| Trim7 | 2.54 |
| Mef2c | 2.54 |
| Asb14 | 2.54 |
| 2010001M09Rik | 2.54 |
| Ehd2 | 2.54 |
| Itpripl2 | 2.53 |
| Ifitm3 | 2.53 |
| Zfp941 | 2.53 |
| Pax5 | 2.53 |
| Cd24a | 2.52 |
| Arhgef40 | 2.51 |
| Rab30 | 2.51 |
| H2-Eb1 | 2.50 |
| Serpinb6a | 2.50 |
| Kynu | 2.50 |
| Klra7 | 2.48 |
| Maf | 2.48 |
| Fcgr2b | 2.48 |
| H2-Ea-ps | 2.47 |
| Crip3 | 2.47 |
| Abhd4 | 2.47 |
| Fcrl1 | 2.47 |
| Egln3 | 2.47 |
| Kif15 | 2.46 |
| H2-Ab1 | 2.46 |
| Jazf1 | 2.46 |
| Dennd5a | 2.46 |
| Hgfac | 2.45 |
| Rtp3 | 2.45 |
| 9130206I24Rik | 2.45 |
| Cited2 | 2.45 |
| Sult4a1 | 2.45 |
| Gls2 | 2.44 |
| Cd80 | 2.44 |
| Tmem176b | 2.44 |
| Vcam1 | 2.44 |
| Ptms | 2.43 |
| Tmem154 | 2.43 |
| Ccl4 | 2.43 |
| Rgs16 | 2.42 |
| Pawr | 2.42 |
| Wdfy4 | 2.42 |
| Olfm1 | 2.42 |
| H2-Aa | 2.42 |
| Stac2 | 2.41 |
| Il18 | 2.40 |
| Fam164a | 2.40 |
| Phlda3 | 2.39 |
| Ctnnal1 | 2.39 |
| Tnfsf10 | 2.39 |
| Cubn | 2.38 |
| Abi2 | 2.38 |
| 2410004P03Rik | 2.38 |
| Trim36 | 2.38 |
| Bmf | 2.37 |
| Adap1 | 2.36 |
| Rarg | 2.36 |
| Tmem151a | 2.36 |
| Ly6d | 2.36 |
| Irf5 | 2.35 |
| Bhlhe40 | 2.35 |
| Zfp608 | 2.34 |
| Fam184a | 2.33 |
| Mafb | 2.33 |
| Coch | 2.32 |
| Gpr179 | 2.32 |
| Ffar1 | 2.32 |
| H2-Ob | 2.32 |
| Nav2 | 2.32 |
| Srpk3 | 2.31 |
| Cd209b | 2.31 |
| Arhgef12 | 2.31 |
| Arvcf | 2.31 |
| Serpina3g | 2.30 |
| Ppp1r3f | 2.30 |
| Cdc25c | 2.30 |
| Tmem159 | 2.30 |
| Atcay | 2.30 |
| Sept4 | 2.29 |
| Cacna1e | 2.29 |
| Plxna1 | 2.29 |
| Fzd6 | 2.29 |
| Zcchc24 | 2.29 |
| Ttbk1 | 2.28 |
| Cd40 | 2.28 |
| Atp6v0a1 | 2.28 |
| Gnao1 | 2.27 |
| Gsg2 | 2.27 |
| Dock5 | 2.26 |
| Tlr9 | 2.26 |
| Fbxo6 | 2.26 |
| 2410022L05Rik | 2.26 |
| Ltbp4 | 2.25 |
| Ncf2 | 2.25 |
| Cela1 | 2.25 |
| Ncr1 | 2.25 |
| Irf4 | 2.25 |
| Bank1 | 2.24 |
| El12 | 2.23 |
| 1190002F15Rik | 2.23 |
| Unc5b | 2.23 |
| Bspry | 2.23 |
| D17H6S56E-3 | 2.23 |
| Chst2 | 2.23 |
| Slc41a3 | 2.22 |
| Ctla2b | 2.22 |
| Gas213 | 2.22 |
| Casp4 | 2.22 |
| Dcxr | 2.21 |
| Ndrg4 | 2.21 |
| Muc1 | 2.21 |
| Lass6 | 2.21 |
| Pls3 | 2.21 |
| Dok3 | 2.20 |
| Carhsp1 | 2.20 |
| Ppfibp1 | 2.20 |
| Stxbp1 | 2.20 |
| Ctsh | 2.20 |
| Edaradd | 2.20 |
| Galnt10 | 2.20 |
| Prr5 | 2.19 |
| Rcbtb2 | 2.19 |
| Pik3c2b | 2.19 |
| Nqo2 | 2.19 |
| Fbxo30 | 2.18 |
| Mki67 | 2.17 |
| Prnp | 2.17 |
| Ahr | 2.16 |
| C030034L19Rik | 2.16 |
| Lgals1 | 2.16 |
| Gm11110 | 2.15 |
| Jup | 2.15 |
| Fam20a | 2.15 |
| Sfpi1 | 2.15 |
| Lrfn4 | 2.14 |
| Cdkn2c | 2.14 |
| Crybg3 | 2.14 |
| Trerf1 | 2.14 |
| Dok4 | 2.14 |
| Cdk6 | 2.13 |
| S1pr3 | 2.13 |
| Rxra | 2.13 |
| Cd163l1 | 2.12 |
| Pwwp2b | 2.12 |
| 1700017B05Rik | 2.12 |
| 2310010M20Rik | 2.12 |
| Nek2 | 2.12 |
| Lix1l | 2.12 |
| Ggta1 | 2.11 |
| Rorc | 2.11 |
| Fosb | 2.11 |
| Rimkla | 2.11 |
| Txlnb | 2.10 |
| Smox | 2.10 |
| Tcf4 | 2.10 |
| Fgd2 | 2.10 |
| Cd3001f | 2.10 |
| Map3k13 | 2.10 |
| Neil3 | 2.09 |
| Optn | 2.09 |
| A930038C07Rik | 2.09 |
| Klra5 | 2.08 |
| Cyp4f18 | 2.08 |
| Akap17b | 2.08 |
| 2700081O15Rik | 2.07 |
| Pcyox1l | 2.07 |
| Cd226 | 2.07 |
| Aurkb | 2.07 |
| St6galnac6 | 2.06 |
| Mfge8 | 2.06 |
| Slamfl | 2.06 |
| Zdhhc2 | 2.06 |
| Bag2 | 2.05 |
| Gpr55 | 2.05 |
| Dip2a | 2.05 |
| BC064078 | 2.05 |
| S1pr5 | 2.05 |
| Mlkl | 2.04 |
| Arap3 | 2.04 |
| Ctla2a | 2.04 |
| Gypc | 2.04 |
| Ccdc88a | 2.04 |
| Serpinb1a | 2.03 |
| Fam19a3 | 2.03 |
| Cdk1 | 2.03 |
| Tribl | 2.01 |
| Gadd45b | 2.01 |
| Pfn2 | 2.01 |
| Klrd1 | 2.01 |
| Trp53i11 | 2.01 |
| Cd22 | 2.00 |
| Mtss1 | 2.00 |
| Adam19 | 2.00 |
| Nusap1 | 2.00 |
| Hk3 | 0.50 |
| Pak1 | 0.50 |
| Irgc1 | 0.50 |
| Zfp296 | 0.50 |
| Il6st | 0.50 |
| Oas2 | 0.49 |
| I16ra | 0.49 |
| Ctsf | 0.49 |
| Lefl | 0.49 |
| Gm4951 | 0.49 |
| Gpr133 | 0.49 |
| Olfr1033 | 0.49 |
| Bambi-ps1 | 0.48 |
| Gtf2ird1 | 0.48 |
| Dtx1 | 0.48 |
| Maml3 | 0.47 |
| Plaur | 0.47 |
| Id3 | 0.47 |
| Tnfrsf25 | 0.47 |
| Susd4 | 0.47 |
| Wfikkn2 | 0.47 |
| 4921525O09Rik | 0.46 |
| Klra3 | 0.46 |
| 1110032F04Rik | 0.46 |
| Car12 | 0.46 |
| Pard6g | 0.46 |
| Slc7a4 | 0.46 |
| Lrp12 | 0.46 |
| Spats21 | 0.45 |
| Dleu7 | 0.45 |
| Cacna2d4 | 0.45 |
| Dst | 0.45 |
| Osbpl6 | 0.45 |
| Tmem108 | 0.44 |
| 4930417O13Rik | 0.44 |
| Angpt12 | 0.44 |
| Adam6b | 0.44 |
| Lrrc9 | 0.44 |
| 2310042E22Rik | 0.44 |
| Lars2 | 0.44 |
| Ankrd55 | 0.44 |
| Ly6k | 0.43 |
| Kazn | 0.43 |
| Dapl1 | 0.43 |
| Slc16a5 | 0.43 |
| Unc13a | 0.42 |
| Gpr113 | 0.42 |
| Siglech | 0.42 |
| Olig3 | 0.42 |
| Ksr2 | 0.42 |
| Card9 | 0.42 |
| Rasip1 | 0.42 |
| Dlc1 | 0.41 |
| Gm19705 | 0.41 |
| Syde2 | 0.41 |
| Actnl | 0.41 |
| Epx | 0.41 |
| Mir5109 | 0.41 |
| Gpr83 | 0.40 |
| Neb | 0.40 |
| Sall2 | 0.40 |
| Fam5b | 0.40 |
| Prickle1 | 0.40 |
| Nme4 | 0.40 |
| Lif | 0.40 |
| Auts2 | 0.39 |
| Col6a4 | 0.39 |
| Ccdc164 | 0.39 |
| Apol11b | 0.39 |
| Plac8 | 0.39 |
| Kcnf1 | 0.38 |
| Gm15708 | 0.38 |
| Wnt5b | 0.38 |
| Plat | 0.38 |
| Nmnat2 | 0.38 |
| 4933440M02Rik | 0.38 |
| Dcaf12l1 | 0.38 |
| Nacc2 | 0.38 |
| She | 0.37 |
| Snord69 | 0.37 |
| Mical3 | 0.37 |
| Cox6a2 | 0.36 |
| Dmbt1 | 0.36 |
| Aldh1l1 | 0.36 |
| Scarna6 | 0.36 |
| Lrp3 | 0.35 |
| Car11 | 0.35 |
| Il1r2 | 0.35 |
| Atp6v1g3 | 0.35 |
| Clec9a | 0.34 |
| Slc6a9 | 0.34 |
| Tnni3 | 0.34 |
| Gpr125 | 0.34 |
| Ikzf2 | 0.34 |
| Slc7a10 | 0.33 |
| Lman1l | 0.33 |
| A430105I19Rik | 0.33 |
| Eng | 0.32 |
| Snora17 | 0.32 |
| Gria4 | 0.32 |
| Fgfr1 | 0.32 |
| BC048644 | 0.31 |
| D830046C22Rik | 0.31 |
| Hs6st2 | 0.31 |
| Hoxa5 | 0.31 |
| Alp1 | 0.30 |
| Pgpep1l | 0.30 |
| Adam6a | 0.29 |
| Hoxa3 | 0.29 |
| Fam110b | 0.28 |
| Padi1 | 0.28 |
| Serpina9 | 0.28 |
| Igfbp4 | 0.28 |
| Pdzk1ip1 | 0.27 |
| Fzd1 | 0.27 |
| 4930546C10Rik | 0.27 |
| Foxp3 | 0.26 |
| Hoxa6 | 0.26 |
| Hoxa7 | 0.26 |
| Edn3 | 0.26 |
| Btc | 0.26 |
| Myl10 | 0.26 |
| Hoxa1 | 0.25 |
| Dnahc7a | 0.24 |
| Mira | 0.24 |
| Pcdhga10 | 0.24 |
| Lrrc3b | 0.23 |
| Slc4a4 | 0.23 |
| Rpr13 | 0.23 |
| Shc2 | 0.22 |
| Expi | 0.22 |
| Shisa2 | 0.22 |
| Gm12709 | 0.22 |
| Fbln2 | 0.21 |
| Sec1 | 0.21 |
| C85492 | 0.21 |
| Lamc3 | 0.21 |
| Dbx1 | 0.20 |
| Atplb1 | 0.20 |
| Nek5 | 0.19 |
| Actg2 | 0.19 |
| Dntt | 0.17 |
| Nrg2 | 0.17 |
| Sostdc1 | 0.14 |
| Trnp1 | 0.14 |
| Lama1 | 0.14 |
| Rpr12 | 0.13 |
| 0610012H03Rik | 0.12 |
| Lrrc32 | 0.10 |

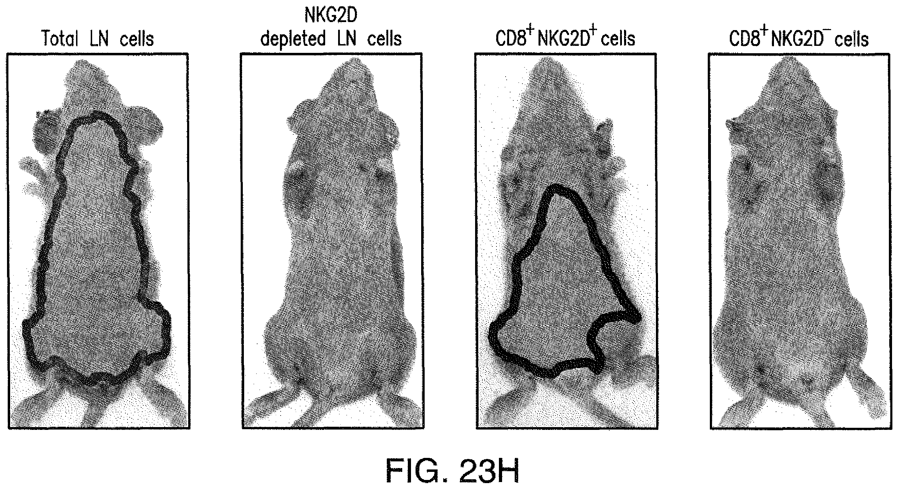

To evaluate the requirement of these 'NK-type' CD8 T cells in disease pathogenesis, an adoptive transfer approach was used. Cytotoxic CD8⁺NKG2D⁺ cells transferred alone, as well as total lymph node cells, were both able to give rise to AA in five recipients, whereas lymph node populations depleted of NKG2D⁺ cells were unable to transfer disease (**FIG. 23H**). Not only are 'NK-type' CD8⁺NKG2D⁺ T cells the dominant cell type in the dermal infiltrate, but, moreover, they are both necessary and sufficient for T cell mediated transfer of alopecia areata.

Using comparative genomics in order to characterize the transcriptional landscape of AA lesional skin from C3H/HeJ mice as well as humans with AA, Affymetrix profiling was first performed of whole skin from C3H/HeJ mice with spontaneous AA versus unaffected C3H/HeJ skin (**FIG**. **24A**, top panel and **FIG**. **29**). In parallel, human skin was similarly profiled from perilesional biopsies of active disease in five AA patients versus normal controls (**Tables 3-5**).

**Table 3.** Differentially expressed genes in the skin of alopecic female C3H/HeJ mice vs. skin obtained from age-matched female C3H/HeJ mice without alopecia (n=3 per group).

| **Affy_ID** | **GeneSymbol** | **FC** |
|---|---|---|
| 1419762_at | Ubd | 135.60 |
| 1418652_at | Cxc19 | 74.98 |
| 1417898_a_at | Gzma | 56.37 |
| 1419697_at | Cxcl11 | 53.33 |
| 1418930_at | Cxcl10 | 37.58 |
| 1419042_at | Iigp1 | 34.65 |
| 1418776_at | Gbp8 | 33.06 |
| 1418126_at | Ccl5 | 27.95 |
| 1419043_a_at | Iigp1 | 25.36 |
| 1438676_at | Gbp6 | 25.11 |
| 1423467_at | Ms4a4b | 23.27 |
| 1453196_a_at | Oas12 | 23.06 |
| 1419060_at | Gzmb | 21.74 |
| 1417141_at | Igtp | 21.08 |
| 1435639_at | 2610528A11Rik | 20.88 |
| 1423555_a_at | Ifi44 | 20.58 |
| 1417292_at | Ifi47 | 18.63 |
| 1425394_at | BC023105 | 18.43 |
| 1420549_at | Gbp1 | 17.81 |
| 1417793_at | Irgm2 | 17.25 |
| 1435906_x_at | Gbp2 | 15.15 |
| 1450033_a_at | Statl | 14.48 |
| 1418825_at | Irgm1 | 14.46 |
| 1444078_at | Cd8a | 14.23 |
| 1422812_at | Cxcr6 | 13.98 |
| 1427747_a_at | Lcn2 | 13.89 |
| 1420699_at | Clec7a | 13.82 |
| 1418240_at | Gbp2 | 13.32 |
| 1450783_at | Ifit1 | 13.13 |
| 1418392_a_at | Gbp3 | 12.61 |
| 1424305_at | Igj | 12.53 |
| 1434380_at | Gbp7 | 12.51 |
| 1424865_at | Pyy | 12.17 |
| 1422588_at | Krt6b | 12.07 |
| 1434046_at | AA467197 | 11.98 |
| 1418580_at | Rtp4 | 11.29 |
| 1419709_at | Stfa3 | 11.04 |
| 1425156_at | Gbp7 | 10.72 |
| 1449254_at | Spp1 | 10.61 |
| 1440481_at | Stat1 | 10.53 |
| 1449153_at | Mmp12 | 10.18 |
| 1424921_at | Bst2 | 9.80 |
| 1429947_a_at | Zbp1 | 9.65 |
| 1456907_at | Cxcl9 | 9.62 |
| 1421075_s_at | Cyp7b1 | 8.74 |
| 1451777_at | Ddx60 | 8.72 |
| 1421688_a_at | Ccl1 | 8.65 |
| 1421074_at | Cyp7b1 | 8.56 |
| 1419604_at | Zbpl | 8.51 |
| 1419714_at | Cd274 | 8.41 |
| 1427102_at | Slfn4 | 8.40 |
| 1424761_at | Fam115c | 8.26 |
| 1419135 at | Ltb | 8.24 |
| 1450034_at | Stat1 | 7.98 |
| 1425832_a_at | Cxcr6 | 7.98 |
| 1420915_at | Statl | 7.67 |
| 1438037_at | Herc6 | 7.62 |
| 1448436_a_at | Irf1 | 7.61 |
| 1448932_at | Krt16 | 7.12 |
| 1435710_at | AI661384 | 7.10 |
| 1451564_at | Parp14 | 7.08 |
| 1452614_at | Bcl2l15 | 7.00 |
| 1417256_at | Mmp13 | 6.88 |
| 1447541_s_at | Itgae | 6.79 |
| 1432026_a_at | Herc6 | 6.70 |
| 1434372_at | AW112010 | 6.62 |
| 1459913_at | Tnfsf10 | 6.51 |
| 1451426_at | Dhx58 | 6.51 |
| 1452405_x_at | Trav9d-3 | 6.44 |
| 1429570_at | Mlkl | 6.41 |
| 1444064_at | Samhd1 | 6.28 |
| 1451860_a_at | Trim30a | 6.23 |
| 1453080_at | Apol7a | 6.22 |
| 1420380_at | Ccl2 | 6.12 |
| 1434438_at | Samhd1 | 6.11 |
| 1450696_at | Psmb9 | 6.06 |
| 1451537_at | Chi3l1 | 5.99 |
| 1450165_at | Slfn2 | 5.88 |
| 1424727_at | Ccr5 | 5.83 |
| 1421256_at | Gzmc | 5.80 |
| 1448162_at | Vcam1 | 5.80 |
| 1453913_a_at | Tap2 | 5.79 |
| 1418191_at | Usp18 | 5.73 |
| 1425005_at | Klrc1 | 5.65 |
| 1439680_at | Tnfsf10 | 5.64 |
| 1433935_at | AU020206 | 5.51 |
| 1426113_ x_ at | Trav9d-3 | 5.51 |
| 1422415_at | Ang2 | 5.44 |
| 1460245_at | Klrd1 | 5.41 |
| 1426971_at | Uba7 | 5.34 |
| 1443698_at | Xafl | 5.32 |
| 1418131_at | Samhd1 | 5.26 |
| 1419591_at | Gsdmc | 5.20 |
| 1416897_at | Parp9 | 5.11 |
| 1417244_a_at | Irf7 | 5.08 |
| 1449216_at | Itgae | 5.06 |
| 1448632_at | Psmb10 | 5.06 |
| 1439825_at | Dtx31 | 5.02 |
| 1416714_at | Irf8 | 5.00 |
| 1425917_at | H28 | 4.99 |
| 1447621_s_at | Tmem173 | 4.93 |
| 1425396_a_at | Lck | 4.88 |
| 1418536_at | H2-Q7 | 4.84 |
| 1449025_at | Ifit3 | 4.75 |
| 1436649_at | Ikzf3 | 4.75 |
| 1425947_at | Ifng | 4.71 |
| 1417172_at | Ube216 | 4.70 |
| 1438855_x_at | Tnfaip2 | 4.70 |
| 1422962_a_at | Psmb8 | 4.69 |
| 1421186_at | Ccr2 | 4.66 |
| 1418204_s_at | Aif1 | 4.61 |
| 1421911_at | Stat2 | 4.57 |
| 1450753_at | Nkg7 | 4.55 |
| 1450424_a_at | Il18bp | 4.53 |
| 1450403_at | Stat2 | 4.52 |
| 1452565_x_at | LOC641050 | 4.44 |
| 1441054_at | Apol8 | 4.43 |
| 1449328_at | Ly75 | 4.39 |
| 1422601_at | Serpinb9 | 4.39 |
| 1422177_at | Il13ra2 | 4.39 |
| 1426170_a_at | Cd8b1 | 4.38 |
| 1460603_at | Samd91 | 4.37 |
| 1448754_at | Rbp1 | 4.31 |
| 1417822_at | D17H6S56E-5 | 4.28 |
| 1435208_at | Dtx31 | 4.26 |
| 1418649_at | Fgln3 | 4.26 |
| 1422160_at | H2-T24 | 4.23 |
| 1456064_at | AI504432 | 4.21 |
| 1449184_at | Pglyrp1 | 4.19 |
| 1438498_at | Zmynd15 | 4.19 |
| 1449195_s_at | Cxcl16 | 4.11 |
| 1439034_at | Spn | 4.10 |
| 1450678_at | Itgb2 | 4.10 |
| 1437176_at | Nlrc5 | 4.09 |
| 1458299_s_at | Nfkbie | 4.07 |
| 1421262_at | Lipg | 4.06 |
| 1426039_a_at | Alox12e | 4.05 |
| 1429184_at | Gvin1 | 4.05 |
| 1448380_at | Lgals3bp | 4.04 |
| 1436576_at | Fam26f | 4.01 |
| 1453939_x_at | Gm9706 | 3.99 |
| 1434457_at | Sp100 | 3.98 |
| 1425295_at | Ear11 | 3.97 |
| 1425065_at | Oas2 | 3.97 |
| 1449846_at | Ear2 | 3.97 |
| 1450582_at | H2-Q5 | 3.96 |
| 1440926_at | Flt1 | 3.94 |
| 1457140_s_at | Rassf10 | 3.91 |
| 1455500_at | Rnf213 | 3.90 |
| 1426970_a_at | Uba7 | 3.89 |
| 1448452_at | Irf8 | 3.88 |
| 1451673_at | Cd8a | 3.88 |
| 1437929_at | Dact2 | 3.86 |
| 1426774_at | Parp12 | 3.84 |
| 1418648_at | Egln3 | 3.82 |
| 1437811_x_at | Cotl1 | 3.78 |
| 1428420_a_at | 1200009I06Rik | 3.78 |
| 1417961_a_at | Trim30a | 3.77 |
| 1425225_at | Fcgr4 | 3.74 |
| 1421812_at | Tapbp | 3.74 |
| 1451905_a_at | Mx1 | 3.72 |
| 1441752_at | Art3 | 3.69 |
| 1448301_s_at | Serpinb1a | 3.69 |
| 1436172_at | Gm20559 | 3.68 |
| 1418133_at | Bcl3 | 3.67 |
| 1418655_at | B4galnt1 | 3.66 |
| 1417821_at | D 17H6S56E-5 | 3.66 |
| 1417171_at | Itk | 3.63 |
| 1460227_at | Timp1 | 3.61 |
| 1455161_at | AI504432 | 3.61 |
| 1421228_at | Ccl7 | 3.60 |
| 1434905_at | Ndufa4l2 | 3.60 |
| 1460218_at | Cd52 | 3.60 |
| 1460437_at | Cyth4 | 3.54 |
| 1430005_a_at | Batf2 | 3.53 |
| 1450188_s_at | Lipg | 3.53 |
| 1419698_at | Cxcll1 | 3.52 |
| 1439790_at | Serpinb9 | 3.51 |
| 1419413_at | Ccl17 | 3.51 |
| 1421009_at | Rsad2 | 3.50 |
| 1436838_x_at | Cotl1 | 3.50 |
| 1455269_a_at | Corola | 3.50 |
| 1422828_at | Cd3d | 3.49 |
| 1440866_at | Eif2ak2 | 3.47 |
| 1420437_at | Idol | 3.45 |
| 1439814_at | Atp8b4 | 3.44 |
| 1441706_at | Dscaml1 | 3.44 |
| 1421322_a_at | Irf9 | 3.43 |
| 1427076_at | Mpeg1 | 3.43 |
| 1435454_a_at | BC006779 | 3.41 |
| 1444619_x_at | Psmb8 | 3.40 |
| 1444350_at | Slfn10-ps | 3.39 |
| 1425335_at | Cd8a | 3.38 |
| 1450484_a_at | Cmpk2 | 3.38 |
| 1435832_at | Lrrc4 | 3.38 |
| 1424067_at | Icam1 | 3.37 |
| 1416318_at | Serpinb1a | 3.37 |
| 1445897_s_at | Ifi35 | 3.35 |
| 1450291_s_at | Ms4a4c | 3.34 |
| 1416273_at | Tnfaip2 | 3.34 |
| 1453304_s_at | Lv6e | 3.33 |
| 1418770_at | Cd2 | 3.32 |
| 1434067_at | AI662270 | 3.32 |
| 1428735_at | Cd69 | 3.31 |
| 1424965_at | Lpxn | 3.31 |
| 1455393_at | Cp | 3.31 |
| 1429567_at | Rassf10 | 3.30 |
| 1436905_x_at | Laptm5 | 3.30 |
| 1428485_at | Car12 | 3.28 |
| 1456211_at | Nlrp10 | 3.28 |
| 1438531_at | A730054J21Rik | 3.26 |
| 1449175_at | Gpr65 | 3.25 |
| 1424923_at | Serpina3g | 3.24 |
| 1417995_at | Ptpn22 | 3.23 |
| 1440990_at | Kif26b | 3.20 |
| 1420338_at | Alox15 | 3.18 |
| 1419300_at | Flt1 | 3.17 |
| 1419128_at | Itgax | 3.16 |
| 1426872_at | Fcgbp | 3.15 |
| 1425336_x_at | H2-K1 | 3.15 |
| 1448759_at | Il2rb | 3.14 |
| 1419178_at | Cd3g | 3.11 |
| 1420412_at | Tnfsf10 | 3.11 |
| 1433465_a_at | AI467606 | 3.11 |
| 1418203_at | Pmaip1 | 3.11 |
| 1430208_at | Krt42 | 3.09 |
| 1421628_at | Il18r1 | 3.08 |
| 1417620_at | Rac2 | 3.08 |
| 1454850_at | Tbc1d10c | 3.07 |
| 1439221_s_at | Cd40 | 3.07 |
| 1438658_a_at | S1pr3 | 3.06 |
| 1426278_at | Ifi2712a | 3.06 |
| 1429692_s_at | Gch1 | 3.04 |
| 1458148_at | Nlrc3 | 3.03 |
| 1440927_x_at | Apol11b | 3.03 |
| 1436236_x_at | Cotl1 | 3.03 |
| 1448940_at | Trim21 | 3.02 |
| 1421217_a_at | Lgals9 | 3.02 |
| 1451756_at | Flt1 | 3.01 |
| 1455048_at | Igsf3 | 3.01 |
| 1435560_at | Itga1 | 3.01 |
| 1425719_a_at | Nmi | 2.99 |
| 1439956_at | Ms4a6b | 2.98 |
| 1436562_at | Ddx58 | 2.98 |
| 1422632_at | Ctsw | 2.98 |
| 1436472_at | Slfn9 | 2.98 |
| 1427013_at | Car9 | 2.98 |
| 1449361_at | Tbx21 | 2.98 |
| 1426025_s_at | Laptm5 | 2.97 |
| 1438052_at | A130071D04Rik | 2.97 |
| 1426276_at | Ifih1 | 2.97 |
| 1456890_at | Ddx58 | 2.96 |
| 1416295_a_at | Il2rg | 2.94 |
| 1434873_a_at | Acap1 | 2.93 |
| 1456103_at | Pml | 2.93 |
| 1418678_at | Has2 | 2.92 |
| 1420697_at | Slc15a3 | 2.91 |
| 1436058_at | Rsad2 | 2.90 |
| 1425294_at | Slamf8 | 2.90 |
| 1459151_x_at | Ifi35 | 2.90 |
| 1418991_at | Bak1 | 2.89 |
| 1439068_at | Erap1 | 2.89 |
| 1424617_at | Ifi35 | 2.89 |
| 1417189_at | Psme2 | 2.89 |
| 1451544_at | Tapbpl | 2.88 |
| 1419412_at | Xcl1 | 2.88 |
| 1419539_at | Irx4 | 2.88 |
| 1452592_at | Mgst2 | 2.87 |
| 1420774_a_at | 4930583H14Rik | 2.86 |
| 1440169_x_at | Ifnar2 | 2.86 |
| 1420499_at | Gch1 | 2.85 |
| 1416002_x_at | Cotl1 | 2.85 |
| 1423754_at | Ifitm3 | 2.84 |
| 1416942_at | Erap1 | 2.84 |
| 1449169_at | Has2 | 2.83 |
| 1419609_at | Ccr1 | 2.83 |
| 1449591_at | Casp4 | 2.81 |
| 1436598_at | Icos | 2.81 |
| 1427381_at | Irg1 | 2.81 |
| 1419879_s_at | Trim25 | 2.81 |
| 1449143_at | Rtp4 | 2.80 |
| 1449839_at | Casp3 | 2.78 |
| 1426599_a_at | Slc2a1 | 2.78 |
| 1452117_a_at | Fyb | 2.77 |
| 1434773_a_at | Slc2a1 | 2.77 |
| 1450378_at | Tapbp | 2.76 |
| 1419561_at | Ccl3 | 2.76 |
| 1427007_at | Sash3 | 2.76 |
| 1422706_at | Pmepa1 | 2.76 |
| 1427892_at | Myolg | 2.75 |
| 1451335_at | Plac8 | 2.73 |
| 1425974_a_at | Trim25 | 2.72 |
| 1426600_at | Slc2a1 | 2.67 |
| 1418826_at | Ms4a6b | 2.67 |
| 1430244_at | 4921509J17Rik | 2.66 |
| 1457664_x_at | C2 | 2.65 |
| 1416564_at | Sox7 | 2.65 |
| 1433466_at | AI467606 | 2.65 |
| 1425801_x_at | Cotl1 | 2.64 |
| 1435945_a_at | Kcnn4 | 2.63 |
| 1434268_at | Adar | 2.63 |
| 1420353_at | Lta | 2.63 |
| 1418741_at | Itgb7 | 2.62 |
| 1416246_a_at | Corola | 2.62 |
| 1422280_at | Gzmk | 2.61 |
| 1421596_s_at | H28 | 2.60 |
| 1415834_at | Dusp6 | 2.60 |
| 1438364_x_at | Ang4 | 2.60 |
| 1439819_at | AU015263 | 2.60 |
| 1451755_a_at | Apobec1 | 2.59 |
| 1416296_at | Il2rg | 2.59 |
| 1439276_at | Adar | 2.59 |
| 1417495_x_at | Cp | 2.59 |
| 1453352_at | Atp10b | 2.58 |
| 1437478_s_at | Efhd2 | 2.58 |
| 1434366_x_at | C1qb | 2.58 |
| 1417185_at | Ly6a | 2.58 |
| 1443858_at | Trim12c | 2.58 |
| 1417494_a_at | Cp | 2.58 |
| 1427746_x_at | H2-K1 | 2.57 |
| 1421034_a_at | Il4ra | 2.56 |
| 1422005_at | Eif2ak2 | 2.56 |
| 1455000_at | Gpr68 | 2.56 |
| 1415803_at | Cx3c11 | 2.55 |
| 1422782_s_at | Tlr3 | 2.55 |
| 1416097_at | Lrrc4 | 2.54 |
| 1460651_at | Lat | 2.53 |
| 1427325_s_at | Akna | 2.53 |
| 1425603_at | Tmem176a | 2.49 |
| 1439595_at | Tcra | 2.49 |
| 1424041_s_at | C1s | 2.49 |
| 1418718_at | Cxc116 | 2.48 |
| 1456061_at | Gimap8 | 2.47 |
| 1417056_at | Psme1 | 2.46 |
| 1418981_at | Casp12 | 2.46 |
| 1421211_a_at | Ciita | 2.46 |
| 1449235_at | Fasl | 2.45 |
| 1449265_at | Casp1 | 2.44 |
| 1424383_at | Tmem51 | 2.44 |
| 1425405_a_at | Adar | 2.43 |
| 1425902_a_at | Nfkb2 | 2.42 |
| 1452817_at | Smyd3 | 2.42 |
| 1416871_at | Adam8 | 2.42 |
| 1457917_at | Lck | 2.42 |
| 1449127_at | Selplg | 2.42 |
| 1416514_a_at | Fscnl | 2.42 |
| 1439773_at | Ly6e | 2.41 |
| 1436223_at | Itgb8 | 2.41 |
| 1454268_a_at | Cyba | 2.41 |
| 1451821_a_at | Sp100 | 2.41 |
| 1444242_at | Slco2a1 | 2.40 |
| 1415983_at | Lcp1 | 2.40 |
| 1418293_at | Ifit2 | 2.39 |
| 1420671_x_at | Ms4a4c | 2.39 |
| 1450534_x_at | H2-K1 | 2.39 |
| 1418077_at | Trim21 | 2.39 |
| 1417496_at | Cp | 2.38 |
| 1435415_x_at | Marcksl1 | 2.37 |
| 1451320_at | Arhgap8 | 2.37 |
| 1427041_at | BC013712 | 2.36 |
| 1422139_at | Plau | 2.36 |
| 1441768_at | 9430051O21Rik | 2.35 |
| 1449455_at | Hck | 2.35 |
| 1421358_at | H2-M3 | 2.34 |
| 1436312_at | Ikzf1 | 2.34 |
| 1444632_at | BC064078 | 2.33 |
| 1450791_at | Nppb | 2.33 |
| 1424927_at | Glipr1 | 2.33 |
| 1419764_at | Chi313 | 2.33 |
| 1426093_at | Trim34a | 2.33 |
| 1420515_a_at | Pglyrp2 | 2.33 |
| 1448061_at | Msr1 | 2.33 |
| 1418641_at | Lep2 | 2.32 |
| 1418842_at | Hcls1 | 2.31 |
| 1434400_at | Tgif2 | 2.31 |
| 1439494_at | Slc5a9 | 2.30 |
| 1448757_at | Pml | 2.28 |
| 1431339_a_at | Efhd2 | 2.28 |
| 1455581_x_at | Gm20559 | 2.28 |
| 1437132_x_at | Nedd9 | 2.27 |
| 1456307_s_at | Adcy7 | 2.27 |
| 1422932_a_at | Vav1 | 2.27 |
| 1450446_a_at | Socs1 | 2.27 |
| 1434068_s_at | AI662270 | 2.26 |
| 1416069_at | Pfkp | 2.26 |
| 1421210_at | Ciita | 2.26 |
| 1437785_at | Adamts9 | 2.25 |
| 1436230_at | Gpr114 | 2.25 |
| 1425119_at | Oaslb | 2.25 |
| 1449310_at | Ptger2 | 2.25 |
| 1440721_at | 5930433N17Rik | 2.25 |
| 1430352_at | Adamts9 | 2.25 |
| 1456377_x_at | Limd2 | 2.25 |
| 1449991_at | Cd244 | 2.25 |
| 1416051_at | C2 | 2.25 |
| 1455132_at | Rasa13 | 2.24 |
| 1422124_a_at | Ptprc | 2.24 |
| 1448576_at | Il7r | 2.23 |
| 1421931_at | Icos | 2.22 |
| 1436199_at | Trim14 | 2.21 |
| 1446509_at | Smox | 2.20 |
| 1430148_at | Rabl9 | 2.20 |
| 1450170_x_at | H2-D1 | 2.19 |
| 1439736_at | 5830453J16Rik | 2.19 |
| 1451567_a_at | Ifi203 | 2.19 |
| 1433920_at | Sema4c | 2.18 |
| 1420913_at | Slco2a1 | 2.18 |
| 1439148_a_at | Pfkl | 2.18 |
| 1452544_x_at | H2-D1 | 2.18 |
| 1441600_at | C920021A13 | 2.17 |
| 1422804_at | Serpinb6b | 2.16 |
| 1453228_at | Stx11 | 2.15 |
| 1427682_a_at | Egr2 | 2.15 |
| 1428378_at | Zc3hav1 | 2.15 |
| 1430165_at | Stk17b | 2.15 |
| 1451321_a_at | Rbm43 | 2.15 |
| 1457780_at | Stx11 | 2.15 |
| 1439764_s_at | Igf2bp2 | 2.14 |
| 1435913_at | B4galnt4 | 2.14 |
| 1449220_at | Gimap3 | 2.13 |
| 1456251_x_at | Tspo | 2.13 |
| 1422781_at | Tlr3 | 2.13 |
| 1423135_at | Thy1 | 2.13 |
| 1424375_s_at | Gimap4 | 2.12 |
| 1428660_s_at | Tor3a | 2.12 |
| 1439012_a_at | Dck | 2.12 |
| 1441912_x_at | C2 | 2.12 |
| 1424704_at | Runx2 | 2.11 |
| 1424953_at | BC021614 | 2.11 |
| 1431843_a_at | Nfkbie | 2.10 |
| 1417219_s_at | Tmsb10 | 2.10 |
| 1448160_at | Lcp1 | 2.10 |
| 1427352_at | Krt79 | 2.10 |
| 1419194_s_at | Gmfg | 2.09 |
| 1460675_at | Igsf8 | 2.09 |
| 1426568_at | Slc2a9 | 2.08 |
| 1424961_at | Tapbpl | 2.08 |
| 1418099_at | Tnfrsf1b | 2.08 |
| 1422527_at | H2-DMa | 2.08 |
| 1426554_a_at | Pgam1 | 2.08 |
| 1438941_x_at | Ampd2 | 2.08 |
| 1450648_s_at | H2-Ab1 | 2.07 |
| 1440165_at | Ptprc | 2.07 |
| 1437724_x_at | Pitpnm1 | 2.07 |
| 1434364_at | Map3k14 | 2.06 |
| 1419537_at | Tfec | 2.06 |
| 1425728_at | Gm12185 | 2.06 |
| 1455966_s_at | Nudt21 | 2.05 |
| 1419282_at | Ccl12 | 2.05 |
| 1420404_at | Cd86 | 2.05 |
| 1415765_at | Hnrnpul2 | 2.05 |
| 1422511_a_at | Ogfr | 2.05 |
| 1425548_a_at | Lst1 | 2.05 |
| 1427683_at | Egr2 | 2.05 |
| 1453757_at | Herc6 | 2.04 |
| 1435331_at | Phin1 | 2.04 |
| 1419119_at | Hcst | 2.03 |
| 1458229_at | Robo2 | 2.03 |
| 1422808_s_at | Dock2 | 2.03 |
| 1437213_at | Nudt21 | 2.02 |
| 1429831_at | Pik3ap1 | 2.01 |
| 1439808_at | Ipcef1 | 2.01 |
| 1428891_at | Parm1 | 2.01 |
| 1427765_a_at | Tcrb-J | 2.01 |
| 1426133_a_at | Mitd1 | 2.00 |
| 1425016_at | Ephb2 | 2.00 |
| 1422562_at | Rrad | 2.00 |
| 1418110_a_at | Inpp5d | 2.00 |
| 1420272_at | Samhd1 | 2.00 |
| 1417928_at | Pdlim4 | 2.00 |
| 1460220_a_at | Csf1 | 2.00 |
| 1451828_a_at | Acsl4 | 2.00 |
| 1435672_at | 3830612M24 | 1.99 |
| 1453181_ x_ at | Plscr1 | 1.99 |
| 1428767_at | Gsdmd | 1.99 |
| 1436861_at | Il7 | 1.99 |
| 1437270_a_at | Clcf1 | 1.99 |
| 1419202_at | Cst7 | 1.98 |
| 1435529_at | Gm14446 | 1.98 |
| 1419810_x_at | Arhgap9 | 1.98 |
| 1429525_s_at | Myo1f | 1.98 |
| 1437308_s_at | F2r | 1.98 |
| 1419676_at | Mx2 | 1.98 |
| 1455019_x_at | Ckap4 | 1.98 |
| 1438948_x_at | Tspo | 1.97 |
| 1448618_at | Mvp | 1.97 |
| 1436365_at | Zbtb7c | 1.97 |
| 1418464_at | Matn4 | 1.97 |
| 1439093_at | Hspa4l | 1.97 |
| 1435193_at | A230050P20Rik | 1.97 |
| 1439065_ x_ at | Gm13152 | 1.97 |
| 1423306_at | 2010002N04Rik | 1.97 |
| 1430658_a_at | Gsdma2 | 1.97 |
| 1419469_at | Gnb4 | 1.96 |
| 1426725_s_at | Ets1 | 1.96 |
| 1417288_at | Plekha2 | 1.96 |
| 1456586_x_at | Mvp | 1.96 |
| 1426415_a_at | Trim25 | 1.95 |
| 1437249_at | Skap1 | 1.95 |
| 1433517_at | Myeov2 | 1.95 |
| 1425374_at | Oas3 | 1.95 |
| 1425681_a_at | Prnd | 1.95 |
| 1436659_at | Dclk1 | 1.94 |
| 1446326_at | Col1a2 | 1.94 |
| 1435596_at | Pion | 1.94 |
| 1416492_at | Ccne1 | 1.94 |
| 1422701_at | Zap70 | 1.93 |
| 1435517_x_at | Ralb | 1.93 |
| 1436902_x_at | Tmsb10 | 1.93 |
| 1425166_at | Rbl1 | 1.93 |
| 1421207_at | Lif | 1.93 |
| 1460134_at | 9330175E14Rik | 1.93 |
| 1420273_x_at | Samhd1 | 1.93 |
| 1443621_at | Xaf1 | 1.92 |
| 1425078_x_at | C130026I21Rik | 1.92 |
| 1427994_at | Cd300lf | 1.91 |
| 1456545_at | Il18rap | 1.91 |
| 1418346_at | Insl6 | 1.91 |
| 1428372_at | St5 | 1.91 |
| 1417045_at | Bid | 1.91 |
| 1433699_at | Tnfaip3 | 1.91 |
| 1440275_at | Runx3 | 1.91 |
| 1460253_at | Cmtm7 | 1.91 |
| 1421188_at | Ccr2 | 1.90 |
| 1427911_at | Tmeml73 | 1.90 |
| 1420653_at | Tgfb1 | 1.90 |
| 1437643_at | Cenpj | 1.90 |
| 1443687_x_at | H2-DMb2 | 1.90 |
| 1440299_at | E330016A19Rik | 1.90 |
| 1425151_a_at | Noxo1 | 1.90 |
| 1441097_at | Vangl1 | 1.90 |
| 1449988_at | Gimap1 | 1.89 |
| 1427404_x_at | Gm5506 | 1.89 |
| 1425617_at | Dhx9 | 1.88 |
| 1426926_at | Plcg2 | 1.88 |
| 1424037_at | Itpka | 1.88 |
| 1449925_at | Cxcr3 | 1.88 |
| 1435394_s_at | Rhoc | 1.87 |
| 1453392_at | Ttc39b | 1.87 |
| 1449473_s_at | Cd40 | 1.87 |
| 1419186_a_at | St8sia4 | 1.86 |
| 1455898_x_at | Slc2a3 | 1.86 |
| 1430447_a_at | Lair1 | 1.86 |
| 1418353_at | Cd5 | 1.85 |
| 1449425_at | Wnt2 | 1.85 |
| 1439114_at | Ddx60 | 1.85 |
| 1425084_at | Gimap7 | 1.85 |
| 1425819_at | Zbtb7c | 1.85 |
| 1416001_a_at | Cotl1 | 1.84 |
| 1422704_at | Gyk | 1.84 |
| 1445452_at | Traf1 | 1.84 |
| 1438852_x_at | Mcm6 | 1.84 |
| 1436423_at | Themis | 1.84 |
| 1417599_at | Cd276 | 1.84 |
| 1421930_at | Icos | 1.84 |
| 1421814_at | Msn | 1.83 |
| 1419132_at | Tlr2 | 1.83 |
| 1448568_a_at | Slc20a1 | 1.83 |
| 1423602_at | Traf1 | 1.83 |
| 1451931_ x _at | H2-L | 1.82 |
| 1456836_at | Itk | 1.82 |
| 1453129_a_at | Rgs12 | 1.82 |
| 1426454_at | Arhgdib | 1.82 |
| 1451385_at | Fam162a | 1.82 |
| 1419470_at | Gnb4 | 1.82 |
| 1435697_a_at | Cytip | 1.82 |
| 1424214_at | Parm1 | 1.81 |
| 1458524_at | Fndc3a | 1.81 |
| 1455051_at | Rnf31 | 1.81 |
| 1452163_at | Ets1 | 1.81 |
| 1448328_at | Sh3bp2 | 1.80 |
| 1456700_ x_ at | Marcks | 1.80 |
| 1451784_x_at | H2-D1 | 1.80 |
| 1437606_at | Btbd19 | 1.80 |
| 1439476_at | Dsg2 | 1.80 |
| 1440878_at | Runx1 | 1.79 |
| 1453472_a_at | Slamf7 | 1.79 |
| 1434653_at | Ptk2b | 1.79 |
| 1436708_x_at | Mcm4 | 1.79 |
| 1454592_at | 9430012M22Rik | 1.79 |
| 1433954_at | 4632419I22Rik | 1.78 |
| 1426315_a_at | 6330416G13Rik | 1.78 |
| 1451289_at | Dclk1 | 1.78 |
| 1443686_at | H2-DMb2 | 1.78 |
| 1449903_at | Crtam | 1.78 |
| 1418090_at | Plvap | 1.78 |
| 1460250_at | Sostdc1 | 1.77 |
| 1435749_at | Gda | 1.77 |
| 1450269_a_at | Pfkl | 1.77 |
| 1438910_a_at | Stom | 1.77 |
| 1437868_at | Fam46a | 1.77 |
| 1448449_at | Ripk3 | 1.77 |
| 1424496_at | 5133401N09Rik | 1.77 |
| 1438854_x at | Pitpnm1 | 1.77 |
| 1424930_s_at | Fam83f | 1.77 |
| 1460378_a_at | Tes | 1.77 |
| 1448914_a_at | Csf1 | 1.76 |
| 1448659_at | Casp7 | 1.76 |
| 1427689_a_at | Tnip1 | 1.76 |
| 1428242_at | Hmha1 | 1.76 |
| 1455975_x_at | Rnf114 | 1.76 |
| 1453076_at | Batf3 | 1.76 |
| 1419125_at | Ptpn18 | 1.76 |
| 1424524_at | Dram1 | 1.76 |
| 1453202_at | E330016A19Rik | 1.76 |
| 1428346_at | Trafd1 | 1.76 |
| 1451987_at | Arrb2 | 1.76 |
| 1417790_at | Dok1 | 1.76 |
| 1418244_at | Naa20 | 1.75 |
| 1416226_at | Arpc1b | 1.75 |
| 1438634_x_at | Lasp1 | 1.75 |
| 1419209_at | Cxcl1 | 1.75 |
| 1451363_a_at | Dennd2d | 1.75 |
| 1430622_at | 4833423F13Rik | 1.74 |
| 1447788_s_at | Tspyl3 | 1.74 |
| 1456310_a_at | 2610002J02Rik | 1.74 |
| 1455251_at | Itga1 | 1.74 |
| 1416750_at | Sigmar1 | 1.74 |
| 1421830_at | Ak4 | 1.74 |
| 1417346_at | Pycard | 1.74 |
| 1436958_x at | Tpm3 | 1.74 |
| 1434069_at | Prex1 | 1.74 |
| 1440249_at | Aknad1 | 1.74 |
| 1442849_at | Lrp1 | 1.74 |
| 1451174_at | Lrrc33 | 1.73 |
| 1451411_at | Gprc5b | 1.73 |
| 1460469_at | Tnfrsf9 | 1.73 |
| 1452661_at | Tfrc | 1.73 |
| 1430534_at | Rnase6 | 1.73 |
| 1456028_x at | Marcks | 1.73 |
| 1448731_at | Il10ra | 1.73 |
| 1418257_at | Slc12a7 | 1.73 |
| 1420170_at | Myh9 | 1.72 |
| 1424032_at | Hvcn1 | 1.72 |
| 1435748_at | Gda | 1.72 |
| 1451285_at | Fus | 1.72 |
| 1454607_s_at | Psat1 | 1.72 |
| 1434089_at | Synpo | 1.71 |
| 1421525_a_at | Naip5 | 1.71 |
| 1458415_at | Clec2e | 1.71 |
| 1419721_at | Niacr1 | 1.71 |
| 1453328_at | 2700008G24Rik | 1.71 |
| 1435337_at | Tshz3 | 1.71 |
| 1456011_x at | Acaa1a | 1.71 |
| 1423986_a_at | Shisa5 | 1.71 |
| 1427386_at | Arhgef16 | 1.70 |
| 1443123_at | Tanc2 | 1.70 |
| 1418674_at | Osmr | 1.70 |
| 1445588_at | Ankrd44 | 1.70 |
| 1424552_at | Casp8 | 1.70 |
| 1439261_ x _at | Mitd1 | 1.70 |
| 1452428_a_at | B2m | 1.70 |
| 1418045_at | Inpp1 | 1.70 |
| 1441317_x_at | Jakmip1 | 1.70 |
| 1436589_x_at | Prkd2 | 1.70 |
| 1437503_a_at | Shisa5 | 1.70 |
| 1421578_at | Ccl4 | 1.70 |
| 1435227_at | Bel11b | 1.70 |
| 1436177_at | Plekha2 | 1.70 |
| 1457644_s_at | Cxcl1 | 1.70 |
| 1429527_a_at | Plscr1 | 1.70 |
| 1433531_at | Acsl4 | 1.69 |
| 1420731_a_at | Csrp2 | 1.69 |
| 1438097_at | Rab20 | 1.69 |
| 1426239_s_at | Arrb2 | 1.69 |
| 1427182_s_at | D18Ertd653e | 1.69 |
| 1435338_at | Cdk6 | 1.69 |
| 1433885_at | Iqgap2 | 1.69 |
| 1450852_s_at | F2r | 1.69 |
| 1446507_at | Kif24 | 1.69 |
| 1450456_at | Il21r | 1.69 |
| 1418612_at | Slfn1 | 1.68 |
| 1438974_x_at | Pitpnm1 | 1.68 |
| 1439030_at | Gmppb | 1.68 |
| 1416619_at | 4632428N05Rik | 1.68 |
| 1441880_ x_ at | Tmem149 | 1.68 |
| 1448797_at | Elk3 | 1.68 |
| 1443794_x_at | Noc41 | 1.68 |
| 1452289_a_at | Rnf135 | 1.68 |
| 1450918_s_at | Src | 1.67 |
| 1420955_at | Vsnl1 | 1.67 |
| 1418751_at | Sit1 | 1.67 |
| 1447568_at | Gatc | 1.67 |
| 1460700_at | Stat3 | 1.67 |
| 1419911_at | Corolc | 1.66 |
| 1452966_at | Bel11b | 1.66 |
| 1421550_a_at | Trim34a | 1.66 |
| 1423686_a_at | Prr13 | 1.66 |
| 1455287_at | Cdk6 | 1.66 |
| 1439422_a_at | Fam132a | 1.66 |
| 1416935_at | Trpv2 | 1.66 |
| 1436183_at | Zc3hav1 | 1.66 |
| 1451314_a_at | Vcam1 | 1.66 |
| 1417300_at | Smpdl3b | 1.66 |
| 1419097_a_at | Stom | 1.66 |
| 1446939_at | Trim12a | 1.65 |
| 1423411_at | Rbm47 | 1.65 |
| 1450355_a_at | Capg | 1.65 |
| 1436220_at | Zfp287 | 1.65 |
| 1429742_at | Rcbtb2 | 1.65 |
| 1455221_at | Abeg1 | 1.65 |
| 1438934_x_at | Sema4a | 1.65 |
| 1452836_at | Lpin2 | 1.65 |
| 1427874_at | Rnf114 | 1.64 |
| 1452425_at | Tnfrsf14 | 1.64 |
| 1422303_a_at | Tnfrsfl8 | 1.64 |
| 1418396_at | Gpsm3 | 1.64 |
| 1451462_a_at | Ifnar2 | 1.64 |
| 1447711_x_at | 4933412E12Rik | 1.64 |
| 1454757_s_at | Ifi27l1 | 1.64 |
| 1424246_a_at | Tes | 1.64 |
| 1459872_x_at | H2-DMa | 1.64 |
| 1424304_at | Tpcn2 | 1.64 |
| 1416695_at | Tspo | 1.64 |
| 1451475_at | Plxnd1 | 1.64 |
| 1428727_at | Cepl92 | 1.64 |
| 1440779_s_at | Slc5a9 | 1.63 |
| 1433451_at | Cdk5r1 | 1.63 |
| 1450531_at | H2-B1 | 1.63 |
| 1458601_at | 8030447M02Rik | 1.63 |
| 1426739_at | Donson | 1.63 |
| 1423350_at | Socs5 | 1.63 |
| 1456467_s_at | Nlk | 1.63 |
| 1447803_ x_ at | Capg | 1.63 |
| 1453201_at | Rassf10 | 1.63 |
| 1438148_at | Cxl3 | 1.63 |
| 1444197_at | Rinl | 1.63 |
| 1428859_at | Paox | 1.63 |
| 1455656_at | Btla | 1.63 |
| 1455080_at | Ppp1r16b | 1.63 |
| 1424857_a_at | Trim34a | 1.62 |
| 1450140_a_at | Cdkn2a | 1.62 |
| 1438095_x_at | Noc4l | 1.62 |
| 1460231_at | Irf5 | 1.62 |
| 1426318_at | Serpinb1b | 1.62 |
| 1427348_at | Zc3h12a | 1.62 |
| 1451862_a_at | Prf1 | 1.62 |
| 1442944_at | C76555 | 1.62 |
| 1420635_a_at | Tcirg1 | 1.62 |
| 1435143_at | Elk3 | 1.62 |
| 1449623_at | Txnrd3 | 1.61 |
| 1458345_s_at | Colec11 | 1.61 |
| 1423812_s_at | Vopp1 | 1.61 |
| 1417561_at | Apoc1 | 1.61 |
| 1455818_at | 4930427A07Rik | 1.60 |
| 1426757_at | Ampd2 | 1.60 |
| 1418265_s_at | Irf2 | 1.60 |
| 1434185_at | Acaca | 1.60 |
| 1437327_ x_ at | Enoph1 | 1.60 |
| 1456240_x_at | Cdca4 | 1.60 |
| 1443086_at | Alcam | 1.60 |
| 1442018_at | Btg1 | 1.60 |
| 1418686_at | Oas1c | 1.60 |
| 1441843_s_at | 5230400M03Rik | 1.60 |
| 1443882_at | Slc26a2 | 1.60 |
| 1420909_at | Vegfe | 1.60 |
| 1439587_at | Lemd3 | 1.60 |
| 1433169_at | 5830456J23Rik | 1.60 |
| 1437451_at | Ecscr | 1.59 |
| 1436074_at | Nfkbid | 1.59 |
| 1437789_at | Birc6 | 1.59 |
| 1450716_at | Adamts1 | 1.59 |
| 1438828_at | Rapgef6 | 1.59 |
| 1453064_at | Etaa1 | 1.59 |
| 1439047_s_at | Recq1 | 1.59 |
| 1435316_at | Psma6 | 1.59 |
| 1428484_at | Osbp13 | 1.59 |
| 1417627_a_at | Limk1 | 1.59 |
| 1459884_at | Cox7c | 1.59 |
| 1455660_at | Csf2rb | 1.59 |
| 1448511_at | Ptprcap | 1.59 |
| 1440245_at | Ccdc88b | 1.59 |
| 1418587_at | Traf3 | 1.59 |
| 1424339_at | Oasl1 | 1.58 |
| 1416166_a_at | Prdx4 | 1.58 |
| 1427095_at | Cdcp1 | 1.58 |
| 1456288_at | Slfn5 | 1.58 |
| 1452239_at | Gt(ROSA)26Sor | 1.58 |
| 1449516_a_at | Rgs3 | 1.58 |
| 1424829_at | A830007P12Rik | 1.58 |
| 1454402_at | Zfp942 | 1.58 |
| 1434139_at | Parp 11 | 1.58 |
| 1428583_at | Nufip2 | 1.58 |
| 1451097_at | Vasp | 1.58 |
| 1458595_at | Intu | 1.58 |
| 1457550_at | 9530059O14Rik | 1.58 |
| 1419530_at | Il12b | 1.58 |
| 1447762_x_at | Car12 | 1.58 |
| 1417588_at | Galnt3 | 1.58 |
| 1437052_s_at | Slc2a3 | 1.58 |
| 1435084_at | C730049014Rik | 1.58 |
| 1419235_s_at | Helb | 1.57 |
| 1440147_at | Lgi2 | 1.57 |
| 1460347_at | Krt14 | 1.57 |
| 1452991_at | Chd2 | 1.57 |
| 1429758_at | 1700017B05Rik | 1.57 |
| 1449508_at | Il27ra | 1.57 |
| 1416087_at | Ap1s1 | 1.57 |
| 1438673_at | Slc4a7 | 1.57 |
| 1438750_at | Atrx | 1.57 |
| 1423869_s_at | Txnrd3 | 1.57 |
| 1420692_at | Il2ra | 1.57 |
| 1449227_at | Ch25h | 1.57 |
| 1425241_a_at | Wsb1 | 1.57 |
| 1450387_s_at | Ak4 | 1.56 |
| 1427164_at | Il13ra1 | 1.56 |
| 1455084_x_at | Shmt2 | 1.56 |
| 1452169_a_at | Dgkz | 1.56 |
| 1420505_a_at | Stxbp1 | 1.56 |
| 1455839_at | Uggt1 | 1.56 |
| 1428118_at | Lingo1 | 1.56 |
| 1427539_a_at | Zwint | 1.56 |
| 1418271_at | Bhlhe22 | 1.56 |
| 1447851_x_at | Atp10a | 1.56 |
| 1452837_at | Lpin2 | 1.56 |
| 1420171_s_at | Myh9 | 1.55 |
| 1439253_x_at | Prelid1 | 1.55 |
| 1452803_at | Glipr2 | 1.55 |
| 1456914_at | Slc16a4 | 1.55 |
| 1448202_x_at | Prelid1 | 1.55 |
| 1452391_at | Cxadr | 1.55 |
| 1435595_at | 1810011O10Rik | 1.55 |
| 1452903_at | 6230427J02Rik | 1.55 |
| 1451110_at | Egln1 | 1.55 |
| 1435564_at | Neurl1b | 1.55 |
| 1422489_at | Mogs | 1.55 |
| 1452337_at | 4930427A07Rik | 1.54 |
| 1459983_at | Impa2 | 1.54 |
| 1432997_at | 5830462P14Rik | 1.54 |
| 1457304_at | D13Ertd787e | 1.54 |
| 1417955_at | Ccdc71 | 1.54 |
| 1428034_a_at | Tnfrsf9 | 1.54 |
| 1439455_x_at | Capza1 | 1.54 |
| 1451608_a_at | Tspan33 | 1.54 |
| 1417878_at | E2f1 | 1.54 |
| 1448894_at | Akr1b8 | 1.54 |
| 1455067_at | Psd4 | 1.53 |
| 1457410_at | Arhgap5 | 1.53 |
| 1436848_x_at | Impa1 | 1.53 |
| 1436395_at | Card6 | 1.53 |
| 1436729_at | Afap1 | 1.53 |
| 1437185_s_at | Tmsb10 | 1.53 |
| 1428018_a_at | AF251705 | 1.53 |
| 1431528_at | 5830427D02Rik | 1.53 |
| 1425760_a_at | Pitpnm1 | 1.53 |
| 1418492_at | Grem2 | 1.53 |
| 1445201_at | Zfp53 | 1.53 |
| 1459894_at | Iqgap2 | 1.53 |
| 1438154_x_at | 2610002J02Rik | 1.53 |
| 1434573_at | Traf3ip3 | 1.53 |
| 1433739_at | Nol10 | 1.53 |
| 1434070_at | Jag1 | 1.52 |
| 1423826_at | Noc41 | 1.52 |
| 1416875_at | Parvg | 1.52 |
| 1447792_x_at | Gpr174 | 1.52 |
| 1417128_at | Plekho1 | 1.52 |
| 1427260_a_at | Tpm3 | 1.52 |
| 1416379_at | Panx1 | 1.52 |
| 1437239_x_at | Phc2 | 1.52 |
| 1451415_at | 1810011O10Rik | 1.52 |
| 1448713_at | Stat4 | 1.52 |
| 1421937_at | Dapp1 | 1.51 |
| 1433748_at | Zdhhc18 | 1.51 |
| 1454350_at | Intu | 1.51 |
| 1429128_x_at | Nfkb2 | 1.51 |
| 1418004_a_at | Tmem176b | 1.51 |
| 1423154_at | BC005537 | 1.51 |
| 1426677_at | Flna | 1.51 |
| 1448600_s_at | Vav3 | 1.51 |
| 1457539_at | D10Ertd709e | 1.51 |
| 1420914_at | Slco2a1 | 1.51 |
| 1429247_at | Anxa6 | 1.51 |
| 1452411_at | Lrrc1 | 1.51 |
| 1449619_s_at | Arhgap9 | 1.51 |
| 1420351_at | Tnfrsf4 | 1.51 |
| 1437242_at | Ttll12 | 1.51 |
| 1439444_x_at | Tmed10 | 1.50 |
| 1448933_at | Pcdhb17 | 1.50 |
| 1434219_at | Stim2 | 1.50 |
| 1455428_at | Fam53b | 1.50 |
| 1419226_at | Cd96 | 1.50 |
| 1435169_at | A930001N09Rik | 0.67 |
| 1451348_at | Deptor | 0.67 |
| 1425411_at | Arl4a | 0.67 |
| 1428864_at | Dusp8 | 0.67 |
| 1426522_at | Hadhb | 0.67 |
| 1417312_at | Dkk3 | 0.67 |
| 1418190_at | Pon1 | 0.67 |
| 1454078_a_at | Gal3st1 | 0.66 |
| 1425145_at | Il1rl1 | 0.66 |
| 1417421_at | S100a1 | 0.66 |
| 1460192_at | Osbp11a | 0.66 |
| 1419963_at | Deptor | 0.66 |
| 1415743_at | Hdac5 | 0.66 |
| 1435559_at | Myo6 | 0.66 |
| 1450395_at | Slc22a5 | 0.66 |
| 1428469_a_at | Dzip1 | 0.66 |
| 1429884_at | Srgap2 | 0.66 |
| 1450971_at | Gadd45b | 0.66 |
| 1436344_at | C2cd2 | 0.66 |
| 1418921_at | Cadm3 | 0.65 |
| 1421471_at | Npy1r | 0.65 |
| 1453571_at | Deptor | 0.65 |
| 1452283_at | Rassf8 | 0.65 |
| 1416268_at | Ets2 | 0.65 |
| 1423364_a_at | Aktip | 0.65 |
| 1453069_at | Pik3cb | 0.65 |
| 1422838_at | Kcnul | 0.65 |
| 1433977_at | Hs3st3b1 | 0.65 |
| 1426010_a_at | Epb4.113 | 0.65 |
| 1455029_at | Kif21a | 0.65 |
| 1460406_at | Pls1 | 0.65 |
| 1434581_at | 2410066E13Rik | 0.65 |
| 1422740_at | Tnfrsf21 | 0.65 |
| 1435396_at | Stxbp6 | 0.65 |
| 1419688_at | Gpc6 | 0.65 |
| 1418658_at | Fam82b | 0.65 |
| 1419457_at | Rgnef | 0.65 |
| 1443904_at | Fads6 | 0.65 |
| 1425186_at | Lmbrd1 | 0.65 |
| 1450286_at | Npr3 | 0.65 |
| 1425037_at | Fgd4 | 0.64 |
| 1437871_at | Pgm5 | 0.64 |
| 1416774_at | Wee1 | 0.64 |
| 1458129_at | Rora | 0.64 |
| 1433460_at | Ttc7b | 0.64 |
| 1451200_at | Kiflb | 0.64 |
| 1448320_at | Stim1 | 0.64 |
| 1452309_at | Cgnl1 | 0.64 |
| 1454824_s_at | Mtus1 | 0.64 |
| 1415981_at | Herpud2 | 0.64 |
| 1419505_a_at | Ggps1 | 0.64 |
| 1457686_at | Akap17b | 0.63 |
| 1437149_at | Slc6a6 | 0.63 |
| 1437661_at | AU021092 | 0.63 |
| 1443906_at | Cd55 | 0.63 |
| 1451714_a_at | Map2k3 | 0.63 |
| 1435693_at | Mall | 0.63 |
| 1451563_at | Emr4 | 0.63 |
| 1452333_at | Smarca2 | 0.63 |
| 1428835_at | Myh14 | 0.63 |
| 1433711_s_at | Sesn1 | 0.63 |
| 1448669_at | Dkk3 | 0.63 |
| 1416778_at | Sdpr | 0.63 |
| 1449082_at | Mfap5 | 0.63 |
| 1425891_a_at | Grtp1 | 0.63 |
| 1418250_at | Arl4d | 0.63 |
| 1458882_at | Serpinb8 | 0.62 |
| 1418714_at | Dusp8 | 0.62 |
| 1431216_s_at | Dnajc6 | 0.62 |
| 1426208_x_at | Plagl1 | 0.62 |
| 1425138_at | Gucalb | 0.62 |
| 1425318_a_at | Tmem116 | 0.62 |
| 1423176_at | Tob1 | 0.62 |
| 1425515_at | Pik3r1 | 0.62 |
| 1455665_at | Lonrf1 | 0.62 |
| 1426576_at | Sgms1 | 0.62 |
| 1440559_at | Hmga2-ps1 | 0.62 |
| 1417545_at | Trpv4 | 0.62 |
| 1418954_at | Camkk1 | 0.62 |
| 1424701_at | Pcdh20 | 0.62 |
| 1454881_s_at | Upk3b | 0.62 |
| 1419378_a_at | Fxyd2 | 0.62 |
| 1448926_at | Hoxa5 | 0.62 |
| 1437731_at | C2cd2 | 0.62 |
| 1436501_at | Mtus1 | 0.61 |
| 1450110_at | Adh7 | 0.61 |
| 1417013_at | Hspb8 | 0.61 |
| 1443969_at | Irs2 | 0.61 |
| 1450206_at | Dlc1 | 0.61 |
| 1428774_at | Gpc6 | 0.61 |
| 1422904_at | Fmo2 | 0.61 |
| 1425321_a_at | Clmn | 0.61 |
| 1426743_at | Appl2 | 0.61 |
| 1427945_at | Dpyd | 0.61 |
| 1428321_at | Eml1 | 0.60 |
| 1431226_a_at | Fndc4 | 0.60 |
| 1421848_at | Slc22a5 | 0.60 |
| 1428731_at | Usp54 | 0.60 |
| 1456939_at | Fam154b | 0.60 |
| 1424408_at | Lims2 | 0.60 |
| 1426139_a_at | Ccrl1 | 0.60 |
| 1426332_a_at | Cldn3 | 0.60 |
| 1416823_a_at | Osbpl1a | 0.60 |
| 1423994_at | Kif1b | 0.60 |
| 1415904_at | Lp1 | 0.60 |
| 1429656_at | Rhobtb1 | 0.60 |
| 1421158_at | Cgnl1 | 0.60 |
| 1456150_at | Jhdm1d | 0.60 |
| 1454604_s_at | Tspan12 | 0.59 |
| 1434307_at | Tmem64 | 0.59 |
| 1431188_a_at | Tom1 | 0.59 |
| 1416926_at | Trp53inp1 | 0.59 |
| 1423447_at | Clpx | 0.59 |
| 1451782_a_at | Slc29a1 | 0.59 |
| 1423174_a_at | Pard6b | 0.59 |
| 1436293_x_at | Ildr2 | 0.59 |
| 1444073_at | Maf | 0.59 |
| 1447462_at | D7Wsu130e | 0.59 |
| 1424280_at | Mospd1 | 0.59 |
| 1451270_at | Dusp18 | 0.59 |
| 1451207_at | Micu1 | 0.59 |
| 1455056_at | Lmo7 | 0.59 |
| 1437409_s_at | Gpr126 | 0.59 |
| 1436988_at | Ism1 | 0.58 |
| 1420718_at | Odz2 | 0.58 |
| 1425456_a_at | Map2k3 | 0.58 |
| 1450318_a_at | P2rv2 | 0.58 |
| 1436853_a_at | Snca | 0.58 |
| 1424437_s_at | Abcg4 | 0.58 |
| 1426285_at | Lama2 | 0.58 |
| 1417962_s_at | Ghr | 0.58 |
| 1455340_at | Dennd5b | 0.58 |
| 1415874_at | Spry1 | 0.58 |
| 1435105_at | Rnf208 | 0.58 |
| 1428240_at | Nrxn1 | 0.58 |
| 1425906_a_at | Sema3e | 0.58 |
| 1454926_at | Sphkap | 0.58 |
| 1416316_at | Slc27a2 | 0.58 |
| 1434917_at | Cobl | 0.57 |
| 1429778_at | Optn | 0.57 |
| 1436350_at | Fam171b | 0.57 |
| 1452207_at | Cited2 | 0.57 |
| 1428547_at | Nt5e | 0.57 |
| 1426263_at | Cadm4 | 0.57 |
| 1429036_at | Otop3 | 0.57 |
| 1435763_at | Tbcldl6 | 0.57 |
| 1420942_s_at | Rgs5 | 0.57 |
| 1450974_at | Timp4 | 0.57 |
| 1434639_at | Klhl29 | 0.56 |
| 1421346_a_at | Slc6a6 | 0.56 |
| 1433742_at | Kank1 | 0.56 |
| 1435207_at | Dixdc1 | 0.56 |
| 1450838_x_at | Rpl37 | 0.56 |
| 1457671_at | Homer2 | 0.56 |
| 1422008_a_at | Aqp3 | 0.56 |
| 1415864_at | Bpgm | 0.56 |
| 1437181_at | Peli2 | 0.56 |
| 1416072_at | Cd34 | 0.56 |
| 1460514_s_at | Ascl2 | 0.56 |
| 1420941_at | Rgs5 | 0.56 |
| 1423966_at | Cd99l2 | 0.55 |
| 1435435_at | Cttnbp2 | 0.55 |
| 1428336_at | Agpat4 | 0.55 |
| 1419458_at | Rgnef | 0.55 |
| 1424567_at | Tspan2 | 0.55 |
| 1419491_at | Defb1 | 0.55 |
| 1415865_s_at | Bpgm | 0.55 |
| 1422667_at | Krt15 | 0.55 |
| 1417355_at | Peg3 | 0.55 |
| 1439478_at | Acot2 | 0.55 |
| 1450460_at | Aqp3 | 0.55 |
| 1426510_at | Sccpdh | 0.55 |
| 1422996_at | Acot2 | 0.55 |
| 1433454_at | Abtb2 | 0.55 |
| 1436640_x_at | Agpat4 | 0.55 |
| 1453008_at | Trnp1 | 0.55 |
| 1424392_at | Adhfe1 | 0.54 |
| 1417332_at | Rfx2 | 0.54 |
| 1419197_x_at | Hamp | 0.54 |
| 1429001_at | Pir | 0.54 |
| 1438496_a_at | Ddx26b | 0.54 |
| 1454699_at | Sesn1 | 0.54 |
| 1437217_at | Ankrd6 | 0.54 |
| 1418412_at | Tpd5211 | 0.54 |
| 1424747_at | Kiflc | 0.54 |
| 1454646_at | Tcp11l2 | 0.54 |
| 1421040_a_at | Gsta2 | 0.53 |
| 1434326_x_at | Coro2b | 0.53 |
| 1417027_at | Trim2 | 0.53 |
| 1460242_at | Cd55 | 0.53 |
| 1453119_at | Otud1 | 0.53 |
| 1420688_a_at | Sgce | 0.53 |
| 1450119_at | Dst | 0.53 |
| 1448119_at | Bpgm | 0.53 |
| 1456481_at | Esyt3 | 0.53 |
| 1422168_a_at | Bdnf | 0.53 |
| 1434542_at | Gpt2 | 0.52 |
| 1422905_s_at | Fmo2 | 0.52 |
| 1425809_at | Fabp4 | 0.52 |
| 1439549_at | Prrg3 | 0.52 |
| 1448416_at | Mgp | 0.52 |
| 1437197_at | Sorbs2 | 0.52 |
| 1422619_at | Ppap2a | 0.52 |
| 1436737_a_at | Sorbs1 | 0.52 |
| 1422620_s_at | Ppap2a | 0.52 |
| 1427378_at | Krt75 | 0.52 |
| 1416779_at | Sdpr | 0.51 |
| 1451501_a_at | Ghr | 0.51 |
| 1423506_a_at | Nnat | 0.51 |
| 1422974_at | Nt5e | 0.51 |
| 1425761_a_at | Nfatc1 | 0.51 |
| 1458268_s_at | Igfbp3 | 0.51 |
| 1460241_a_at | St3gal5 | 0.50 |
| 1417028_a_at | Trim2 | 0.50 |
| 1456397_at | Cdh4 | 0.50 |
| 1434156_at | Rab11fip4 | 0.50 |
| 1429024_at | Rbm20 | 0.50 |
| 1441254_at | Pard3b | 0.50 |
| 1419289_a_at | Syngr1 | 0.50 |
| 1422112_at | Ccbp2 | 0.50 |
| 1444559_at | Phtf2 | 0.50 |
| 1451410_a_at | Crip3 | 0.50 |
| 1420017_at | Tspan8 | 0.50 |
| 1416194_at | Cyp4b1 | 0.50 |
| 1434013_at | Ablim3 | 0.49 |
| 1419706_a_at | Akap12 | 0.49 |
| 1451762_a_at | Kif1b | 0.49 |
| 1427371_at | Abca8a | 0.49 |
| 1424007_at | Gdf10 | 0.49 |
| 1424155_at | Fabp4 | 0.48 |
| 1428357_at | 2610019F03Rik | 0.48 |
| 1454654_at | Dirc2 | 0.48 |
| 1456796_at | Snai3 | 0.48 |
| 1417702_a_at | Hnmt | 0.48 |
| 1450738_at | Kif21a | 0.48 |
| 1422007_at | Aqp3 | 0.48 |
| 1417014_at | Hspb8 | 0.47 |
| 1438422_at | Lrrc20 | 0.47 |
| 1417029_a_at | Trim2 | 0.47 |
| 1417673_at | Grb14 | 0.47 |
| 1428471_at | Sorbs 1 | 0.47 |
| 1439117_at | Clmn | 0.47 |
| 1427946_s_at | Dpyd | 0.46 |
| 1418317_at | Lhx2 | 0.46 |
| 1424367_a_at | Homer2 | 0.46 |
| 1454691_at | Nrxn1 | 0.46 |
| 1419082_at | Serpinb2 | 0.46 |
| 1434651_a_at | Cldn3 | 0.46 |
| 1460569_x_at | Cldn3 | 0.45 |
| 1431056_a_at | Lp1 | 0.45 |
| 1443832_s_at | Sdpr | 0.45 |
| 1451701_xat | Cldn3 | 0.45 |
| 1419490_at | Fam19a5 | 0.45 |
| 1428792_at | Bcas1 | 0.45 |
| 1419492_s_at | Defb1 | 0.45 |
| 1424393_s_at | Adhfe1 | 0.44 |
| 1448978_at | Ngef | 0.44 |
| 1429344_at | 9.13E+15 | 0.44 |
| 1437637_at | Phtf2 | 0.44 |
| 1425826_a_at | Sorbs1 | 0.44 |
| 1451620_at | Clql3 | 0.44 |
| 1450759_at | Bmp6 | 0.43 |
| 1433638_s_at | Hoxd8 | 0.43 |
| 1415996_at | Txnip | 0.43 |
| 1448551_aat | Trim2 | 0.43 |
| 1426981_at | Pcsk6 | 0.43 |
| 1427673_a_at | Sema3e | 0.43 |
| 1451527_at | Pcolce2 | 0.43 |
| 1434877_at | Nptx1 | 0.42 |
| 1453435_a_at | Fmo2 | 0.42 |
| 1449621_s_at | Thsd1 | 0.42 |
| 1431805_a_at | Rhpn2 | 0.42 |
| 1449824_at | Prg4 | 0.41 |
| 1416129_at | Errfi1 | 0.41 |
| 1434628_a_at | Rhpn2 | 0.41 |
| 1455214_at | Mitf | 0.41 |
| 1427345_a_at | Sult1a1 | 0.41 |
| 1418205_at | Thsd1 | 0.40 |
| 1419414_at | Gng13 | 0.40 |
| 1437840_s_at | D2hgdh | 0.40 |
| 1423635_at | Bmp2 | 0.40 |
| 1423679_at | 2810432L12Rik | 0.39 |
| 1418063_at | Kera | 0.39 |
| 1434667_at | Col8a2 | 0.39 |
| 1424649_a_at | Tspan8 | 0.39 |
| 1446498_at | Il20ra | 0.39 |
| 1441918_x_at | Serpinb6e | 0.39 |
| 1415997_at | Txnip | 0.39 |
| 1431833_a_at | Hmgcs2 | 0.38 |
| 1418589_a_at | Mlfl | 0.38 |
| 1419717_at | Sema3e | 0.38 |
| 1429166_s_at | Clmn | 0.38 |
| 1435459_at | Fmo2 | 0.38 |
| 1434669_at | Ralgps1 | 0.38 |
| 1431099_at | Hoxd8 | 0.38 |
| 1434657_at | Gls | 0.37 |
| 1418595_at | Plin4 | 0.36 |
| 1434474_at | Abca5 | 0.36 |
| 1417168_a_at | Usp2 | 0.36 |
| 1427029_at | Htra3 | 0.35 |
| 1456895_at | Cd209b | 0.34 |
| 1416225_at | Adh1 | 0.34 |
| 1454969_at | Lypd6 | 0.34 |
| 1456741_s_at | Gpm6a | 0.34 |
| 1442226_at | Sema3e | 0.33 |
| 1451478_at | Angpt17 | 0.33 |
| 1418706_at | Slc38a3 | 0.32 |
| 1436279_at | Slc26a7 | 0.32 |
| 1460244_at | Upb1 | 0.32 |
| 1459737_s_at | Ttr | 0.31 |
| 1419816_s_at | Errfi1 | 0.31 |
| 1417765_a_at | Amy1 | 0.30 |
| 1446681_at | BB086117 | 0.30 |
| 1433837_at | 8430408G22Rik | 0.30 |
| 1454965_at | Fam171b | 0.29 |
| 1433596_at | Dnajc6 | 0.29 |
| 1425357_a_at | Grem1 | 0.29 |
| 1455913_x_at | Ttr | 0.29 |
| 1433836_a_at | 8430408G22Rik | 0.28 |
| 1451382_at | Chacl | 0.28 |
| 1454608_x_at | Ttr | 0.26 |
| 1433877_at | Fam46b | 0.26 |
| 1425281_a_at | Tsc22d3 | 0.25 |
| 1451535_at | Il31ra | 0.24 |
| 1423405_at | Timp4 | 0.23 |
| 1435184_at | Npr3 | 0.23 |
| 1437665_at | Il22ra2 | 0.22 |
| 1456512_at | Pdzrn4 | 0.22 |
| 1417788_at | Sncg | 0.21 |
| 1460012_at | Wfdc3 | 0.20 |
| 1452543_a_at | Scgb1a1 | 0.19 |
| 1419332_at | Egfl6 | 0.19 |
| 1436643_x_at | Hamp2 | 0.18 |
| 1453327_at | Krt24 | 0.17 |
| 1456487_at | Adcy1 | 0.14 |
| 1449545_at | Fgf18 | 0.12 |

**Table 4.** Differentially expressed genes in skin from human alopecia areata vs. skin from normal individuals. Perilesional punch biopsies from 5 patients with patchy alopecia areata who were not undergoing local or systemic treatments were collected and compared to scalp biopsies from 5 unrelated unaffected individuals.

| **Affy_ID** | **GeneSymbol** | **FC** |
|---|---|---|
| 221728_x_at | XIST | 56.06 |
| 211696_x_at | HBB | 13.20 |
| 204533_at | CXCL10 | 12.37 |
| 203915_at | CXCL9 | 9.53 |
| 204580_at | MMP 12 | 4.04 |
| 235763_at | SLC44A5 | 3.83 |
| 205488_at | GZMA | 3.65 |
| 223809_at | RGS18 | 3.54 |
| 201858_s_at | SRGN | 3.33 |
| 215784_at | CD1E | 3.19 |
| 216714_at | CCL13 | 3.15 |
| 232024_at | GIMAP2 | 3.13 |
| 1555745_a_at | LYZ | 3.09 |
| 205758_at | CD8A | 3.04 |
| 214059_at | IFI44 | 3.03 |
| 226736_at | CHURC1 | 3.02 |
| 206749_at | CD1B | 2.96 |
| 242943_at | ST8SIA4 | 2.94 |
| 204439_at | IFI44L | 2.88 |
| 210656_at | EED | 2.87 |
| 220122_at | MCTP1 | 2.78 |
| 210164_at | GZMB | 2.75 |
| 206134_at | ADAMDEC1 | 2.70 |
| 209875_s_at | SPP1 | 2.69 |
| 206666_at | GZMK | 2.68 |
| 204110_at | HNMT | 2.67 |
| 217147_s_at | TRAT1 | 2.66 |
| 204057_at | IRF8 | 2.64 |
| 212588_at | PTPRC | 2.63 |
| 214467_at | GPR65 | 2.60 |
| 1554899_s_at | FCER1G | 2.60 |
| 242557_at | ZNRD1-AS1 | 2.58 |
| 209541_at | IGF1 | 2.58 |
| 205991_s at | PRRX1 | 2.56 |
| 228956_at | UGT8 | 2.55 |
| 207651_at | GPR171 | 2.54 |
| 216598_s_at | CCL2 | 2.54 |
| 211742_s_at | EVI2B | 2.52 |
| 231879_at | COL12A1 | 2.52 |
| 1555827_at | CCNL1 | 2.51 |
| 1552398_a_at | CLEC12A | 2.50 |
| 213797_at | RSAD2 | 2.49 |
| 211559_s_at | CCNG2 | 2.47 |
| 209612_s_at | ADH1B | 2.45 |
| 230422_at | FPR3 | 2.44 |
| 221978_at | HLA-F | 2.44 |
| 212230_at | PPAP2B | 2.44 |
| 210198_s_at | PLP1 | 2.43 |
| 220330_s_at | SAMSN1 | 2.42 |
| 203561_at | FCGR2A | 2.41 |
| 239345_at | SLC19A3 | 2.41 |
| 227609_at | EPSTI1 | 2.41 |
| 205226_at | PDGFRL | 2.37 |
| 1553678_a_at | ITGB1 | 2.37 |
| 206247_at | MICB | 2.37 |
| 208016_s_at | AGTR1 | 2.36 |
| 1405_i_at | CCL5 | 2.36 |
| 214972_at | MGEA5 | 2.35 |
| 204774_at | EVI2A | 2.34 |
| 1557116_at | APOL6 | 2.34 |
| 1558604_a_at | SSBP2 | 2.33 |
| 208894_at | HLA-DRA | 2.32 |
| 219607_s_at | MS4A4A | 2.32 |
| 238461_at | EIF4E3 | 2.31 |
| 222587_s_at | GALNT7 | 2.30 |
| 214617_at | PRF1 | 2.30 |
| 204222_s_at | GLIPR1 | 2.29 |
| 205039_s_at | IKZF1 | 2.29 |
| 215073_s_at | NR2F2 | 2.29 |
| 220477_s_at | TMEM230 | 2.29 |
| 205270_s_at | LCP2 | 2.29 |
| 226757_at | IFIT2 | 2.28 |
| 1555229_a_at | C1S | 2.28 |
| 1559584_a_at | C16orf54 | 2.28 |
| 203504_s_at | ABCA1 | 2.28 |
| 202902_s_at | CTSS | 2.27 |
| 1552612_at | CDC42SE2 | 2.27 |
| 205158_at | RNASE4 | 2.26 |
| 203708_at | PDE4B | 2.26 |
| 206271_at | TLR3 | 2.25 |
| 205992_s_at | IL15 | 2.24 |
| 228938_at | MBP | 2.24 |
| 1554712_a_at | GLYATL2 | 2.24 |
| 214279_s_at | NDRG2 | 2.23 |
| 205831_at | CD2 | 2.22 |
| 239364_at | ETV6 | 2.22 |
| 206070_s_at | EPHA3 | 2.22 |
| 224976_at | NFIA | 2.22 |
| 229584_at | LRRK2 | 2.21 |
| 206693_at | IL7 | 2.20 |
| 1558820_a_at | C18orf34 | 2.20 |
| 213882_at | TM2D1 | 2.20 |
| 229450_at | IFIT3 | 2.19 |
| 203680_ at | PRKAR2B | 2.17 |
| 210889_s_at | FCGR2B | 2.17 |
| 227458_at | CD274 | 2.17 |
| 203000_at | STMN2 | 2.16 |
| 226603_at | SAMD9L | 2.16 |
| 208885_at | LCP1 | 2.15 |
| 222726_s_at | EXOC5 | 2.15 |
| 222722_at | OGN | 2.15 |
| 1558972_s_at | THEMIS | 2.14 |
| 204118_at | CD48 | 2.13 |
| 217649_at | ZFAND5 | 2.13 |
| 204972_at | OAS2 | 2.12 |
| 231577_s_at | GBP1 | 2.11 |
| 228218_at | LSAMP | 2.11 |
| 230867_at | COL6A6 | 2.11 |
| 229383_at | 1-Mar | 2.11 |
| 227677_at | JAK3 | 2.10 |
| 230391_at | CD84 | 2.09 |
| 204923_at | SASH3 | 2.09 |
| 203416_at | CD53 | 2.08 |
| 204116_at | IL2RG | 2.08 |
| 206307_ s_at | FOXD1 | 2.08 |
| 206120_ at | CD33 | 2.08 |
| 204563_at | SELL | 2.07 |
| 225957_at | CREBRF | 2.07 |
| 220416_at | ATP8B4 | 2.07 |
| 200605_ s_at | PRKAR1A | 2.07 |
| 209006_s_at | C1orf63 | 2.07 |
| 232911_at | ZFP14 | 2.06 |
| 1556209_at | CLEC2B | 2.06 |
| 1555938_x_at | VIM | 2.05 |
| 219179_at | DACT1 | 2.05 |
| 206366_x_at | XCL1 | 2.05 |
| 235737_at | TSLP | 2.04 |
| 206978_at | CCR2 | 2.04 |
| 235175_at | GBP4 | 2.04 |
| 220868_s_at | SLC7A10 | 2.03 |
| 211676_s_at | IFNGR1 | 2.03 |
| 214887_at | N4BP2L1 | 2.03 |
| 230206_at | DOCK5 | 2.03 |
| 203100_ s_at | CDYL | 2.02 |
| 203471_s_at | PLEK | 2.02 |
| 208925_at | CLDND1 | 2.02 |
| 202820_ at | AHR | 2.02 |
| 231366_at | FDPSL2A | 2.02 |
| 204607_at | HMGCS2 | 2.01 |
| 220199_s_at | AIDA | 2.01 |
| 204070_at | RARRES3 | 2.01 |
| 227870_ at | IGDCC4 | 2.01 |
| 204865_at | CA3 | 2.01 |
| 205842_s_at | JAK2 | 2.00 |
| 241925_x_at | SLC16A7 | 2.00 |
| 214533_at | CMA1 | 2.00 |
| 209770_at | BTN3A1 | 1.99 |
| 229543_at | FAM26F | 1.99 |
| 226847_at | FST | 1.99 |
| 210971_s at | ARNTL | 1.98 |
| 204279_at | PSMB9 | 1.98 |
| 205291_at | IL2RB | 1.98 |
| 204959_at | MNDA | 1.98 |
| 227467_at | RDH10 | 1.98 |
| 206545_at | CD28 | 1.98 |
| 203758_at | CTSO | 1.98 |
| 214724_at | DIXDC1 | 1.98 |
| 216231_s_at | B2M | 1.97 |
| 210029_at | IDO1 | 1.97 |
| 206914_at | CRTAM | 1.97 |
| 211734_s_at | FCER1A | 1.97 |
| 218404 _at | SNX10 | 1.96 |
| 206960_at | LPAR4 | 1.96 |
| 202742_s_at | PRKACB | 1.96 |
| 213293_s_at | TRIM22 | 1.96 |
| 1555778_a_at | POSTN | 1.96 |
| 227266_s_at | FYB | 1.96 |
| 217465_at | NCKAP1 | 1.95 |
| 244774_at | PHACTR2 | 1.95 |
| 232392_at | SRSF3 | 1.94 |
| 213193_x_at | TRBC 1 | 1.94 |
| 216511_s_at | TCF7L2 | 1.94 |
| 228504_at | SCN7A | 1.93 |
| 201151_s_at | MBNL1 | 1.93 |
| 242268_at | CELF2 | 1.92 |
| 220777_at | KIF13A | 1.92 |
| 205987_at | CD1C | 1.92 |
| 201008_s_at | TXNIP | 1.92 |
| 202527_s_at | SMAD4 | 1.91 |
| 224480_s_at | AGPAT9 | 1.91 |
| 223220_s_at | PARP9 | 1.91 |
| 209960_at | HGF | 1.91 |
| 210057_at | SMG1 | 1.91 |
| 203305_at | F13A1 | 1.91 |
| 213649_at | SRSF7 | 1.90 |
| 226392_at | RASA2 | 1.90 |
| 205159_at | CSF2RB | 1.90 |
| 213435_at | SATB2 | 1.90 |
| 226743_at | SLFN11 | 1.90 |
| 204205_at | APOBEC3G | 1.90 |
| 203936_s_at | MMP9 | 1.90 |
| 223501_at | TNFSF13B | 1.90 |
| 212208 _at | MED13L | 1.89 |
| 203966_s_at | PPM1A | 1.89 |
| 204438_at | MRC1 | 1.89 |
| 235458_at | HAVCR2 | 1.89 |
| 207610s_at | EMR2 | 1.89 |
| 222859_s_at | DAPP1 | 1.89 |
| 1552628_a_at | HERPUD2 | 1.89 |
| 221207s_at | NBEA | 1.88 |
| 205101 _at | CIITA | 1.88 |
| 205083_at | AOX1 | 1.88 |
| 230023_at | NSUN4 | 1.88 |
| 214450_at | CTSW | 1.88 |
| 1568752_s_at | RGS13 | 1.88 |
| 237919_at | RFFL | 1.88 |
| 232898_at | DAB2 | 1.87 |
| 234975_at | GSPT1 | 1.87 |
| 227198_at | AFF3 | 1.87 |
| 205799_s_at | SLC3A1 | 1.87 |
| 242974_at | CD47 | 1.87 |
| 1555852_at | LOC100507463 | 1.87 |
| 201604_s_at | PPP1R12A | 1.87 |
| 244455_at | KCNT2 | 1.86 |
| 210163_at | CXCL11 | 1.86 |
| 219279_at | DOCK10 | 1.86 |
| 203165_s_at | SLC33A1 | 1.86 |
| 222161_at | NAALAD2 | 1.86 |
| 221724_s_at | CLEC4A | 1.86 |
| 226841_at | MPEG 1 | 1.86 |
| 229713_at | PIP4K2A | 1.85 |
| 231776_at | EOMES | 1.85 |
| 209040_ s_at | PSMB8 | 1.85 |
| 221760_at | MAN1A1 | 1.85 |
| 215303_at | DCLK1 | 1.85 |
| 208191_x_at | PSG4 | 1.85 |
| 228057_at | DDIT4L | 1.85 |
| 230364_at | CHPT1 | 1.85 |
| 206637_at | P2RY14 | 1.84 |
| 218806_s_at | VAV3 | 1.84 |
| 224451_x_at | ARHGAP9 | 1.84 |
| 215363_x_at | FOLH1 | 1.84 |
| 212298_at | NRP1 | 1.84 |
| 206082_at | HCP5 | 1.84 |
| 223271_s_at | CTDSPL2 | 1.84 |
| 222235_s_at | CSGALNACT2 | 1.84 |
| 237753_at | IL21R | 1.84 |
| 210895_s_at | CD86 | 1.84 |
| 201619_at | PRDX3 | 1.84 |
| 237189_at | HOXB-AS1 | 1.84 |
| 1552658_a_at | NAV3 | 1.84 |
| 205803_s_at | TRPC1 | 1.83 |
| 208121_s_at | PTPRO | 1.83 |
| 243352_at | ALPK1 | 1.83 |
| 1555749_at | SF1 | 1.83 |
| 227629_at | PRLR | 1.83 |
| 210896_s_at | ASPH | 1.83 |
| 211671_s_at | NR3C1 | 1.83 |
| 221423_s_at | YIPF5 | 1.82 |
| 204821_at | BTN3A3 | 1.82 |
| 204951_at | RHOH | 1.82 |
| 219093_at | PID1 | 1.82 |
| 230177_at | GTF2H2B | 1.82 |
| 234305_s_at | GSDMC | 1.82 |
| 204149_s_at | GSTM4 | 1.82 |
| 221428_s_at | TBL1XR1 | 1.82 |
| 219427_at | FAT4 | 1.82 |
| 1565939_at | C5orf22 | 1.81 |
| 238725_at | IRF1 | 1.81 |
| 205681_at | BCL2A1 | 1.81 |
| 203302_at | DCK | 1.81 |
| 220005_at | P2RY13 | 1.80 |
| 208671_at | SERINC1 | 1.80 |
| 215318_at | MINOS1P1 | 1.80 |
| 223217_s_at | NFKBIZ | 1.80 |
| 242172_at | MEIS1 | 1.80 |
| 214038_at | CCL8 | 1.80 |
| 201688_s_at | TPD52 | 1.80 |
| 239384_at | FLJ44342 | 1.80 |
| 214917_at | PRKAA1 | 1.80 |
| 201720_ s_at | LAPTM5 | 1.80 |
| 1554462_a_at | DNAJB9 | 1.79 |
| 226702_at | CMPK2 | 1.79 |
| 208131_s_at | PTGIS | 1.79 |
| 239297_at | KIAA1456 | 1.79 |
| 223802_s_at | RBBP6 | 1.79 |
| 1555193_a_at | ZNF277 | 1.78 |
| 225415_at | DTX3L | 1.78 |
| 203932_at | HLA-DMB | 1.78 |
| 203320_at | SH2B3 | 1.78 |
| 228704_s_at | CLDN23 | 1.78 |
| 228094_at | AMICA1 | 1.78 |
| 204955_at | SRPX | 1.77 |
| 206752_s_at | DFFB | 1.77 |
| 1557071_s_at | NUB1 | 1.77 |
| 204011_at | SPRY2 | 1.77 |
| 210280_ at | MPZ | 1.77 |
| 214855_s_at | RALGAPA1 | 1.77 |
| 227900_at | CBLB | 1.77 |
| 1558523_at | FAM184A | 1.77 |
| 205280_ at | GLRB | 1.77 |
| 228225_at | PEX2 | 1.77 |
| 218986_s_at | DDX60 | 1.76 |
| 210184_at | ITGAX | 1.76 |
| 242900_at | ALG10B | 1.76 |
| 213016_at | BBX | 1.76 |
| 208146_s_at | CPVL | 1.76 |
| 1556498_at | FAM69A | 1.76 |
| 206420_at | IGSF6 | 1.76 |
| 205660_at | OASL | 1.76 |
| 225700_ at | GLCCI1 | 1.76 |
| 1554479_a_at | CARD8 | 1.76 |
| 210176_at | TLR1 | 1.76 |
| 203157_s_at | GLS | 1.76 |
| 207113_s_at | TNF | 1.76 |
| 228617_at | XAF1 | 1.76 |
| 205907_s_at | OMD | 1.76 |
| 202411_at | IFI27 | 1.75 |
| 204567_s_at | ABCG1 | 1.75 |
| 209788_s_at | ERAP1 | 1.75 |
| 213426_s_at | CAV2 | 1.75 |
| 225980_at | C14orf43 | 1.75 |
| 220560_at | C11orf21 | 1.75 |
| 1559265_at | SKIDA1 | 1.75 |
| 238858_at | TIFA | 1.75 |
| 210538_s_at | BIRC3 | 1.75 |
| 207957_s_at | PRKCB | 1.75 |
| 207175_ at | ADIPOQ | 1.74 |
| 229866_at | STK32A | 1.74 |
| 216198_at | ATF7IP | 1.74 |
| 235228_at | CCDC85A | 1.74 |
| 205821_at | KLRK1 | 1.74 |
| 203387_s_at | TBC1D4 | 1.74 |
| 226459_at | PIK3AP1 | 1.74 |
| 233898_s_at | FGFR1OP2 | 1.74 |
| 220103_s_at | MRPS18C | 1.73 |
| 239921_at | COL28A1 | 1.73 |
| 227910_at | XPNPEP3 | 1.73 |
| 203741_s_at | ADCY7 | 1.73 |
| 229431_at | RFXAP | 1.73 |
| 226641_at | ANKRD44 | 1.73 |
| 205934_at | PLCL1 | 1.73 |
| 225975_at | PCDH18 | 1.73 |
| 215990_ s_at | BCL6 | 1.73 |
| 201417_at | SOX4 | 1.73 |
| 241440_ at | FLJ30375 | 1.73 |
| 231955_s_at | HIBADH | 1.72 |
| 234165_at | PTGDR | 1.72 |
| 219292_at | THAP1 | 1.72 |
| 1567628_at | CD74 | 1.72 |
| 203434_s_at | MME | 1.72 |
| 211922_s_at | CAT | 1.72 |
| 210571_s_at | CMAHP | 1.72 |
| 206030_ at | ASPA | 1.72 |
| 1554547_at | FAM13C | 1.72 |
| 207977_s_at | DPT | 1.72 |
| 219230_at | TMEM100 | 1.72 |
| 213475_s_at | ITGAL | 1.72 |
| 227529_s_at | AKAP12 | 1.72 |
| 227148_at | PLEKHH2 | 1.72 |
| 243407_at | MFSD8 | 1.72 |
| 223620_at | GPR34 | 1.72 |
| 214181_x_at | LST1 | 1.72 |
| 208510_s_at | PPARG | 1.72 |
| 235165_at | PARD6B | 1.71 |
| 218400_at | OAS3 | 1.71 |
| 228826_at | BZRAP1-AS1 | 1.71 |
| 206098_at | ZBTB6 | 1.71 |
| 209869_at | ADRA2A | 1.71 |
| 1552497_a_at | SLAMF6 | 1.71 |
| 204614_at | SERPINB2 | 1.71 |
| 213496_at | LPPR4 | 1.71 |
| 244395_at | FLJ41455 | 1.71 |
| 225163_at | FRMD4A | 1.71 |
| 219528_s_at | BCL11B | 1.71 |
| 220014_ at | PRR16 | 1.71 |
| 221024_s_at | SLC2A10 | 1.71 |
| 229523_at | TMEM200C | 1.71 |
| 201951_at | ALCAM | 1.71 |
| 222538_s_at | APPL1 | 1.71 |
| 222791_at | RSBN1 | 1.71 |
| 229568_at | MOB3B | 1.71 |
| 238449_at | LOC595101 | 1.70 |
| 1555349_a_at | ITGB2 | 1.70 |
| 217816_s_at | PCNP | 1.70 |
| 1556364_at | ADAMTS9-AS2 | 1.70 |
| 228368_at | ARHGAP20 | 1.70 |
| 206157_at | PTX3 | 1.70 |
| 242852_at | LOC285147 | 1.70 |
| 207606_s_at | ARHGAP12 | 1.69 |
| 210425_x_at | GOLGA8B | 1.69 |
| 224583_at | COTL1 | 1.69 |
| 222839_s_at | PAPOLG | 1.69 |
| 1557954_at | TXLNG | 1.69 |
| 213289_at | APOOL | 1.69 |
| 225579_at | PQLC3 | 1.69 |
| 1554464_a_at | CRTAP | 1.69 |
| 228618_at | PEAR1 | 1.69 |
| 233005_at | ZNHIT6 | 1.68 |
| 203991_s_at | KDM6A | 1.68 |
| 227857_at | APMAP | 1.68 |
| 241698_at | RFTN2 | 1.68 |
| 215046_at | KANSL1L | 1.68 |
| 212850_s_at | LRP4 | 1.68 |
| 205098_at | CCR1 | 1.68 |
| 204646_at | DPYD | 1.68 |
| 232563_at | ZNF684 | 1.68 |
| 206181_at | SLAMF1 | 1.67 |
| 228167_at | KLHL6 | 1.67 |
| 210839_s_at | ENPP2 | 1.67 |
| 221039_s_at | ASAP1 | 1.67 |
| 202957_at | HCLS1 | 1.67 |
| 210321_at | GZMH | 1.67 |
| 228948_at | EPHA4 | 1.67 |
| 223854_at | PCDHB10 | 1.67 |
| 229222_at | ACSS3 | 1.67 |
| 220327_at | VGLL3 | 1.67 |
| 1552370_at | C4orf33 | 1.67 |
| 238907_at | ZNF780A | 1.67 |
| 206715_at | TFEC | 1.67 |
| 213603_s_at | RAC2 | 1.67 |
| 210279_at | GPR18 | 1.67 |
| 238520_ at | TRERF1 | 1.67 |
| 232137_at | ZNF616 | 1.67 |
| 225769_at | COG6 | 1.67 |
| 235970_at | LCORL | 1.67 |
| 213566_at | RNASE6 | 1.67 |
| 216944_s_at | ITPR1 | 1.66 |
| 202983_at | HLTF | 1.66 |
| 220532_s_at | TMEM176B | 1.66 |
| 235226_at | CDK19 | 1.66 |
| 205839_s_at | BZRAP1 | 1.66 |
| 219033_at | PARP8 | 1.66 |
| 207277_at | CD209 | 1.66 |
| 218236_s_at | PRKD3 | 1.66 |
| 37793_r_at | RAD51D | 1.66 |
| 230403_at | RFX3 | 1.66 |
| 225992_at | MLLT10 | 1.66 |
| 243664_at | TXNL1 | 1.65 |
| 229234_at | ZC3H12B | 1.65 |
| 1569652_at | MLLT3 | 1.65 |
| 201311_s_at | SH3BGRL | 1.65 |
| 212262_at | QKI | 1.65 |
| 244572_at | KY | 1.65 |
| 1553764_a_at | AJUBA | 1.65 |
| 207861_at | CCL22 | 1.65 |
| 203639_s_at | FGFR2 | 1.65 |
| 229828_at | CDC73 | 1.65 |
| 220146_at | TLR7 | 1.65 |
| 241808_at | ZC2HC1A | 1.65 |
| 229309_at | ADRB1 | 1.65 |
| 218614_at | KIAA1551 | 1.65 |
| 213415_at | CLIC2 | 1.65 |
| 224339_s_at | ANGPTL1 | 1.65 |
| 223696_at | ARSD | 1.64 |
| 1566472_s_at | RETSAT | 1.64 |
| 218197_s_at | OXR1 | 1.64 |
| 242940_x_at | DLX6 | 1.64 |
| 226932_at | SSPN | 1.64 |
| 34210_at | CD52 | 1.64 |
| 209555_s_at | CD36 | 1.64 |
| 232234_at | SLA2 | 1.64 |
| 205234_at | SLC16A4 | 1.64 |
| 211776_s_at | EPB41L3 | 1.64 |
| 235304_at | LOC100507486 | 1.64 |
| 224682_at | ANKIB1 | 1.64 |
| 204719_at | ABCA8 | 1.64 |
| 212558_at | SPRY1 | 1.64 |
| 219743_at | HEY2 | 1.64 |
| 1555600_s_at | APOL4 | 1.64 |
| 215013_s_at | USP34 | 1.64 |
| 230783_at | LOC283713 | 1.64 |
| 223595_at | TMEM133 | 1.64 |
| 213539_at | CD3D | 1.64 |
| 209357_at | CITED2 | 1.64 |
| 218345_at | TMEM176A | 1.64 |
| 226655_at | STX17 | 1.63 |
| 225499_at | RALGAPA2 | 1.63 |
| 213915_at | NKG7 | 1.63 |
| 209821_at | IL33 | 1.63 |
| 217257_at | SH3BP2 | 1.63 |
| 214470_at | KLRB1 | 1.63 |
| 228869_at | SNX20 | 1.63 |
| 217478_s_at | HLA-DMA | 1.63 |
| 243747_at | ZNF599 | 1.63 |
| 37145_at | GNLY | 1.63 |
| 209949_at | NCF2 | 1.63 |
| 1563458_at | PARVA | 1.63 |
| 226508_at | PHC3 | 1.63 |
| 206373_at | ZIC1 | 1.63 |
| 203372_s_at | SOCS2 | 1.63 |
| 218344_s_at | RCOR3 | 1.63 |
| 204174_at | ALOX5AP | 1.63 |
| 212655_at | ZCCHC14 | 1.63 |
| 206513_at | AIM2 | 1.62 |
| 205486_at | TESK2 | 1.62 |
| 207782_s_at | PSEN1 | 1.62 |
| 238438_at | CNOT6L | 1.62 |
| 230381_at | C1orf186 | 1.62 |
| 202765_s_at | FBN1 | 1.62 |
| 1565162_s_at | MGST1 | 1.62 |
| 235840_at | ZKSCAN3 | 1.62 |
| 1558346_at | COX17 | 1.62 |
| 205624_at | CPA3 | 1.62 |
| 202579_x_at | HMGN4 | 1.62 |
| 214913_at | ADAMTS3 | 1.62 |
| 1556069_s_at | HIF3A | 1.62 |
| 235051_at | CCDC50 | 1.62 |
| 236782_at | SAMD3 | 1.62 |
| 236673_at | TIFAB | 1.62 |
| 233641_s_at | FAM167A | 1.62 |
| 225426_at | PPP6C | 1.62 |
| 215310_ at | APC | 1.61 |
| 1556277_a_at | PAPD4 | 1.61 |
| 227553_at | PIK3R5 | 1.61 |
| 1553718_at | ZNF548 | 1.61 |
| 212746_s_at | CEP170 | 1.61 |
| 219684_at | RTP4 | 1.61 |
| 205225_at | ESR1 | 1.61 |
| 204103_at | CCL4 | 1.61 |
| 205859_at | LY86 | 1.61 |
| 212704_at | ZCCHC11 | 1.61 |
| 208893_s_at | DUSP6 | 1.61 |
| 206618_at | IL18R1 | 1.61 |
| 212502_at | ADO | 1.61 |
| 213160_at | DOCK2 | 1.61 |
| 204042_at | WASF3 | 1.61 |
| 203275_at | IRF2 | 1.61 |
| 201741_x_at | SRSF1 | 1.61 |
| 219023_at | AP1AR | 1.61 |
| 217989_at | HSD17B11 | 1.61 |
| 203156_at | AKAP11 | 1.61 |
| 201133_s_at | PJA2 | 1.61 |
| 231558_at | INSM1 | 1.60 |
| 226381_at | LOC100506748 | 1.60 |
| 231779_at | IRAK2 | 1.60 |
| 242680_at | AVPR1A | 1.60 |
| 232197_x_at | ARSB | 1.60 |
| 203199_s_at | MTRR | 1.60 |
| 235171_at | LOC100505501 | 1.60 |
| 206974_at | CXCR6 | 1.60 |
| 1561226_at | XCR1 | 1.60 |
| 206584_at | LY96 | 1.60 |
| 202995_s_at | FBLN1 | 1.60 |
| 217504_at | ABCA6 | 1.60 |
| 207375_s_at | IL15RA | 1.60 |
| 1559369_at | C5orf44 | 1.60 |
| 216250_s_at | LPXN | 1.60 |
| 235648_at | ZNF567 | 1.59 |
| 224797_at | ARRDC3 | 1.59 |
| 1552670_a_at | PPP1R3B | 1.59 |
| 223320_s_at | ABCB10 | 1.59 |
| 202430_s_at | PLSCR1 | 1.59 |
| 209708_at | MOXD1 | 1.59 |
| 206589_at | GFI1 | 1.59 |
| 222414_at | MLL3 | 1.59 |
| 223588_at | THAP2 | 1.59 |
| 213208_at | KIAA0240 | 1.59 |
| 203153_at | IFIT1 | 1.59 |
| 223343_at | MS4A7 | 1.59 |
| 155599_s_at | EIF4A2 | 1.59 |
| 219666_at | MS4A6A | 1.59 |
| 215446_s_at | LOX | 1.59 |
| 235160_at | ATF7 | 1.59 |
| 229480_at | MAGI2-AS3 | 1.59 |
| 204122_at | TYROBP | 1.59 |
| 228967_at | EIF1 | 1.59 |
| 230141_at | ARID4A | 1.59 |
| 226366_at | SHPRH | 1.58 |
| 218854_at | DSE | 1.58 |
| 219895_at | FAM70A | 1.58 |
| 226344_at | ZMAT1 | 1.58 |
| 207068_at | ZFP37 | 1.58 |
| 227025_at | PPHLN1 | 1.58 |
| 222496_s_at | RBM47 | 1.58 |
| 219694_at | FAM105A | 1.58 |
| 205220_ at | HCAR3 | 1.58 |
| 203645_s_at | CD163 | 1.58 |
| 239651_at | ANAPC5 | 1.58 |
| 1555812_a_at | ARHGDIB | 1.58 |
| 226908_at | LRIG3 | 1.58 |
| 212956_at | TBC1D9 | 1.58 |
| 229560_at | TLR8 | 1.58 |
| 231610_at | TTBK2 | 1.58 |
| 204176_at | KLHL20 | 1.58 |
| 205307_s_at | KMO | 1.58 |
| 229618_at | SNX16 | 1.58 |
| 213596_at | CASP4 | 1.58 |
| 208666_s_at | ST13 | 1.58 |
| 238678_at | FLJ45340 | 1.58 |
| 219161_s_at | CKLF | 1.58 |
| 217777_s_at | PTPLAD1 | 1.58 |
| 209582_s_at | CD200 | 1.57 |
| 235155_at | BDH2 | 1.57 |
| 205407_at | RECK | 1.57 |
| 215493_x_at | BTN2A1 | 1.57 |
| 242979_at | IRS1 | 1.57 |
| 212277_at | MTMR4 | 1.57 |
| 213170_at | GPX7 | 1.57 |
| 214146_s_at | PPBP | 1.57 |
| 210354_at | IFNG | 1.57 |
| 226181_at | TUBE 1 | 1.57 |
| 224600_at | CGGBP1 | 1.57 |
| 206519_x_at | SIGLEC6 | 1.57 |
| 212672_at | ATM | 1.57 |
| 207431_s_at | DEGS1 | 1.56 |
| 226358_at | APH1B | 1.56 |
| 208450_at | LGALS2 | 1.56 |
| 212820_at | DMXL2 | 1.56 |
| 205191_at | RP2 | 1.56 |
| 218486_at | KLF11 | 1.56 |
| 225931_s_at | RNF213 | 1.56 |
| 241399_at | FAM19A2 | 1.56 |
| 224701_at | PARP14 | 1.56 |
| 205127_at | PTGS1 | 1.56 |
| 226799_at | FGD6 | 1.56 |
| 227475_at | FOXQ1 | 1.56 |
| 230413_s_at | AP1S2 | 1.56 |
| 242521_at | LOC100505812 | 1.56 |
| 44790_s_at | KIAA0226L | 1.56 |
| 204032_at | BCAR3 | 1.56 |
| 224865_at | FAR1 | 1.56 |
| 221556_at | CDC14B | 1.56 |
| 230158_at | DPY19L2 | 1.56 |
| 217915_s_at | RSL24D1 | 1.56 |
| 243370_at | CAPRJN1 | 1.56 |
| 227722_at | RPS23 | 1.55 |
| 212486_s_at | FYN | 1.55 |
| 220992_s_at | TRMT1L | 1.55 |
| 205421_at | SLC22A3 | 1.55 |
| 214070_s_at | ATP10B | 1.55 |
| 1556300_s_at | SIM1 | 1.55 |
| 206170_ at | ADRB2 | 1.55 |
| 217104_at | ST20 | 1.55 |
| 212530_at | NEK7 | 1.55 |
| 227226_at | MRAP2 | 1.55 |
| 201455_s_at | NPEPPS | 1.55 |
| 1553150_at | KDM1B | 1.55 |
| 202181_at | KIAA0247 | 1.55 |
| 222279_at | HLA-F-AS1 | 1.55 |
| 214732_at | SP1 | 1.55 |
| 205433_at | BCHE | 1.55 |
| 206682_at | CLEC10A | 1.54 |
| 236539_at | PTPN22 | 1.54 |
| 212598_at | WDFY3 | 1.54 |
| 226251_at | ASXL2 | 1.54 |
| 215743_at | NMT2 | 1.54 |
| 1559490_at | LRCH3 | 1.54 |
| 238453_at | FGFBP3 | 1.54 |
| 226878_at | HLA-DOA | 1.54 |
| 220770_s_at | C5orf54 | 1.54 |
| 209892_at | FUT4 | 1.54 |
| 206765_at | KCNJ2 | 1.54 |
| 202377_at | LEPROT | 1.54 |
| 235450_ at | FBXL4 | 1.54 |
| 219599_at | EIF4B | 1.54 |
| 238581_at | GBP5 | 1.54 |
| 221231_s_at | C14orf102 | 1.54 |
| 212764_at | ZEB1 | 1.54 |
| 214162_at | LOC284244 | 1.54 |
| 227425_at | REPS2 | 1.53 |
| 221823_at | C5orf30 | 1.53 |
| 227428_at | GABPA | 1.53 |
| 204451_at | FZD1 | 1.53 |
| 211089_s_at | NEK3 | 1.53 |
| 203799_at | CD302 | 1.53 |
| 224965_at | GNG2 | 1.53 |
| 206159_at | GDF10 | 1.53 |
| 207181_s_at | CASP7 | 1.53 |
| 1556770_a_at | FBXL13 | 1.53 |
| 212408_at | TOR1AIP1 | 1.53 |
| 201653_at | CNIH | 1.53 |
| 235520_at | ZNF280C | 1.53 |
| 1553906_s_at | FGD2 | 1.53 |
| 233241_at | PLK1S1 | 1.53 |
| 228728_at | CPED1 | 1.53 |
| 225872_at | SLC35F5 | 1.53 |
| 229067_at | SRGAP2C | 1.53 |
| 209969_s_at | STAT1 | 1.53 |
| 1554062_at | XG | 1.53 |
| 211548_s_at | HPGD | 1.53 |
| 215023_s_at | PEX1 | 1.53 |
| 210135_s_at | SHOX2 | 1.53 |
| 215307_ at | ZNF529 | 1.53 |
| 226326_at | PCGF5 | 1.53 |
| 222659_at | IPO11 | 1.52 |
| 227351_at | C16orf52 | 1.52 |
| 202510_ s_at | TNFAIP2 | 1.52 |
| 204114_at | NID2 | 1.52 |
| 210355_at | PTHLH | 1.52 |
| 221950_at | EMX2 | 1.52 |
| 226884_at | LRRN1 | 1.52 |
| 226741_at | SLC12A6 | 1.52 |
| 202073_ at | OPTN | 1.52 |
| 236244_at | HNRNPU | 1.52 |
| 210340_s_at | CSF2RA | 1.52 |
| 228788_at | YPEL1 | 1.52 |
| 214581_x_at | TNFRSF21 | 1.52 |
| 201422_at | IFI30 | 1.52 |
| 204172_at | CPOX | 1.52 |
| 225207_ at | PDK4 | 1.52 |
| 205590_at | RASGRP1 | 1.52 |
| 227210_ at | SFMBT2 | 1.52 |
| 231975_s_at | MIER3 | 1.52 |
| 212067_s_at | C1R | 1.52 |
| 202026_at | SDHD | 1.52 |
| 1555858_at | LOC440944 | 1.52 |
| 219940_s_at | PCID2 | 1.52 |
| 207339_s_at | LTB | 1.52 |
| 235537_at | OCIAD1 | 1.52 |
| 238081_at | WDFY3-AS2 | 1.52 |
| 218769_s_at | ANKRA2 | 1.51 |
| 212406_s_at | PCMTD2 | 1.51 |
| 235885_at | P2RY12 | 1.51 |
| 227171_at | CCT4 | 1.51 |
| 209546_s_at | APOL1 | 1.51 |
| 204591_at | CHL1 | 1.51 |
| 213974_at | ADAMTSL3 | 1.51 |
| 221757_at | PIK3IP1 | 1.51 |
| 220350_ at | ZNF235 | 1.51 |
| 213605_s_at | LOC728093 | 1.51 |
| 235151_at | LOC283357 | 1.51 |
| 239237_at | LOC100506776 | 1.51 |
| 219209_at | IFIH1 | 1.51 |
| 205498_at | GHR | 1.51 |
| 218918_at | MAN1C1 | 1.51 |
| 203097_s_at | RAPGEF2 | 1.51 |
| 1556116_s_at | TNPO1 | 1.51 |
| 239843_at | RIT1 | 1.51 |
| 228010_at | PPP2R2C | 1.51 |
| 209687_at | CXCL12 | 1.51 |
| 213095_x_at | AIF1 | 1.51 |
| 208018_s_at | HCK | 1.51 |
| 211958_at | IGFBP5 | 1.51 |
| 238923_at | SPOP | 1.51 |
| 234888_at | CACHD1 | 1.51 |
| 201218_at | CTBP2 | 1.51 |
| 218311_at | MAP4K3 | 1.51 |
| 202171_at | VEZF1 | 1.51 |
| 229969_at | SEC63 | 1.51 |
| 230780_at | LOC730091 | 1.51 |
| 1564276_at | C5orf56 | 1.50 |
| 209168_at | GPM6B | 1.50 |
| 222938_x_at | ENPP3 | 1.50 |
| 205051_s_at | KIT | 1.50 |
| 231993_at | ITGBL1 | 1.50 |
| 221965_at | MPHOSPH9 | 1.50 |
| 222218_s_at | PILRA | 1.50 |
| 222572_at | PDP1 | 1.50 |
| 222075_s_at | OAZ3 | 0.67 |
| 1555677_s_at | SMC1A | 0.67 |
| 223894_s_at | AKTIP | 0.67 |
| 218488_at | EIF2B3 | 0.67 |
| 226355_at | POC1A | 0.67 |
| 239319_at | LOC728342 | 0.67 |
| 202041_s_at | FIBP | 0.67 |
| 229119_s_at | ZSWIM7 | 0.67 |
| 212123_at | TCTN3 | 0.67 |
| 206653_at | POLR3G | 0.67 |
| 202570_s_at | DLGAP4 | 0.66 |
| 213546_at | DKFZP58611420 | 0.66 |
| 220021_at | TMC7 | 0.66 |
| 219944_at | CLIP4 | 0.66 |
| 219693_at | AGPAT4 | 0.66 |
| 229900_at | CD109 | 0.66 |
| 219395_at | ESRP2 | 0.66 |
| 227844_at | FMNL3 | 0.66 |
| 202870_s_at | CDC20 | 0.66 |
| 200643_at | HDLBP | 0.66 |
| 40850_at | FKBP8 | 0.66 |
| 229635_at | LOC100505702 | 0.66 |
| 227410_at | FAM43A | 0.66 |
| 204328_at | TMC6 | 0.66 |
| 220244_at | LINC00312 | 0.66 |
| 225496_s_at | SYTL2 | 0.66 |
| 213571_s_at | EIF4E2 | 0.66 |
| 208297_s_at | EVI5 | 0.66 |
| 208932_at | PPP4C | 0.66 |
| 230362_at | INPP5F | 0.66 |
| 222810_s_at | RASAL2 | 0.66 |
| 200601_at | ACTN4 | 0.66 |
| 210480_s_at | MYO6 | 0.66 |
| 214752_x_at | FLNA | 0.66 |
| 213067_at | MYH10 | 0.66 |
| 227477_at | ZMYND19 | 0.66 |
| 224898_at | WDR26 | 0.66 |
| 223701_ s_at | USP47 | 0.66 |
| 207865_s_at | BMP8B | 0.66 |
| 220203_at | BMP8A | 0.66 |
| 206312_at | GUCY2C | 0.66 |
| 203727_at | SKIV2L | 0.66 |
| 202756_s_at | GPC1 | 0.66 |
| 213899_at | METAP2 | 0.66 |
| 242624_at | ABLIM2 | 0.66 |
| 205423_at | AP1B1 | 0.66 |
| 207321_s_at | ABCB9 | 0.66 |
| 235536_at | SNORD89 | 0.66 |
| 241436_at | SCNN1G | 0.66 |
| 213008_at | FANCI | 0.66 |
| 201291_s_at | TOP2A | 0.66 |
| 206034_at | SERPINB8 | 0.66 |
| 239196_at | ANKRD22 | 0.66 |
| 225788_at | RRP36 | 0.66 |
| 235086_at | THBS1 | 0.66 |
| 204616_at | UCHL3 | 0.66 |
| 221063_x_at | RNF123 | 0.66 |
| 203382_s_at | APOE | 0.66 |
| 1552314_a_at | EYA3 | 0.66 |
| 1554049_s_at | DCAF8 | 0.66 |
| 232074_at | PRSS27 | 0.66 |
| 229936_at | GFRA3 | 0.65 |
| 205138_s_at | UST | 0.65 |
| 227060_ at | RELT | 0.65 |
| 205268_s_at | ADD2 | 0.65 |
| 213822_s_at | UBE3B | 0.65 |
| 219842_at | ARL15 | 0.65 |
| 204840_ s_at | EEA1 | 0.65 |
| 235948_at | RIMKLA | 0.65 |
| 208676_s_at | PA2G4 | 0.65 |
| 210020_x_at | CALML3 | 0.65 |
| 227898_s_at | ZFP41 | 0.65 |
| 200710_ at | ACADVL | 0.65 |
| 207390_s_at | SMTN | 0.65 |
| 218360_at | RAB22A | 0.65 |
| 204454_at | LDOC1 | 0.65 |
| 211564_s_at | PDLIM4 | 0.65 |
| 201911_s_at | FARP1 | 0.65 |
| 214374_s_at | PPFIBP1 | 0.65 |
| 212374_at | FEM1B | 0.65 |
| 208555_x_at | CST2 | 0.65 |
| 225591_at | FBXO25 | 0.65 |
| 217933_s_at | LAP3 | 0.65 |
| 1569064_at | C15orf62 | 0.65 |
| 1569868_s_at | EME2 | 0.65 |
| 226615_at | XPR1 | 0.65 |
| 226697_at | FAM114A1 | 0.65 |
| 235998_at | RHPN1 | 0.65 |
| 200598_s_at | HSP90B 1 | 0.65 |
| 235709_at | GAS2L3 | 0.65 |
| 212878_s_at | KLC1 | 0.65 |
| 221005_s_at | PTDSS2 | 0.65 |
| 238528_at | UBR1 | 0.65 |
| 225955_at | METRNL | 0.65 |
| 202107_s_at | MCM2 | 0.65 |
| 202806_at | DBN1 | 0.65 |
| 1554333_at | DNAJA4 | 0.65 |
| 223682_s_at | EIF1AD | 0.65 |
| 214324_at | GP2 | 0.65 |
| 202109_at | ARFIP2 | 0.65 |
| 214337_at | COPA | 0.65 |
| 203634_s_at | CPT1A | 0.65 |
| 227318_at | TMEM178B | 0.65 |
| 222240_s_at | ISYNA1 | 0.65 |
| 221713_s_at | MAP6D1 | 0.65 |
| 209912_s_at | AP5Z1 | 0.65 |
| 218489_s_at | ALAD | 0.65 |
| 227880_s_at | TMEM185A | 0.65 |
| 223419_at | FBXW9 | 0.65 |
| 222348_at | MAST4 | 0.65 |
| 209848_s_at | PMEL | 0.65 |
| 219904_at | ZSCAN5A | 0.65 |
| 231928_at | HES2 | 0.65 |
| 244889_at | LOC388210 | 0.65 |
| 205521_at | EXOG | 0.65 |
| 1552296_at | BEST4 | 0.65 |
| 203722_at | ALDH4A1 | 0.65 |
| 221585_at | CACNG4 | 0.65 |
| 202714_s_at | KIAA0391 | 0.65 |
| 232270_at | C9orf3 | 0.65 |
| 219084_at | NSD1 | 0.65 |
| 202356_s_at | GTF2F1 | 0.65 |
| 208190_s_at | LSR | 0.64 |
| 221521_s_at | GINS2 | 0.64 |
| 228236_at | SLC52A3 | 0.64 |
| 227217_at | WNK2 | 0.64 |
| 238460_at | FAM83A | 0.64 |
| 206277_at | P2RY2 | 0.64 |
| 226359_at | GTPBP1 | 0.64 |
| 1552578_a_at | MYO3B | 0.64 |
| 216212_s_at | DKC1 | 0.64 |
| 215235_at | SPTAN1 | 0.64 |
| 221981_s_at | WDR59 | 0.64 |
| 203263_s_at | ARHGEF9 | 0.64 |
| 210428_s_at | HGS | 0.64 |
| 225794_s_at | C22orf32 | 0.64 |
| 226313_at | C10orf35 | 0.64 |
| 204608_at | ASL | 0.64 |
| 230674_at | LGR4 | 0.64 |
| 219560_at | C22orf29 | 0.64 |
| 225301_s_at | MYO5B | 0.64 |
| 219555_s_at | CENPN | 0.64 |
| 219358_s_at | ADAP2 | 0.64 |
| 1559399_s_at | ZCCHC10 | 0.64 |
| 233167_at | SELO | 0.64 |
| 202315_s_at | BCR | 0.64 |
| 226009_at | DPCD | 0.64 |
| 229380_at | ILDR2 | 0.64 |
| 223518_at | DFFA | 0.64 |
| 1558173_a_at | LUZP1 | 0.64 |
| 202391_at | BASP 1 | 0.64 |
| 224987_at | C6orf89 | 0.64 |
| 204378_at | BCAS1 | 0.64 |
| 201353_s_at | BAZ2A | 0.64 |
| 1569659_at | LOC100506895 | 0.64 |
| 238594_x_at | DUSP8 | 0.64 |
| 202580_x_at | FOXM1 | 0.64 |
| 208998_at | UCP2 | 0.64 |
| 1557165_s_at | KLHL18 | 0.64 |
| 205164_at | GCAT | 0.64 |
| 212563_at | BOP1 | 0.64 |
| 211846_s_at | PVRL1 | 0.64 |
| 240233_at | LOC100506714 | 0.64 |
| 212983_at | HRAS | 0.64 |
| 242283_at | DNAH14 | 0.64 |
| 228922_at | SHF | 0.64 |
| 202924_s_at | PLAGL2 | 0.64 |
| 208693_s_at | GARS | 0.64 |
| 218874_s_at | ATAT1 | 0.64 |
| 222245_s_at | FER1L4 | 0.64 |
| 225193_at | KIAA1967 | 0.64 |
| 225096_at | C17orf79 | 0.64 |
| 202461_at | EIF2B2 | 0.64 |
| 219916_s_at | RNF39 | 0.64 |
| 217785_s_at | YKT6 | 0.64 |
| 202705_at | CCNB2 | 0.64 |
| 211531_x_at | PRB1 | 0.64 |
| 226027_at | SWI5 | 0.64 |
| 203201_at | PMM2 | 0.64 |
| 209667_at | CES2 | 0.64 |
| 203903_s_at | HEPH | 0.64 |
| 1552803_a_at | STMN1 | 0.64 |
| 205348_s_at | DYNC1I1 | 0.64 |
| 231406_at | ORAI2 | 0.64 |
| 232203_at | NKD 1 | 0.64 |
| 213467_at | RND2 | 0.64 |
| 209495_at | CEP250 | 0.63 |
| 216555_at | PRR14L | 0.63 |
| 229494_s_at | PHLDA2 | 0.63 |
| 231233_at | KDM5B-AS1 | 0.63 |
| 242729_at | TTN-AS1 | 0.63 |
| 38710_at | OTUB1 | 0.63 |
| 204256_at | ELOVL6 | 0.63 |
| 218619_s_at | SUV39H1 | 0.63 |
| 1557136_at | ATP13A4 | 0.63 |
| 242722_at | LMO7 | 0.63 |
| 1569044_at | CDC42BPG | 0.63 |
| 1553226_at | LINC00052 | 0.63 |
| 1552389_at | C8orf47 | 0.63 |
| 219752_at | RASAL1 | 0.63 |
| 213096_at | TMCC2 | 0.63 |
| 209679_s_at | SMAGP | 0.63 |
| 224227_s_at | BDP1 | 0.63 |
| 204879_at | PDPN | 0.63 |
| 203235_at | THOP1 | 0.63 |
| 220482_s_at | SERGEF | 0.63 |
| 205864_at | SLC7A4 | 0.63 |
| 205796_at | TCP11L1 | 0.63 |
| 229405_at | KIF7 | 0.63 |
| 205375_at | MDFI | 0.63 |
| 228587_at | FAM83G | 0.63 |
| 1553211_at | ANKFN1 | 0.63 |
| 227437_at | LOC100506668 | 0.63 |
| 225682_s_at | POLR3H | 0.63 |
| 232322_x_at | STARD10 | 0.63 |
| 219722_s_at | GDPD3 | 0.63 |
| 225751_at | RBM17 | 0.63 |
| 223346_at | VPS18 | 0.63 |
| 208637_x_at | ACTN1 | 0.63 |
| 204958_at | PLK3 | 0.63 |
| 207525_s_at | GIPC1 | 0.63 |
| 201797_s_at | VARS | 0.63 |
| 2028_s_at | E2F1 | 0.62 |
| 205145_s_at | MYL5 | 0.62 |
| 205436_s_at | H2AFX | 0.62 |
| 203668_at | MAN2C1 | 0.62 |
| 229581_at | ELFN1 | 0.62 |
| 55065_at | MARK4 | 0.62 |
| 229933_at | Clorf74 | 0.62 |
| 218257_s_at | UGGT1 | 0.62 |
| 238909_at | S100A10 | 0.62 |
| 235461_at | TET2 | 0.62 |
| 220308_at | CCDC19 | 0.62 |
| 205547_s_at | TAGLN | 0.62 |
| 223919_at | TP53AIP1 | 0.62 |
| 230104_s_at | TPPP | 0.62 |
| 235852_at | STON2 | 0.62 |
| 1564231_at | IFT80 | 0.62 |
| 38340_at | HIP1R | 0.62 |
| 219785_s_at | FBXO31 | 0.62 |
| 226828_s_at | HEYL | 0.62 |
| 210081_at | AGER | 0.62 |
| 227283_at | EFR3B | 0.62 |
| 221779_at | MICALL1 | 0.62 |
| 215714_s_at | SMARCA4 | 0.62 |
| 229004_at | ADAMTS15 | 0.62 |
| 238703_at | FAM207A | 0.62 |
| 204284_at | PPP1R3C | 0.62 |
| 217080_s_at | HOMER2 | 0.62 |
| 213270_ at | MPP2 | 0.62 |
| 222622_at | PGP | 0.62 |
| 1554469_at | ZBTB44 | 0.62 |
| 1552755_at | C9orf66 | 0.62 |
| 231230_at | KCNK10 | 0.62 |
| 1568617_a_at | CAMSAP3 | 0.62 |
| 226373_at | SFXN5 | 0.62 |
| 220620_at | CRCT1 | 0.62 |
| 1553023_a_at | NOX5 | 0.62 |
| 238028_at | C6orf132 | 0.62 |
| 212570_at | ENDOD1 | 0.62 |
| 1569787_at | RFTN1 | 0.62 |
| 204745_x_at | MT1G | 0.62 |
| 230847_at | WRNIP1 | 0.62 |
| 227744_s_at | HNRNPD | 0.61 |
| 223078_s_at | TMOD3 | 0.61 |
| 205592_at | SLC4A1 | 0.61 |
| 201149_s_at | TIMP3 | 0.61 |
| 210065_s_at | UPK1B | 0.61 |
| 203612_at | BYSL | 0.61 |
| 240288_at | KCNRG | 0.61 |
| 227672_at | C8orf73 | 0.61 |
| 210689_ at | CLDN14 | 0.61 |
| 1554804_a_at | CLDN19 | 0.61 |
| 31846_at | RHOD | 0.61 |
| 214657_s_at | NEAT1 | 0.61 |
| 222783_s_at | SMOC1 | 0.61 |
| 227069_at | CUX1 | 0.61 |
| 243362_s_at | LEF1-AS1 | 0.61 |
| 215471_s_at | MAP7 | 0.61 |
| 1552618_at | STX6 | 0.61 |
| 214073_at | CTTN | 0.61 |
| 221389_at | PLA2G2E | 0.61 |
| 218376_s_at | MICAL1 | 0.61 |
| 225317_at | ACBD6 | 0.61 |
| 200611_s_at | WDR1 | 0.61 |
| 235683_at | SESN3 | 0.61 |
| 206315_at | CRLF 1 | 0.61 |
| 230092_at | UBXN10 | 0.61 |
| 220953_s_at | MTMR12 | 0.61 |
| 210357_s_at | SMOX | 0.61 |
| 203004_s_at | MEF2D | 0.61 |
| 205214_at | STK17B | 0.61 |
| 209945_s_at | GSK3B | 0.61 |
| 238625_at | Clorf168 | 0.61 |
| 212154_at | SDC2 | 0.61 |
| 229854_at | OBSCN | 0.61 |
| 228274_at | SDSL | 0.61 |
| 1563796_s_at | EARS2 | 0.61 |
| 222671_s_at | JMJD4 | 0.61 |
| 219844_at | C10orf118 | 0.60 |
| 212810_ s_at | SLC1A4 | 0.60 |
| 209922_at | BRAP | 0.60 |
| 219271_at | GALNT14 | 0.60 |
| 222222_s_at | HOMER3 | 0.60 |
| 205918_at | SLC4A3 | 0.60 |
| 210609_s_at | TP53I3 | 0.60 |
| 1554038_at | LARP1B | 0.60 |
| 239061_at | TPRXL | 0.60 |
| 200720_s_at | ACTR1A | 0.60 |
| 218961_s_at | PNKP | 0.60 |
| 210624_s_at | ILVBL | 0.60 |
| 202525_at | PRSS8 | 0.60 |
| 233208_x_at | CPSF2 | 0.60 |
| 204736_s_at | CSPG4 | 0.60 |
| 202005_at | ST14 | 0.60 |
| 1558281_a_at | TMEM184A | 0.60 |
| 239263_at | LOC100506813 | 0.60 |
| 218960_at | TMPRSS4 | 0.60 |
| 201516_at | SRM | 0.60 |
| 239174_at | LOC100505912 | 0.60 |
| 210021_s_at | CCNO | 0.60 |
| 217150_s_at | NF2 | 0.60 |
| 201861_s_at | LRRFIP 1 | 0.60 |
| 219976_at | HOOK1 | 0.60 |
| 212834_at | DDX52 | 0.60 |
| 37408_at | MRC2 | 0.60 |
| 218906_x_at | KLC2 | 0.60 |
| 228068_at | GOLGA7B | 0.60 |
| 1554095_at | RBM33 | 0.60 |
| 238636_at | CACNA1C | 0.60 |
| 1560068_a_at | LOC729870 | 0.60 |
| 237169_at | TNC | 0.60 |
| 218872_at | TESC | 0.60 |
| 65635_at | ENGASE | 0.60 |
| 229084_at | CNTN4 | 0.60 |
| 1558050_at | EIF2B5 | 0.60 |
| 206828_at | TXK | 0.60 |
| 239707_at | SLC5A10 | 0.60 |
| 208622_s_at | EZR | 0.60 |
| 214763_at | ACOT11 | 0.60 |
| 208002_s_at | ACOT7 | 0.59 |
| 217930_s_at | TOLLIP | 0.59 |
| 219194_at | SEMA4G | 0.59 |
| 204857_at | MAD1L1 | 0.59 |
| 209037_s_at | EHD1 | 0.59 |
| 219806_s_at | C11orf75 | 0.59 |
| 1553672_at | ENAH | 0.59 |
| 1556308_at | PRRT3 | 0.59 |
| 203367_at | DUSP14 | 0.59 |
| 224097_s_at | F11R | 0.59 |
| 1554701_a_at | TBC1D16 | 0.59 |
| 232069_at | KIF26A | 0.59 |
| 228515_at | LOC90784 | 0.59 |
| 216804_s_at | PDLIM5 | 0.59 |
| 226584_s_at | FAM110A | 0.59 |
| 227506_at | SLC16A9 | 0.59 |
| 202198_s_at | MTMR3 | 0.59 |
| 226661_at | CDCA2 | 0.59 |
| 219529_at | CLIC3 | 0.59 |
| 228900_at | SPECC1 | 0.59 |
| 211702_s_at | USP32 | 0.59 |
| 225249_at | SPPL2B | 0.59 |
| 225160_x_at | MDM2 | 0.59 |
| 203027_s_at | MVD | 0.59 |
| 222435_s_at | UBE2J1 | 0.59 |
| 223471_at | RAB3IP | 0.59 |
| 232300_at | LOC100133190 | 0.59 |
| 214838_at | SFT2D2 | 0.59 |
| 228176_at | S1PR3 | 0.59 |
| 200924_s_at | SLC3A2 | 0.59 |
| 222874_s_at | CLN8 | 0.59 |
| 206858_s_at | HOXC6 | 0.59 |
| 229275_at | IGFN1 | 0.59 |
| 217924_at | C6orf106 | 0.59 |
| 228303_at | GALNT6 | 0.59 |
| 214490_at | ARSF | 0.58 |
| 228996_at | RC3H1 | 0.58 |
| 203287_at | LAD1 | 0.58 |
| 219229_at | SLCO3A1 | 0.58 |
| 1553352_x_at | ERVW-1 | 0.58 |
| 227174_at | WDR72 | 0.58 |
| 231609_at | C10orf82 | 0.58 |
| 1570156_s_at | FMN1 | 0.58 |
| 228235_at | MGC16121 | 0.58 |
| 209442_x_at | ANK3 | 0.58 |
| 212915_at | PDZRN3 | 0.58 |
| 229715_at | NCR3LG1 | 0.58 |
| 204657_s_at | SHB | 0.58 |
| 233123_at | SLC40A1 | 0.58 |
| 201050_at | PLD3 | 0.58 |
| 1553785_at | RASGEF1B | 0.58 |
| 228360_ at | LYPD6B | 0.58 |
| 1554821_a_at | ZBED1 | 0.58 |
| 203842_s_at | MAPRE3 | 0.58 |
| 1555783_x_at | PQLC2 | 0.58 |
| 205872_x_at | PDE4DIP | 0.58 |
| 212807_s_at | SORT1 | 0.58 |
| 205406_s_at | SPA17 | 0.58 |
| 235095_at | CCDC64B | 0.58 |
| 203707_ at | ZNF263 | 0.58 |
| 237981_at | CMYA5 | 0.58 |
| 237423_at | RSPO4 | 0.58 |
| 243955_at | LOC388820 | 0.57 |
| 216222_s_at | MYO10 | 0.57 |
| 230976_at | AK8 | 0.57 |
| 225258_at | FBLIM1 | 0.57 |
| 206044_s_at | BRAF | 0.57 |
| 226535_at | ITGB6 | 0.57 |
| 202402_s_at | CARS | 0.57 |
| 215547_at | TSC22D2 | 0.57 |
| 201043_s_at | ANP32A | 0.57 |
| 205948_at | PTPRT | 0.57 |
| 223461_at | TBC1D7 | 0.57 |
| 1553710_at | FAM218A | 0.57 |
| 212686_at | PPM1H | 0.57 |
| 239569_at | FLJ31485 | 0.57 |
| 202275_at | G6PD | 0.57 |
| 211538_s_at | HSPA2 | 0.57 |
| 202895_s_at | SIRPA | 0.57 |
| 204556_s_at | DZIP 1 | 0.57 |
| 214433_s_at | SELENBP1 | 0.57 |
| 206472_s_at | TLE3 | 0.57 |
| 220193_at | SH3D21 | 0.57 |
| 230144_at | GRIA3 | 0.57 |
| 204420_ at | FOSL1 | 0.57 |
| 234925_at | LOC732275 | 0.57 |
| 200906_s_at | PALLD | 0.56 |
| 229929_at | SPSB4 | 0.56 |
| 209589_s_at | EPHB2 | 0.56 |
| 213793_s_at | HOMER1 | 0.56 |
| 218876_at | TPPP3 | 0.56 |
| 213385_at | CHN2 | 0.56 |
| 223468_s_at | RGMA | 0.56 |
| 217966_s_at | FAM129A | 0.56 |
| 213607_x_at | NADK | 0.56 |
| 204730_at | RIMS3 | 0.56 |
| 203160_s_at | RNF8 | 0.56 |
| 229163_at | CAMK2N1 | 0.56 |
| 243209_at | KCNQ4 | 0.56 |
| 213358_at | SOGA2 | 0.56 |
| 213030_s_at | PLXNA2 | 0.56 |
| 226187_at | CDS1 | 0.56 |
| 224008_ s_at | KCNK7 | 0.56 |
| 236263_at | SHH | 0.55 |
| 229518_at | FAM46B | 0.55 |
| 201481_s_at | PYGB | 0.55 |
| 208653_s_at | CD164 | 0.55 |
| 228720_ at | SORCS2 | 0.55 |
| 236275_at | KRBA1 | 0.55 |
| 210402_at | KCNJ1 | 0.55 |
| 219554_at | RHCG | 0.55 |
| 236926_at | TBX1 | 0.55 |
| 207144_s_at | CITED1 | 0.55 |
| 209696_at | FBP1 | 0.55 |
| 206758_at | EDN2 | 0.55 |
| 219478_at | WFDC1 | 0.55 |
| 223575_at | KIAA1549 | 0.55 |
| 228486_at | SLC44A1 | 0.55 |
| 206046_at | ADAM23 | 0.54 |
| 229892_at | EP400NL | 0.54 |
| 220245_at | SLC45A2 | 0.54 |
| 228473_at | MSX1 | 0.54 |
| 211071_s_at | MLLT11 | 0.54 |
| 222027_at | NUCKS1 | 0.54 |
| 206623_at | PDE6A | 0.54 |
| 220317_at | LRAT | 0.54 |
| 215729_s_at | VGLL1 | 0.54 |
| 219511_s_at | SNCAIP | 0.54 |
| 229909_at | B4GALNT3 | 0.54 |
| 230498_at | MCHR1 | 0.54 |
| 230469_at | RTKN2 | 0.54 |
| 209438_at | PHKA2 | 0.54 |
| 36829_at | PER1 | 0.54 |
| 228262_at | MAP7D2 | 0.54 |
| 217395_at | MT4 | 0.54 |
| 203827_at | WIPI1 | 0.53 |
| 33767_at | NEFH | 0.53 |
| 209698_at | CCHCR1 | 0.53 |
| 237899_at | LOC100506374 | 0.53 |
| 223427_s_at | EPB41L4B | 0.53 |
| 200744_s_at | GNB1 | 0.53 |
| 204157_s_at | SIK3 | 0.53 |
| 215779_s_at | HIST1H2BG | 0.53 |
| 1553388_at | FAM26D | 0.53 |
| 221031_s_at | APOLD1 | 0.53 |
| 222871_at | KLHDC8A | 0.53 |
| 235479_at | CPEB2 | 0.53 |
| 1552496_a_at | COBL | 0.53 |
| 231371_at | TDRD10 | 0.53 |
| 220795_s_at | BEGAIN | 0.53 |
| 234978_at | SLC36A4 | 0.53 |
| 206132_at | MCC | 0.52 |
| 230087_at | PRIMA1 | 0.52 |
| 221114_at | AMBN | 0.52 |
| 206912_at | FOXE1 | 0.52 |
| 225142_at | JHDM1D | 0.52 |
| 229160_at | MUM1L1 | 0.52 |
| 229596_at | AMDHD1 | 0.52 |
| 212479_s_at | RMND5A | 0.52 |
| 242660_at | C10orf112 | 0.52 |
| 210048_at | NAPG | 0.52 |
| 1555007_s_at | WDR66 | 0.52 |
| 207192_at | DNASE1L2 | 0.51 |
| 205125_at | PLCD1 | 0.51 |
| 204682_at | LTBP2 | 0.51 |
| 203650_at | PROCR | 0.51 |
| 240024_at | SEC14L2 | 0.51 |
| 220011_at | AUNIP | 0.51 |
| 208399_s_at | EDN3 | 0.51 |
| 203184_at | FBN2 | 0.51 |
| 1569852_at | C7orf53 | 0.51 |
| 204105_s_at | NRCAM | 0.51 |
| 203021_at | SLPI | 0.51 |
| 206306_at | RYR3 | 0.51 |
| 211782_at | IDS | 0.51 |
| 232067_at | FAXC | 0.50 |
| 216665_s_at | TTTY2 | 0.50 |
| 210236_at | PPFIA1 | 0.50 |
| 222774_s_at | NETO2 | 0.50 |
| 223720_at | SPINK7 | 0.49 |
| 239136_at | LOC728978 | 0.49 |
| 206994_at | CST4 | 0.49 |
| 219250_s_at | FLRT3 | 0.49 |
| 1553539_at | KRT74 | 0.49 |
| 224762_at | SERINC2 | 0.49 |
| 210221_at | CHRNA3 | 0.49 |
| 220289_s_at | AIM1L | 0.49 |
| 218834_s_at | TMEM132A | 0.49 |
| 227482_at | ADCK1 | 0.49 |
| 222277_at | C1QTNF9B-AS1 | 0.48 |
| 200790_at | ODC1 | 0.48 |
| 224293_at | TTTY10 | 0.48 |
| 226698_at | FCHSD1 | 0.48 |
| 232682_at | MREG | 0.48 |
| 214168_s_at | TJP1 | 0.48 |
| 226899_at | UNC5B | 0.48 |
| 204351_at | S100P | 0.47 |
| 209591_s_at | BMP7 | 0.47 |
| 226473_at | CBX2 | 0.47 |
| 236651_at | KALRN | 0.47 |
| 208701_ at | APLP2 | 0.47 |
| 237466_s_at | HHIP | 0.47 |
| 214357_at | C1orf105 | 0.47 |
| 228977_at | LOC729680 | 0.47 |
| 1552960_at | LRRC15 | 0.47 |
| 223991_s_at | GALNT2 | 0.47 |
| 205534_at | PCDH7 | 0.47 |
| 231867_at | ODZ2 | 0.47 |
| 1557321_a_at | CAPN14 | 0.46 |
| 208613_s_at | FLNB | 0.46 |
| 218980_ at | FHOD3 | 0.46 |
| 203304_at | BAMBI | 0.46 |
| 207703_at | NLGN4Y | 0.46 |
| 206196_s_at | RUNDC3A | 0.46 |
| 215253_s_at | RCAN 1 | 0.46 |
| 205376_at | INPP4B | 0.46 |
| 214636_at | CALCB | 0.46 |
| 213913_s_at | TBC1D30 | 0.46 |
| 233586_s_at | KLK12 | 0.46 |
| 213556_at | PINLYP | 0.45 |
| 207213_s_at | USP2 | 0.45 |
| 227450_at | ERP27 | 0.45 |
| 203921_at | CHST2 | 0.45 |
| 208250_ s_at | DMBT1 | 0.45 |
| 224412_s_at | TRPM6 | 0.45 |
| 206774_at | FRMPD 1 | 0.45 |
| 214313_s_at | EIF5B | 0.44 |
| 227650_at | HSPA14 | 0.44 |
| 1562247_at | LOC286058 | 0.44 |
| 202208_s_at | ARL4C | 0.44 |
| 217276_x_at | SERHL2 | 0.44 |
| 1553494_at | TDH | 0.44 |
| 213774_s_at | PPP1R2 | 0.44 |
| 220624_s_at | ELF5 | 0.43 |
| 239913_at | SLC10A4 | 0.43 |
| 207147_at | DLX2 | 0.43 |
| 219946_x_at | MYH14 | 0.43 |
| 244760_at | HERC6 | 0.43 |
| 236255_at | PLEKHG4B | 0.43 |
| 219463_at | LAMP5 | 0.43 |
| 239202_at | RAB3B | 0.43 |
| 242138_at | DLX1 | 0.43 |
| 204903_x_at | ATG4B | 0.43 |
| 206882_at | SLC1A6 | 0.43 |
| 209343_at | EFHD1 | 0.43 |
| 229252_at | ATG9B | 0.43 |
| 221416_at | PLA2G2F | 0.42 |
| 227736_at | C10orf99 | 0.42 |
| 1553829_at | CYP1B1-AS1 | 0.42 |
| 224279_s_at | CABYR | 0.42 |
| 215785_s_at | CYFIP2 | 0.42 |
| 221627_at | TRIM10 | 0.42 |
| 205290_s_at | BMP2 | 0.42 |
| 223739_at | PADI1 | 0.42 |
| 203130_s_at | KIF5C | 0.42 |
| 232555_at | CREB5 | 0.42 |
| 220067_at | SPTBN5 | 0.41 |
| 210311_at | FGF5 | 0.41 |
| 1558687_a_at | FOXN1 | 0.41 |
| 231875_at | KIF21A | 0.40 |
| 220129_at | SOHLH2 | 0.40 |
| 209800_ at | KRT16 | 0.40 |
| 1552807_a_at | SIGLEC10 | 0.40 |
| 210335_at | RASSF9 | 0.40 |
| 213713_s_at | GLB1L2 | 0.40 |
| 1556533_at | C17orf52 | 0.39 |
| 242329_at | LOC401317 | 0.39 |
| 209859_at | TRIM9 | 0.39 |
| 229730_at | SMTNL2 | 0.39 |
| 239572_at | GJA3 | 0.39 |
| 241985_at | JMY | 0.38 |
| 215711_s_at | WEE1 | 0.38 |
| 206604_at | OVOL1 | 0.38 |
| 214226_at | PRSS53 | 0.37 |
| 205724_at | PKP1 | 0.37 |
| 225809_at | PARM1 | 0.36 |
| 227764_at | LYPD6 | 0.36 |
| 218935_at | EHD3 | 0.36 |
| 219659_at | ATP8A2 | 0.36 |
| 231778_at | DLX3 | 0.36 |
| 202376_at | SERPINA3 | 0.36 |
| 222015_at | CSNK1E | 0.36 |
| 220984_s_at | SLCO5A1 | 0.35 |
| 224262_at | IL1F10 | 0.35 |
| 206375_s_at | HSPB3 | 0.35 |
| 237225_at | ZFY-AS1 | 0.35 |
| 226872_at | RFX2 | 0.35 |
| 202752_x_at | SLC7A8 | 0.35 |
| 210948_s_at | LEF1 | 0.35 |
| 208216_at | DLX4 | 0.34 |
| 229823_at | RIMS2 | 0.34 |
| 214983_at | TTTY15 | 0.34 |
| 237003_at | BEST3 | 0.34 |
| 205637_s_at | SH3GL3 | 0.34 |
| 1555019_at | CDHR1 | 0.34 |
| 205555_s_at | MSX2 | 0.33 |
| 228975_at | SP6 | 0.33 |
| 214576_at | KRT36 | 0.33 |
| 204720_s_at | DNAJC6 | 0.33 |
| 217031_at | KRT84 | 0.32 |
| 218266_s at | NCS1 | 0.32 |
| 219612_s_at | FGG | 0.31 |
| 219301_s_at | CNTNAP2 | 0.31 |
| 233301_at | OXCT2 | 0.31 |
| 211429_s_at | SERPINA1 | 0.31 |
| 219832_s_at | HOXC13 | 0.31 |
| 232684_at | ZNF503-AS1 | 0.30 |
| 222878_s_at | OTUB2 | 0.30 |
| 220090_at | CRNN | 0.30 |
| 206279_at | PRKY | 0.30 |
| 222351_at | PPP2R1B | 0.30 |
| 206423_at | ANGPTL7 | 0.29 |
| 209981_at | CSDC2 | 0.29 |
| 205374_at | SLN | 0.28 |
| 243445_at | BNC2 | 0.28 |
| 206004_at | TGM3 | 0.27 |
| 240967_at | KRTAP19-3 | 0.26 |
| 229158_at | WNK4 | 0.26 |
| 207065_at | KRT75 | 0.26 |
| 220635_at | PSORS1C2 | 0.26 |
| 228705_at | CAPN12 | 0.25 |
| 155641_a_at | KRTAP19-1 | 0.24 |
| 229030_ at | CAPN8 | 0.24 |
| 21451_at | KRTAP5-9 | 0.24 |
| 209921_at | SLC7A11 | 0.23 |
| 210082_at | ABCA4 | 0.23 |
| 206677_at | KRT31 | 0.23 |
| 230760_at | ZFY | 0.23 |
| 230720_at | RNF182 | 0.22 |
| 1564435_a_at | KRT72 | 0.22 |
| 237507_at | KRT73 | 0.21 |
| 208092_s_at | FAM49A | 0.21 |
| 234683_at | KRTAP4-6 | 0.21 |
| 234635_at | KRTAP4-1 | 0.21 |
| 233640_x_at | KRTAP9-4 | 0.20 |
| 205713_s_at | COMP | 0.20 |
| 233681_at | KRTAP3-3 | 0.20 |
| 216810_at | KRTAP4-7 | 0.19 |
| 234639_x_at | KRTAP9-8 | 0.19 |
| 234631_at | KRTAP4-8 | 0.19 |
| 206969_at | KRT34 | 0.19 |
| 224269_at | KRTAP4-12 | 0.18 |
| 208483_x_at | KRT33A | 0.18 |
| 211149_at | UTY | 0.18 |
| 211029_x_at | FGF18 | 0.18 |
| 234772_s_at | KRTAP2-2 | 0.17 |
| 234678_at | KRTAP4-3 | 0.17 |
| 1564803_at | KRTAP11-1 | 0.17 |
| 233534_at | KRTAP3-2 | 0.17 |
| 220976_s_at | KRTAP1-1 | 0.17 |
| 234684_s_at | KRTAP4-4 | 0.17 |
| 1554398_at | LYG2 | 0.17 |
| 206224_at | CST1 | 0.17 |
| 208532_x_at | KRTAP5-8 | 0.17 |
| 1561330_at | DSG4 | 0.16 |
| 207146_at | KRT32 | 0.16 |
| 242301_at | CBLN2 | 0.16 |
| 234880_x_at | KRTAP1-3 | 0.16 |
| 234691_at | KRTAP2-1 | 0.16 |
| 234679_at | KRTAP9-3 | 0.16 |
| 234680_at | KRTAP17-1 | 0.15 |
| 206027_at | S100A3 | 0.15 |
| 1555775_a_at | ZAR1 | 0.15 |
| 234633_at | KRTAP4-11 | 0.15 |
| 234637_at | KRTAP4-5 | 0.14 |
| 220779_at | PADI3 | 0.14 |
| 1555673_at | KRTAP2-3 | 0.14 |
| 220972_s_at | KRTAP9-9 | 0.14 |
| 234671_at | KRTAP4-2 | 0.13 |
| 232887_at | PIRT | 0.13 |
| 207670_at | KRT85 | 0.13 |
| 233537_at | KRTAP3-1 | 0.13 |
| 221297_at | GPRC5D | 0.12 |
| 233158_at | KRT82 | 0.12 |
| 237853_x_at | KRTAP10-12 | 0.12 |
| 219270_at | CHAC1 | 0.12 |
| 1562629_a_at | KRT40 | 0.11 |
| 207787_at | KRT33B | 0.11 |
| 228492_at | USP9Y | 0.11 |
| 1564960_at | KRTAP7-1 | 0.11 |
| 207457_s_at | LY6G6D | 0.11 |
| 213711_at | KRT81 | 0.10 |
| 216921_s_at | KRT35 | 0.10 |
| 1560897_a_at | KRTAP10-11 | 0.10 |
| 1564974_at | KRTAP8-1 | 0.09 |
| 207669_at | KRT83 | 0.09 |
| 207063_at | NCRNA00185 | 0.08 |
| 232618_at | TXLNG2P | 0.07 |
| 205000_at | DDX3Y | 0.05 |
| 206700_s_at | KDM5D | 0.04 |
| 201909_ at | RPS4Y1 | 0.04 |
| 204409_s_at | EIF1AY | 0.04 |
| 208331_at | BPY2 | 0.03 |

By comparing the corresponding lists of genes identified as differentially expressed to each other **(****FIG. 24A****,** bottom panel), three striking gene expression signatures were uncovered in this analysis. First, many of the upregulated genes in alopecic skin of both species were IFN-response genes, including the IFN-inducible chemokines CXCL 9,10 and 11¹¹ **(****FIG. 24A****,** **FIG. 29****).** Secondly, several key CTL-specific transcripts were identified, including CD8A and granzymes A and B **(****FIG. 24A****).**

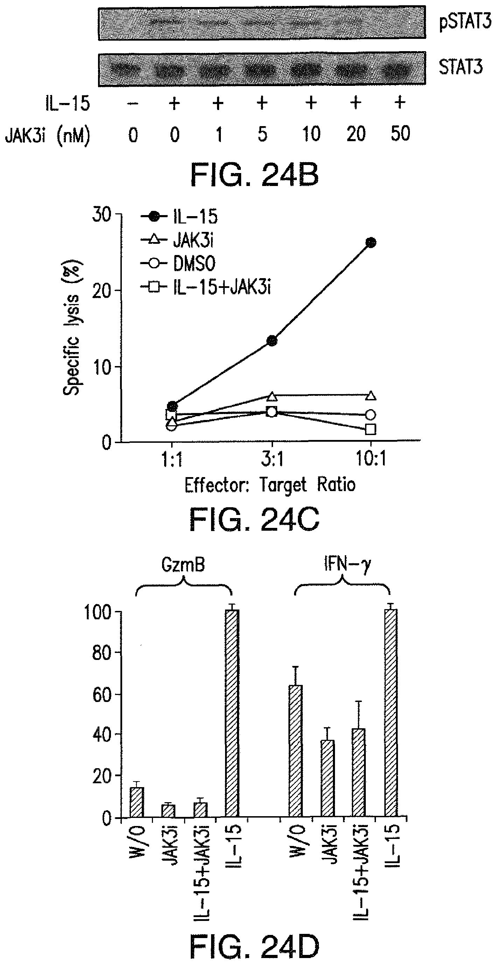

In turn, using CTL effectors, the small molecule JAK3 inhibitor, tofacitinib (JAK3>JAK1>>JAK2 selectivity)¹⁸, was shown to block IL-15 triggered pSTAT3 activation **(****FIG. 24B****),** and inhibit CTL effector functions, including dermal sheath cell cytotoxicity **(****FIG. 24C****),** and granzyme B/IPNγ production **(****FIG. 24D****).** Thus, hair follicle production of IL-15 provides a cytokine axis that can be interrupted pharmacologically by inhibiting its signaling in immune effectors.

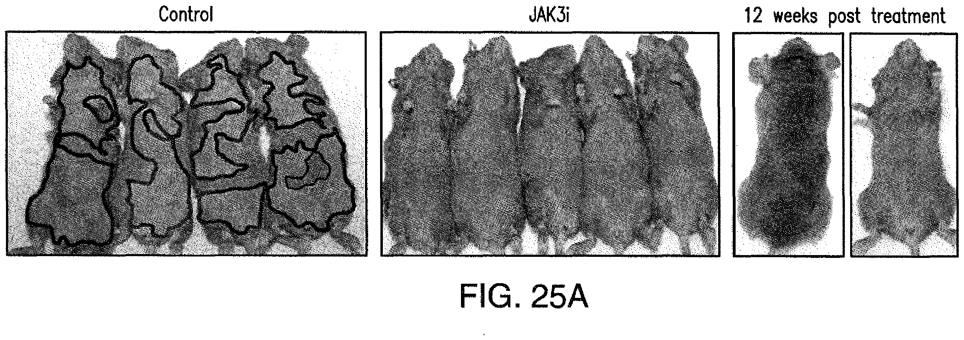

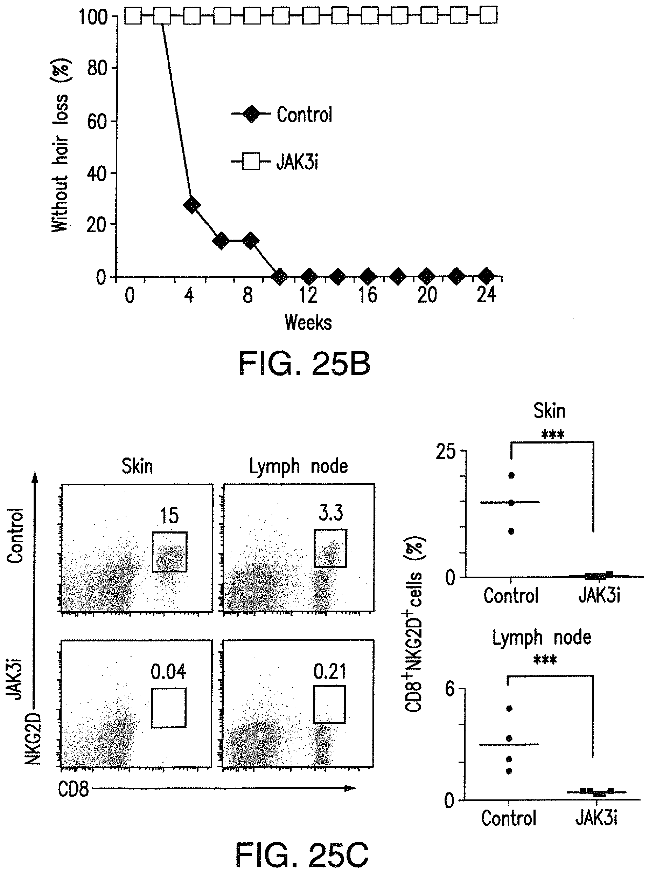

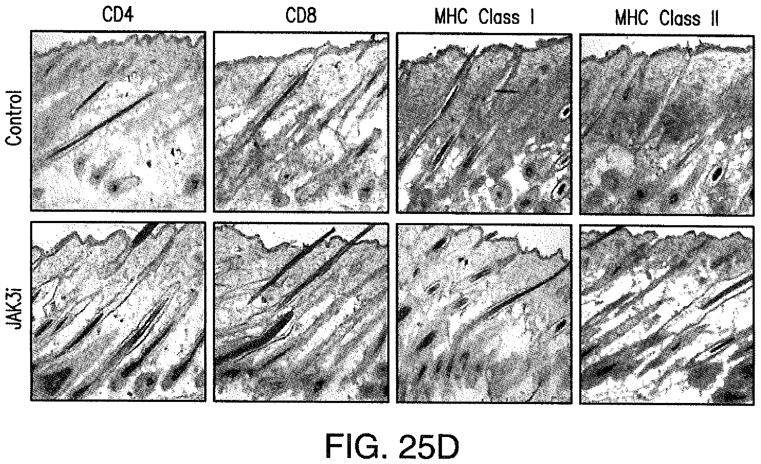

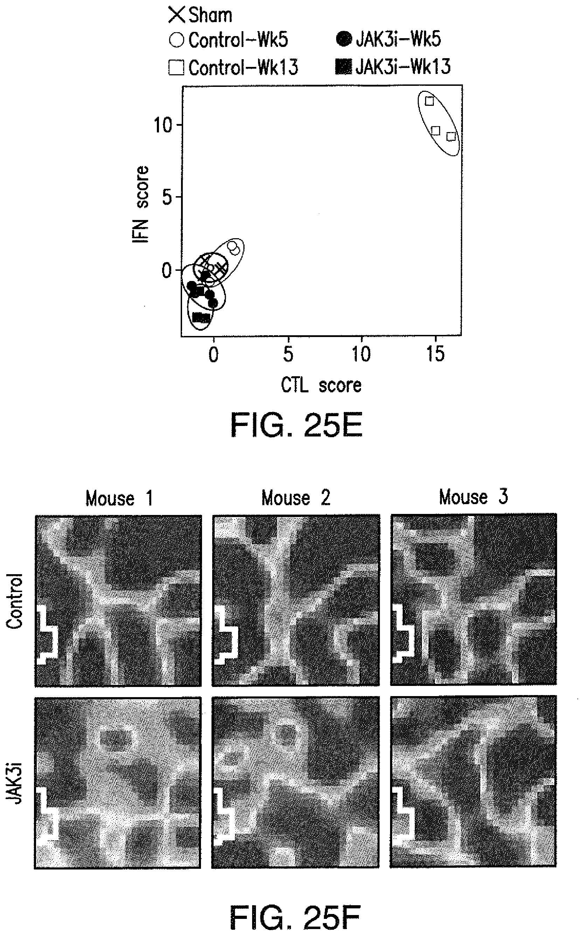

Next, it was determined whether the effects of IL-15 blockade could be recapitulated by intervening downstream using small molecule inhibitors of JAK3. Using tofacitinib, systemic administration of JAK3i **(****FIG. 25****)** treatment prevented the development of AA and the expansion of CD8⁺NKG2D⁺ T cells in all grafted recipients. Skin of mice treated with the protein tyrosine kinase inhibitor (PTKi) showed no histological signs of inflammation and prevented the emergence of the skin ALADIN (*Al*opecia *A*reata *D*isease Activity *In*dex) transcriptional signature and Gene Expression Dynamic Index (GEDI) analysis in all mice **(****FIG. 25****).**

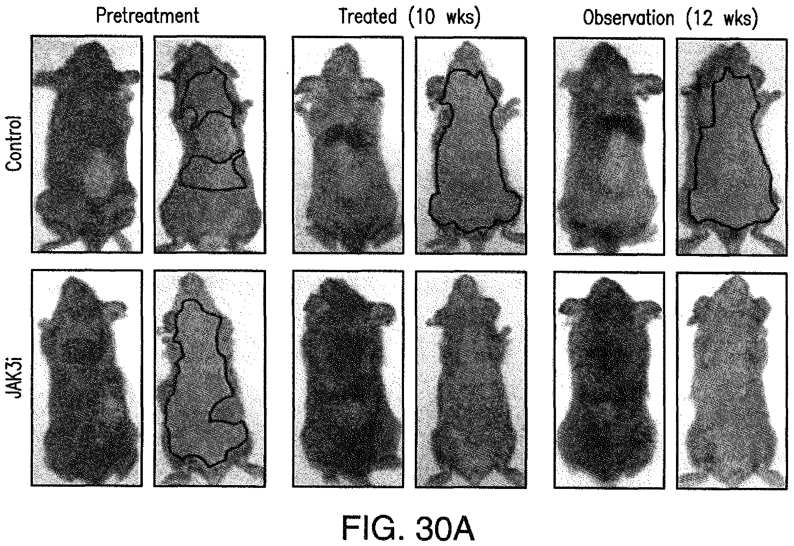

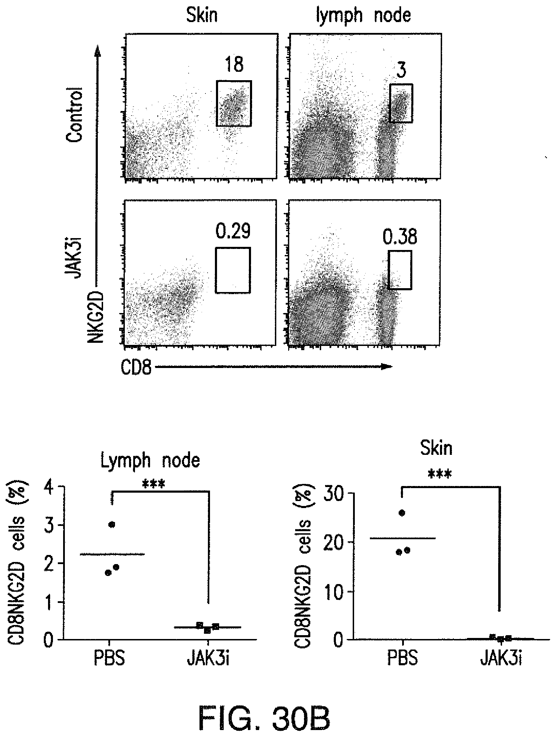

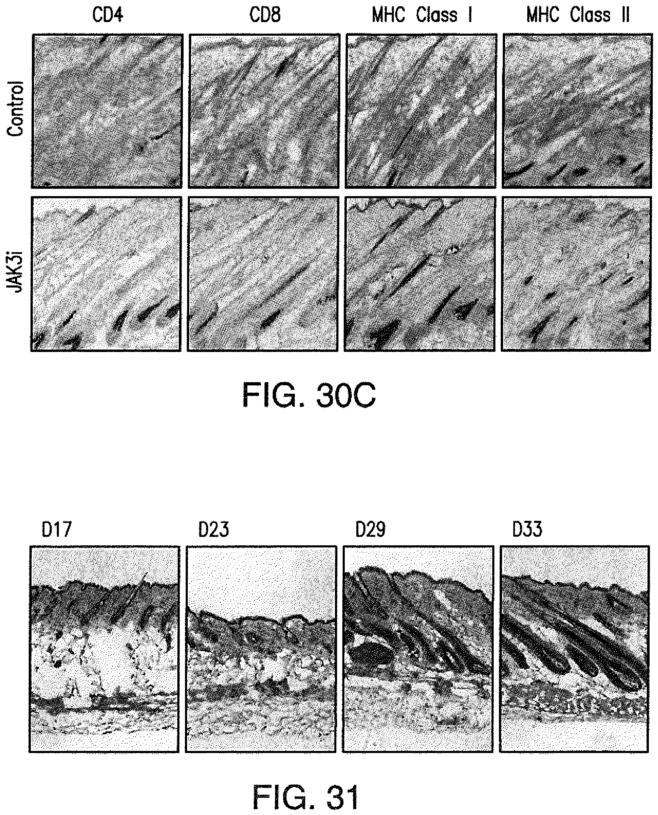

To evaluate the efficacy of JAK3i in the clinical context of AA, it was determined whether systemic JAK3i treatment could reverse established disease by initiating therapy 7 weeks after grafting, a time point at which all mice had developed extensive AA. Importantly, systemic therapy induced hair regrowth all over the body, and likewise eliminated the expansion of CD8⁺NKG2D⁺ T cells and reversed histological markers of disease **(****FIG. 30****),** an effect that persisted 2-3 months after the cessation of treatment.

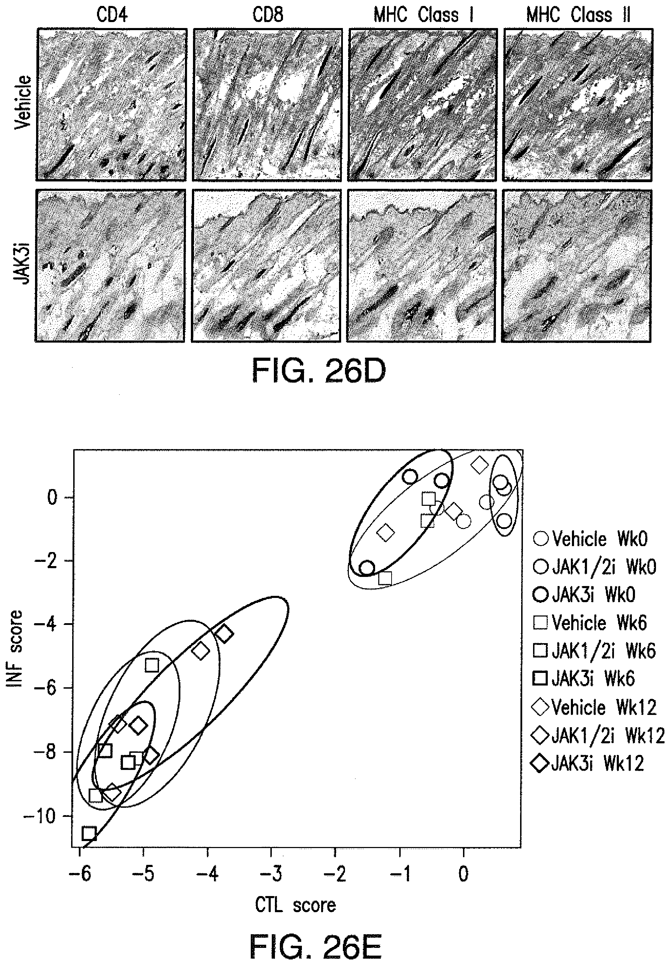

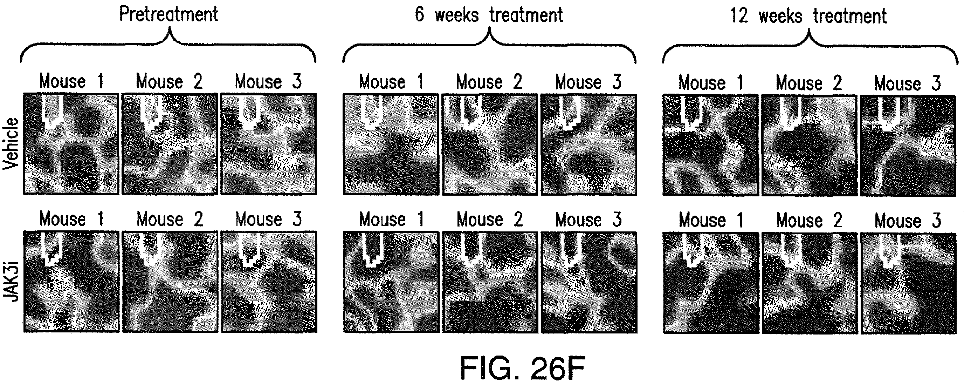

Finally, to test a more clinically convenient route of delivery, it was determined whether topical administration of PTKis could reverse long-standing alopecia areata with similar kinetics as systemic delivery. In established disease, topical tofacitinib, was highly effective in reversing disease in treated lesions (applied to back skin), and complete hair regrowth was observed within 12 weeks following topical therapy **(****FIG. 26A-B).** Topical therapy was associated with the disappearance of CD8⁺NKG2D⁺ T cells in the treated skin **(****FIG. 26C****)** and the reversal of histological markers of disease (**FIG. 26D****),** as well as the normalization of the ALADIN transcriptional signature **(****FIG. 26E****),** and the GEDI index in all treated mice (**FIG. 26F**). Notably, untreated areas on the abdomen remained alopecic **(****FIG. 26A****,** middle panels), demonstrating that topical therapy was locally effective and therapeutic effects were not the result of systemic absorption. Further, these effects were durable 2-3 months after the cessation of treatment **(****FIG. 26A****,** right panels).

Taken together, the data identify CD8⁺NKG2D⁺ T cells as the immune cellular effectors responsible for autoimmune attack of the hair follicle and provide support for a model of AA pathogenesis.

Importantly, these common mechanistic underpinnings were first revealed by in the GWAS study, which placed AA squarely among this group of allied autoimmune diseases involving NKG2DL mediated recruitment of CD8 T cell effectors, including type I diabetes^{23,24}, celiac disease^{16,25} and rheumatoid arthritis²⁶. These pathways can be interrupted by small molecule inhibitors of the IL-15 downstream effector JAK kinases, the latter being particularly appealing as a topical therapeutic approach in this cutaneous disease. FDA-approved JAK inhibitors were used to show the therapeutic effect, arguing for clinical evaluation in AA with these compounds or other JAK PTKis in clinical development²⁸.

In a very short time since the GWAS findings, not only have the specific subset of cytotoxic T cells that give rise to AA been identified, but they have successfully targeted them for elimination using clinically relevant, rational therapies selected on the basis of our mechanistic studies. The findings illustrate the power of GWAS studies in uncovering new disease pathways, and, coupled with translational approaches²⁹, have rapidly opened a new and unexpected avenue for intervention in AA.

### Methods

Mice. 7-10 week old female C57B1.6 and C3H/HeJ mice (Jackson Laboratories, Bar Harbor, ME) were used and maintained under specific pathogen-free conditions.

**Transfer of Alopecia Areata using grafted C3H/HeJ skin.** Normal-haired C3H/HeJ mice were grafted at 8 weeks of age (during the second telogen) with skin from a C3H/HeJ mouse that developed AA spontaneously, as described previously⁷. In brief, mice spontaneously affected with AA were euthanized, full thickness skin grafts of approximately 2 cm in diameter were removed and grafted to normal-haired C3H/HeJ mice. Hair loss typically began at around 4-6 weeks after grafting.

**Flow cytometric analysis of skin and cutaneous lymph nodes.** To make a single cell suspension of mouse skin, fat was removed from the overlying skin in cold PBS and then incubated in collagenase type I (2 mg/ml in PBS) at 32°C for 75 minutes. After digestion the skin was minced in RPMI/10% fetal bovine serum, filtered through a 70mM cell strainer, and centrifuged at 1100g for 5 minutes. The pellet was resuspended in RPMI/10% fetal bovine serum, filtered through a 40mM cell strainer and spun at 400g for 5mins. The pellet was resuspended in FACs buffer (PBS/5% BSA), DAPI to gate on live cells and staining antibodies (listed in Supplemental Data). Cutaneous lymph nodes were pooled minced in RPMI, filtered through a 40mM cell strainer, centrifuged at 400g for 5 minutes stained and analysed on a BD LSR II flow cytometer.

**Transfer of T cell populations into recipient C3H/HeJ mice.** For positive selection of T cell populations, lymph node cells were obtained from 5 C3H/HeJ alopecic mice, stained with anti-CD4, anti-CD8, and anti-NKG2D antibodies, then sorted (BD Influx) to obtain two fractions: CD8⁺NKG2D⁺T cells, and CD8⁺NKG2D⁻T cells. Three to five 7-week-old C3H/HeJ mice per group were injected subcutaneously with two million sorted cells of each population. For negatively selected populations, NKG2D⁺ cells were depleted by incubating total lymph node cells from 3 alopecic C3H/HeJ mice with biotinylated anti-NKG2D (CX5) and then with streptavidin-conjugated beads (Miltenyi) prior to removal on a Miltenyi magnetic column. Five million cells (either CD8/NKG2D-depleted or total lymph node cells) were suspended in 100ul PBS and transferred into each of 5 mice by subcutaneous injection.

**Prevention and Treatment Studies.** For prevention studies, mice were treatment beginning the day of grafting (n=5-10 mice per group). For JAK3i experiments: mice were implanted subcutaneously with Alzet osmotic mini-pumps (pumps, model 2004, Durect Corporation) on the back of each mouse to deliver vehicle (poly(ethylene glycol) (PEG)300) or vehicle containing the JAK3i tofacitinib (Abmole) at 15 mg/kg/day for 12 weeks.

For topical treatment studies, grafted mice with long-standing AA (more than 8 weeks) were treated once daily for 12 weeks to affected skin on the dorsal back with vehicle (Aquaphor) or vehicle containing the JAK3 inhibitor (0.5% ointment). Full-thickness skin biopsies were excised from the dorsal surface of each mouse at interim time points, and skin samples were either snap frozen in liquid nitrogen for RNA extraction or snap frozen in OCT for immunostaining. Hair status was examined twice weekly and hair growth index calculated as described³⁰.

**Immunohistochemistry and immunofluorescence.** 8 mM acetone-fixed frozen mouse skin sections were air-dried and stained overnight with anti-mouse Abs (see below) at 4°C in a moist chamber. Human hair follicles were microdissected and embedded in OCT compound prior to sectioning and staining (see below).

**Primary dermal sheath and lymphokine-activated killer (LAK) cell culture.** Dermal sheath (DS) cells were isolated from microdissected mouse vibrissa follicles and cultured in 20% FBS DMEM with 5ng/ml murine FGF (Pepro Tech). LAK cells were generated from splenocytes plated at 4×10⁶ in 6-well plates with 50nM JAK3i (tofacitinib) or 50ng/ml murine IL-15 plus 50nM JAK3i, and incubated at 37°C in a 5% CO₂ incubator for 96 hours.

**In vitro cytotoxicity assays.** Determination of specific killing of target cells was performed using CFSE-labeled DS cells at targets mixed with different ratios of effector cells incubated for 5 hours at 37°C 5% CO2 with or without neutralizing rat anti-mouse NKG2D antibody (20ug/ml) (Biolegend, CX5). Specific lysis of DS cells was determined flow cytometrically by measuring cell death of CFSE+ DS cells using Annexin V/7-AAD.

**Gene expression analysis in human and mouse skin and T cells.** Total RNA was isolated using the miRNeasy Mini Kit (Qiagen Inc., Valencia, CA, USA) with on-column DNA digestion using the RNase-free DNase set (Qiagen, Inc.). For RNAseq analysis CD3+CD8+CD44+NKG2D+ and CD3+ CD8+CD44+NKG2D- cells were flow-sorted from lymph nodes of alopecic C3H/HeJ mice. RNA was extracted as above and prepared for RNA-seq using the TruSeq RNA Sample Prep Kit v2. Samples were sequenced on the HiSeq 2000 sequencer (Illumina, San Diego, CA) for 50 cycles. RNA-Seq files were demultiplexed by the Rockefeller University Genomics Core Facility.

For global transcriptional profiling in mouse skin, total extracted RNA was processed using the 3' IVT Express Kit from Affymetrix. Resulting biotinylated cDNA samples were hybridized to the Mouse Genome 430 2.0 gene chips and subsequently washed, stained with streptavidin-phycoerythrin, and scanned on an HP GeneArray Scanner (Hewlett-Packard Company, Palo Alto, CA).

For human AA samples, perilesional punch biopsies from 5 patients with patchy alopecia areata who were not undergoing local or systemic treatments were collected and compared to scalp biopsies from 5 unrelated unaffected individuals.

Extracted total RNA were reverse transcribed and amplified using the Ovation RNA Amplification V2 kit (NuGEN Technologies, Inc., San Carlos, CA). Amplified cDNA was biotinylated with the Encore Biotin Module (NuGEN Technologies) and then hybridized to the U133A Plus 2.0 gene chips.

**Antibodies used for flow cytometry and immunostaining.** Flow cytometric analysis used the following anti-mouse antibodies: CD3 (17A2, Ebioscience), CD4 (GK1.5, BD), CD8a (53-6.7, BD), CD8a (YTS156.7.7, Biolegend), NKG2D (CX5, Ebioscience), NKG2A/C/E (clone 20d5, Ebioscience), CD44 (IM7, BD), CD45 (30-F11, BD), CD49b (Dx5, BD), CD62L (MEL-14, BD), CD69 (H1.2F3, BD), CD103 (2E7, eBioscience), IFNγ (XMG1.2, Ebioscience), Granzyme B (NGZB, eBioscience), Rae-1 (186107, R&D).

For immunohistochemical studies of mouse skin, 8 µM methanol-fixed frozen skin sections were stained with primary rat antibodies (Biolegend) including: anti-CD4 (clone RM4-5), anti-CD8 (clone 53-6.7), Biotin anti-MHC class I (clone 36-7.5), anti-MHC class II (clone M5/114.15.2). Biotinylated goat anti-rat IgG (Life Technologies) was used as secondary antibody. For immunofluorescence studies anti-H60 (R&D, clone 205326), anti-Pan Rae-1(R&D, clone 186107), anti-NKG2D (R&D clone 191004), anti-K71 (Abcam) primary antibody were used in immunofluorescence. Alexa Fluor 488 or Alexa Fluor 594-conjugated goat anti-Rat, donkey anti-Rabbit or donkey anti-Goat antibody was used as secondary antibody (Life Technologies).

Human hair follicles were microdissected and embedded in OCT compound prior to sectioning and staining. 8 µM methanol-fixed frozen sections were stained with CD8 (SCBT, C8/144B) followed by staining with Alexa Fluor 488 or Alexa Fluor 594-conjugated secondary antibody (Life Technologies). All images were captured with an SDRC Zeiss Exciter Confocal Microscope.

### Statistical Analysis and Quality Control of Gene Expression Signatures

**RNA-Seq analysis.** Samples were sequenced on the HiSeq 2000 sequencer (Illumina, San Diego, CA) for 50 cycles. RNA-Seq files were demultiplexed by the Rockefeller University Genomics Core Facility. Quality control of the sample fastq files was performed using fastqc'^{S1}. TopHat'^{S2} was used to map transcripts to the UCSC mm9 reference genome from iGenome. The RefSeq gene annotation packaged with this iGenome version of the UCSC mm9 were used. The htseq-count utility from the HTSeq package was used to convert TopHat bam files to counts that could be used as input for downstream analysis of differential expression with edgeR'^{S3}. Absent genes were removed and a pseudocount of 1 was added in order to avoid division by zero in downstream analysis. EdgeR was used to identify differentially expressed genes using a matched pairs design with three biological replicates.

### Microarray Analysis.

*Quality Control, Preprocessing.* For the mouse cDNA samples were hybridized to the Mouse Genome 430 2.0 gene chips and subsequently washed, stained with streptavidin-phycoerythrin, and scanned on an HP GeneArray Scanner (Hewlett-Packard Company, Palo Alto, CA). For the human, amplified cDNA was hybridized to the U133A 2.0 gene chips.

Quality control was performed using the affyanalysisQC package from http://arrayanalysis.org/. AffyanalysisQC uses R/BioConductor packages: affy, affycomp, affypdnn, affyPLM, affyQCReport, ArrayTools, bioDistm biomaRt, simpleaffy, yaqcaffy to perform QC within a single script. RMA normalization'^{S4} was performed on each experimental group separately. Batch effect correction using ComBat was required for the prevention experiments.

Microarray preprocessing was performed using BioConductor in R. Preprocessing of the three experiments, 1) spontaneous AA mice vs. normal mice, 2) prevention mice with three treatments vs placebo and sham-operated mice, and 3) treatment mice for two treatments vs. placebo were performed separately using the same pipeline. In addition to the preprocessing that was done for the mouse skin samples, Harshlight was used to correct for image defects for the human skin samples.

### Identification of Gene Signatures.

*Unsupervised analysis.* Hierarchical clustering was performed using Cluster'^{S5} on the 363 genes from the human 5x5 and 583 genes from the spontaneous mouse 3x3 in order that met the threshold abs(logFC) > 1, unadjusted p-value <= 0.05. Genes were first selected that met the threshold logFC > 1, and unadjusted p-value <= 0.05.Genes were median centered and normalized. Spearman rank correlation was used as the similarity measure and average linkage was used to perform row (genes) and column (sample) clustering. Visualization of the hierarchical clusters was performed with java TreeView'^{S6}. Gene Expression Dynamic Index (GEDI) analysis was used to visualize how "metagenes" identified with a self organizing map algorithm vary across samples'^{S7}. Metagenes are clusters of genes that show similar expression patterns across samples and that are assigned to a single pixel in a two dimensional grid. Neighboring pixels demonstrate similar expression patterns to one another.

*Supervised analysis.* Initial analysis of differential gene expression was performed on the spontaneous mouse 3x3 and the human 5x5 data sets using limma'^{S8}. A threshold of 1.5 fold change and unadjusted p-value of 0.05.

*RT-PCR Validation.* Predicted differentially expressed genes in human and mouse were confirmed using RT-PCR. First-strand cDNA was synthesized using a ratio of 2:1 random primers: Oligo (dT) primer and SuperScript III RT (Invitrogen) according to the manufacturer's instructions. qRT-PCR was performed on an ABI 7300 machine and analyzed with ABI Relative Quantification Study software (Applied Biosystems, Foster City, CA, USA). Primers were designed according to ABI guidelines and all reactions were performed using *Power* SYBR Green PCR Master Mix (Applied Biosystems), 250 nM primers (Invitrogen) and 20 ng cDNA in a 20µL reaction volume. Primer sequences are provided in **Table 5.**

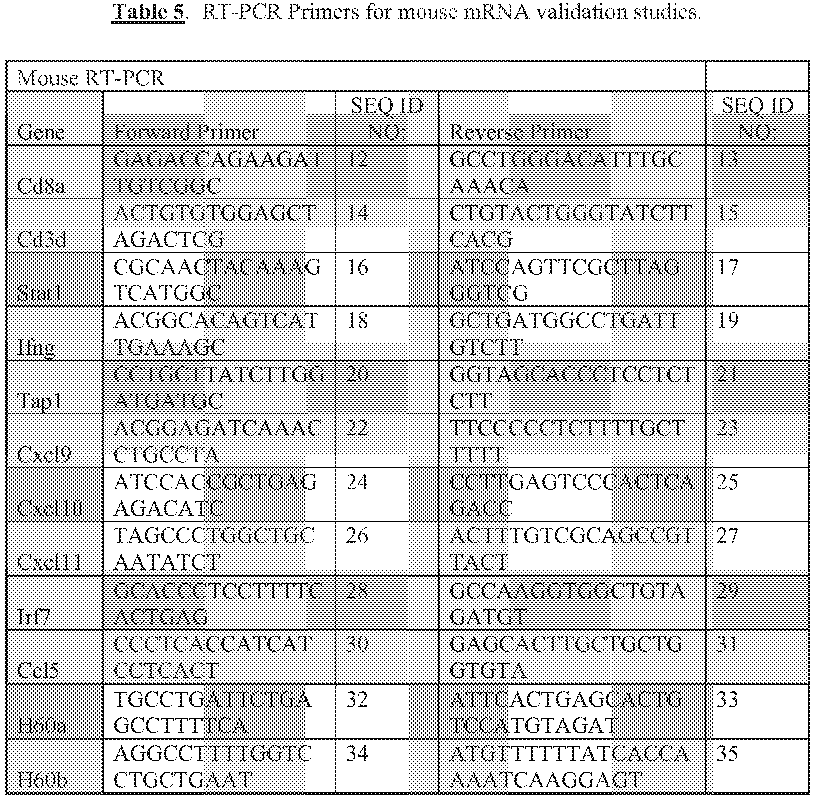

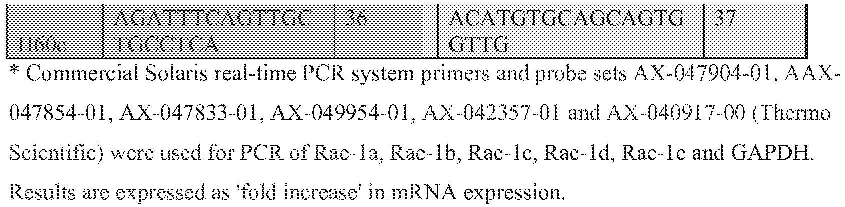

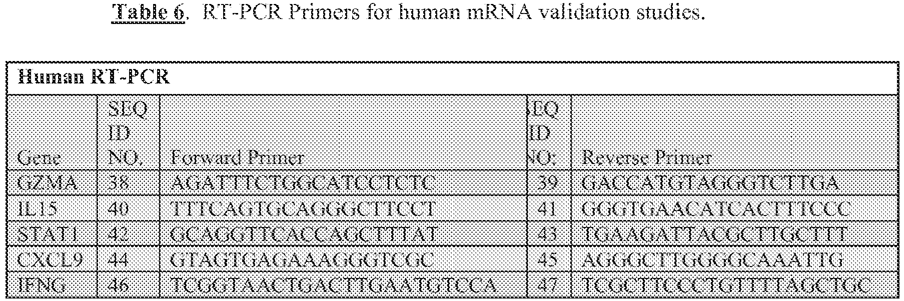

The following PCR protocol was used: step 1: 50°C for 2 min; step 2: 95°C for 10 min; step 3: 95°C for 15 s; step 4: 60°C for 1 min; repeat steps 3 and 4 for 40 cycles. All samples were run in quadruplicate for three independent runs and normalized against an endogenous internal control as indicated.

*ALADIN scores.* The IFN and CTL signatures were used to develop a bivariate score statistic. Individual signature IFN and CTL scores were determined following procedures used in human SLE'^{S9,S10}. The sets of genes selected to comprise our IFN and CTL signatures were CD8A, GZMB, and ICOS for the CTL signature, and CXCL9, CXCL10, CXCL11, STAT1, and MX1 for the IFN signature. The scores for the prevention mice were calculated in relation to the sham mice; whereas, the scores for the topical treatment experiments were calculated relative to all the samples at week zero.

### References

1 McDonagh, A. J. & Messenger, A. G. The pathogenesis of alopecia areata. Dermatol Clin 14, 661-670 (1996).
2 McElwee, K. J., Tobin, D. J., Bystryn, J. C., King, L. E., Jr. & Sundberg, J. P. Alopecia areata: an autoimmune disease? Exp Dermatol 8, 371-379 (1999).
3 Petukhova, L. et al. Genome-wide association study in alopecia areata implicates both innate and adaptive immunity. Nature 466, 113-117 (2010).
4 Gilhar, A., Ullmann, Y., Berkutzki, T., Assy, B. & Kalish, R. S. Autoimmune hair loss (alopecia areata) transferred by T lymphocytes to human scalp explants on SCID mice. J Clin Invest 101, 62-67 (1998).
5 McElwee, K. J. et al. Transfer of CD8(+) cells induces localized hair loss whereas CD4(+)/CD25(-) cells promote systemic alopecia areata and CD4(+)/CD25(+) cells blockade disease onset in the C3H/HeJ mouse model. J Invest Dermatol 124, 947-957 (2005).
6 Sundberg, J. P., Cordy, W. R. & King, L. E., Jr. Alopecia areata in aging C3H/HeJ mice. J Invest Dermatol 102, 847-856 (1994).
7 McElwee, K. J., Boggess, D., King, L. E., Jr. & Sundberg, J. P. Experimental induction of alopecia areata-like hair loss in C3H/HeJ mice using full-thickness skin grafts. J Invest Dermatol 111, 797-803 (1998).
8 Best, J. A. et al. Transcriptional insights into the CD8(+) T cell response to infection and memory T cell formation. Nat Immunol, 4, 404-12 (2013).
9 Bezman, N. A. et al. Molecular definition of the identity and activation of natural killer cells. Nat Immunol 13, 1000-1009 (2012).
10 Heng, T. S. & Painter, M. W. The Immunological Genome Project: networks of gene expression in immune cells. Nat Immunol 9, 1091-1094 (2008).
11 Kota, R. S. et al. Regulation of gene expression in RAW 264.7 macrophage cell line by interferon-gamma. Biochem Biophys Res Commun 342, 1137-1146 (2006).
12 Fehniger, T. A. & Caligiuri, M. A. Interleukin 15: biology and relevance to human disease. Blood 97, 14-32 (2001).
13 Ye, W., Young, J. D. & Liu, C. C. Interleukin-15 induces the expression of mRNAs of cytolytic mediators and augments cytotoxic activities in primary murine lymphocytes. Cell Immunol 174, 54-62 (1996).
14 Meresse, B. et al. Reprogramming of CTLs into natural killer-like cells in celiac disease. J Exp Med 203, 1343-1355 (2006).
15 Saikali, P., Antel, J. P., Pittet, C. L., Newcombe, J. & Arbour, N. Contribution of astrocyte-derived IL-15 to CD8 T cell effector functions in multiple sclerosis. J Immunol 185, 5693-5703 (2010).
16 Meresse, B. et al. Coordinated induction by IL15 of a TCR-independent NKG2D signaling pathway converts CTL into lymphokine-activated killer cells in celiac disease. Immunity 21, 357-366 (2004).
17 Quintas-Cardama, A. et al. Preclinical characterization of the selective JAK1/2 inhibitor INCB018424: therapeutic implications for the treatment of myeloproliferative neoplasms. Blood 115, 3109-3117 (2010).
18 Ghoreschi, K. et al. Modulation of innate and adaptive immune responses by tofacitinib (CP-690,550). Journal of Immunology 186, 4234-4243 (2011).
19 Eichler, G. S., Huang, S. & Ingber, D. E. Gene Expression Dynamics Inspector (GEDI): for integrative analysis of expression profiles. Bioinformatics 19, 2321-2322 (2003).
20 Paus, R., Nickoloff, B. J. & Ito, T. A 'hairy' privilege. Trends Immunol 26, 32-40 (2005).
21 Freyschmidt-Paul, P. et al. Interferon-gamma-deficient mice are resistant to the development of alopecia areata. The British journal of dermatology 155, 515-521 (2006).
22 Waldmann, T. A. The biology of interleukin-2 and interleukin-15: implications for cancer therapy and vaccine design. Nature reviews. Immunology 6, 595-601 (2006).
23 Ogasawara, K. et al. NKG2D blockade prevents autoimmune diabetes in NOD mice. Immunity 20, 757-767 (2004).
24 Markiewicz, M. A. et al. RAElepsilon ligand expressed on pancreatic islets recruits NKG2D receptor-expressing cytotoxic T cells independent of T cell receptor recognition. Immunity 36, 132-141 (2012).
25 Hue, S. et al. A direct role for NKG2D/MICA interaction in villous atrophy during celiac disease. Immunity 21, 367-377 (2004).
26 Andersson, A. K., Feldmann, M. & Brennan, F. M. Neutralizing IL-21 and IL-15 inhibits pro-inflammatory cytokine production in rheumatoid arthritis. Scand J Immunol 68, 103-111 (2008).
27 Baslund, B. et al. Targeting interleukin-15 in patients with rheumatoid arthritis: a proof-of-concept study. Arthritis and rheumatism 52, 2686-2692 (2005).
28 Dolgin, E. Companies hope for kinase inhibitor JAKpot. Nature reviews. Drug discovery 10, 717-718 (2011).
29 Fugger, L., McVean, G. & Bell, J. I. Genomewide association studies and common disease-realizing clinical utility. The New England Journal of Medicine 367, 2370-2371 (2012).
30 Tang, L. et al. Topical mechlorethamine restores autoimmune-arrested follicular activity in mice with an alopecia areata-like disease by targeting infiltrated lymphocytes. J Invest Dermatol 120, 400-406 (2003).

S1 Andrews, S. <http://www.bioinformatics.babraham.ac.uk/projects/fastqc/>
S2 Trapnell, C., Pachter, L. & Salzberg, S. L. TopHat: discovering splice junctions with RNA-Seq. Bioinformatics 25, 1105-1111, doi:10.1093/bioinformatics/btp120 (2009).
S3 Robinson, M. D., McCarthy, D. J. & Smyth, G. K. edgeR: a Bioconductor package for differential expression analysis of digital gene expression data. Bioinformatics 26, 139-140, doi:10.1093/bioinformatics/btp616 (2010).
S4 Irizarry, R. A. et al. Summaries of Affymetrix GeneChip probe level data. Nucleic acids research 31, e15 (2003).
S5 Eisen, M. B., Spellman, P. T., Brown, P. O. & Botstein, D. Cluster analysis and display of genome-wide expression patterns. Proceedings of the National Academy of Sciences of the United States of America 95, 14863-14868 (1998).
S6 Saldanha, A. J. Java Treeview--extensible visualization of microarray data. Bioinformatics 20, 3246-3248, doi:10.1093/bioinformatics/bth349 (2004).
S7 Eichler, G. S., Huang, S. & Ingber, D. E. Gene Expression Dynamics Inspector (GEDI): for integrative analysis of expression profiles. Bioinformatics 19, 2321-2322 (2003).
S8 Smyth, G. K. Limma: linear models for microarray data. In: "Bioinformatics and Computational biology Solutions using R and Bioconductor". 397-420 (Springer, New York, 2005).
S9 Kirou, K. A. et al. Coordinate overexpression of interferon-alpha-induced genes in systemic lupus erythematosus. Arthritis and rheumatism 50, 3958-3967, doi:10.1002/art20798 (2004).
S10 Feng, X. et al. Association of increased interferon-inducible gene expression with disease activity and lupus nephritis in patients with systemic lupus erythematosus. Arthritis and rheumatism 54, 2951-2962, doi:10.1002/art.22044 (2006).

### EXAMPLE 5 - Effect of JAK3 inhibitor on the induction of hair growth

The JAK-STAT signaling pathway has been implicated in several developmental processes, and most recently in stem cell maintenance, activation and differentiation. In the course of the inventor's topical treatment studies using JAK3 in the C3H/HeJ AA mouse model, it was noticed that the hairs that regrew did so with two striking features that were different from systemic administration: 1) hair regrowth was very rapid; and 2) the hair coat was darkly pigmented. Without being bound by theory, in addition to the JAK3 inhibitor eliminating pathogenic T cells from the skin, the JAK3 inhibitor also has a direct effect on the hair follicle itself, for example, via an anagen-promoting effect

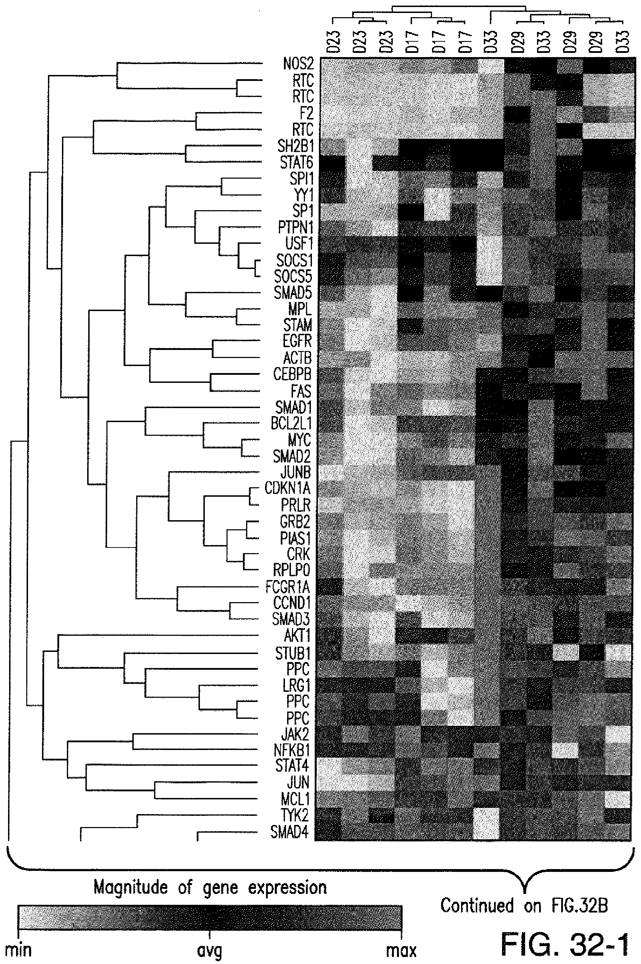

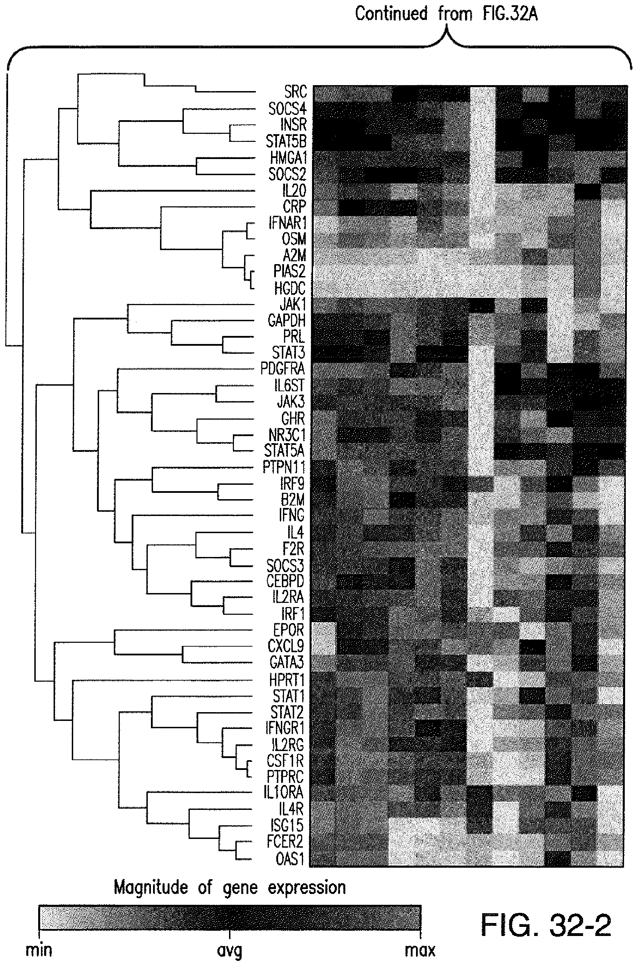

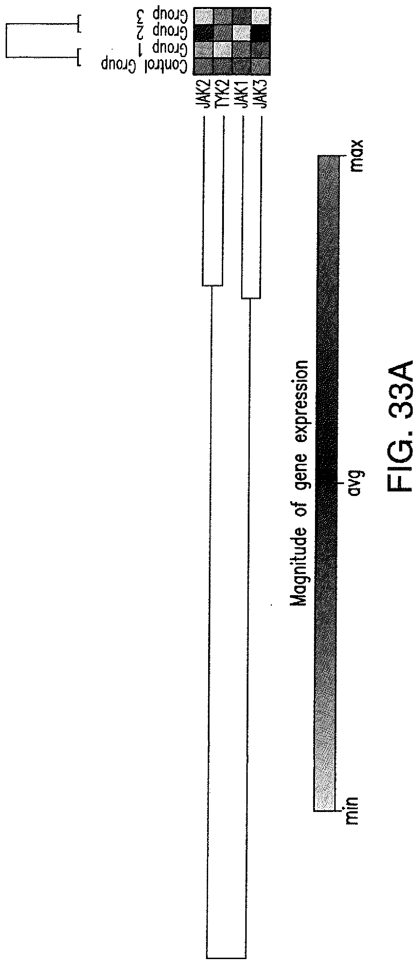

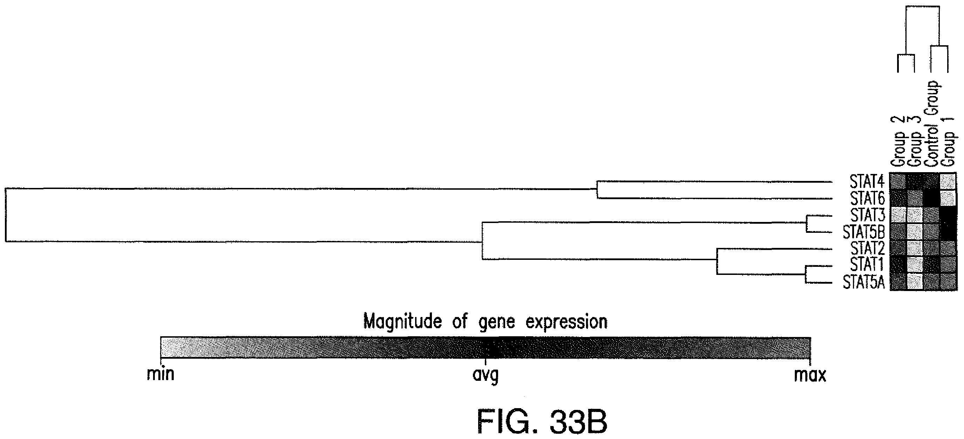

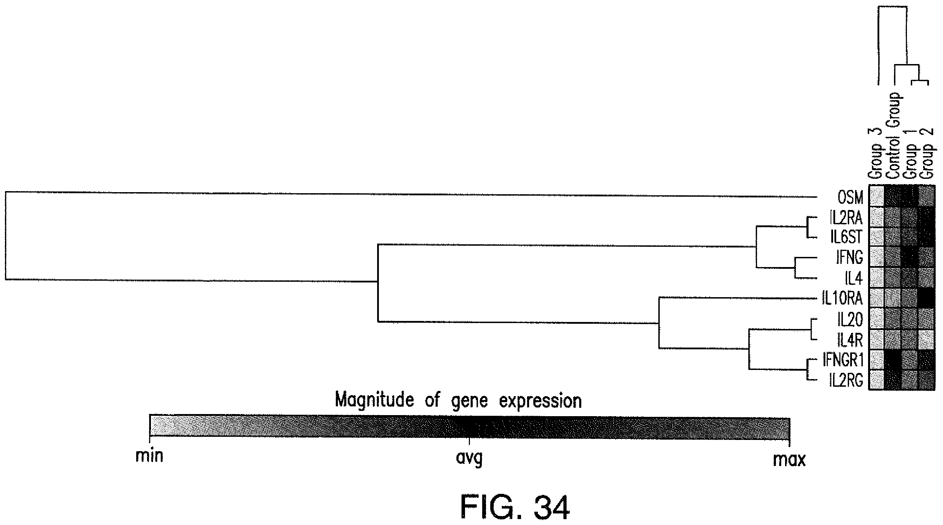

The dynamics of the JAK-STAT signaling pathway were first interrogated using a targeted RT-PCR array containing readouts of JAK-STAT signaling **(****FIG. 32****),** and compared gene expression between the telogen vs. anagen stages of the normal hair cycle **(FIGS. 32-35).**

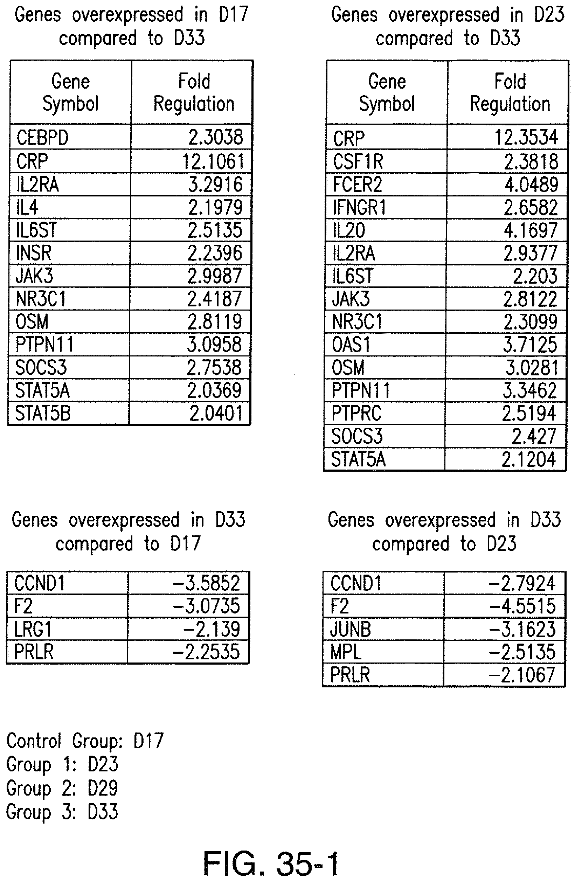

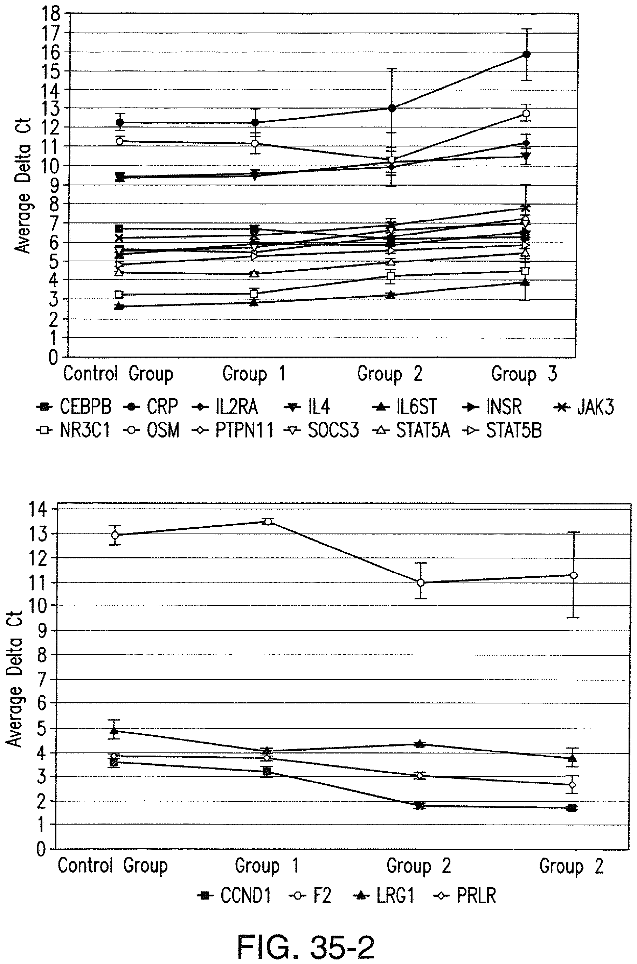

This data revealed that many components of JAK-STAT signaling were upregulated in telogen and downregulated in anagen phase of the normal hair cycle, indicating that in the context of the hair cycle, JAK-STAT signaling can be associated with maintaining stem cell quiescence in telogen **(****FIG. 35****).**

To test whether inhibition of JAK-STAT signaling could therefore trigger the telogen-to-anagen transition, a topical JAK3 inhibitor was applied to test whether anagen could be induced in normal mouse skin in telogen.

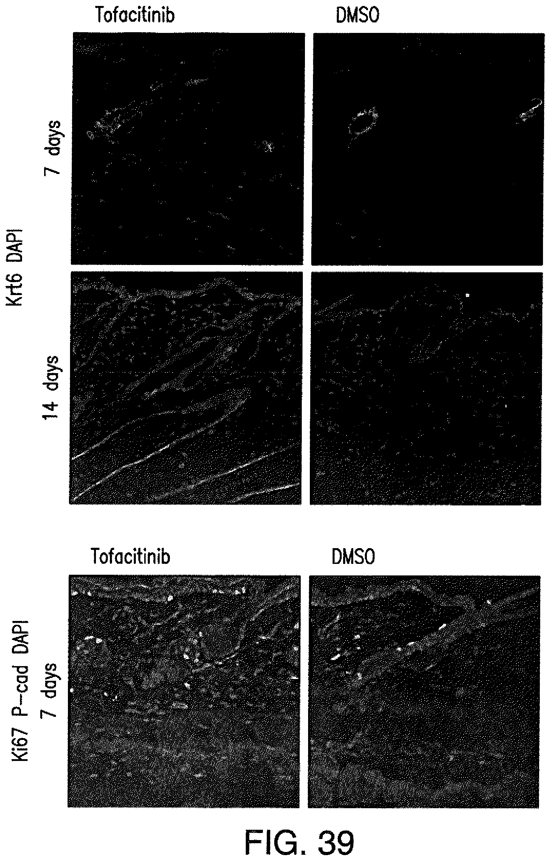

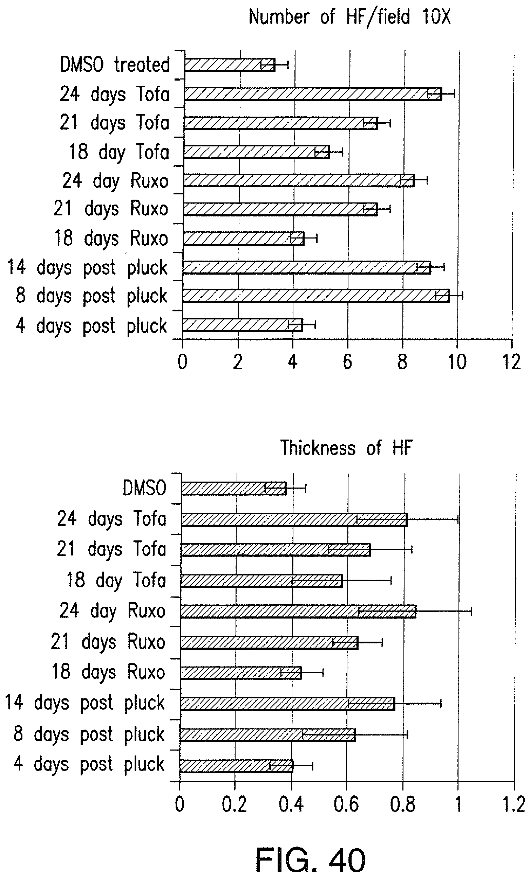

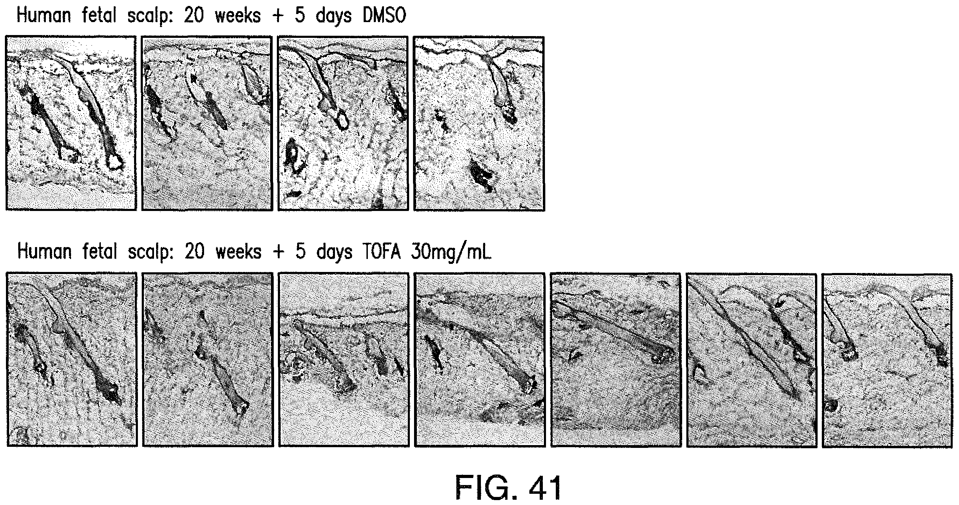

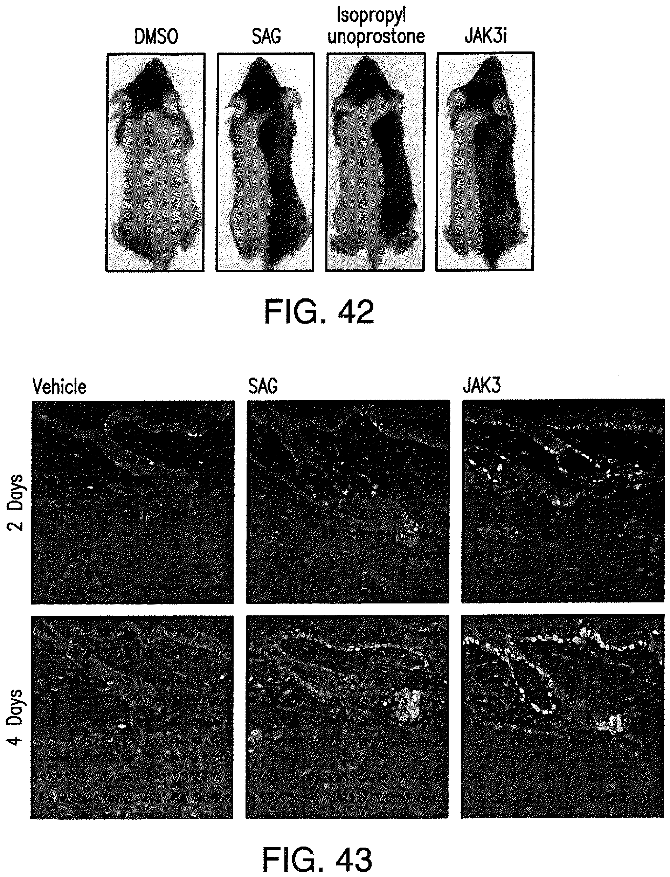

Indeed, topical administration of a JAK3 inhibitor resulted in a striking anagen induction in mouse skin in telogen, compared to vehicle alone. This was associated with marked proliferation of keratinocyte matrix cells, and the induction and growth of robust pigmented anagen hairs after 1-2 weeks (*see* **FIGS. 38-43**).

This observation was compared to a positive control SAG (sonic hedgehog agonist) which is known to have the same effect, and the JAK3 inhibitor was comparable in its effect on anagen induction **(****FIGS. 38****,** **42, 43****).**

Without being bound by theory, these findings indicate that blockade of JAK-STAT signaling in telogen mimics in part the molecular events of anagen initiation, and can be a useful therapeutic agent for hair growth, using topical JAK inhibitors to induce telogen hairs to re-enter anagen.

## Claims

1. A Jak3 inhibitor selected from the group consisting of: decernotinib; tofacitinib; JAK3 Inhibitor IV (ZM-39923); NSC114792; PF-956980; an antisense RNA or antisense DNA that is specific for a nucleic acid encoding a JAK3 polypeptide; and a siRNA that specifically targets the Jak3 gene for use in the treatment of a hair-loss disorder selected from the group consisting of alopecia areata and androgenetic alopecia.

2. The Jak3 inhibitor for use according to claim 1, wherein the inhibitor is decernotinib.

3. The Jak3 inhibitor for use according to claim 1, wherein the inhibitor is tofacitinib.

4. The Jak3 inhibitor for use according to claim 1, wherein the inhibitor is JAK3 Inhibitor IV (ZM-39923); NSC114792; or PF-956980.

5. The Jak3 inhibitor for use according to claim 1, wherein the hair loss disorder is alopecia areata.

6. The Jak3 inhibitor for use according to claim 1, wherein the hair loss disorder is androgenetic alopecia.

7. The Jak3 inhibitor for use according to any one of claims 1 to 6, wherein the Jak3 inhibitor is intended to be administered subcutaneously, intra-muscularly, intraperitoneally, intradermally, by intravenous injection; by an infusion; parenterally, transdermally, transmucosally, rectally, orally, nasally, or by topical delivery; or a combination thereof.

8. The Jak3 inhibitor for use according to any one of claims 1 to 7, wherein the Jak3 inhibitor is intended to be administered 1 time per week, 2 times per week, 3 times per week, 4 times per week, 5 times per week, 6 times per week, 7 times per week, 8 times per week, 9 times per week, 10 times per week, 11 times per week, 12 times per week, 13 times per week, or 14 times per week.

9. The Jak3 inhibitor for use according to any one of claims 1 to 8, wherein the Jak3 inhibitor is intended to be administered for at least 1 week, at least 2 weeks, at least 3 weeks, at least 4 weeks, at least 5 weeks, at least 6 weeks, at least 8 weeks, at least 12 weeks, or at least 16 weeks.

10. The Jak3 inhibitor for use according to any one of claims 1 to 9, wherein the Jak3 inhibitor is intended to be administered with a Jak 1/2 inhibitor to the subject.

11. The Jak3 inhibitor for use according to claim 10, wherein the Jakl/2 inhibitor is intended to be administered simultaneously with the Jak3 inhibitor; or wherein the Jak1/2 inhibitor is intended to be administered in any order with the Jak3 inhibitor.

12. The Jak3 inhibitor for use according to any one of claims 10 or 11, wherein the Jak1/2 inhibitor is ruxolitinib, figlotinib, AG490, momelotinib, pacritinib, baricitinib, fedratinib, BMS-911543, or lestaurtinib.

13. The Jak3 inhibitor for use according to claim 1, wherein the inhibitor is tofacitinib, the hair loss disorder is alopecia areata, and the inhibitor is intended to be administered topically.

14. The Jak3 inhibitor for use according to claim 1, wherein the inhibitor is administered to the subject in a pharmaceutical composition selected from solution, ointment, salve, gel, and cream.

15. The Jak3 inhibitor for use according to claim 1, wherein the inhibitor is contained in a topical pharmaceutical composition at a concentration selected from about 0.1%, about 0.2%, about 0.3%, about 0.4%, about 0.5%, about 0.6%, about 0.7%, about 0.8%, about 0.9%, about 1.0%, about 1.1%, about 1.2%, about 1.3%, about 1.4%, about 1.5%, about 1.6%, about 1.7%, about 1.8%, about 1.9%, about 2.0%, about 2.1%, about 2.2%, about 2.3%, about 2.4%, about 2.5%, about 2.6%, about 2.7%, about 2.8%, about 2.9%, about 3.0%, about 3.5%, about 4%, about 4.5%, about 5%, about 5.5%, about 6%, about 6.5%, about 7%, about 7.5%, about 8%, about 8.5%, about 9%, about 9.5%, or about 10%.

16. The Jak3 inhibitor for use according to claim 1, wherein the inhibitor is administered to the subject in an amount of about 0.0001 µg/kg body weight, about 0.00025 µg/kg body weight, about 0.0005 µg/kg body weight, about 0.00075 µg/kg body weight, about 0.001 µg/kg body weight, about 0.0025 µg/kg body weight, about 0.005 µg/kg body weight, about 0.0075 µg/kg body weight, about 0.01 µg/kg body weight, about 0.025 µg/kg body weight, about 0.05 µg/kg body weight, about 0.075 µg/kg body weight, about 0.1 µg/kg body weight, about 0.25 µg/kg body weight, about 0.5 µg/kg body weight, about 0.75 µg/kg body weight, about 1 µg/kg body weight, about 5 µg/kg body weight, about 10 µg/kg body weight, about 25 µg/kg body weight, about 50 µg/kg body weight, about 75 µg/kg body weight, about 100 µg/kg body weight, about 150 µg/kg body weight, about 200 µg/kg body weight, about 250 µg/kg body weight, about 300 µg/kg body weight, about 350 µg/kg body weight, about 400 µg/kg body weight, about 450 µg/kg body weight, about 500 µg/kg body weight, about 550 µg/kg body weight, about 600 µg/kg body weight, about 650 µg/kg body weight, about 700 µg/kg body weight, about 750 µg/kg body weight, about 800 µg/kg body weight, about 850 µg/kg body weight, about 900 µg/kg body weight, about 950 µg/kg body weight, about 1000 µg/kg body weight, about 2000 µg/kg body weight, about 3000 µg/kg body weight, about 4000 µg/kg body weight, about 5000 µg/kg body weight, about 6000 µg/kg body weight, about 7000 µg/kg body weight, about 8000 µg/kg body weight, about 9500 µg/kg body weight, or about 10,000 µg/kg body weight, about 1 mg/kg body weight, about 1.5 mg/kg body weight, about 2 mg/kg body weight, about 2.5 mg/kg body weight, about 3 mg/kg body weight, about 3.5 mg/kg body weight, about 4 mg/kg body weight, about 4.5 mg/kg body weight, about 5 mg/kg body weight, about 5.5 mg/kg body weight, about 6 mg/kg body weight, about 6.5 mg/kg body weight, about 7 mg/kg body weight, about 7.5 mg/kg body weight, about 8 mg/kg body weight, about 9.5 mg/kg body weight, about 10 mg/kg body weight, about 10.5 mg/kg body weight, about 11.0 mg/kg body weight, about 11.5 mg/kg body weight, about 12 mg/kg body weight, about 12.5 mg/kg body weight, about 13 mg/kg body weight, about 13.5 mg/kg body weight, about 14 mg/kg body weight, about 14.5 mg/kg body weight, about 15 mg/kg body weight, about 15.5 mg/kg body weight, about 16 mg/kg body weight, about 16.5 mg/kg body weight, about 17 mg/kg body weight, about 17.5 mg/kg body weight, about 18 mg/kg body weight, about 19.5 mg/kg body weight, about 20 mg/kg body weight, about 21.5 mg/kg body weight, about 22 mg/kg body weight, about 22.5 mg/kg body weight, about 23 mg/kg body weight, about 23.5 mg/kg body weight, about 24 mg/kg body weight, about 24.5 mg/kg body weight, about 25 mg/kg body weight, about 25.5 mg/kg body weight, about 26 mg/kg body weight, about 26.5 mg/kg body weight, about 27 mg/kg body weight, about 27.5 mg/kg body weight, about 28 mg/kg body weight, about 29.5 mg/kg body weight, or about 30 mg/kg body weight.

## Patentansprüche

1. Jak3-Inhibitor, der ausgewählt ist aus der Gruppe bestehend aus: Decernotinib; Tofacitinib; JAK3-Inhibitor IV (ZM-39923); NSC114792; PF-956980; einer Antisense-RNA oder Antisense-DNA, die für eine Nukleinsäure spezifisch ist, die ein JAK3-Polypeptid codiert; und einer siRNA, die spezifisch auf das Jak3-Gen abzielt, zur Verwendung in der Behandlung einer Haarausfallstörung, ausgewählt aus der Gruppe bestehend aus Alopecia areata und androgenetischer Alopezie.

2. Jak3-Inhibitor zur Verwendung nach Anspruch 1, wobei der Inhibitor Decernotinib ist.

3. Jak3-Inhibitor zur Verwendung nach Anspruch 1, wobei der Inhibitor Tofacitinib ist.

4. Jak3-Inhibitor zur Verwendung nach Anspruch 1, wobei der Inhibitor JAK3-Inhibitor IV (ZM-39923); NSC114792; oder PF-956980 ist.

5. Jak3-Inhibitor zur Verwendung nach Anspruch 1, wobei die Haarausfallstörung Alopecia areata ist.

6. Jak3-Inhibitor zur Verwendung nach Anspruch 1, wobei die Haarausfallstörung androgenetische Alopezie ist.

7. Jak3-Inhibitor zur Verwendung nach einem beliebigen der Ansprüche 1 bis 6, wobei der Jak3-Inhibitor dazu bestimmt ist, subkutan, intramuskulär, intraperitoneal, intradermal, durch intravenöse Injektion; durch eine Infusion; parenteral, transdermal, transmukosal, rektal, oral, nasal oder durch topische Zuführung; oder eine Kombination davon, verabreicht zu werden.

8. Jak3-Inhibitor zur Verwendung nach einem beliebigen der Ansprüche 1 bis 7, wobei der Jak3-Inhibitor dazu bestimmt ist, 1 Mal pro Woche, 2 Mal pro Woche, 3 Mal pro Woche, 4 Mal pro Woche, 5 Mal pro Woche, 6 Mal pro Woche, 7 Mal pro Woche, 8 Mal pro Woche, 9 Mal pro Woche, 10 Mal pro Woche, 11 Mal pro Woche, 12 Mal pro Woche, 13 Mal pro Woche oder 14 Mal pro Woche verabreicht zu werden.

9. Jak3-Inhibitor zur Verwendung nach einem beliebigen der Ansprüche 1 bis 8, wobei der Jak3-Inhibitor dazu bestimmt ist, während mindestens 1 Woche, mindestens 2 Wochen, mindestens 3 Wochen, mindestens 4 Wochen, mindestens 5 Wochen, mindestens 6 Wochen, mindestens 8 Wochen, mindestens 12 Wochen oder mindestens 16 Wochen verabreicht zu werden.

10. Jak3-Inhibitor zur Verwendung nach einem beliebigen der Ansprüche 1 bis 9, wobei der Jak3-Inhibitor dazu bestimmt ist, mit einem Jak1/2-Inhibitor an das Individuum verabreicht zu werden.

11. Jak3-Inhibitor zur Verwendung nach Anspruch 10, wobei der Jak1/2-Inhibitor dazu bestimmt ist, gleichzeitig mit dem Jak3-Inhibitor verabreicht zu werden; oder wobei der Jak1/2-Inhibitor dazu bestimmt ist, in irgendeiner Reihenfolge mit dem Jak3-Inhibitor verabreicht zu werden.

12. Jak3-Inhibitor zur Verwendung nach einem beliebigen der Ansprüche 10 oder 11, wobei der Jak1/2-Inhibitor Ruxolitinib, Figotinib, AG490, Momelotinib, Pacritinib, Baricitinib, Fedratinib, BMS-911543 oder Lestaurtinib ist.

13. Jak3-Inhibitor zur Verwendung nach Anspruch 1, wobei der Inhibitor Tofacitinib ist, die Haarausfallstörung Alopecia areata ist und der Inhibitor dazu bestimmt ist, topisch verabreicht zu werden.

14. Jak3-Inhibitor zur Verwendung nach Anspruch 1, wobei der Inhibitor dem Individuum in einer pharmazeutischen Zusammensetzung verabreicht wird, die aus einer Lösung, einem Balsam, einer Salbe, einem Gel und einer Creme ausgewählt ist.

15. Jak3-Inhibitor zur Verwendung nach Anspruch 1, wobei der Inhibitor in einer topischen pharmazeutischen Zusammensetzung in einer Konzentration von etwa 0,1%, etwa 0,2%, etwa 0,3%, etwa 0,4%, etwa 0,5%, etwa 0,6%, etwa 0,7%, etwa 0,8%, etwa 0,9%, etwa 1,0%, etwa 1,1%, etwa 1,2%, etwa 1,3%, etwa 1,4%, etwa 1,5%, etwa 1,6%, etwa 1,7%, etwa 1,8%, etwa 1,9%, etwa 2,0%, etwa 2,1%, etwa 2,2%, etwa 2,3%, etwa 2,4%, etwa 2,5%, etwa 2,6%, etwa 2,7%, etwa 2,8%, etwa 2,9%, etwa 3,0%, etwa 3,5%, etwa 4%, etwa 4,5%, etwa 5%, etwa 5,5%, etwa 6%, etwa 6,5%, etwa 7%, etwa 7,5%, etwa 8%, etwa 8,5%, etwa 9%, etwa 9,5% oder etwa 10% enthalten ist.

16. Jak3-Inhibitor zur Verwendung nach Anspruch 1, wobei der Inhibitor dem Individuum in einer Menge von etwa 0,0001 µg/kg Körpergewicht, etwa 0,00025 µg/kg Körpergewicht, etwa 0,0005 µg/kg Körpergewicht, etwa 0,00075 µg/kg Körpergewicht, etwa 0,001 µg/kg Körpergewicht, etwa 0,0025 µg/kg Körpergewicht, etwa 0,005 µg/kg Körpergewicht, etwa 0,0075 µg/kg Körpergewicht, etwa 0,01 µg/kg Körpergewicht, etwa 0,025 µg/kg Körpergewicht, etwa 0,05 µg/kg Körpergewicht, etwa 0,075 µg/kg Körpergewicht, etwa 0,1 µg/kg Körpergewicht, etwa 0,25 µg/kg Körpergewicht, etwa 0,5 µg/kg Körpergewicht, etwa 0,75 µg/kg Körpergewicht, etwa 1 µg/kg Körpergewicht, etwa 5 µg/kg Körpergewicht, etwa 10 µg/kg Körpergewicht, etwa 25 µg/kg Körpergewicht, etwa 50 µg/kg Körpergewicht, etwa 75 µg/kg Körpergewicht, etwa 100 µg/kg Körpergewicht, etwa 150 µg/kg Körpergewicht, etwa 200 µg/kg Körpergewicht, etwa 250 µg/kg Körpergewicht, etwa 300 µg/kg Körpergewicht, etwa 350 µg/kg Körpergewicht, etwa 400 µg/kg Körpergewicht, etwa 450 µg/kg Körpergewicht, etwa 500 µg/kg Körpergewicht, etwa 550 µg/kg Körpergewicht, etwa 600 µg/kg Körpergewicht, etwa 650 µg/kg Körpergewicht, etwa 700 µg/kg Körpergewicht, etwa 750 µg/kg Körpergewicht, etwa 800 µg/kg Körpergewicht, etwa 850 µg/kg Körpergewicht, etwa 900 µg/kg Körpergewicht, etwa 950 µg/kg Körpergewicht, etwa 1000 µg/kg Körpergewicht, etwa 2000 µg/kg Körpergewicht, etwa 3000 µg/kg Körpergewicht, etwa 4000 µg/kg Körpergewicht, etwa 5000 µg/kg Körpergewicht, etwa 6000 µg/kg Körpergewicht, etwa 7000 µg/kg Körpergewicht, etwa 8000 µg/kg Körpergewicht, etwa 9500 µg/kg Körpergewicht, oder etwa 10.000 µg/kg Körpergewicht, etwa 1 mg/kg Körpergewicht, etwa 1,5 mg/kg Körpergewicht, etwa 2 mg/kg Körpergewicht, etwa 2,5 mg/kg Körpergewicht, etwa 3 mg/kg Körpergewicht, etwa 3,5 mg/kg Körpergewicht, etwa 4 mg/kg Körpergewicht, etwa 4,5 mg/kg Körpergewicht, etwa 5 mg/kg Körpergewicht, etwa 5,5 mg/kg Körpergewicht, etwa 6 mg/kg Körpergewicht, etwa 6,5 mg/kg Körpergewicht, etwa 7 mg/kg Körpergewicht, etwa 7,5 mg/kg Körpergewicht, etwa 8 mg/kg Körpergewicht, etwa 9,5 mg/kg Körpergewicht, etwa 10 mg/kg Körpergewicht, etwa 10,5 mg/kg Körpergewicht, etwa 11,0 mg/kg Körpergewicht, etwa 11,5 mg/kg Körpergewicht, etwa 12 mg/kg Körpergewicht, etwa 12,5 mg/kg Körpergewicht, etwa 13 mg/kg Körpergewicht, etwa 13,5 mg/kg Körpergewicht, etwa 14 mg/kg Körpergewicht, etwa 14,5 mg/kg Körpergewicht, etwa 15 mg/kg Körpergewicht, etwa 15,5 mg/kg Körpergewicht, etwa 16 mg/kg Körpergewicht, etwa 16,5 mg/kg Körpergewicht, etwa 17 mg/kg Körpergewicht, etwa 17,5 mg/kg Körpergewicht, etwa 18 mg/kg Körpergewicht, etwa 19,5 mg/kg Körpergewicht, etwa 20 mg/kg Körpergewicht, etwa 21,5 mg/kg Körpergewicht, etwa 22 mg/kg Körpergewicht, etwa 22,5 mg/kg Körpergewicht, etwa 23 mg/kg Körpergewicht, etwa 23,5 mg/kg Körpergewicht, etwa 24 mg/kg Körpergewicht, etwa 24,5 mg/kg Körpergewicht, etwa 25 mg/kg Körpergewicht, etwa 25,5 mg/kg Körpergewicht, etwa 26 mg/kg Körpergewicht, etwa 26,5 mg/kg Körpergewicht, etwa 27 mg/kg Körpergewicht, etwa 27,5 mg/kg Körpergewicht, etwa 28 mg/kg Körpergewicht, etwa 29,5 mg/kg Körpergewicht oder etwa 30 mg/kg Körpergewicht verabreicht wird.

## Revendications

1. Inhibiteur de Jak3 choisi dans le groupe constitué des : décernotinib ; tofacitinib ; inhibiteur de JAK3 IV (ZM-39923) ; NSC114792 ; PF-956980 ; un ARN antisens ou un ADN antisens qui est spécifique pour un acide nucléique codant pour un polypeptide JAK3 ; et un ARNsi qui cible spécifiquement le gène Jak3 pour utilisation dans le traitement d'un trouble de perte des cheveux choisi dans le groupe constitué de l'alopécie areata et l'alopécie androgénétique.

2. Inhibiteur de Jak3 pour utilisation selon la revendication 1, l'inhibiteur étant le décernotinib.

3. Inhibiteur de Jak3 pour utilisation selon la revendication 1, l'inhibiteur étant le tofacitinib.

4. Inhibiteur de Jak3 pour utilisation selon la revendication 1, l'inhibiteur étant l'inhibiteur de JAK3 IV (ZM-39923) ; NSC114792 ; ou PF-956980.

5. Inhibiteur de Jak3 pour utilisation selon la revendication 1, le trouble de perte des cheveux étant l'alopécie areata.

6. Inhibiteur de Jak3 pour utilisation selon la revendication 1, le trouble de perte des cheveux étant l'alopécie androgénétique.

7. Inhibiteur de Jak3 pour utilisation selon l'une quelconque des revendications 1 à 6, l'inhibiteur de Jak3 étant destiné à être administré par voie sous-cutanée, intramusculaire, intrapéritonéale, intradermique, par injection intraveineuse ; par perfusion ; par voie parentérale, transdermique, transmuqueuse, rectale, orale, nasale, ou par administration topique ; ou une combinaison de celles-ci.

8. Inhibiteur de Jak3 pour utilisation selon l'une quelconque des revendications 1 à 7, l'inhibiteur de Jak3 étant destiné à être administré 1 fois par semaine, 2 fois par semaine, 3 fois par semaine, 4 fois par semaine, 5 fois par semaine, 6 fois par semaine, 7 fois par semaine, 8 fois par semaine, 9 fois par semaine, 10 fois par semaine, 11 fois par semaine, 12 fois par semaine, 13 fois par semaine ou 14 fois par semaine.

9. Inhibiteur de Jak3 pour utilisation selon l'une quelconque des revendications 1 à 8, l'inhibiteur de Jak3 étant destiné à être administré pendant au moins 1 semaine, au moins 2 semaines, au moins 3 semaines, au moins 4 semaines, au moins 5 semaines, au moins 6 semaines, au moins 8 semaines, au moins 12 semaines ou au moins 16 semaines.

10. Inhibiteur de Jak3 pour utilisation selon l'une quelconque des revendications 1 à 9, l'inhibiteur de Jak3 étant destiné à être administré avec un inhibiteur de Jak1/2 au sujet.

11. Inhibiteur de Jak3 pour utilisation selon la revendication 10, l'inhibiteur de Jak1/2 étant destiné à être administré simultanément avec l'inhibiteur de Jak3 ; ou l'inhibiteur de Jak1/2 étant destiné à être administré dans un ordre quelconque avec l'inhibiteur de Jak3.

12. Inhibiteur de Jak3 pour utilisation selon l'une quelconque des revendications 10 ou 11, l'inhibiteur de Jak1/2 étant le ruxolitinib, le figlotinib, AG490, le momélotinib, le pacritinib, le baricitinib, le fédratinib, BMS-911543 ou le lestaurtinib.

13. Inhibiteur de Jak3 pour utilisation selon la revendication 1, l'inhibiteur étant le tofacitinib, le trouble de perte des cheveux étant l'alopécie areata, et l'inhibiteur étant destiné à être administré par voie topique.

14. Inhibiteur de Jak3 pour utilisation selon la revendication 1, l'inhibiteur étant administré au sujet dans une composition pharmaceutique choisie parmi une solution, une pommade, un onguent, un gel et une crème.

15. Inhibiteur de Jak3 pour utilisation selon la revendication 1, l'inhibiteur étant contenu dans une composition pharmaceutique topique à une concentration choisie parmi environ 0,1 %, environ 0,2 %, environ 0,3 %, environ 0,4 %, environ 0,5 %, environ 0,6 %, environ 0,7 %, environ 0,8 %, environ 0,9 %, environ 1,0 %, environ 1,1 %, environ 1,2 %, environ 1,3 %, environ 1,4 %, environ 1,5 %, environ 1,6 %, environ 1,7 %, environ 1,8 %, environ 1,9 %, environ 2,0 %, environ 2,1 %, environ 2,2 %, environ 2,3 %, environ 2,4 %, environ 2,5 %, environ 2,6 %, environ 2,7 %, environ 2,8 %, environ 2,9 %, environ 3,0 %, environ 3,5 %, environ 4 %, environ 4,5 %, environ 5 %, environ 5,5 %, environ 6 %, environ 6,5 %, environ 7 %, environ 7,5 %, environ 8 %, environ 8,5 %, environ 9 %, environ 9,5 %, ou environ 10 %.

16. Inhibiteur de Jak3 pour utilisation selon la revendication 1, l'inhibiteur étant administré au sujet en une quantité d'environ 0,0001 µg/kg de poids corporel, environ 0,00025 µg/kg de poids corporel, environ 0,0005 µg/kg de poids corporel, environ 0,00075 µg/kg de poids corporel, environ 0,001 µg/kg de poids corporel, environ 0,0025 µg/kg de poids corporel, environ 0,005 µg/kg de poids corporel, environ 0,0075 µg/kg de poids corporel, environ 0,01 µg/kg de poids corporel, environ 0,025 µg/kg de poids corporel, environ 0,05 µg/kg de poids corporel, environ 0,075 µg/kg de poids corporel, environ 0,1 µg/kg de poids corporel, environ 0,25 µg/kg de poids corporel, environ 0,5 µg/kg de poids corporel, environ 0,75 µg/kg de poids corporel, environ 1 µg/kg de poids corporel, environ 5 µg/kg de poids corporel, environ 10 µg/kg de poids corporel, environ 25 µg/kg de poids corporel, environ 50 µg/kg de poids corporel, environ 75 µg/kg de poids corporel, environ 100 µg/kg de poids corporel, environ 150 µg/kg de poids corporel, environ 200 µg/kg de poids corporel, environ 250 µg/kg de poids corporel, environ 300 µg/kg de poids corporel, environ 350 µg/kg de poids corporel, environ 400 µg/kg de poids corporel, environ 450 µg/kg de poids corporel, environ 500 µg/kg de poids corporel, environ 550 µg/kg de poids corporel, environ 600 µg/kg de poids corporel, environ 650 µg/kg de poids corporel, environ 700 µg/kg de poids corporel, environ 750 µg/kg de poids corporel, environ 800 µg/kg de poids corporel, environ 850 µg/kg de poids corporel, environ 900 µg/kg de poids corporel, environ 950 µg/kg de poids corporel, environ 1000 µg/kg de poids corporel, environ 2000 µg/kg de poids corporel, environ 3000 µg/kg de poids corporel, environ 4000 µg/kg de poids corporel, environ 5000 µg/kg de poids corporel, environ 6000 µg/kg de poids corporel, environ 7000 µg/kg de poids corporel, environ 8000 µg/kg de poids corporel, environ 9500 µg/kg de poids corporel, ou environ 10 000 µg/kg de poids corporel, environ 1 mg/kg de poids corporel, environ 1,5 mg/kg de poids corporel, environ 2 mg/kg de poids corporel, environ 2,5 mg/kg de poids corporel, environ 3 mg/kg de poids corporel, environ 3,5 mg/kg de poids corporel, environ 4 mg/kg de poids corporel, environ 4,5 mg/kg de poids corporel, environ 5 mg/kg de poids corporel, environ 5,5 mg/kg de poids corporel, environ 6 mg/kg de poids corporel, environ 6,5 mg/kg de poids corporel, environ 7 mg/kg de poids corporel, environ 7,5 mg/kg de poids corporel, environ 8 mg/kg de poids corporel, environ 9,5 mg/kg de poids corporel, environ 10 mg/kg de poids corporel, environ 10,5 mg/kg de poids corporel, environ 11,0 mg/kg de poids corporel, environ 11,5 mg/kg de poids corporel, environ 12 mg/kg de poids corporel, environ 12,5 mg/kg de poids corporel, environ 13 mg/kg de poids corporel, environ 13,5 mg/kg de poids corporel, environ 14 mg/kg de poids corporel, environ 14,5 mg/kg de poids corporel, environ 15 mg/kg de poids corporel, environ 15,5 mg/kg de poids corporel, environ 16 mg/kg de poids corporel, environ 16,5 mg/kg de poids corporel, environ 17 mg/kg de poids corporel, environ 17,5 mg/kg de poids corporel, environ 18 mg/kg de poids corporel, environ 19,5 mg/kg de poids corporel, environ 20 mg/kg de poids corporel, environ 21,5 mg/kg de poids corporel, environ 22 mg/kg de poids corporel, environ 22,5 mg/kg de poids corporel, environ 23 mg/kg de poids corporel, environ 23,5 mg/kg de poids corporel, environ 24 mg/kg de poids corporel, environ 24,5 mg/kg de poids corporel, environ 25 mg/kg de poids corporel, environ 25,5 mg/kg de poids corporel, environ 26 mg/kg de poids corporel, environ 26,5 mg/kg de poids corporel, environ 27 mg/kg de poids corporel, environ 27,5 mg/kg de poids corporel, environ 28 mg/kg de poids corporel, environ 29,5 mg/kg de poids corporel, ou environ 30 mg/kg de poids corporel.
